(19) Europäisches Patentamt
European Patent Office
Office européen des brevets

(11) EP 3 121 167 A1

(12) **EUROPEAN PATENT APPLICATION**
published in accordance with Art. 153(4) EPC

(43) Date of publication:
**25.01.2017 Bulletin 2017/04**

(21) Application number: **15764627.4**

(22) Date of filing: **27.02.2015**

(51) Int Cl.:
***C07D 265/36*** *(2006.01)* ***C07D 215/04*** *(2006.01)*
***A61K 31/538*** *(2006.01)* ***A61P 3/10*** *(2006.01)*

(86) International application number:
**PCT/KR2015/001941**

(87) International publication number:
**WO 2015/141958 (24.09.2015 Gazette 2015/38)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **20.03.2014 KR 20140033041**

(71) Applicant: **Hyundai Pharm Co., Ltd.**
**Cheonan-si, Chungcheongnam-do 330-911 (KR)**

(72) Inventors:
- **LEE, In Hee**
**Hwaseong-si**
**Gyeonggi-do 445-733 (KR)**
- **CHAE, Hee Il**
**Suwon-si**
**Gyeonggi-do 442-816 (KR)**
- **KIM, Se hoan**
**Suwon-si**
**Gyeonggi-do 442-819 (KR)**
- **MOON, Soon Young**
**Suwon-si**
**Gyeonggi-do 443-370 (KR)**
- **HA, Tae Young**
**Suwon-si**
**Gyeonggi-do 442-754 (KR)**
- **CHOI, Hyo sun**
**Suwon-si**
**Gyeonggi-do 442-816 (KR)**
- **KIM, Young Seok**
**Seoul 135-782 (KR)**
- **KIM, Chun hwa**
**Yongin-si**
**Gyeonggi-do 448-991 (KR)**
- **RHEE, Jae Keol**
**Hwaseong-si**
**Gyeonggi-do 445-856 (KR)**

(74) Representative: **Pearson, Samuel John et al**
**Abel & Imray**
**20 Red Lion Street**
**London WC1R 4PQ (GB)**

(54) **COMPOUND BINDING TO PPARG BUT NOT ACTING AS PROMOTER AND PHARMACEUTICAL COMPOSITION FOR TREATING PPARG-RELATED DISEASES CONTAINING SAME AS ACTIVE INGREDIENT**

(57) The present invention relates to a compound inhibiting CDK5-mediated PPARG phosphorylation and a pharmaceutical composition for treating PPARG-related diseases containing the same as an active ingredient. A compound represented by formula 1 or an optical isomer thereof according to the present invention binds to PPARG at a high affinity level, but does not act as an agonist, thereby inducing no gene transcription; blocks CDK5, which is a cause of PPARG phosphorylation, thereby causing no side effects of existing anti-diabetics; can be formulated into drugs due to improved pharmacological properties thereof; and can be favorably used as a pharmaceutical composition for treating PPARG-related diseases due to an excellent treatment effect on PPARG-related diseases.

EP 3 121 167 A1

## Description

## BACKGROUND OF THE INVENTION

### 1. Field of the Invention

The present invention relates to a compound binding to PPARG but not acting as a promoter and a pharmaceutical composition for treating PPARG-related diseases comprising the compound as an active ingredient.

### 2. Description of the Related Art

According to CDC (Centers for Disease Control and Prevention, USA) data, 24 million people, which are 8% of the total US population, are diabetic patients in US. Over the past decades, the number of diabetic patients has been growing, and so did the diabetic drug market. The dominant diabetic treating drug has been insulin so far, but the treatment using insulin injection has a problem of the inconvenience of using a syringe. Besides, it can simply supplement the shortage of insulin but cannot be a fundamental solution for diabetes. Therefore, a variety of alternative drugs including sulfonylurea increasing insulin secretion, metformin slowly isolating glucose deposited in the liver, acarbose suppressing glycolysis to inhibit the adsorption of sugar, and rosiglitazone increasing the sensitivity of insulin receptor have been developed, which are on the market now. In spite of these drugs, treating diabetes is yet unsatisfactory and cannot meet the need of diabetic patients. Recently understanding of the cause and the mechanism of diabetes is enlarged, and thereby the diabetic drug market turns into a new land of opportunity.

Thiazolidinedione (TZD) drugs such as rosiglitazone and pioglitazone activate the transcription of PPARG (peroxisome proliferator activated receptor-gamma), the nuclear receptor, and thereby increase insulin sensitivity to bring the anti-diabetic effect. It is well known that this kind of drug is effective in dealing with PPARG but recently it is also confirmed that this kind of drug affects another biochemical pathway. The involvement of this drug in the obesity-like insulin resistance mechanism is an example. The known cause of the insulin resistance observed in diabetic patients is the phosphorylation of the 273$^{rd}$ amino acid of PPARG, serine, of CDK5 (cyclin-dependant kinase 5) induced by mutation. It has been additionally confirmed that the blocking of CDK5 working on PPARG is an important approach to develop an efficient anti-diabetic agent.

A patent document (US2012-0309757 A1) introduces those compounds that bind to PPARG at a high affinity level but do not induce the transcription of PPARG by acting as an agonist and thereby suppress insulin resistance by blocking CDK5, so that they display anti-diabetic effect (SR1664, SR1824, etc). Those compounds were also confirmed from the comparative experiment to have significantly lower chance of developing side effects like weight gain and fluid retention, etc, which are frequently accompanied by the conventional drugs such as rosiglitazone. From the cell culture test, it was confirmed that SR1664 did not show such a problem as fat generation in bone cells, which is one of the side effects of rosiglitazone. The results above confirm that SR1664 can block specifically CDK5 mediated PPARG phosphorylation.

Even though the said SR1664 has many advantages, there is a problem in developing it as a drug because of poor pharmaco-kinetic (PK) pharmacophysical properties that make *in vivo* absorption difficult.

Thus, the present inventors tried to develop a compound that can be formulated as a drug with improved pharmaco-kinetic pharmacophysical properties. In the course of the study, the present inventors confirmed that a compound with a specific structure that binds to PPARG with high affinity but does not induce transcriptional activity, suggesting that the compound does not act as an agonist but can block CDK5 activity so as to improve insulin resistance, and thereby confirmed that the compound is more excellent in lowering blood sugar and weight than SR1664 and SR1824 and does not significantly inhibit CYP enzyme activity, the drug-drug interaction index. As a result, the inventors confirmed that the compound above can be effectively used for treating PPARG related diseases, leading to the completion of the present invention.

## SUMMARY OF THE INVENTION

It is an object of the present invention to provide a compound that binds to PPARG but does not act as a promoter, an optical isomer thereof, or a pharmaceutically acceptable salt of the same.

It is another object of the present invention to provide a method for preparing the compound above.

It is also an object of the present invention to provide a pharmaceutical composition for treating PPARG related disease which comprises the compound above, the optical isomer thereof, or the pharmaceutically acceptable salt of the same as an active ingredient.

To achieve the above objects, the present invention provides a compound represented by formula 1 below, an optical isomer thereof, or a pharmaceutically acceptable salt of the same.

[Formula 1]

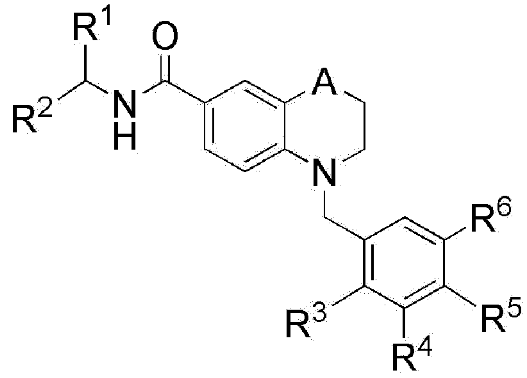

In the formula 1,

$R^1$ is H, unsubstituted or substituted $C_{1-10}$ straight or branched alkyl wherein one or more halogens are substituted, or unsubstituted or substituted $C_{1-10}$ straight or branched alkoxy wherein one or more halogens are substituted;
$R^2$ is unsubstituted or substituted $C_{6-10}$ aryl, unsubstituted or substituted $C_{6-10}$ aryl $C_{1-10}$ straight or branched alkyl, or unsubstituted or substituted 5 ~ 10 membered heteroaryl containing one or more hetero atoms selected from the group consisting of N, O, and S.

In the said substituted $C_{6-10}$ aryl, the substituted $C_{6-10}$ aryl $C_{1-10}$ straight or branched alkyl, and the substituted 5 ~ 10 membered heteroaryl, one or more substituents selected from the group consisting of $C_{1-10}$ straight or branched alkyl unsubstituted or substituted with one or more halogens, $C_{1-10}$ straight or branched alkoxy unsubstituted or substituted with one or more halogens, $C_{1-10}$ straight or branched alkylsulfonyl unsubstituted or substituted with one or more halogens, $C_{1-10}$ straight or branched alkoxycarbonyl, halogen, nitrile, and nitro can be substituted,
In the said unsubstituted or substituted $C_{6-10}$ aryl, phenyl or 5 ~ 8 membered heterocycloalkyl containing one or more hetero atoms selected from the group consisting of N, O, and S can be fused;
$R^1$ and $R^2$ can form $C_{5-10}$ cycloalkyl, 5 ~ 10 membered heterocycloalkyl containing one or more hetero atoms selected from the group consisting of N, O, and S, or 5 ~ 10 membered heteroaryl containing one or more hetero atoms selected from the group consisting of N, O, and S along with the carbon atoms which are conjugated to the same,
In the said $C_{5-10}$ cycloalkyl, the 5 ~ 10 membered heterocycloalkyl, or the 5 ~ 10 membered heteroaryl, phenyl can be fused;
$R^3$, $R^4$, $R^5$, and $R^6$ are independently H, halogen, unsubstituted or substituted $C_{1-10}$ straight or branched alkyl wherein one or more halogens are substituted, unsubstituted or substituted $C_{1-10}$ straight or branched alkoxy wherein one or more halogens are substituted, or unsubstituted or substituted $C_{6-10}$ aryl,
In the said substituted $C_{6-10}$ aryl, one or more substituents selected from the group consisting of halogen, unsubstituted or substituted $C_{1-10}$ straight or branched alkyl wherein one or more halogens are substituted, hydroxycarbonyl, aminocarbonyl, and sodiumoxycarbonyl can be substituted;

A is $-CH_2-$ or -O-.

The present invention also provides a method for preparing the compound represented by formula 1 comprising the following steps, as shown in reaction formula 1 below:

preparing the compound represented by formula 4 by reacting the compound represented by formula 2 with the compound represented by formula 3 (step 1);
preparing the compound represented by formula 5 by substituting methoxycarbonyl group of the compound represented by formula 4 prepared in step 1 with carboxy group (step 2);
preparing the compound represented by formula 7 by reacting the compound represented by formula 5 prepared in step 2 with the compound represented by formula 6 (step 3); and
substituting t-butoxy group of the compound represented by formula 7 prepared in step 3 with -OH group (step 4).

[Reaction Formula 1]

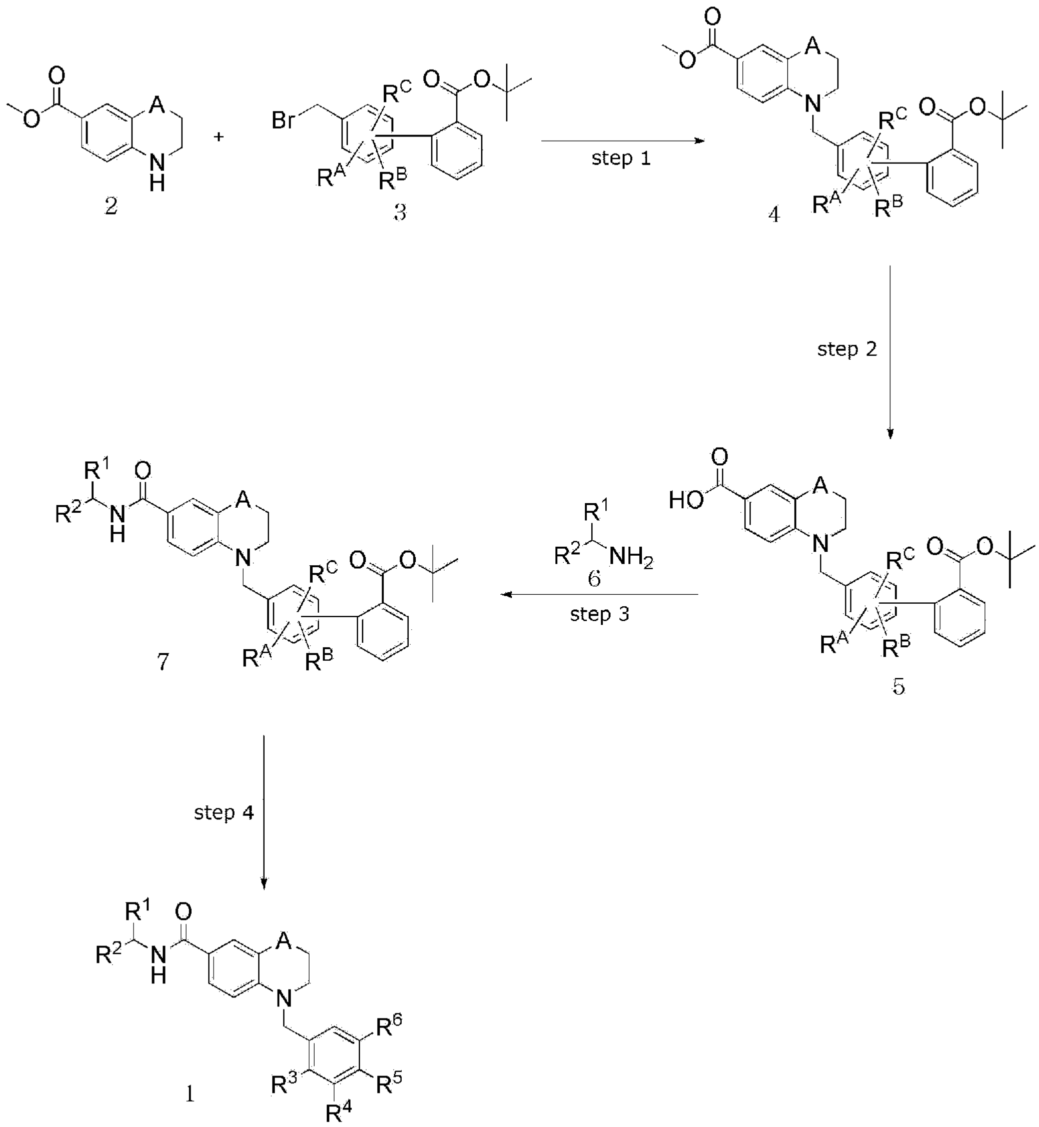


(In the reaction formula 1,

$R^1 \sim R^6$ and A are as defined in formula 1, and
$R^A$, $R^B$, and $R^C$ are independently as defined in $R^3 \sim R^6$).

The present invention also provides a method for preparing the compound represented by formula 1 comprising the following steps, as shown in reaction formula 2 below:

preparing the compound represented by formula 9 by reacting the compound represented by formula 8 with the compound represented by formula 6 (step 1);
preparing the compound represented by formula 7 by reacting the compound represented by formula 9 prepared in step 1 with the compound represented by formula 3 (step 2); and
substituting t-butoxy group of the compound represented by formula 7 prepared in step 2 with -OH group (step 3).

[Reaction Formula 2]

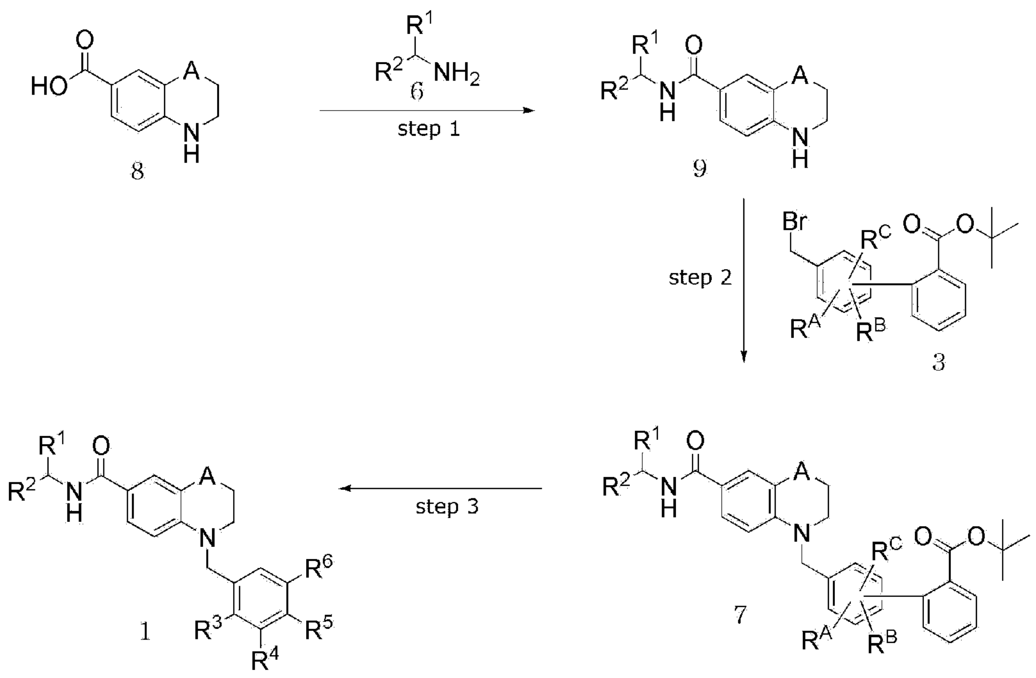


(In the reaction formula 2,

$R^1 \sim R^6$ and A are as defined in formula 1, and
$R^A$, $R^B$, and $R^C$ are independently as defined in $R^3 \sim R^6$).

The present invention further provides a method for preparing the compound represented by formula 1, as shown in reaction formula 3 below, comprising the step of substituting carboxyl group of the compound represented by formula 1A with sodiumoxycarbonyl group or amide group (step 1).

[Reaction Formula 3]

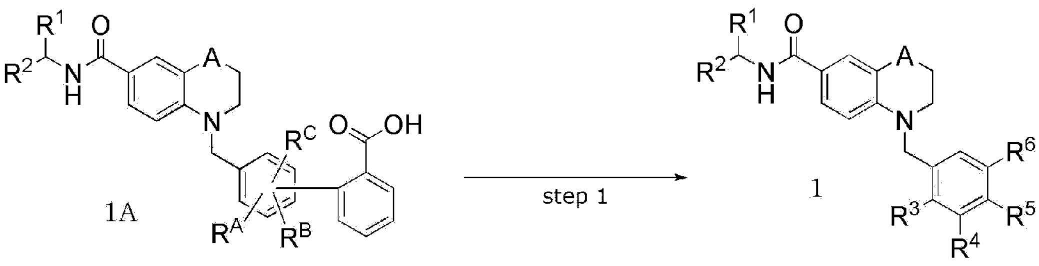


(In the reaction formula 3,

$R^1 \sim R^6$ and A are as defined in formula 1, and
$R^A$, $R^B$, and $R^C$ are independently as defined in $R^3 \sim R^6$).

In addition, the present invention provides a pharmaceutical composition for treating PPARG-related diseases comprising the compound represented by formula 1, the optical isomer thereof, or the pharmaceutically acceptable salt of the same as an active ingredient.

## ADVANTAGEOUS EFFECT

The compound represented by formula 1 or the optical isomer thereof of the present invention binds to PPARG with a high affinity but does not act as an agonist and thereby does not induce gene transcription of the same and blocks CDK5 that is a cause of PPARG phosphorylation. Therefore, the compound or the optical isomer thereof of the invention does not cause side effects of the conventional anti-diabetic agents but is easily formulated as a drug with improved pharmacophysical properties and is excellent in treating PPARG-related diseases, indicating that the compound or the optical isomer thereof of the invention can be effectively used as a pharmaceutical composition for PPARG-related diseases.

## DESCRIPTION OF THE PREFERRED EMBODIMENTS

Hereinafter, the present invention is described in detail.

The present invention provides a compound represented by formula 1 below, an optical isomer thereof, or a pharmaceutically acceptable salt of the same.

[Formula 1]

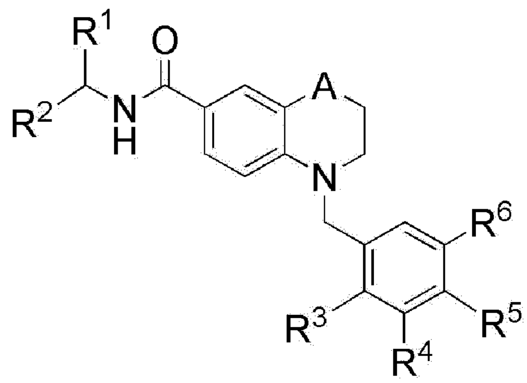

In the formula 1,

$R^1$ is H, unsubstituted or substituted $C_{1-10}$ straight or branched alkyl wherein one or more halogens are substituted, or unsubstituted or substituted $C_{1-10}$ straight or branched alkoxy wherein one or more halogens are substituted;
$R^2$ is unsubstituted or substituted $C_{6-10}$ aryl, unsubstituted or substituted $C_{6-10}$ aryl $C_{1-10}$ straight or branched alkyl, or unsubstituted or substituted 5 ~ 10 membered heteroaryl containing one or more hetero atoms selected from the group consisting of N, O, and S.

In the said substituted $C_{6-10}$ aryl, the substituted $C_{6-10}$ aryl $C_{1-10}$ straight or branched alkyl, and the substituted 5 ~ 10 membered heteroaryl, one or more substituents selected from the group consisting of $C_{1-10}$ straight or branched alkyl unsubstituted or substituted with one or more halogens, $C_{1-10}$ straight or branched alkoxy unsubstituted or substituted with one or more halogens, $C_{1-10}$ straight or branched alkylsulfonyl unsubstituted or substituted with one or more halogens, $C_{1-10}$ straight or branched alkoxycarbonyl, halogen, nitrile, and nitro can be substituted,
In the said unsubstituted or substituted $C_{6-10}$ aryl, phenyl or 5 ~ 8 membered heterocycloalkyl containing one or more hetero atoms selected from the group consisting of N, O, and S can be fused;
$R^1$ and $R^2$ can form $C_{5-10}$ cycloalkyl, 5 ~ 10 membered heterocycloalkyl containing one or more hetero atoms selected from the group consisting of N, O, and S, or 5 ~ 10 membered heteroaryl containing one or more hetero atoms selected from the group consisting of N, O, and S along with the carbon atoms which are conjugated to the same,
In the said $C_{5-10}$ cycloalkyl, the 5 ~ 10 membered heterocycloalkyl, or the 5 ~ 10 membered heteroaryl, phenyl can be fused;
$R^3$, $R^4$, $R^5$, and $R^6$ are independently H, halogen, unsubstituted or substituted $C_{1-10}$ straight or branched alkyl wherein one or more halogens are substituted, unsubstituted or substituted $C_{1-10}$ straight or branched alkoxy wherein one or more halogens are substituted, or unsubstituted or substituted $C_{6-10}$ aryl,
In the said substituted $C_{6-10}$ aryl, one or more substituents selected from the group consisting of halogen, unsubstituted or substituted $C_{1-10}$ straight or branched alkyl wherein one or more halogens are substituted, hydroxycarbonyl, aminocarbonyl, and sodiumoxycarbonyl can be substituted;

A is $-CH_2-$ or -O-.

Preferably,
$R^1$ is H, unsubstituted or substituted $C_{1-5}$ straight or branched alkyl wherein one or more halogens are substituted, or unsubstituted or substituted $C_{1-5}$ straight or branched alkoxy wherein one or more halogens are substituted;
$R^2$ is unsubstituted or substituted $C_{6-8}$ aryl, unsubstituted or substituted $C_{6-8}$ aryl $C_{1-5}$ straight or branched alkyl, or unsubstituted or substituted 5 ~ 8 membered heteroaryl containing one or more hetero atoms selected from the group consisting of N, O, and S,
In the said substituted $C_{6-8}$ aryl, the substituted $C_{6-8}$ aryl $C_{1-5}$ straight or branched alkyl, and the substituted 5 ~ 8 membered heteroaryl, one or more substituents selected from the group consisting of $C_{1-5}$ straight or branched alkyl unsubstituted or substituted with one or more halogens, $C_{1-5}$ straight or branched alkoxy unsubstituted or substituted with one or more halogens, $C_{1-5}$ straight or branched alkylsulfonyl unsubstituted or substituted with one or more halogens, $C_{1-5}$ straight or branched alkoxycarbonyl, halogen, nitrile, and nitro can be substituted,
In the said unsubstituted or substituted $C_{6-8}$ aryl, phenyl or 5 ~ 8 membered heterocycloalkyl containing one or more hetero atoms selected from the group consisting of N, O, and S can be fused;
$R^1$ and $R^2$ can form $C_{5-8}$ cycloalkyl, 5 ~ 8 membered heterocycloalkyl containing one or more hetero atoms selected from the group consisting of N, O, and S, or 5 ~ 8 membered heteroaryl containing one or more hetero atoms selected from the group consisting of N, O, and S along with the carbon atoms which are conjugated to the same,
In the said $C_{5-8}$ cycloalkyl, the 5 ~ 8 membered heterocycloalkyl, or the 5 ~ 8 membered heteroaryl, phenyl can be fused;
$R^3$, $R^4$, $R^5$, and $R^6$ are independently H, halogen, unsubstituted or substituted $C_{1-5}$ straight or branched alkyl wherein one or more halogens are substituted, unsubstituted or substituted $C_{1-5}$ straight or branched alkoxy wherein one or more halogens are substituted, or unsubstituted or substituted $C_{6-8}$ aryl,
In the said substituted $C_{6-8}$ aryl, one or more substituents selected from the group consisting of halogen, unsubstituted or substituted $C_{1-5}$ straight or branched alkyl wherein one or more halogens are substituted, hydroxycarbonyl, aminocarbonyl, and sodiumoxycarbonyl can be substituted;

A is $-CH_2-$ or -O-.

More preferably,
$R^1$ is H, methyl, or ethyl;
$R^2$ is unsubstituted or substituted phenyl, unsubstituted or substituted benzyl, or unsubstituted or substituted 5 ~ 6 membered heteroaryl containing one or more hetero atoms selected from the group consisting of N and O,
In the said substituted phenyl, the substituted benzyl, and the substituted 5 ~ 6 membered heteroaryl, one or more substituents selected from the group consisting of $C_{1-5}$ straight or branched alkyl unsubstituted or substituted with one or more halogens, $C_{1-5}$ straight or branched alkoxy unsubstituted or substituted with one or more halogens, $C_{1-5}$ straight or branched alkylsulfonyl unsubstituted or substituted with one or more halogens, $C_{1-5}$ straight or branched alkoxycarbonyl, halogen, nitrile, and nitro can be substituted,
In the said unsubstituted or substituted phenyl, phenyl or 6 membered heterocycloalkyl containing one or more hetero atoms selected from the group consisting of N and O can be fused;
$R^1$ and $R^2$ can form $C_{5-8}$ cycloalkyl, 5 ~ 8 membered heterocycloalkyl containing one or more hetero atoms selected from the group consisting of N, O, and S, or 5 ~ 8 membered heteroaryl containing one or more hetero atoms selected from the group consisting of N, O, and S along with the carbon atoms which are conjugated to the same,
In the said $C_{5-8}$ cycloalkyl, the 5 ~ 8 membered heterocycloalkyl, or the 5 ~ 8 membered heteroaryl, phenyl can be fused;
$R^3$, $R^4$, $R^5$, and $R^6$ are independently H, fluoro, or unsubstituted or substituted phenyl,
In the said substituted phenyl, one or more substituents selected from the group consisting of hydroxycarbonyl, aminocarbonyl, and sodiumoxycarbonyl can be substituted;

A is $-CH_2-$ or -O-.

More preferably,
$R^1$ is H, methyl, or ethyl;
$R^2$ is

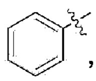, 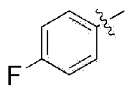, 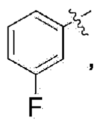, 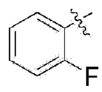, 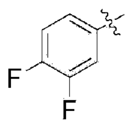, 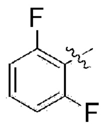, 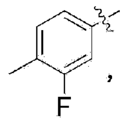,

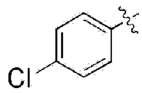, 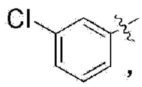, 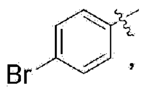, 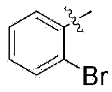, 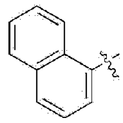, 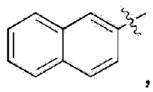,

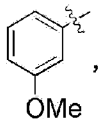, 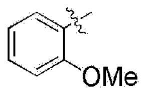, 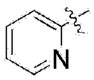, 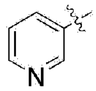, 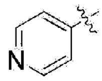, 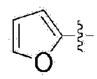,

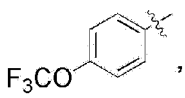, 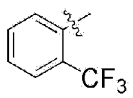, 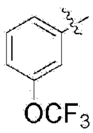, 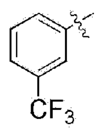, 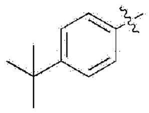, 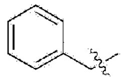,

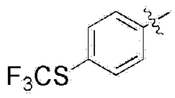, 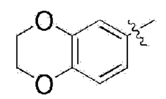, 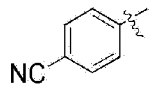, 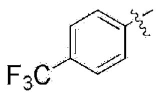, 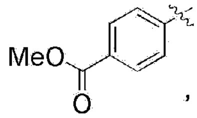,

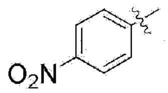 or 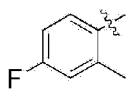;

$R^3$ is H, F,

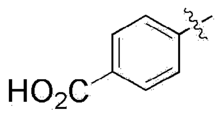

or

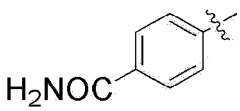;

$R^4$ is H, F,

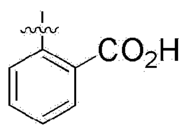

or

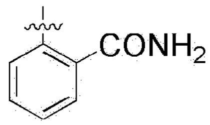

;

$R^5$ is H,

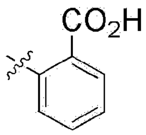

,

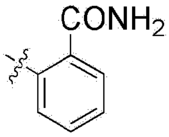

or

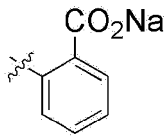

;

$R^6$ is H,

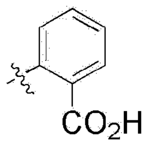

or

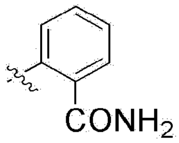

;

and

A is $-CH_2-$ or -O-.

The compound represented by formula 1 or the optical isomer thereof of the present invention can be exemplified by the following compounds:

(1) (S)-4'-((6-((1-phenylethyl)carbamoyl)-3,4-dihydroquinoline-1(2H)-yl)methyl)-[1,1'-biphenyl]-2-carboxylic acid;
(2) (S)-1-((2'-carbamoyl-[1,1'-biphenyl]-4-yl)methyl)-N-(1-phenylethyl)-1,2,3,4-tetrahydroquinoline-6-carboxamide;
(3) (S)-4'-((6-((1-(4-nitrophenyl)ethyl)carbamoyl)-3,4-dihydroquinoline-1(2H)-yl)methyl)-[1,1'-biphenyl]-2-carboxylic acid;
(4) (S)-4'-((6-((1-(4-fluoro-2-methylphenyl)ethyl)carbamoyl)-3,4-dihydroquinoline-1(2H)-yl)methyl)-[1,1'-biphenyl]-2-carboxylic acid;
(5) (S)-4'-((6-((1-(3-fluoro-4-methylphenyl)ethyl)carbamoyl)-3,4-dihydroquinoline-1(2H)-yl)methyl)-[1,1'-biphenyl]-2-carboxylic acid;
(6) (S)-4'-((6-((1-(4-fluorophenyl)ethyl)carbamoyl)-3,4-dihydroquinoline-1(2H)-yl)methyl)-[1,1'-biphenyl]-2-carboxylic acid;
(7) (S)-4'-((6-((1-(4-bromophenyl)ethyl)carbamoyl)-3,4-dihydroquinoline-1(2H)-yl)methyl)-[1,1'-biphenyl]-2-carboxylic acid;
(8) 4'-((6-((4-nitrobenzyl)carbamoyl)-3,4-dihydroquinoline-1(2H)-yl)methyl)-[1,1'-biphenyl]-2-carboxylic acid;

(9) (S)-4'-((6-((1-(3-chlorophenyl)ethyl)carbamoyl)-3,4-dihydroquinoline-1(2H)-yl)methyl)-[1,1'-biphenyl]-2-carboxylic acid;
(10) (S)-4'-((6-((1-(naphthalene-1-yl)ethyl)carbamoyl)-3,4-dihydroquinoline-1(2H)-yl)methyl)-[1,1'-biphenyl]-2-carboxylic acid;
(11) (S)-4'-((6-((1-(4-(trifluoromethoxy)phenyl)ethyl)carbamoyl)-3,4-dihydroquinoline-1(2H)-yl)methyl)-[1,1'-biphenyl]-2-carboxylic acid;
(12) (S)-4'-((6-((1-(4-(trifluoromethyl)phenyl)ethyl)carbamoyl)-3,4-dihydroquinoline-1(2H)-yl)methyl)-[1,1'-biphenyl]-2-carboxylic acid;
(13) (S)-4'-((6-((1-(4-((trifluoromethyl)thio)phenyl)ethyl)carbamoyl)-3,4-dihydroquinoline-1 (2H)-yl)methyl)-[1,1'-biphenyl]-2-carboxylic acid;
(14) (S)-4'-((6-((1-(4-cyanophenyl)ethyl)carbamoyl)-3,4-dihydroquinoline-1(2H)-yl)methyl)-[1,1'-biphenyl]-2-carboxylic acid;
(15) 4'-((6-(cromene-3-ylcarbamoyl)-3,4-dihydroquinoline-1(2H)-yl)methyl)-[1,1'-biphenyl]-2-carboxylic acid;
(16) (S)-4'-((6-((1-(2,6-difluorophenyl)ethyl)carbamoyl)-3,4-dihydroquinoline-1(2H)-yl)methyl)-[1,1'-biphenyl]-2-carboxylic acid;
(17) (S)-4'-((6-((1-(3-fluorophenyl)ethyl)carbamoyl)-3,4-dihydroquinoline-1(2H)-yl)methyl)-[1,1'-biphenyl]-2-carboxylic acid;
(18) (S)-4'-((6-((1-(3-(trifluoromethoxy)phenyl)ethyl)carbamoyl)-3,4-dihydroquinoline-1(2H)-yl)methyl)-[1,1'-biphenyl]-2-carboxylic acid;
(19) (S)-4'-((6-((1-(3-(trifluoromethyl)phenyl)ethyl)carbamoyl)-3,4-dihydroquinoline-1(2H)-yl)methyl)-[1,1'-biphenyl]-2-carboxylic acid;
(20) (S)-4'-((6-((1-(naphthalene-2-yl)ethyl)carbamoyl)-3,4-dihydroquinoline-1(2H)-yl)methyl)-[1,1'-biphenyl]-2-carboxylic acid;
(21) (S)-4'-((6-((1-(3,4-difluorophenyl)ethyl)carbamoyl)-3,4-dihydroquinoline-1(2H)-yl)methyl)-[1,1'-biphenyl]-2-carboxylic acid;
(22) (S)-4'-((6-((1-(3-methoxyphenyl)ethyl)carbamoyl)-3,4-dihydroquinoline-1(2H)-yl)methyl)-[1,1'- biphenyl]-2-carboxylic acid;
(23) (S)-4'-((6-((1-(2-methoxyphenyl)ethyl)carbamoyl)-3,4-dihydroquinoline-1(2H)-yl)methyl)-[1,1'-biphenyl]-2-carboxylic acid;
(24) (S)-4'-((6-((1-(2-fluorophenyl)ethyl)carbamoyl)-3,4-dihydroquinoline-1(2H)-yl)methyl)-[1,1'-biphenyl]-2-carboxylic acid;
(25) (S)-4'-((6-((1-(2-(trifluoromethyl)phenyl)ethyl)carbamoyl)-3,4-dihydroquinoline-1(2H)-yl)methyl)-[1,1'-biphenyl]-2-carboxylic acid;
(26) 4'-((6-((pyridine-2-ylmethyl)carbamoyl)-3,4-dihydroquinoline-1(2H)-yl)methyl)-[1,1'-biphenyl]-2-carboxylic acid;
(27) 4'-((6-((pyridine-4-ylmethyl)carbamoyl)-3,4-dihydroquinoline-1(2H)-yl)methyl)-[1,1'-biphenyl]-2-carboxylic acid;
(28) 4'-((6-(benzylcarbamoyl)-3,4-dihydroquinoline-1(2H)-yl)methyl)-[1,1'-biphenyl]-2-carboxylic acid;
(29) 4'-((6-((2-bromobenzyl)carbamoyl)-3,4-dihydroquinoline-1(2H)-yl)methyl)-[1,1'-biphenyl]-2-carboxylic acid;
(30) 4'-((6-(((2,3-dihydrobenzo[b][1,4]dioxin-6-yl)methyl)carbamoyl)-3,4-dihydroquinoline-1(2H)-yl)methyl)-[1,1'-biphenyl]-2-carboxylic acid;
(31) 4'-((6-((4-bromobenzyl)carbamoyl)-3,4-dihydroquinoline-1(2H)-yl)methyl)-[1,1'-biphenyl]-2-carboxylic acid;
(32) 4'-((6-((3-(trifluoromethoxy)benzyl)carbamoyl)-3,4-dihydroquinoline-1(2H)-yl)methyl)-[1,1'-biphenyl]-2-carboxylic acid;
(33) (S)-1-((2'-carbamoyl-[1,1'-biphenyl]-4-yl)methyl)-N-(1-(4-(trifluoromethyl)phenyl)ethyl)-1,2,3,4-tetrahydroquinoline-6-carboxamide
(34) (S)-1-((2'-carbamoyl-[1,1'-biphenyl]-4-yl)methyl)-N-(1-(4-(trifluoromethoxy)phenyl)ethyl)-1,2,3,4-tetrahydroquinoline-6-carboxamide;
(35) (S)-1-((2'-carbamoyl-[1,1'-biphenyl]-4-yl)methyl)-N-(1-(3-chlorophenyl)ethyl)-1,2,3,4-tetrahydroquinoline-6-carboxamide;
(36) (S)-1-((2'-carbamoyl-[1,1'-biphenyl]-4-yl)methyl)-N-(1-(4-fluorophenyl)ethyl)-1,2,3,4-tetrahydroquinoline-6-carboxamide;
(37) (S)-N-(1-(4-bromophenyl)ethyl)-1-((2'-carbamoyl-[1,1'-biphenyl]-4-yl)methyl)-1,2,3,4-tetrahydroquinoline-6-carboxamide;
(38) (S)-1-((2'-carbamoyl-[1,1'-biphenyl]-4-yl)methyl)-N-(1-(naphthalene-1-yl)ethyl)-1,2,3,4-tetrahydroquinoline-6-carboxamide;
(39) (S)-1-((2'-carbamoyl-[1,1'-biphenyl]-4-yl)methyl)-N-(1-(3,4-difluorophenyl)ethyl)-1,2,3,4-tetrahydroquinoline-6-carboxamide;

(40) (S)-1-((2'-carbamoyl-[1,1'-biphenyl]-4-yl)methyl)-N-(1-(3-methoxyphenyl)ethyl)-1,2,3,4-tetrahydroquinoline-6-carboxamide;
(41) (S)-1-((2'-carbamoyl-[1,1'-biphenyl]-4-yl)methyl)-N-(1-(2-methoxyphenyl)ethyl)-1,2,3,4-tetrahydroquinoline-6-carboxamide;
(42) (S)-1-((2'-carbamoyl-[1,1'-biphenyl]-4-yl)methyl)-N-(1-(2-fluorophenyl)ethyl)-1,2,3,4-tetrahydroquinoline-6-carboxamide;
(43) (S)-1-((2'-carbamoyl-[1,1'-biphenyl]-4-yl)methyl)-N-(1-(2-(trifluoromethyl)phenyl)ethyl)-1,2,3,4-tetrahydroquinoline-6-carboxamide;
(44) (S)-1-((2'-carbamoyl-[1,1'-biphenyl]-4-yl)methyl)-N-(1-(naphthalene-2-yl)ethyl)-1,2,3,4-tetrahydroquinoline-6-carboxamide;
(45) (S)-1- ((2'-carbamoyl-[1,1'-biphenyl]-4-yl)methyl)-N-(1-(3-fluorophenyl)ethyl)-1,2,3,4-tetrahydroquinoline-6-carboxamide;
(46) (S)-1-((2'-carbamoyl-[1,1'-biphenyl]-4-yl)methyl)-N-(1-(3-(trifluoromethoxy)phenyl)ethyl)-1,2,3,4-tetrahydroquinoline-6-carboxamide;
(47) (S)-1-((2'-carbamoyl-[1,1'-biphenyl]-4-yl)methyl)-N-(1-(3-(trifluoromethyl)phenyl)ethyl)-1,2,3,4-tetrahydroquinoline-6-carboxamide;
(48) 1-((2'-carbamoyl-[1,1'-biphenyl]-4-yl)methyl)-N-(pyridine-4-ylmethyl)-1,2,3,4-tetrahydroquinoline-6-carboxamide;
(49) (S)-1-((2'-carbamoyl-[1,1'-biphenyl]-4-yl)methyl)-N-(1-(3-fluoro-4-methylphenyl)ethyl)-1,2,3,4-tetrahydroquinoline-6-carboxamide;
(50) N-benzyl-1-((2'-carbamoyl-[1,1'-biphenyl]-4-yl)methyl)-1,2,3,4-tetrahydroquinoline-6-carboxamide;
(51) N-(2-bromobenzyl)-1-((2'-carbamoyl-[1,1'-biphenyl]-4-yl)methyl)-1,2,3,4-tetrahydroquinoline-6-carboxamide;
(52) 1-((2'-carbamoyl-[1,1'-biphenyl]-4-yl)methyl)-N-((2,3-dihydrobenzo[b][1,4]dioxin-6-yl)methyl)-1,2,3,4-tetrahydroquinoline-6-carboxamide;
(53) N-(4-bromobenzyl)-1-((2'-carbamoyl-[1,1'-biphenyl]-4-yl)methyl)-1,2,3,4-tetrahydroquinoline-6-carboxamide;
(54) 1-((2'-carbamoyl-[1,1'-biphenyl]-4-yl)methyl)-N-(3-(trifluoromethoxy)benzyl)-1,2,3,4-tetrahydroquinoline-6-carboxamide;
(55) sodium (S)-4'-((6-((1-phenylethyl)carbamoyl)-3,4-dihydroquinoline-1(2H)-yl)methyl)-[1,1'-biphenyl]-2-carboxylate;
(56) sodium (S)-4'-((6-((1-(3-fluoro-4-methylphenyl)ethyl)carbamoyl)-3,4-dihydroquinoline-1(2H)-yl)methyl)-[1,1'-biphenyl]-2-carboxylate;
(57) sodium (S)-4'-((6-((1-(4-cyanophenyl)ethyl)carbamoyl)-3,4-dihydroquinoline-1(2H)-yl)methyl)-[1,1'-biphenyl]-2-carboxylate;
(58) (S)-4'-((7-((1-phenylethyl)carbamoyl)-2H-benzo[b][1,4]oxazine-4(3H)-yl)methyl)-[1,1'-biphenyl]-2-carboxylic acid;
(59) (S)-4-((2'-carbamoyl-[1,1'-biphenyl]-4-yl)methyl)-N-(1-phenylethyl)-3,4-dihydro-2H-benzo[b][1,4]oxazine-7-carboxamide;
(60) (S)-4'-((7-((1-(4-nitrophenyl)ethyl)carbamoyl)-2H-benzo[b][1,4]oxazine-4(3H)-yl)methyl)-[1,1'-biphenyl]-2-carboxylic acid;
(61) (S)-4'-((7-((1-(4-bromophenyl)ethyl)carbamoyl)-2H-benzo[b][1,4]oxazine-4(3H)-yl)methyl)-[1,1'-biphenyl]-2-carboxylic acid;
(62) (S)-4'-((7-((1-phenylpropyl)carbamoyl)-2H-benzo[b][1,4]oxazine-4(3H)-yl)methyl)-[1,1'-biphenyl]-2- carboxylic acid;
(63) (S)-4'-((7-((1-(4-fluorophenyl)ethyl)carbamoyl)-2H-benzo[b][1,4]oxazine-4(3H)-yl)methyl)-[1,1'-biphenyl]-2-carboxylic acid;
(64) (S)-4'-((7-((1-(4-chlorophenyl)ethyl)carbamoyl)-2H-benzo[b][1,4]oxazine-4(3H)-yl)methyl)-[1,1'-biphenyl]-2-carboxylic acid;
(65) (S)-4'-((7-((1-(3-chlorophenyl)ethyl)carbamoyl)-2H-benzo[b][1,4]oxazine-4(3H)-yl)methyl)-[1,1'-biphenyl]-2-carboxylic acid;
(66) 4'-((7-((4-nitrobenzyl)carbamoyl)-2H-benzo[b][1,4]oxazine-4(3H)-yl)methyl)-[1,1'-biphenyl]-2-carboxylic acid;
(67)4'-((7-(benzylcarbamoyl)-2H-benzo[b][1,4]oxazine-4(3H)-yl)methyl)-[1,1'-biphenyl]-2-carboxylic acid;
(68) 4'-((7-(phenethylcarbamoyl)-2H-benzo[b][1,4]oxazine-4(3H)-yl)methyl)-[1,1'-biphenyl]-2-carboxylic acid;
(69) 4'-((7-((furan-2-ylmethyl)carbamoyl)-2H-benzo[b][1,4]oxazine-4(3H)-yl)methyl)-[1,1'-biphenyl]-2-carboxylic acid;
(70) (S)-4'-((7-((1-(naphthalene-1-yl)ethyl)carbamoyl)-2H-benzo[b][1,4]oxazine-4(3H)-yl)methyl)-[1,1'-biphenyl]-2-carboxylic acid;
(71) 4'-((7-((4-(methoxycarbonyl)benzyl)carbamoyl)-2H-benzo[b][1,4]oxazine-4(3H)-yl)methyl)-[1,1'-biphenyl]-2-carboxylic acid;

(72) 4'-((7-((3-methoxybenzyl)carbamoyl)-2H-benzo[b][1,4]oxazine-4(3H)-yl)methyl)-[1,1'-biphenyl]-2-carboxylic acid;
(73) 4'-((7-((4-bromobenzyl)carbamoyl)-2H-benzo[b][1,4]oxazine-4(3H)-yl)methyl)-[1,1'-biphenyl]-2-carboxylic acid;
(74) (S)-4'-((7-((1-(4-cyanophenyl)ethyl)carbamoyl)-2H-benzo[b][1,4]oxazine-4(3H)-yl)methyl)-[1,1'-biphenyl]-2-carboxylic acid;
(75) (S)-4'-((7-((1-(4-(trifluoromethoxy)phenyl)ethyl)carbamoyl)-2H-benzo[b][1,4]oxazine-4(3H)-yl)methyl)-[1,1'-biphenyl]-2-carboxylic acid;
(76) (S)-4'-((7-((1-(4-(trifluoromethyl)phenyl)ethyl)carbamoyl)-2H-benzo [b][1,4]oxazine-4(3H)-yl)methyl)-[1,1'-biphenyl]-2-carboxylic acid;
(77) (S)-4'-((7-((1-(4-((trifluoromethyl)thio)phenyl)ethyl)carbamoyl)-2H-benzo[b][1,4]oxazine-4(3H)-yl)methyl)-[1,1'-biphenyl]-2-carboxylic acid ;
(78) (S)-4'-((7-((1-(4-(tert-butyl)phenyl)ethyl)carbamoyl)-2H-benzo[b][1,4]oxazine-4(3H)-yl)methyl)-[1,1'-biphenyl]-2-carboxylic acid;
(79) (S)-4'-((7-((1-(4-fluoro-2-methylphenyl)ethyl)carbamoyl)-2H-benzo[b][1,4]oxazine-4(3H)-yl)methyl)-[1,1'-biphenyl]-2-carboxylic acid;
(80) (S)-4'-((7-((1-(3-fluoro-4-methylphenyl)ethyl)carbamoyl)-2H-benzo[b][1,4]oxazine-4(3H)-yl)methyl)-[1,1'-biphenyl]-2-carboxylic acid;
(81) (R)-4'-((7-((1-(4-cyanophenyl)ethyl)carbamoyl)-2H-benzo[b][1,4]oxazine-4(3H)-yl)methyl)-[1,1'-biphenyl]-2-carboxylicacid;
(82) 4'-((7-(cromene-3-ylcarbamoyl)-2H-benzo[b][1,4]oxazine-4(3H)-yl)methyl)-[1,1'-biphenyl]-2-carboxylic acid;
(83) (S)-4'-((7-((1-(2,6-difluorophenyl)ethyl)carbamoyl)-2H-benzo[b][1,4]oxazine-4(3H)-yl)methyl)-[1,1'-biphenyl]-2-carboxylic acid;
(84) (S)-4'-((7-((1-(3-fluorophenyl)ethyl)carbamoyl)-2H-benzo[b][1,4]oxazine-4(3H)- yl)methyl)-[1,1'-biphenyl]-2-carboxylic acid;
(85) (S)-4'-((7-((1-(3-(trifluoromethoxy)phenyl)ethyl)carbamoyl)-2H-benzo[b][1,4]oxazine-4(3H)-yl)methyl)-[1,1'-biphenyl]-2-carboxylic acid;
(86) (S)-4'-((7-((1-(3-(trifluoromethyl)phenyl)ethyl)carbamoyl)-2H-benzo[b][1,4]oxazine-4(3H)-yl)methyl)-[1,1'-biphenyl]-2-carboxylic acid;
(87) (S)-4'-((7-((1-(naphthalene-2-yl)ethyl)carbamoyl)-2H-benzo[b][1,4]oxazine-4(3H)-yl)methyl)-[1,1'-biphenyl]-2-carboxylic acid;
(88) 4'-((7-((pyridine-2-ylmethyl)carbamoyl)-2H-benzo[b][1,4]oxazine-4(3H)-yl)methyl)-[1,1'-biphenyl]-2-carboxylic acid;
(89) 4'-((7-((pyridine-3-ylmethyl)carbamoyl)-2H-benzo[b][1,4]oxazine-4(3H)-yl)methyl)-[1,1'-biphenyl]-2-carboxylic acid;
(90) 4'-((7-((pyridine-4-ylmethyl)carbamoyl)-2H-benzo[b][1,4]oxazine-4(3H)-yl)methyl)-[1,1'-biphenyl]-2-carboxylic acid;
(91) 4'-((7-(benzylcarbamoyl)-2H-benzo[b][1,4]oxazine-4(3H)-yl)methyl)-[1,1'-biphenyl]-2-carboxylic acid;
(92) 4'-((7-((2-bromobenzyl)carbamoyl)-2H-benzo[b][1,4]oxazine-4(3H)-yl)methyl)-[1,1'-biphenyl]-2-carboxylic acid;
(93) 4'-((7-(((2,3-dihydrobenzo[b][1,4]dioxin-6-yl)methyl)carbamoyl)-2H-benzo[b][1,4]oxazine-4(3H)-yl)methyl)-[1,1'-biphenyl]-2-carboxylic acid;
(94) (R)-4-((2'-carbamoyl-[1,1'-biphenyl]-4-yl)methyl)-N-(1-(4-cyanophenyl)ethyl)-3,4-dihydro-2H-benzo[b][1,4]oxazine-7-carboxamide;
(95) (S)-4-((2'-carbamoyl-[1,1'-biphenyl]-4-yl)methyl)-N-(1-(3-fluorophenyl)ethyl)-3,4-dihydro-2H-benzo[b] [1,4]oxazine-7-carboxamide;
(96) (S)-4-((2'-carbamoyl-[1,1'-biphenyl]-4-yl)methyl)-N-(1-(naphthalene-2-yl)ethyl)-3,4-dihydro-2H-benzo[b][1,4]oxazine-7-carboxamide;
(97) 4-((2'-carbamoyl-[1,1'-biphenyl]-4-yl)methyl)-N-(pyridine-3-ylmethyl)-3,4-dihydro-2H-benzo[b][1,4]oxazine-7-carboxamide;
(98) 4-((2'-carbamoyl-[1,1'-biphenyl]-4-yl)methyl)-N-(pyridine-4-ylmethyl)-3,4-dihydro-2H-benzo[b][1,4]oxazine-7-carboxamide;
(99) (S)-4-((2'-carbamoyl-[1,1'-biphenyl]-4-yl)methyl)-N-(1-(4-((trifluoromethyl)thio)phenyl)ethyl)-3,4-dihydro-2H-benzo[b][1,4]oxazine-7-carboxamide;
(100) N-benzyl-4-((2'-carbamoyl-[1,1'-biphenyl]-4-yl)methyl)-3,4-dihydro-2H-benzo[b][1,4]oxazine-7-carboxamide;
(101) N-(2-bromobenzyl)-4-((2'-carbamoyl-[1,1'-biphenyl]-4-yl)methyl)-3,4-dihydro-2H-benzo[b][1,4]oxazine-7-carboxamide;
(102) 4-((2'-carbamoyl-[1,1'-biphenyl]-4-yl)methyl)-N-((2,3-dihydrobenzo[b][l,4]dioxin-6-yl)methyl)-3,4-dihydro-2H-benzo[b][1,4]oxazine-7-carboxamide;
(103) sodium (S)-4'-((7-((1-phenylethyl)carbamoyl)-2H-benzo[b][1,4]oxazine-4(3H)-yl)methyl)-[1,1'-biphenyl]-2-carboxylate;
(104) sodium (S)-4'-((7-((1-(4-cyanophenyl)ethyl)carbamoyl)-2H-benzo[b][1,4]oxazine-4(3H)-yl)methyl)-[1,1'-biphenyl]-2-carboxylate;
(105) sodium (S)-4'-((7-((1-(4-nitrophenyl)ethyl)carbamoyl)-2H-benzo[b][1,4]oxazine-4(3H)-yl)methyl)-[1,1'-biphenyl]-2-carboxylate;
(106) (S)-1-((2'-carbamoyl-3-fluoro-[1,1'-biphenyl]-4-yl)methyl)-N-(1-phenylethyl)-1,2,3,4-tetrahydroquinoline-6-carboxylic acid;
(107) (S)-1-((2'-carbamoyl-3-fluoro-[1,1'-biphenyl]-4-yl)methyl)-N-(1-phenylethyl)-1,2,3,4-tetrahydroquinoline-6-carboxamide;
(108) (S)-3'-fluoro-4'-((6-((1-(naphthalene-2-yl)ethyl)carbamoyl)-3,4-dihydro quinoline-1(2H)-yl)methyl)-[1,1'-biphenyl]-2-carboxylic acid;
(109) (S)-3'-fluoro-4'-((6-((1-(4-nitrophenyl)ethyl)carbamoyl)-3,4-dihydroquinoline-1(2H)-yl)methyl)-[1,1'-biphenyl]-2-carboxylic acid;
(110) (S)-4'-((6-((1-(4-cyanophenyl)ethyl)carbamoyl)-3,4-dihydroquinoline-1(2H)-yl)methyl)-3'-fluoro-[1,1'-biphenyl]-2-carboxylic acid;
(111) (S)-3'-fluoro-4'-((6-((1-(3-fluoro-4-methylphenyl)ethyl)carbamoyl)-3,4-dihydroquinoline-1(2H)-yl)methyl)-[1,1'-biphenyl]-2-carboxylic acid;
(112) (S)-1-((2'-carbamoyl-3-flucre-[1,1'-biphenyl]-4-yl)methyl)-N-(1-(4-cyanophenyl)ethyl)-1,2,3,4-tetrahydroquinoline-6-carboxamide;
(113) (S)-1-((2'-carbamoyl-3-fluoro-[1,1'-biphenyl]-4-yl)methyl)-N-(1-(4-nitrophenyl)ethyl)-1,2,3,4-tetrahydroquinoline-6-carboxamide;
(114) (S)-1-((2'-carbamoyl-3-flucre-[1,1'-biphenyl]-4-yl)methyl)-N-(1-(3-fluoro-4-methylphenyl)ethyl)-1,2,3,4-tetrahydroquinoline-6-carboxamide;
(115) (S)-1-((2'-carbamoyl-3-fluoro-[1,1'-biphenyl]-4-yl)methyl)-N-(1-(naphthalene-2-yl)ethyl)-1,2,3,4-tetrahydroquinoline-6-carboxamide;
(116) (S)-3'-((6-((1-phenylethyl)carbamoyl)-3,4-dihydroquinoline-1(2H)-yl)methyl)-[1,1'-biphenyl]-2-carboxylic acid;
(117) (S)-1-((2'-carbamoyl-[1,1'-biphenyl]-3-yl)methyl)-N-(1-phenylethyl)-1,2,3,4-tetrahydroquinoline-6-carboxamide;
(118) (S)-3'-((6-((1-(4-cyanophenyl)ethyl)carbamoyl)-3,4-dihydroquinoline-1(2H)-yl)methyl)-[1,1'-biphenyl]-2-carboxylic acid;
(119) (S)-3'-((6-((1-(4-nitrophenyl)ethyl)carbamoyl)-3,4-dihydroquinoline-1(2H)-yl)methyl)-[1,1'-biphenyl]-2-carboxylic acid;
(120) (S)-3'-((6-((1-(naphthalene-2-yl)ethyl)carbamoyl)-3,4-dihydroquinoline-1(2H)-yl)methyl)-[1,1'-biphenyl]-2-carboxylic acid;
(121) (S)-3'-((6-((1-(3-fluoro-4-methylphenyl)ethyl)carbamoyl)-3,4-dihydroquinoline-1(2H)-yl)methyl)-[1,1'-biphenyl]-2-carboxylic acid;
(122) (S)-1-((2'-carbamoyl-[1,1'-biphenyl]-3-yl)methyl)-N-(1-(4-cyanophenyl)ethyl)-1,2,3,4-tetrahydroquinoline-6-carboxamide;
(123) (S)-1-((2'-carbamoyl-[1,1'-biphenyl]-3-yl)methyl)-N-(1-(4-nitrophenyl)ethyl)-1,2,3,4-tetrahydroquinoline-6-carboxamide;
(124) (S)-1-((2'-carbamoyl-[1,1'-biphenyl]-3-yl)methyl)-N-(1-(naphthalene-2-yl)ethyl)-1,2,3,4-tetrahydroquinoline-6-carboxamide;
(125) (S)-1-((2'-carbamoyl-[1,1'-biphenyl]-3-yl)methyl)-N-(1-(3-fluoro-4-methylphenyl)ethyl)-1,2,3,4-tetrahydroquinoline-6-carboxamide;
(126) (S)-2'-fluoro-3'-((6-((1-phenylethyl)carbamoyl)-3,4-dihydroquinoline- 1(2H)-yl)methyl)-[1,1'-biphenyl]-2-carboxylic acid;
(127) (S)-1-((2'-carbamoyl-2-fluoro-[1,1'-biphenyl]-3-yl)methyl)-N-(1-phenylethyl)-1,2,3,4-tetrahydroquinoline-6-carboxamide compound;
(128) (S)-2'-fluoro-3'-((6-((1-(4-nitrophenyl)ethyl)carbamoyl)-3,4-dihydroquinoline-1(2H)-yl)methyl)-[1,1'-biphenyl]-2-carboxylic acid;
(129) (S)-2'-fluoro-3'-((6-((1-(naphthalene-2-yl)ethyl)carbamoyl)-3,4-dihydroquinoline-1(2H)-yl)methyl)-[1,1'-biphenyl]-2-carboxylic acid;
(130) (S)-2'-fluoro-3'-((6-((1-(3-fluoro-4-methylphenyl)ethyl)carbamoyl)-3,4-dihydroquinoline-1(2H)-yl)methyl)-[1,1'-biphenyl]-2-carboxylic acid ;
(131) (S)-1-((2'-carbamoyl-2-fluoro-[1,1'-biphenyl]-3-yl)methyl)-N-(1-(4-nitrophenyl)ethyl)-1,2,3,4-tetrahydroquinoline-6-carboxamide;

(132) (S)-1-((2'-carbamoyl-2-fluoro-[1,1'-biphenyl]-3-yl)methyl)-N-(1-(naphthalene-2-yl)ethyl)-1,2,3,4-tetrahydroquinoline-6-carboxamide;

(133) (S)-1-((2'-carbamoyl-2-fluoro-[1,1'-biphenyl]-3-yl)methyl)-N-(1-(3-fluoro-4-methylphenyl)ethyl)-1,2,3,4-tetrahydroquinoline-6-carboxamide;

(134) (S)-4'-fluoro-3'-((6-((1-phenylethyl)carbamoyl)-3,4-dihydroquinoline-1(2H)-yl)methyl)-[1,1'-biphenyl]-2-carboxylic acid;

(135) (S)-1-((2'-carbamoyl-4-fluoro-[1,1'-biphenyl]-3-yl)methyl)-N-(1-phenylethyl)-1,2,3,4-tetrahydroquinoline-6-carboxamide;

(136) (S)-4'-fluoro-3'-((6-((1-(4-nitrophenyl)ethyl)carbamoyl)-3,4-dihydroquinoline-1(2H)-yl)methyl)-[1,1'-biphenyl]-2-carboxylic acid;

(137) (S)-4'-fluoro-3'-((6-((1-(naphthalene-2-yl)ethyl)carbamoyl)-3,4-dihydroquinoline-1(2H)-yl)methyl)-[1,1'-biphenyl]-2-carboxylic acid;

(138) (S)-4'-fluoro-3'-((6-((1-(3-fluoro-4-methylphenyl)ethyl)carbamoyl)-3,4-dihydroquinoline-1(2H)-yl)methyl)-[1,1'-biphenyl]-2-carboxylic acid;

(139) (S)-1-((2'-carbamoyl-4-fluoro-[1,1'-biphenyl]-3-yl)methyl)-N-(1-(4-nitrophenyl)ethyl)-1,2,3,4-tetrahydroquinoline-6-carboxamide;

(140) (S)-1-((2'-carbamoyl-4-fluoro-[1,1'-biphenyl]-3-yl)methyl)-N-(1-(naphthalene-2-yl)ethyl)-1,2,3,4-tetrahydroquinoline-6-carboxamide;

(141) (S)-1-((2'-carbamoyl-4-fluoro-[1,1'-biphenyl]-3-yl)methyl)-N-(1-(3-fluoro-4-methylphenyl)ethyl)-1,2,3,4-tetrahydroquinoline-6-carboxamide;

(142) (S)-2'-((7-((1-phenylethyl)carbamoyl)-2H-benzo[b][1,4]oxazine-4(3H)-yl)methyl)-[1,1'-biphenyl]-4-carboxylic acid;

(143) (S)-4-((4'-carbamoyl-[1,1'-biphenyl]-2-yl)methyl)-N-(1-phenylethyl)-3,4-dihydro-2H-benzo[b][1,4]oxazine-7-carboxamide;

(144) (S)-2'-((7-((1-(4-cyanophenyl)ethyl)carbamoyl)-2H-benzo[b][1,4]oxazine-4(3H)-yl)methyl)-[1,1'-biphenyl]-4-carboxylic acid;

(145) (S)-2'-((7-((1-(3-fluoro-4-methylphenyl)ethyl)carbamoyl)-2H-benzo[b][1,4]oxazine-4(3H)-yl)methyl)-[1,1'-biphenyl]-4-carboxylic acid;

(146) (S)-2'-((7-((1-(4-nitrophenyl)ethyl)carbamoyl)-2H-benzo[b][1,4]oxazine-4(3H)-yl)methyl)-[1,1'-biphenyl]-4-carboxylic acid;

(147) (S)-2'-((7-((1-(naphthalene-2-yl)ethyl)carbamoyl)-2H-benzo[b][1,4]oxazine-4(3H)-yl)methyl)-[1,1'-biphenyl]-4-carboxylic acid;

(148) (S)-4-((4'-carbamoyl-[1,1'-biphenyl]-2-yl)methyl)-N-(1-(3-fluoro-4-methylphenyl)ethyl)-3,4-dihydro-2H-benzo[b] [1,4]oxazine-7-carboxamide;

(149) (S)-4-((4'-carbamoyl-[1,1'-biphenyl]-2-yl)methyl)-N-(1-(4-cyanophenyl)ethyl)-3,4-dihydro-2H-benzo [b][1,4]oxazine-7-carboxamide;

(150) (S)-4-((4'-carbamoyl-[1,1'-biphenyl]-2-yl)methyl)-N-(1-(4-nitrophenyl)ethyl)-3,4-dihydro-2H-benzo[b][1,4]oxazine-7-carboxamide;

(151) (S)-4-((4'-carbamoyl-[1,1'-biphenyl]-2-yl)methyl)-N-(1-(naphthalene-2-yl)ethyl)-3,4-dihydro-2H-benzo [b] [1,4]oxazine-7-carboxamide;

(152) (S)-2'-((6-((1-phenylethyl)carbamoyl)-3,4-dihydroquinoline-1(2H)-yl)methyl)-[1,1'-biphenyl]-4-carboxylic acid;

(153) (S)-1-((4'-carbamoyl-[1,1'-biphenyl]-2-yl)methyl)-N-(1-phenylethyl)-1,2,3,4-tetrahydroquinoline-6-carboxamide;

(154) (S)-2'-((6-((1-(3-fluoro-4-methylphenyl)ethyl)carbamoyl)-3,4-dihydroquinoline-1(2H)-yl)methyl)-[1,1'-biphenyl]-4-carboxylic acid;

(155) (S)-2'-((6-((1-(4-nitrophenyl)ethyl)carbamoyl)-3,4-dihydroquinoline-1(2H)-yl)methyl)-[1,1'-biphenyl]-4-carboxylic acid;

(156) (S)-2'-((6-((1-(naphthalene-2-yl) ethyl)carbamoyl)-3,4-dihydroquinoline-1(2H)-yl)methyl)-[1,1'-biphenyl]-4-carboxylic acid;

(157) (S)-2'-((6-((1-(4-cyanophenyl)ethyl)carbamoyl)-3,4-dihydroquinoline-1(2H)-yl)methyl)-[1,1'-biphenyl]-4-carboxylic acid;

(158) (S)-1-((4'-carbamoyl-[1,1'-biphenyl]-2-yl)methyl)-N-(1-(3-fluoro-4-methylphenyl)ethyl)-1,2,3,4 -tetrahydroquinoline-6-carboxamide;

(159) (S)-1-((4'-carbamoyl-[1,1'-biphenyl]-2-yl)methyl)-N-(1-(4-nitrophenyl)ethyl)-1,2,3,4-tetrahydroquinoline-6-carboxamide;

(160) (S)-1-((4'-carbamoyl-[1,1'-biphenyl]-2-yl)methyl)-N-(1-(naphthalene-2-yl)ethyl)-1,2,3,4-tetrahydroquinoline-6-carboxamide;

(161) (S)-1-((4'-carbamoyl-[1,1'-biphenyl]-2-yl)methyl)-N-(1-(4-cyanophenyl)ethyl)-1,2,3,4-tetrahydroquinoline-6-carboxamide;

(162) (S)-2'-fluoro-4'-((7-((1-phenylethyl)carbamoyl)-2H-benzo[b][1,4]oxazine-4(3H)-yl)methyl)-[1,1'-biphenyl]-2-carboxylic acid;

(163) (S)-2'-fluoro-4'-((7-((1-(naphthalene-2-yl)ethyl)carbamoyl)-2H-benzo[b][1,4]oxazine-4(3H)-yl)methyl)-[1,1'-biphenyl]-2-carboxylic acid; and

(164) (S)-2'-fluoro-4'-((7-(((3-fluoro-4-methylphenyl)ethyl)carbamoyl)-2H-benzo[b][1,4]oxazine-4(3H)-yl)methyl)-[1,1'-biphenyl]-2-carboxylic acid.

The compound represented by formula 1 of the present invention can be used as a form of a pharmaceutically acceptable salt, in which the salt is preferably an acid addition salt formed by pharmaceutically acceptable free acids. The acid addition salt herein can be obtained from inorganic acids such as hydrochloric acid, nitric acid, phosphoric acid, sulfuric acid, hydrobromic acid, hydroiodic acid, nitrous acid, and phosphorous acid; non-toxic organic acids such as aliphatic mono/dicarboxylate, phenyl-substituted alkanoate, hydroxy alkanoate, alkandioate, aromatic acids, and aliphatic/aromatic sulfonic acids; or organic acids such as acetic acid, benzoic acid, citric acid, lactic acid, maleic acid, gluconic acid, methanesulfonic acid, 4-toluenesulfonic acid, tartaric acid, and fumaric acid. The pharmaceutically non-toxic salts are exemplified by sulfate, pyrosulfate, bisulfate, sulphite, bisulphite, nitrate, phosphate, monohydrogen phosphate, dihydrogen phosphate, metaphosphate, pyrophosphate, chloride, bromide, iodide, fluoride, acetate, propionate, decanoate, caprylate, acrylate, formate, isobutylate, caprate, heptanoate, propiolate, oxalate, malonate, succinate, suberate, sebacate, fumarate, maliate, butyne-1,4-dioate, hexane-1,6-dioate, benzoate, chlorobenzoate, methylbenzoate, dinitrobenzoate, hydroxybenzoate, methoxybenzoate, phthalate, terephthalate, benzenesulfonate, toluenesulfonate, chlorobenzenesulfonate, xylenesulfonate, phenylacetate, phenylpropionate, phenylbutylate, citrate, lactate, hydroxybutylate, glycolate, malate, tartrate, methanesulfonate, propanesulfonate, naphthalene-1-sulfonate, naphthalene-2-sulfonate, and mandelate.

The acid addition salt in this invention can be prepared by the conventional method known to those in the art. For example, the compound represented by formula 1 is dissolved in an organic solvent such as methanol, ethanol, acetone, methylenechloride, or acetonitrile, to which an organic acid or an inorganic acid is added to induce precipitation. Then, the precipitate is filtered and dried to give the salt. Or the solvent and the excessive acid are distillated under reduced pressure, and dried to give the salt. Or the precipitate is crystallized in an organic solvent to give the same.

The present invention includes not only the compound represented by formula 1 but also a pharmaceutically acceptable salt thereof, and a solvate, a hydrate, or an isomer possibly produced from the same.

The present invention also provides a method for preparing the compound represented by formula 1 comprising the following steps, as shown in reaction formula 1 below:

preparing the compound represented by formula 4 by reacting the compound represented by formula 2 with the compound represented by formula 3 (step 1);

preparing the compound represented by formula 5 by substituting methoxycarbonyl group of the compound represented by formula 4 prepared in step 1 with carboxy group (step 2);

preparing the compound represented by formula 7 by reacting the compound represented by formula 5 prepared in step 2 with the compound represented by formula 6 (step 3); and

substituting t-butoxy group of the compound represented by formula 7 prepared in step 3 with -OH group (step 4).

[Reaction Formula 1]

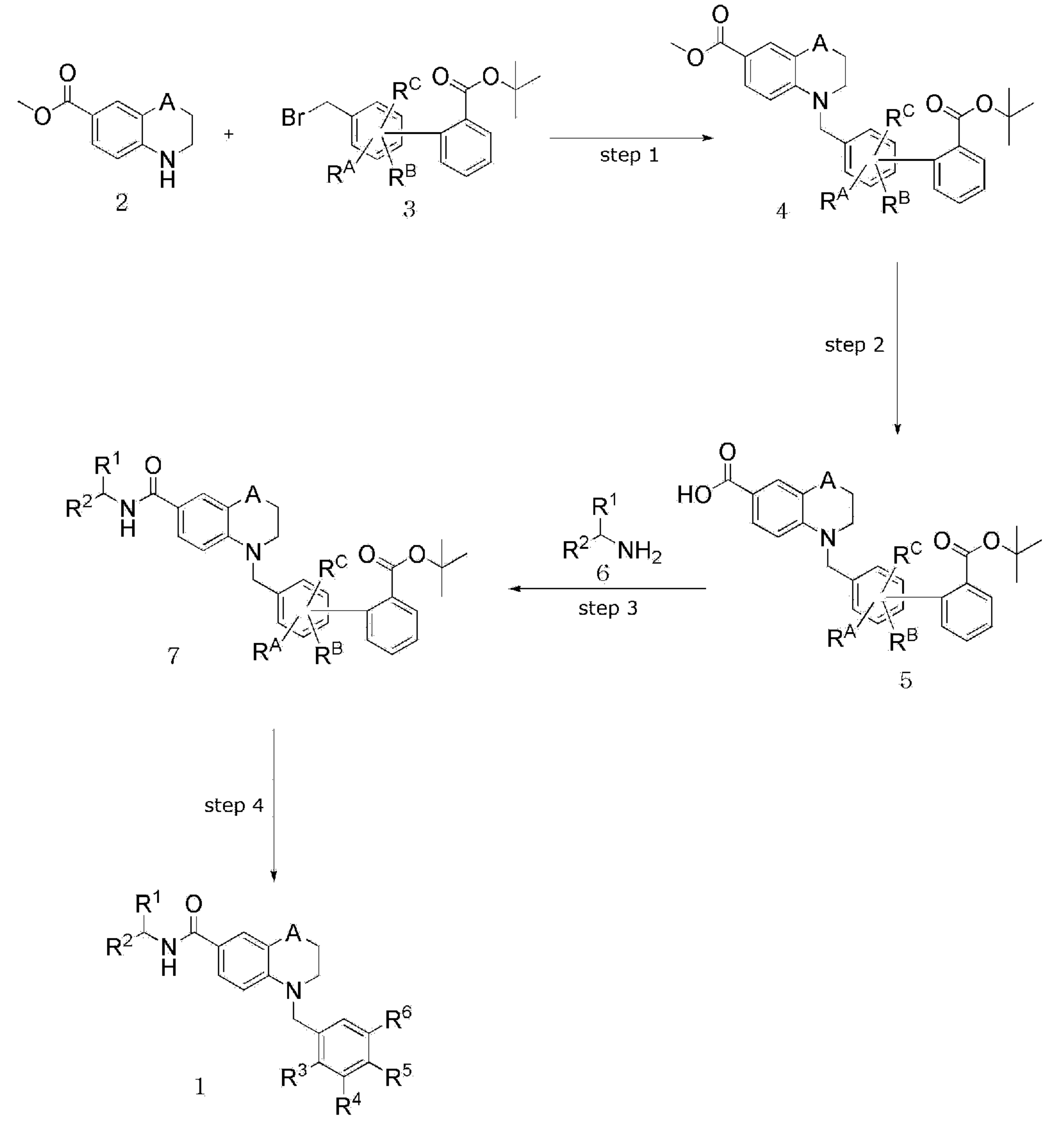


2 3 4 5 6 7 1

(In the reaction formula 1,

$R^1 \sim R^6$ and A are as defined in formula 1, and
$R^A$, $R^B$, and $R^C$ are independently as defined in $R^3 \sim R^6$).

Hereinafter, the method for preparing the compound of the invention is described in more details step by step.

In the preparation method of the invention, step 1 is to prepare the compound represented by formula 4 by reacting the compound represented by formula 2 with the compound represented by formula 3.

Particularly, the compound represented by formula 2 was reacted with the compound represented by formula 3 via alkylation in an organic solvent in the presence of a base, and as a result the compound represented by formula 4 was obtained. At this time, the reaction time was 1 ~ 30 hours and the reaction temperature was -20 ~ 100**°C**.

The organic solvent used herein was selected from the group consisting of tetrahydrofuran; dioxan; ether solvents including ethyl ether and 1,2-dimethoxyethane, dimethylformamide (DMF), dimethylsulfoxide (DMSO), dichloromethane (DCM), dichloroethane, acetonitrile, toluene, chlorobenzene, and acetone.

The base used herein was selected from the group consisting of organic bases such as pyridine, triethylamine, N,N-diisopropylethylamine (DIPEA), and 1,8-diazabicyclo[5.4.0]-7-undecene (DBU); and inorganic bases such as sodium hydroxide, sodium carbonate, potassium carbonate, cesium carbonate, and sodium hydride, which can be used by equivalent or excessive amount.

Upon completion of the reaction above, extraction was performed using an organic solvent, followed by drying, filtering, and distillation under reduced pressure. Column chromatography was additionally performed to give the compound represented by formula 4.

In the preparation method of the invention, step 2 is to substitute methoxycarbonyl group of the compound represented by formula 4 with carboxyl group.

Particularly, the compound represented by formula 4 was stirred in an organic solvent in the presence of a base. Upon completion of the reaction, the compound was acidified to give the compound represented by formula 5. At this time, the reaction time was 1 ~ 30 hours and the reaction temperature was -20 ~ 100**°C**.

The organic solvent used herein was selected from the group consisting of lower alcohols including ethanol, propanol, and butanol; tetrahydrofuran, and water.

The base used herein was selected from the group consisting of inorganic bases such as sodium hydroxide and lithium hydroxide, which can be used by equivalent or excessive amount.

Further, the acid used herein was HCl.

Upon completion of the reaction above, extraction was performed using an organic solvent, followed by drying, filtering, and distillation under reduced pressure. Column chromatography was additionally performed to give the compound represented by formula 5.

In the preparation method of the invention, step 3 is to prepare the compound represented by formula 7 by reacting the compound represented by formula 5 with the compound represented by formula 6.

Particularly, the compound represented by formula 5 was reacted with the compound represented by formula 6 via dehydrating condensation in an organic solvent to form a peptide bond, leading to the preparation of the compound represented by formula 7. At this time, the reaction time was 1 ~ 30 hours and the reaction temperature was -20 ~ 100**°C**.

The organic solvent used herein was selected from the group consisting of dimethylformamide (DMF), dimethylsulfoxide (DMSO), dichloromethane (DCM), and dichloroethane.

The base used herein was selected from the group consisting of organic bases such as DIPEA (N,N'-Diisopropylethylamine), pyridine, and triethylamine, which can be used by equivalent or excessive amount.

The catalyst used herein was HATU (1-[Bis(dimethylamino)methylene]-1H-1,2,3-triazolo[4,5-b]pyridinium 3-oxid hexafluorophosphate).

Upon completion of the reaction above, extraction was performed using an organic solvent, followed by drying, filtering, and distillation under reduced pressure. Column chromatography was additionally performed to give the compound represented by formula 7.

In the preparation method of the invention, step 4 is to substitute t-butoxy group of the compound represented by formula 7 prepared in step 3 with -OH group.

Particularly, the compound represented by formula 7 was reacted in an organic solvent in the presence of an acid to prepare the compound represented by formula 1. At this time, the reaction time was 1 ~ 30 hours and the reaction temperature was -20 ~ 100**°C**.

The organic solvent used herein was selected from the group consisting of dichloromethane (DCM) and dichloroethane.

The acid used herein was selected from the group consisting of TFA (trifluoroacetic acid), HCl, and $H_2SO_4$.

Upon completion of the reaction above, extraction was performed using an organic solvent, followed by drying, filtering, and distillation under reduced pressure. Column chromatography was additionally performed to give the compound represented by formula 1.

The present invention also provides a method for preparing the compound represented by formula 1 comprising the following steps, as shown in reaction formula 2 below:

preparing the compound represented by formula 9 by reacting the compound represented by formula 8 with the compound represented by formula 6 (step 1);
preparing the compound represented by formula 7 by reacting the compound represented by formula 9 prepared in step 1 with the compound represented by formula 3 (step 2); and
substituting t-butoxy group of the compound represented by formula 7 prepared in step 2 with -OH group (step 3).

[Reaction Formula 2]

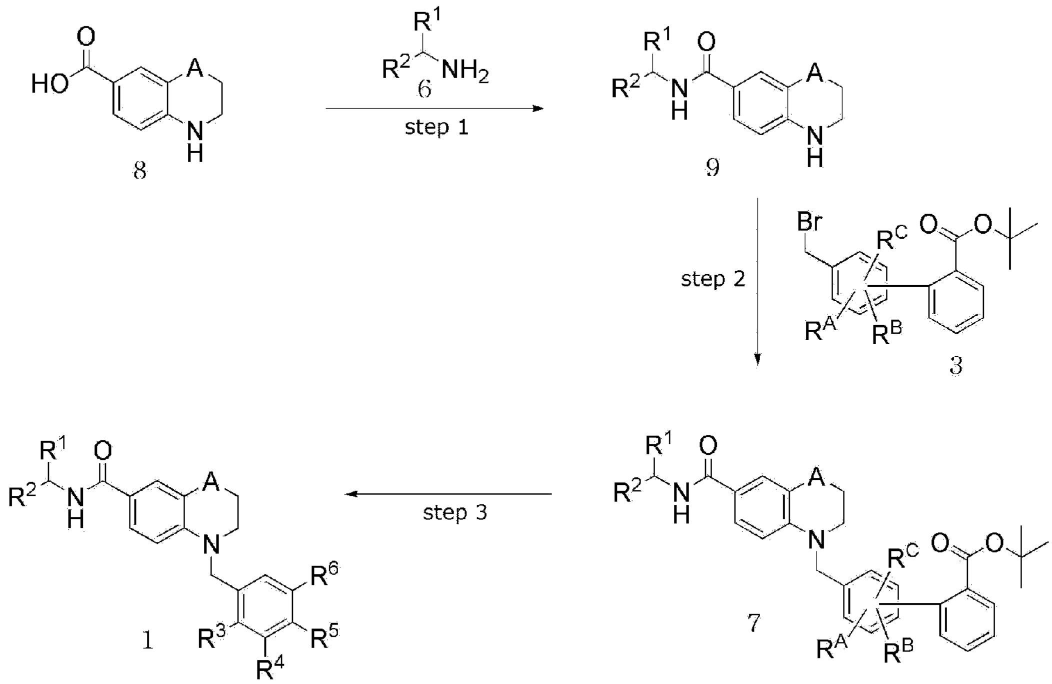


(In the reaction formula 2,

$R^1$ ~ $R^6$ and A are as defined in formula 1, and
$R^A$, $R^B$, and $R^C$ are independently as defined in $R^3$ ~ $R^6$).

Hereinafter, the method for preparing the compound of the invention is described in more details step by step.

In the preparation method of the invention, step 1 is to prepare the compound represented by formula 9 by reacting the compound represented by formula 8 with the compound represented by formula 6.

Particularly, the compound represented by formula 8 was reacted with the compound represented by formula 6 via dehydrating condensation in an organic solvent to form a peptide bond, leading to the preparation of the compound represented by formula 9. At this time, the reaction time was 1 ~ 30 hours and the reaction temperature was -20 ~ 100**°C**.

The organic solvent used herein was selected from the group consisting of dimethylformamide (DMF), dimethylsulfoxide (DMSO), dichloromethane (DCM), and dichloroethane.

The base used herein was selected from the group consisting of organic bases such as DIPEA (N,N'-Diisopropylethylamine), pyridine, and triethylamine, which can be used by equivalent or excessive amount.

The catalyst used herein was HATU (1-[Bis(dimethylamino)methylene]-1H-1,2,3-triazolo[4,5-b]pyridinium 3-oxid hexafluorophosphate).

Upon completion of the reaction above, extraction was performed using an organic solvent, followed by drying, filtering, and distillation under reduced pressure. Column chromatography was additionally performed to give the compound represented by formula 9.

In the preparation method of the invention, step 2 is to prepare the compound represented by formula 7 by reacting the compound represented by formula 9 with the compound represented by formula 3.

Particularly, the compound represented by formula 9 was reacted with the compound represented by formula 3 via alkylation in an organic solvent in the presence of a base, and as a result the compound represented by formula 7 was obtained. At this time, the reaction time was 1 ~ 30 hours and the reaction temperature was -20 ~ 100**°C**.

The organic solvent used herein was selected from the group consisting of tetrahydrofuran; dioxan; ether solvents including ethyl ether and 1,2-dimethoxyethane, dimethylformamide (DMF), dimethylsulfoxide (DMSO), dichloromethane (DCM), dichloroethane, acetonitrile, toluene, chlorobenzene, and acetone.

The base used herein was selected from the group consisting of organic bases such as pyridine, triethylamine, N,N-diisopropylethylamine (DIPEA), and 1,8-diazabicyclo[5.4.0]-7-undecene (DBU); and inorganic bases such as sodium hydroxide, sodium carbonate, potassium carbonate, cesium carbonate, and sodium hydride, which can be used by equivalent or excessive amount.

Upon completion of the reaction above, extraction was performed using an organic solvent, followed by drying,

filtering, and distillation under reduced pressure. Column chromatography was additionally performed to give the compound represented by formula 7.

In the preparation method of the invention, step 3 is to substitute t-butoxy group of the compound represented by formula 7 prepared in step 2 with -OH group.

Particularly, the compound represented by formula 7 was reacted in an organic solvent in the presence of an acid to prepare the compound represented by formula 1. At this time, the reaction time was 1 ~ 30 hours and the reaction temperature was -20 ~ 100°**C**.

The organic solvent used herein was selected from the group consisting of dichloromethane (DCM) and dichloroethane.

The acid used herein was selected from the group consisting of TFA (trifluoroacetic acid), HCl, and $H_2SO_4$.

Upon completion of the reaction above, extraction was performed using an organic solvent, followed by drying, filtering, and distillation under reduced pressure. Column chromatography was additionally performed to give the compound represented by formula 1.

The present invention further provides a method for preparing the compound represented by formula 1, as shown in reaction formula 3A below, comprising the step of substituting carboxyl group of the compound represented by formula 1A with sodiumoxycarbonyl group (step 1).

[Reaction Formula 3A]

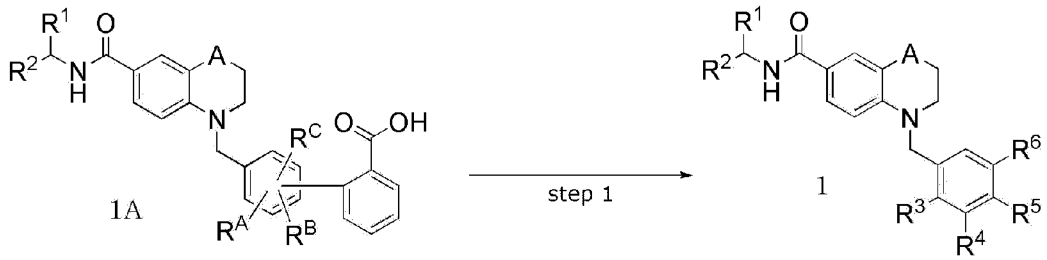


1A

1

(In the reaction formula 3A,

$R^1$ ~ $R^6$ and A are as defined in formula 1, and
$R^A$, $R^B$, and $R^C$ are independently as defined in $R^3$ ~ $R^6$).

Particularly, the compound represented by formula 1A was stirred in an organic solvent in the presence of a base to give the compound represented by formula 1. At this time, the reaction time was 1 ~ 30 hours and the reaction temperature was -20 ~ 100°**C**.

The organic solvent used herein was selected from the group consisting of lower alcohols including methanol, ethanol, propanol, and butanol; dichloromethane (DCM), dichloroethane, and ethylether.

The base used herein was sodium hydroxide, which can be used by equivalent or excessive amount.

Upon completion of the reaction above, extraction was performed using an organic solvent, followed by drying, filtering, and distillation under reduced pressure. Column chromatography was additionally performed to give the compound represented by formula 1.

The present invention further provides a method for preparing the compound represented by formula 1, as shown in reaction formula 3B below, comprising the step of substituting carboxyl group of the compound represented by formula 1A with amide group (step 1).

[Reaction Formula 3B]

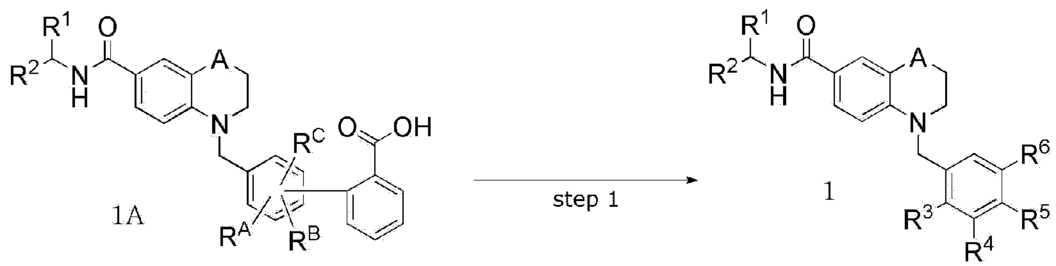


1A

1

(In the reaction formula 3B,

$R^1 \sim R^6$ and A are as defined in formula 1, and
$R^A$, $R^B$, and $R^C$ are independently as defined in $R^3 \sim R^6$).

Particularly, the compound represented by formula 1A was stirred in an organic solvent in the presence of a catalyst and a base to give the compound represented by formula 1. At this time, the reaction time was 1 ~ 30 hours and the reaction temperature was -20 ~ 100°**C**.

The organic solvent used herein was selected from the group consisting of ammonium hydroxide, dimethylformamide (DMF), dimethylsulfoxide (DMSO), dichloromethane (DCM), dichloroethane, and water.

The catalyst used herein was selected from the group consisting of EDCI (1-Ethyl-3-(3-dimethylaminopropyl)carbodi-imide), DMAP (4-Dimethylamino pyridine), and HOBt (Hydroxybenzotriazole).

Upon completion of the reaction above, extraction was performed using an organic solvent, followed by drying, filtering, and distillation under reduced pressure. Column chromatography was additionally performed to give the compound represented by formula 1.

The present invention also provides a pharmaceutical composition for treating PPARG-related disease which comprises the compound represented by formula 1, the optical isomer thereof, or the pharmaceutically acceptable salt of the same as an active ingredient. Particularly, the PPARG-related disease herein includes diabetes, insulin resistance, impaired glucose tolerance, pre-diabetes, hyperglycemia, hyperinsulinemia, obesity, and inflammation, but not always limited thereto.

The compound represented by formula 1 or the optical isomer thereof binds to PPARG with a high affinity but does not act as an agonist and thereby does not induce gene transcription thereof, so that it can block CDK5 which is a cause of PPARG phosphorylation to suppress the side effects of the conventional anti-diabetic agents. Particularly, the said side effects are exemplified by weight gain, edema, impairment of bone growth or formation, and cardiac hypertrophy, but not always limited thereto.

The cause of the said side effects is the phosphorylation of the $273^{rd}$ amino acid serine of PPARG of CDK5 kinase induced by various gene mutations. Therefore, it will be an important step for the development of an anti- diabetic agent to suppress PPARG gene transcription or to block CDK5 mediated phosphorylation. In particular, when CDK5 binds to S273 among the sites that are available for the conjugation specifically with PPARG structure (helix, H11, H12, and S273), it induces phosphorylation there to cause side effects.

The present inventors performed an experiment to investigate whether or not the compounds prepared in the example of the invention could inhibit the phosphorylation of serine, the $273^{rd}$ amino acid of PPARG. As a result, it was confirmed that the compounds of the examples of the invention bound to PPARG (peroxisome proliferator activated receptor-gamma) but did not act as a CDK5 (cyclin-dependant kinase 5) promoter and thereby exhibited excellent inhibitory effect on the phosphorylation of serine, the $273^{rd}$ amino acid of PPARG (see Experimental Example 1).

The present inventors also investigated the PPARG (peroxisome proliferator activated receptor-gamma) transcriptional activity of the compounds prepared in the example of the invention. As a result, it was confirmed that the compounds prepared in the example of the invention had excellent activity of binding to PPARG (peroxisome proliferator activated receptor-gamma) but did not induce the transcription or activation of PPARG (peroxisome proliferator activated receptor-gamma) (see Experimental Example 2).

The present inventors also investigated the inhibitory effect of the compounds prepared in the examples of the invention on CYP (cytochrome P450) activity. As a result, it was confirmed that the compounds of examples 14, 36, 44, 56, 57, 58, 60, 74, 96, 110, 113, 115, 118, 123, 131, and 139 inhibited CYP isozymes 1A2, 2C9, 2C19, and 2D6 activity comparatively less strongly than the compounds of Comparative Examples (SR1664 and SR1824).

Particularly, SR1664 inhibited the activity of CYP isozyme 2C9 so that 96% of the substance was not decomposed and instead stayed, suggesting that when SR1664 is administered as a drug the amount of the drug that reaches a target is way too much than a proper dose, so that it might induce toxicity.

However, the compounds of the present invention inhibited CYP activity properly, better than SR1664 could, so the drug was decomposed at least 10 times more than SR1664 could be, suggesting that only a proper amount of a drug reaches a target (see Experimental Example 3).

Further, the present inventors performed another experiment to evaluate the cardiotoxicity of the compounds prepared in the examples of the invention. As a result, the compounds of the invention displayed a significantly low $IC_{50}$ (2.7 $\mu$M), which was significantly lower than $IC_{50}$ that induces cardiotoxicity (less than 10 uM) of the compound of Comparative Example (SR-1664). The above result indicates that the compound of Comparative Example has a high chance of causing cardiotoxicity as a drug.

However, the compounds prepared in the examples of the invention displayed very high $IC_{50}$, which was much higher than $IC_{50}$ that could cause cardiotoxicity (less than 10 uM), indicating that the compounds had a significantly low chance of causing cardiotoxicity (see Experimental Example 4).

The present inventors also investigated the blood sugar lowering effect and the weight reducing effect of the compounds prepared in the examples of the invention. As a result, the compounds of examples 56, 155, 156, and 157 (10 mpk respectively) reduced body weight at least 5% even at a comparatively low dose, while the compound of Comparative Example (SR-1664, 20 mpk) reduced body weight by 1.6%.

The compounds of the examples 56, 156, and 157 (10 mpk respectively) reduced blood sugar at least 24% even at a comparatively low dose, while the compound of Comparative Example (SR-1664, 20 mpk) reduced blood sugar by 14% (see Experimental Example 5).

The compound represented by formula 1, the optical isomer thereof, or the pharmaceutically acceptable salt thereof of the present invention can be prepared for oral or parenteral administration.

The formulations for oral administration are exemplified by tablets, pills, hard/soft capsules, solutions, suspensions, emulsions, syrups, granules, elixirs, and troches, etc. These formulations can include diluents (for example, lactose, dextrose, sucrose, mannitol, sorbitol, cellulose, and/or glycine) and lubricants (for example, silica, talc, stearate and its magnesium or calcium salt, and/or polyethylene glycol) in addition to the active ingredient. Tablets can include binding agents such as magnesium aluminum silicate, starch paste, gelatin, methylcellulose, sodium carboxymethylcellulose and/or polyvinylpyrolidone, and if necessary disintegrating agents such as starch, agarose, alginic acid or its sodium salt or azeotropic mixtures and/or absorbents, coloring agents, flavours, and sweeteners can be additionally included thereto.

The pharmaceutical composition comprising the compound represented by formula 1 as an active ingredient can be administered by parenterally and the parenteral administration includes subcutaneous injection, intravenous injection, intramuscular injection, or intrathoracic injection.

To prepare the composition as a formulation for parenteral administration, the compound represented by formula 1, the optical isomer thereof, or the pharmaceutically acceptable salt thereof is mixed with a stabilizer or a buffering agent in water to produce a solution or suspension, which is then formulated as ampoules or vials. The composition herein can be sterilized and additionally contains preservatives, stabilizers, wettable powders or emulsifiers, salts and/or buffers for the regulation of osmotic pressure, and other therapeutically useful materials, and the composition can be formulated by the conventional mixing, granulating or coating method.

The effective dosage of the pharmaceutical composition comprising the compound represented by formula 1 as an active ingredient of the present invention can be determined according to age, weight, gender, administration method, health condition, and severity of disease. The dosage is preferably 0.01 ~ 1000 mg/kg/day, which can be administered several times a day or preferably 1 ~ 3 times a day.

The preparation method of the compound represented by formula 1 of the present invention is described in more detail in preparative examples or examples. The following preparative examples or examples are only examples to describe the method for preparing the compound represented by formula 1 and the present invention is not limited thereto. The preparation methods described in the following preparative examples or examples are performed under the conditions with proper reagents well-known in the field of organic synthesis.

**<Preparative Example 1> 1-((2'-(tert-butoxycarbonyl)-[1,1'-biphenyl]-4-yl)methyl)-1,2,3,4-tetrahydroquinoline-6-carboxylic acid**

<u>Step 1 : Methyl 1,2,3,4-tetrahydroquinoline-6-carboxylate</u>

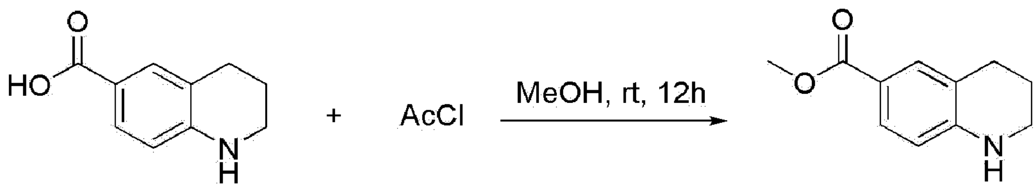


1,2,3,4-tetrahydroquinoline-6-carboxylic acid (50 g, 282 mmol) was dissolved in MeOH (500 mL) in a 2 L flask, to which AcCl (100 mL) was added at 0°**C,** followed by stirring at room temperature for 12 hours. Upon completion of the reaction, the reaction mixture was concentrated under reduced pressure to give methyl 1,2,3,4-tetrahydroquinoline-6-carboxylate (45 g).

<u>Step 2 : Methyl 1-((2'-(tert-butoxycarbonyl)-[1,1'-biphenyl]-4-yl)methyl)-1,2,3,4-tetrahydroquinoline-6-carboxylate</u>

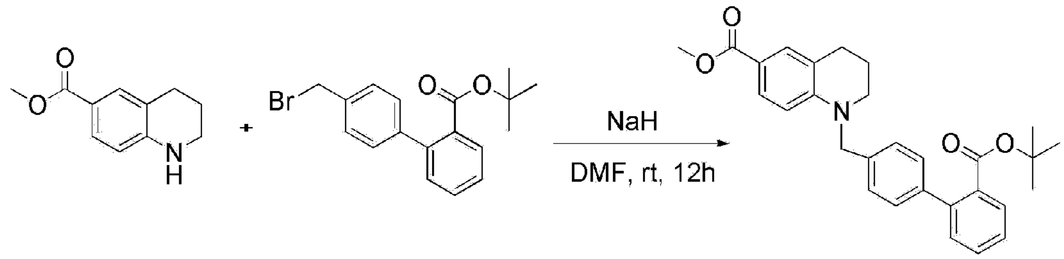


Methyl 1,2,3,4-tetrahydroquinoline-6-carboxylate (2 g, 10.46 mmol) was dissolved in DMF (20 ml) in a 1 L flask, to which NaH (628 mg, 15.69 mmol) and tert-butyl 4'-(bromomethyl)-[1,1'-biphenyl]-2-carboxylate (4.36 g, 12.55 mmol) were added, followed by stirring at room temperature for 12 hours. The organic layer was separated by using ethyl acetate and brine. The organic layer was dried over $MgSO_4$ and filtered. The solvent was eliminated from the mixture via vacuum distillation, followed by column chromatography to give methyl 1-((2'-(tert-butoxycarbonyl)-[1,1'-biphenyl]-4-yl)methyl)-1,2,3,4-tetrahydroquinoline-6-carboxylate (4 g, 8.55 mmol, 85%).

Step 3 : 1-((2'-(tert-butoxycarbonyl)-[1,1'-biphenyl]-4-yl)methyl)-1,2,3,4-tetrahydroquinoline-6-carboxylic acid

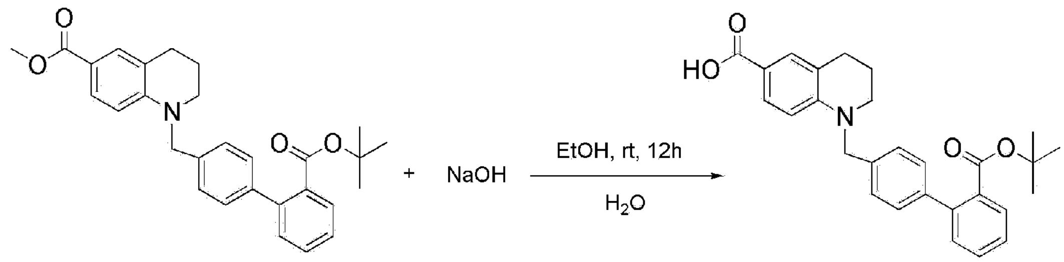


Methyl 1-((2'-(tert-butoxycarbonyl)-[1,1'-biphenyl]-4-yl)methyl)-1,2,3,4-tetrahydroquinoline-6-carboxylate (4 g, 8.55 mmol) was mixed with NaOH (1.48 g, 37 mmol) dissolved in EtOH (50 mL) / $H_2O$ (50 mL) in a 250 mL flask, followed by stirring at room temperature for 12 hours. Upon completion of the reaction, the solvent was concentrated and pH was adjusted to be 5 with 2N-HCl. Extraction was performed with EA and the organic layer was dried over $MgSO_4$ and filtered. As a result, 1-((2'-(tert-butoxycarbonyl)-[1,1'-biphenyl]-4-yl)methyl)-1,2,3,4-tetrahydroquinoline-6-carboxylic acid was obtained (3.55 g, 8.0 mmol, 90%).

**<Preparative Example 2> 4-((2'-(tert-butoxycarbonyl)-[1,1'-biphenyl]-4-yl)methyl)-3,4-dihydro-2H-benzo[b][1,4]oxazine-7-carboxylic** acid

Step 1 Methyl 4-amino-3-hydroxybenzoate

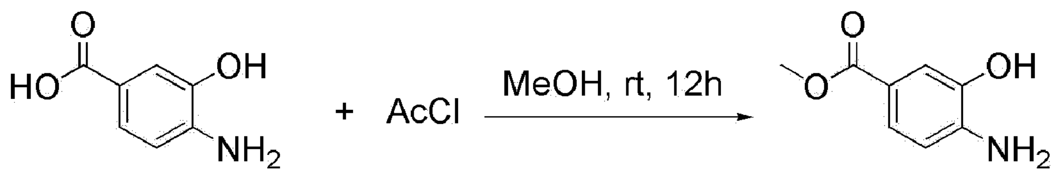


4-amino-3-hydroxybenzoic acid (50 g, 326 mmol) was dissolved in MeOH (500 mL) in a 2 L flask, to which AcCL (100 mL) was added at 0°C, followed by stirring at room temperature for 12 hours. Upon completion of the reaction, the reaction mixture was concentrated under reduced pressure to give 4-amino-3-hydroxybenzoate (50 g).

Step 2 : Methyl 3,4-dihydro-2H-benzo[b][1,4]oxazine-7-carboxylate

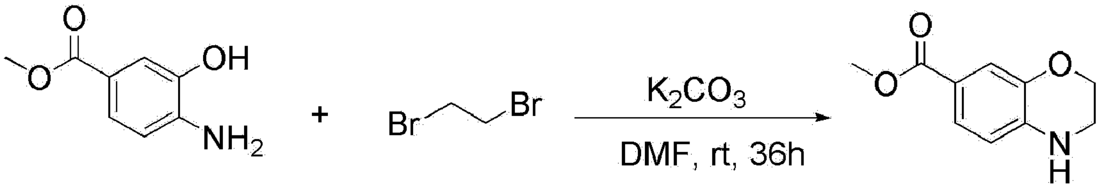


Methyl 4-amino-3-hydroxybenzoate (55 g, 329 mmol) prepared in step 1 and 1,2-dibromoethane (185 g, 986 mmol) were dissolved in DMF (1 L) in a 2 L flask, to which $K_2CO_3$ (227 g, 1645 mmol) was added, followed by stirring at room temperature for 36 hours. The organic layer was separated by using ethyl acetate and brine. The separated organic layer was dried over $MgSO_4$ and filtered. The solvent was eliminated from the mixture via vacuum distillation, followed by column chromatography to give methyl 3,4-dihydro-2H-benzo[b][1,4]oxazine-7-carboxylate (41.3 g, 213 mmol, 65%).

Step 3 : Methyl 4-((2'-(tert-butoxycarbonyl)-[1,1'-biphenyl]-4-yl)methyl)-3,4-dihydro-2H-benzo[b][1,4]oxazine-7-carboxylate

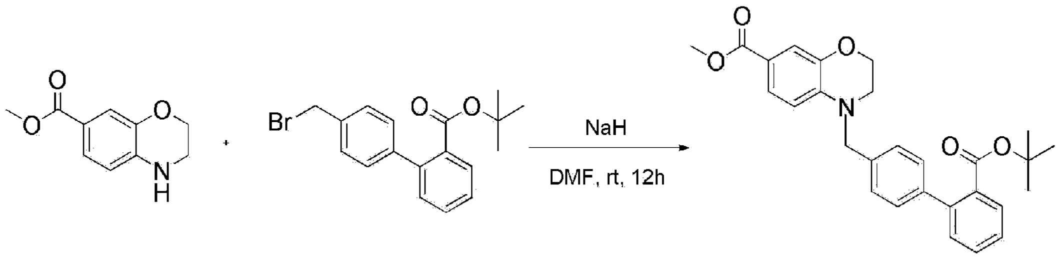


Methyl 3,4-dihydro-2H-benzo[b][1,4]oxazine-7-carboxylate (8.08 g, 41.8 mmol) obtained in step 2 was dissolved in DMF (50 ml) in a 500 mL flask, to which NaH (2 g, 50.2 mmol) and tert-butyl 4'-(bromomethyl)-[1,1'-biphenyl]-2-carboxylate (17.4 g, 50.2 mmol) were added, followed by stirring at room temperature for 12 hours. The organic layer was separated by using ethyl acetate and brine. The separated organic layer was dried over $MgSO_4$ and filtered. The solvent was eliminated from the mixture via vacuum distillation, followed by column chromatography to give methyl 4-((2'-(tert-butoxycarbonyl)-[1,1'-biphenyl]-4-yl)methyl)-3,4-dihydro-2H-benzo[b][1,4]oxazine-7-carboxylate (28.4 g, 68.03 mmol, 68%).

Step 4 : 4-((2'-(tert-butoxycarbonyl)-[1,1'-biphenyl]-4-yl)methyl)-3,4-dihydro-2H-benzo[b][1,4]oxazine-7-carboxylic acid

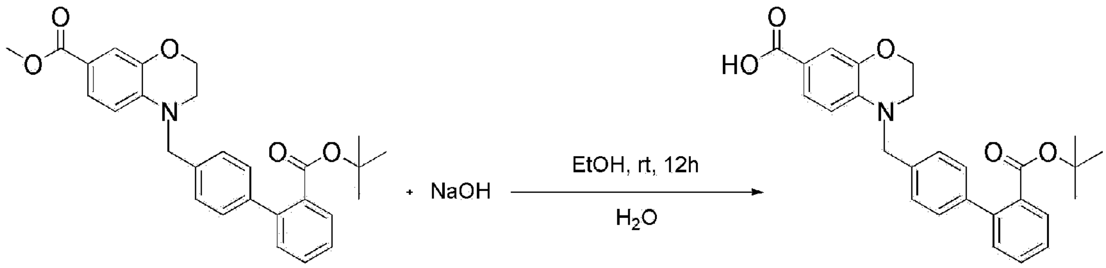


Methyl 4-((2'-(tert-butoxycarbonyl)-[1,1'-biphenyl]-4-yl)methyl)-3,4-dihydro-2H-benzo[b][1,4]oxazine-7-carboxylate (11.8 g, 28.43 mmol) obtained in step 3 was mixed with NaOH (5.68 g, 142 mmol) dissolved in EtOH (50 ml)

and $H_2O$ (50 ml) in a 250 mL flask, followed by stirring at room temperature for 12 hours. Upon completion of the reaction, the solvent was concentrated. PH of the reaction mixture was adjusted to 5 by using 2*N*-HCl. The mixture was extracted with EA. The organic layer was dried over $MgSO_4$ and filtered. As a result, 4-((2'-(tert-butoxycarbonyl)-[1,1'-biphenyl]-4-yl)methyl)-3,4-dihydro-2H-benzo[b][1,4]oxazine-7-carboxylic acid was obtained (10.3 g, 25.53 mmol, 90%).

**<Preparative Example 3> 1-((2'-(tert-butoxycarbonyl)-3-fluoro-[1,1'-biphenyl]-4-yl)methyl)-1,2,3,4-tetrahydroquinoline-6-carboxylic acid**

Step 1 : tert-butyl 2-bromobenzoate

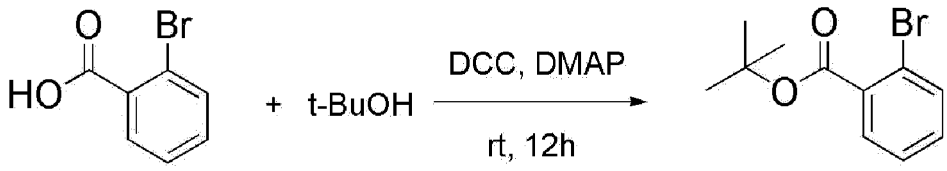


2-bromobenzoic acid (25 g, 124 mmol), DMAP (1.52 g, 12.4 mmol), and t-butanol (9.2 g, 124 mmol) were dissolved in dichloromethane (500 ml) in a 1 L flask, to which DCC (25.7 g, 124 mmol) was added, followed by stirring at room temperature for 12 hours. The organic layer was separated by using ethyl acetate and brine. The separated organic layer was dried over $MgSO_4$ and filtered. The solvent was eliminated from the mixture via vacuum distillation, followed by column chromatography to give tert-butyl 2-bromobenzoate (30 g).

Step 2 : tert-butyl 3'-fluoro-4'-methyl-[1,1'-biphenyl]-2-carboxylate

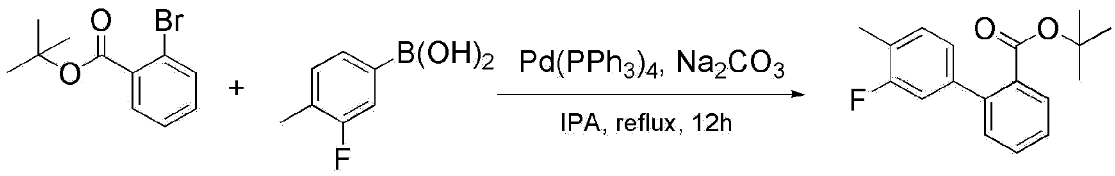


Tert-butyl 2-bromobenzoate (10 g, 64.8 mmol), (3-fluoro-4-methylphenyl)boronic acid (14 g, 54 mmol), $Pd(PPh_3)_4$ (62 4 mg, 0.54 mmol), and $Na_2CO_3$ (11.4 g, 108 mmol) were dissolved in IPA (60 ml) and $H_2O$ (60 ml) in a 1 L flask, followed by reflux-stirring for 12 hours. The reaction mixture was concentrated under reduced pressure. The organic layer was separated by using ethyl acetate and brine. The separated organic layer was dried over $MgSO_4$ and filtered. The mixture was separated by column chromatography to give tert-butyl 3'-fluoro-4'-methyl-[1,1'-biphenyl]-2-carboxylate (14 g).

Step 3 : tert-butyl 4'-(bromomethyl)-3'-fluoro-[1,1'-biphenyl]-2-carboxylate

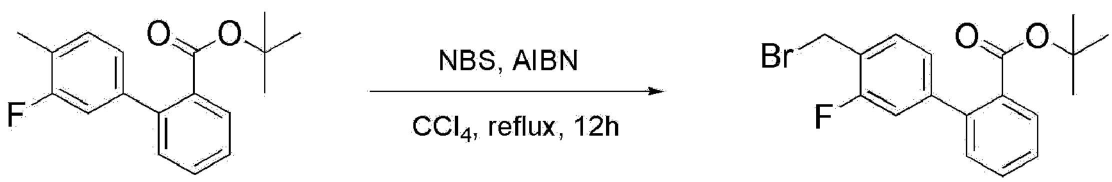


Tert-butyl 3'-fluoro-4'-methyl-[1,1'-biphenyl]-2-carboxylate (14 g, 48.9 mmol), NBS (9.57 g, 53.79 mmol), and AIBN (2.4 g, 14.67 mmol) were dissolved in $CCl_4$ (50 ml) in a 1 L flask, followed by reflux-stirring for 12 hours. The reaction mixture was concentrated under reduced pressure. The organic layer was separated by using dichloromethane and brine. The separated organic layer was dried over $MgSO_4$ and filtered. The mixture was separated by column

chromatography to give tert-butyl 4'-(bromomethyl)-3'-fluoro-[1,1'-biphenyl]-2-carboxylate (7.4 g).

Step 4 : methyl 1-((2'-(tert-butoxycarbonyl)-3-fluoro-[1,1'-biphenyl]-4-yl)methyl)-1,2,3,4-tetrahydroquinoline-6-carboxylate

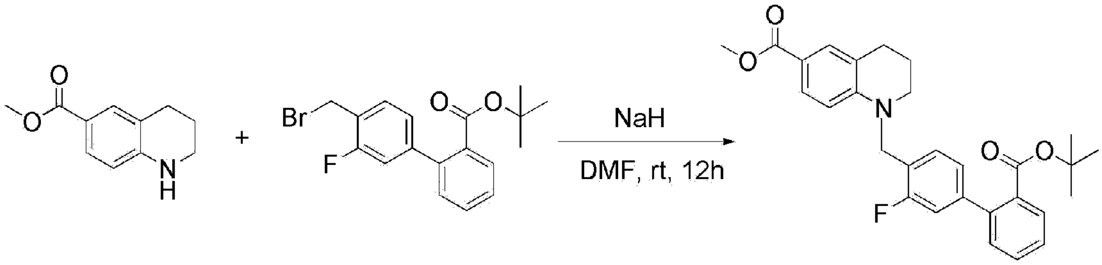


Methyl 1,2,3,4-tetrahydroquinoline-6-carboxylate (3 g, 15.69 mmol) was dissolved in DMF (20 ml) in a 250 mL flask, to which NaH (753 mg, 18.83 mmol) and tert-butyl 4'-(bromomethyl)-3'-fluoro-[1,1'-biphenyl]-2-carboxylate (5.7 g, 15.69 mmol) were added, followed by stirring at room temperature for 12 hours. The organic layer was separated by using ethyl acetate and brine. The separated organic layer was dried over $MgSO_4$ and filtered. The solvent was eliminated from the mixture via vacuum distillation, followed by column chromatography to give methyl 1-((2'-(tert-butoxycarbonyl)-3-fluoro-[1,1'-biphenyl]-4-yl)methyl)-1,2,3,4-tetrahydroquinoline-6-carboxylate (4 g).

Step 5 : 1-((2'-(tert-butoxycarbonyl)-3-fluoro-[1,1'-biphenyl]-4-yl)methyl)-1,2,3,4-tetrahydroquinoline-6-carboxylic acid

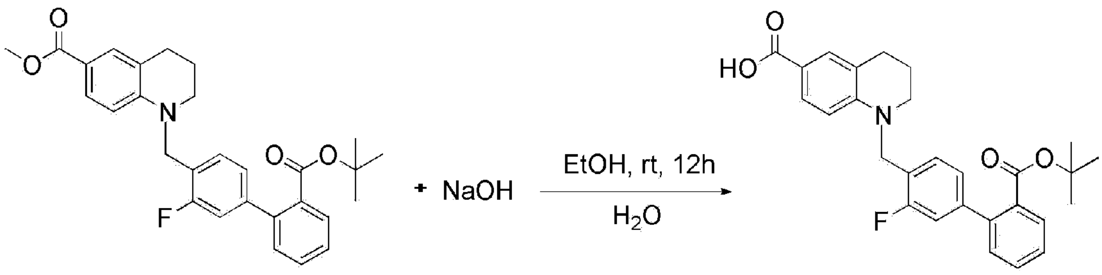


Methyl 1-((2'-(tert-butoxycarbonyl)-3-fluoro-[1,1'-biphenyl]-4-yl)methyl)-1,2,3,4-tetrahydroquinoline-6-carboxylate (4 g, 8.4 mmol) was mixed with NaOH (1.48 g, 37 mmol) dissolved in EtOH (50 ml) and $H_2O$ (50 ml) in a 250 mL flask, followed by stirring at room temperature for 12 hours. Upon completion of the reaction, the solvent was concentrated. PH of the reaction mixture was adjusted to 5 by using 2*N*-HCl. The mixture was extracted with EA. The organic layer was dried over $MgSO_4$ and filtered. As a result, 1-((2'-(tert-butoxycarbonyl)-3-fluoro-[1,1'-biphenyl]-4-yl)methyl)-1,2,3,4-tetrahydroquinoline-6-carboxylic acid was obtained (3 g).

**<Preparative Example 4> 1-((2'-(tert-butoxycarbonyl)-[1,1'-biphenyl]-3-yl)methyl)-1,2,3,4-tetrahydroquinoline-6-carboxylic acid**

Step 1 : tert-butyl 2-bromobenzoate

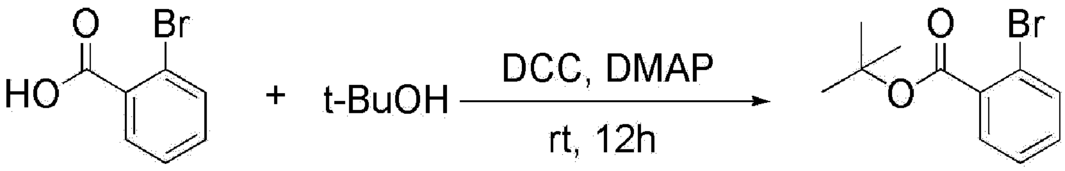

2-bromobenzoic acid (25 g, 124 mmol), DMAP (1.52 g, 12.4 mmol), and t-butanol (9.2 g, 124 mmol) were dissolved in dichloromethane (500 ml) in a 1 L flask, to which DCC (25.7 g, 124 mmol) was added, followed by stirring at room temperature for 12 hours. The organic layer was separated by using ethyl acetate and brine. The separated organic layer was dried over $MgSO_4$ and filtered. The solvent was eliminated from the mixture via vacuum distillation, followed by column chromatography to give tert-butyl 2-bromobenzoate (30 g).

Step 2 : tert-butyl 3'-methyl-[1,1'-biphenyl]-2-carboxylate

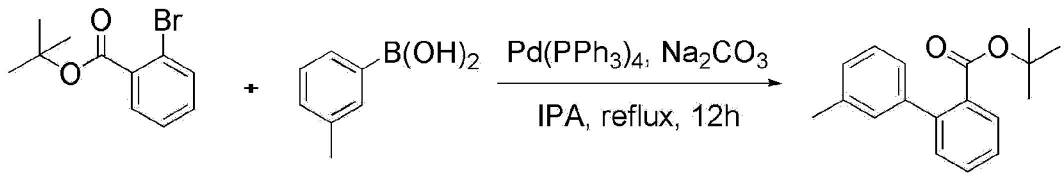


Tert-butyl 2-bromobenzoate (22.7 g, 88.26 mmol), meta-toluoylboronic acid (8 g, 58.84 mmol), $Pd(PPh_3)_4$ (680 mg, 0.58 mmol), and $Na_2CO_3$ (12.47 g, 117 mmol) were dissolved in IPA (50 ml) and $H_2O$ (50 ml) in a 1 L flask, followed by reflux-stirring for 12 hours. The reaction mixture was concentrated under reduced pressure. The organic layer was separated by using ethyl acetate and brine. The separated organic layer was dried over $MgSO_4$ and filtered. The mixture was separated by column chromatography to give tert-butyl 3'-methyl-[1,1'-biphenyl]-2-carboxylate (7.2 g).

Step 3 : tert-butyl 3'-(bromomethyl)-[1,1'-biphenyl]-2-carboxylate

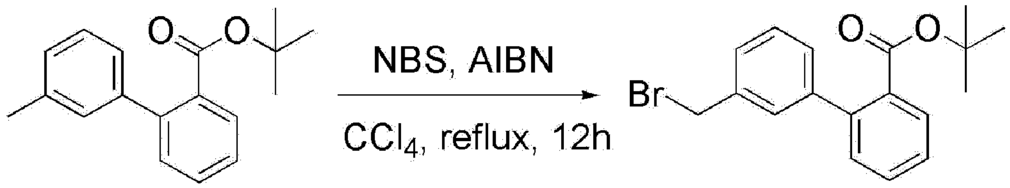


Tert-butyl 3'-methyl-[1,1'-biphenyl]-2-carboxylate (7.2 g, 26.83 mmol), NBS (5.73 g, 32.2 mmol), and AIBN (1.32 g, 8.05 mmol) were dissolved in $CCl_4$ (100 ml) in a 500 mL flask, followed by reflux-stirring for 12 hours. The reaction mixture was concentrated under reduced pressure. The organic layer was separated by using dichloromethane and brine. The separated organic layer was dried over $MgSO_4$ and filtered. The mixture was separated by column chromatography to give tert-butyl 3'-(bromomethyl)-[1,1'-biphenyl]-2-carboxylate (5.2 g).

Step 4 : methyl 1-((2'-(tert-butoxycarbonyl)-[1,1'-biphenyl]-3-yl)methyl)-1,2,3,4-tetrahydroquinoline-6-carboxylate

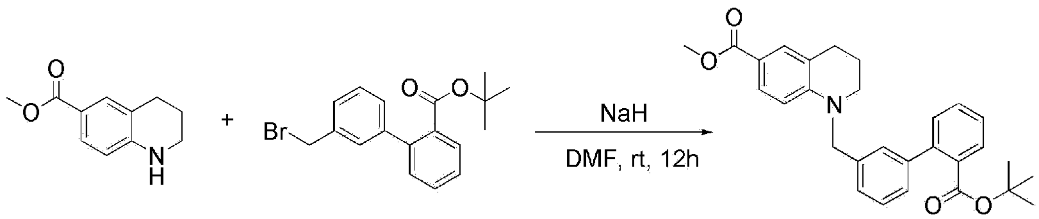


Methyl 1,2,3,4-tetrahydroquinoline-6-carboxylate (3 g, 15.69 mmol) was dissolved in DMF (20 ml) in a 100 mL flask, to which NaH (753 mg, 18.83 mmol) and tert-butyl 3'-(bromomethyl)-[1,1'-biphenyl]-2-carboxylate (5.45 g, 15.69 mmol) were added, followed by stirring at room temperature for 12 hours. The organic layer was separated by using ethyl acetate and brine. The separated organic layer was dried over $MgSO_4$ and filtered. The solvent was eliminated from the mixture via vacuum distillation, followed by column chromatography to give methyl 1-((2'-(tert-butoxycarbonyl)-[1,1'-biphenyl]-3-yl)methyl)-1,2,3,4-tetrahydroquinoline-6-carboxylate (4.76 g).

Step 5 : 1-((2'-(tert-butoxycarbonyl)-[1,1'-biphenyl]-3-yl)methyl)-1,2,3,4-tetrahydroquinoline-6-carboxylic acid

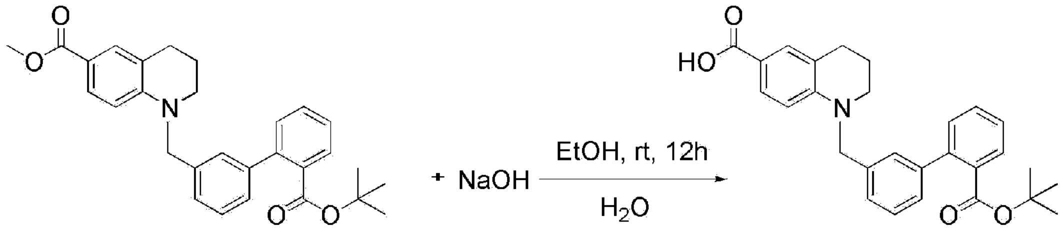


Methyl 1-((2'-(tert-butoxycarbonyl)-[1,1'-biphenyl]-3-yl)methyl)-1,2,3,4-tetrahydroquinoline-6-carboxylate (4.76 g, 10.4 mmol) was mixed with NaOH (2.08 g, 52 mmol) dissolved in EtOH (50 ml) and $H_2O$ (50 ml) in a 250 mL flask, followed by stirring at room temperature for 12 hours. Upon completion of the reaction, the solvent was concentrated. PH of the reaction mixture was adjusted to 5 by using 2*N*-HCl. The mixture was extracted with EA. The organic layer was dried over $MgSO_4$ and filtered. As a result, 1-((2'-(tert-butoxycarbonyl)-[1,1'-biphenyl]-3-yl)methyl)-1,2,3,4-tetrahydroquinoline-6-carboxylic acid was obtained (2.7 g).

**<Preparative Example 5> 1-((2'-(tert-butoxycarbonyl)-2-fluoro-[1,1'-biphenyl]-3-yl)methyl)-1,2,3,4-tetrahydroquinoline-6-carboxylic acid**

Step 1 : tert-butyl 2-bromobenzoate

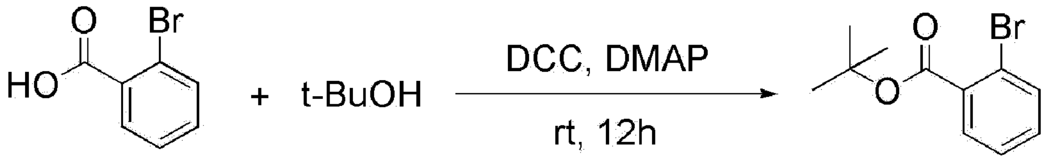


2-bromobenzoic acid (25 g, 124 mmol), DMAP (1.52 g, 12.4 mmol), and t-butanol (9.2 g, 124 mmol) were dissolved in dichloromethane (500 ml) in a 1 L flask, to which DCC (25.7 g, 124 mmol) was added, followed by stirring at room temperature for 12 hours. The organic layer was separated by using ethyl acetate and brine. The separated organic layer was dried over $MgSO_4$ and filtered. The solvent was eliminated from the mixture via vacuum distillation, followed by column chromatography to give tert-butyl 2-bromobenzoate (30 g).

Step 2 : tert-butyl 2'-fluoro-3'-methyl-[1,1'-biphenyl]-2-carboxylate

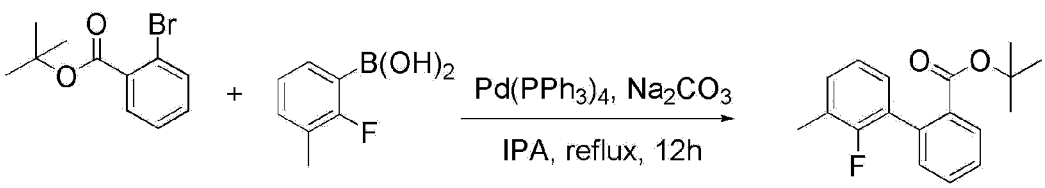


Tert-butyl 2-bromobenzoate (33.38 g, 129.8 mmol), (2-fluoro-3-methylphenyl)boronic acid (10 g, 64.9 mmol), Pd $(PPh_3)_4$ (749 mg, 0.65 mmol), and $Na_2CO_3$ (13.75 g, 129.8 mmol) were dissolved in IPA (60 ml) and $H_2O$ (60 ml) in a 1 L flask, followed by reflux-stirring for 12 hours. The reaction mixture was concentrated under reduced pressure. The organic layer was separated by using ethyl acetate and brine. The separated organic layer was dried over $MgSO_4$ and filtered. The mixture was separated by column chromatography to give tert-butyl 2'-fluoro-3'-methyl-[1,1'-biphenyl]-2-carboxylate (15 g).

Step 3 : tert-butyl 3'-(bromomethyl)-2'-fluoro-[1,1'-biphenyl]-2-carboxylate

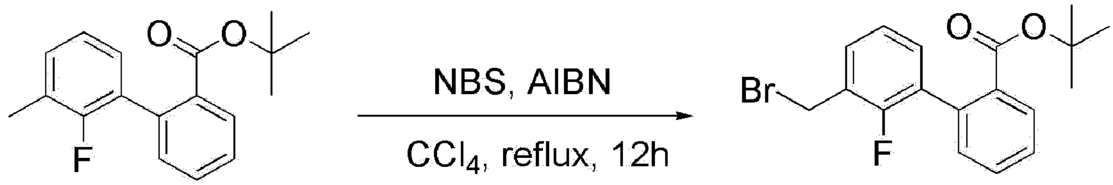


Tert-butyl 2'-fluoro-3'-methyl-[1,1'-biphenyl]-2-carboxylate (18.58 g, 64.89 mmol), NBS (17.3 g, 97.33 mmol), and AIBN (3.2 g, 19.46 mmol) were dissolved in $CCl_4$ (100 ml) in a 500 mL flask, followed by reflux-stirring for 12 hours. The reaction mixture was concentrated under reduced pressure. The organic layer was separated by using dichloromethane and brine. The separated organic layer was dried over $MgSO_4$ and filtered. The mixture was separated by column chromatography to give tert-butyl 3'-(bromomethyl)-2'-fluoro-[1,1'-biphenyll-2-carboxylate (12.5 g).

Step 4 : methyl 1-((2'-(tert-butoxycarbonyl)-2-fluoro-[1,1'-biphenyl]-3-yl)methyl)-1,2,3,4-tetrahydroquinoline-6-carboxylate

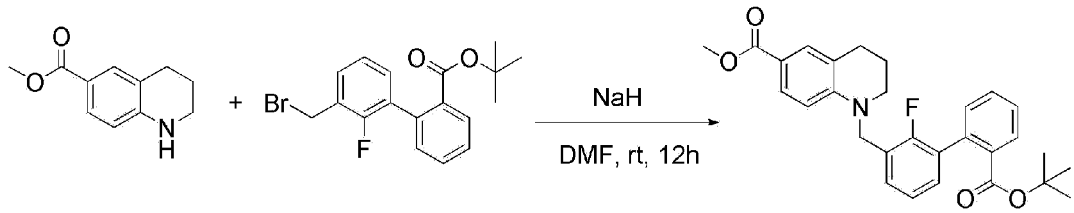


Methyl 1,2,3,4-tetrahydroquinoline-6-carboxylate (4 g, 20.9 mmol) was dissolved in DMF (20 ml) in a 250 mL flask, to which NaH (1 g, 25.08 mmol) and tert-butyl 3'-(bromomethyl)-2'-fluoro-[1,1'-biphenyl]-2-carboxylate (12.5 g, 34.22 mmol) were added, followed by stirring at room temperature for 12 hours. The organic layer was separated by using ethyl acetate and brine. The separated organic layer was dried over $MgSO_4$ and filtered. The solvent was eliminated from the mixture via vacuum distillation, followed by column chromatography to give methyl 1-((2'-(tert-butoxycarbonyl)-2-fluoro-[1,1'-biphenyl]-3-yl)methyl)-1,2,3,4-tetrahydroquinoline-6-carboxylate (6.2 g).

Step 5 : 1-((2'-(tert-butoxycarbonyl)-2-fluoro-[1,1'-biphenyl]-3-yl)methyl)-1,2,3,4-tetrahydroquinoline-6-carboxylic acid

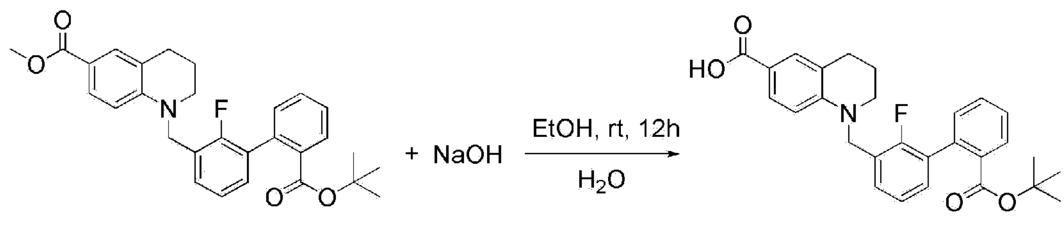


Methyl 1-((2'-(tert-butoxycarbonyl)-2-fluoro-[1,1'-biphenyl]-3-yl)methyl)-1,2,3,4-tetrahydroquinoline-6-carboxylate (6.2 g, 13.04 mmol) was mixed with NaOH (2.6 g, 65.2 mmol) dissolved in EtOH (50 ml) and $H_2O$ (50 ml) in a 250 mL flask, followed by stirring at room temperature for 12 hours. Upon completion of the reaction, the solvent was concentrated. PH of the reaction mixture was adjusted to 5 by using 2*N*-HCl. The mixture was extracted with EA. The organic layer was dried over $MgSO_4$ and filtered. As a result, 1-((2'-(tert-butoxycarbonyl)-2-fluoro-[1,1'-biphenyl]-3-yl)methyl)-1,2,3,4-tetrahydroquinoline-6-carboxylic acid was obtained (4 g).

**<Preparative Example 6> 1-((2'-(tert-butoxycarbonyl)-4-fluoro-[1,1'-biphenyl]-3-yl)methyl)-1,2,3,4-tetrahydroquinoline-6-carboxylic acid**

Step 1 : tert-butyl 2-bromobenzoate

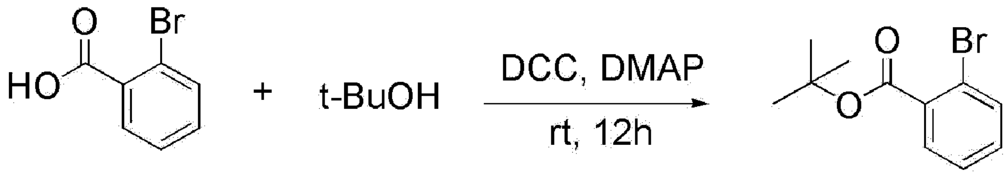


2-bromobenzoic acid (25 g, 124 mmol), DMAP (1.52 g, 12.4 mmol), and t-butanol (9.2 g, 124 mmol) were dissolved in dichloromethane (500 ml) in a 1 L flask, to which DCC (25.7 g, 124 mmol) was added, followed by stirring at room temperature for 12 hours. The organic layer was separated by using ethyl acetate and brine. The separated organic layer was dried over $MgSO_4$ and filtered. The solvent was eliminated from the mixture via vacuum distillation, followed by column chromatography to give tert-butyl 2-bromobenzoate (30 g).

Step 2 : tert-butyl 4'-fluoro-3'-methyl-[1,1'-biphenyl]-2-carboxylate

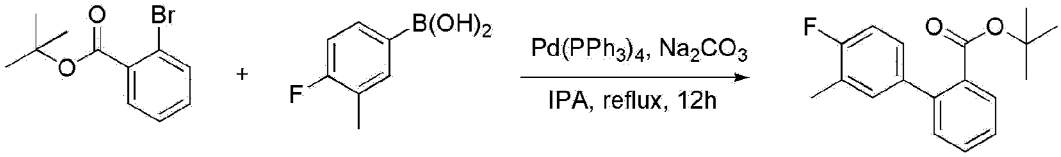


Tert-butyl 2-bromobenzoate (33.38 g, 129.8 mmol), (4-fluoro-3-methylphenyl)boronic acid (10 g, 64.9 mmol), $Pd(PPh_3)$ (749 mg, 0.64 mmol), and $Na_2CO_3$ (13.7 g, 129.8 mmol) were dissolved in IPA (50 ml) and $H_2O$ (50 ml) in a 1 L flask, followed by reflux-stirring for 12 hours. The reaction mixture was concentrated under reduced pressure. The organic layer was separated by using ethyl acetate and brine. The separated organic layer was dried over $MgSO_4$ and filtered. The mixture was separated by column chromatography to give tert-butyl 4'-fluoro-3'-methyl-[1,1'-biphenyl]-2-carboxylate (13 g).

Step 3 : tert-butyl 3'-(bromomethyl)-4'-fluoro-[1,1'-biphenyl]-2-carboxylate

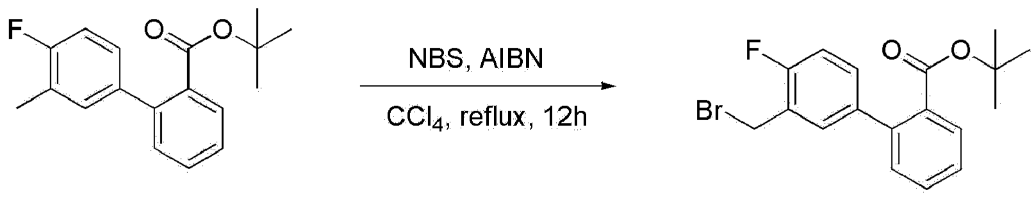


Tert-butyl 4'-fluoro-3'-methyl-[1,1'-biphenyl]-2-carboxylate (13 g, 45.4 mmol), NBS (12.12 g, 68.1 mmol), and AIBN (2.24 g, 13.62 mmol) were dissolved in $CCl_4$ (100 ml) in a 500 mL flask, followed by reflux-stirring for 12 hours. The reaction mixture was concentrated under reduced pressure. The organic layer was separated by using ethyl acetate and brine. The separated organic layer was dried over $MgSO_4$ and filtered. The mixture was separated by column chromatography to give tert-butyl 3'-(bromomethyl)-4'-fluoro-[1,1'-biphenyl]-2-carboxylate (11.25 g).

Step 4 : methyl 1-((2'-(tert-butoxycarbonyl)-4-fluoro-[1,1'-biphenyl]-3-yl)methyl)-1,2,3,4-tetrahydroquinoline-6-carboxylate

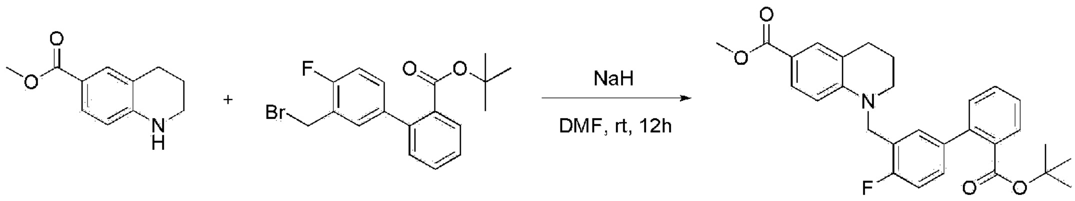


Methyl 1,2,3,4-tetrahydroquinoline-6-carboxylate (4 g, 20.9 mmol) was dissolved in DMF (20 ml) in a 250 mL flask, to which NaH (1 g, 25.08 mmol) and tert-butyl 3'-(bromomethyl)-4'-fluoro-[1,1'-biphenyl]-2-carboxylate (11.25 g, 30.8 mmol) were added, followed by stirring at room temperature for 12 hours. The organic layer was separated by using ethyl acetate and brine. The separated organic layer was dried over $MgSO_4$ and filtered. The solvent was eliminated from the mixture via vacuum distillation, followed by column chromatography to give methyl 1-((2'-(tert-butoxycarbonyl)-4-fluoro-[1,1'-biphenyl]-3-yl)methyl)-1,2,3,4-tetrahydroquinoline-6-carboxylate (6.45 g).

Step 5 : 1-((2'-(tert-butoxycarbonyl)-4-fluoro-[1,1'-biphenyl]-3-yl)methyl)-1,2,3,4-tetrahydroquinoline-6-carboxylic acid

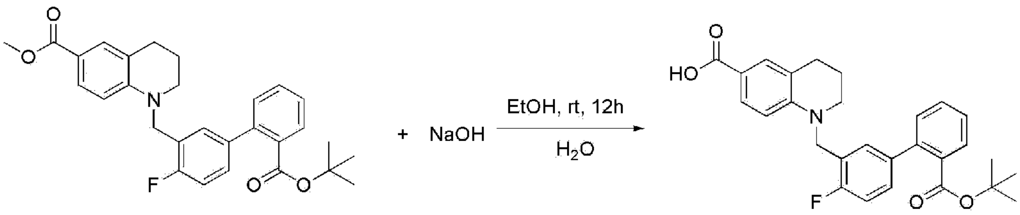


Methyl 1-((2'-(tert-butoxycarbonyl)-4-fluoro-[1,1'-biphenyl]-3-yl)methyl)-1,2,3,4-tetrahydroquinoline-6-carboxylate (6.45 g, 13.56 mmol) was mixed with NaOH (2.71 g, 67.8 mmol) dissolved in EtOH (50 ml) and $H_2O$ (50 ml) in a 250 mL flask, followed by stirring at room temperature for 12 hours. Upon completion of the reaction, the solvent was concentrated. PH of the reaction mixture was adjusted to 5 by using 2*N*-HCl. The mixture was extracted with EA. The organic layer was dried over $MgSO_4$ and filtered. As a result, 1-((2'-(tert-butoxycarbonyl)-4-fluoro-[1,1'-biphenyl]-3-yl)methyl)-1,2,3,4-tetrahydroquinoline-6-carboxylic acid was obtained (5.2 g).

**<Preparative Example 7> 3,4-dihydro-2H-benzo[b][1,4]oxazine-7-carboxylic acid**

Step 1 : methyl 2'-methyl-[1,1'-biphenyl]-4-carboxylate

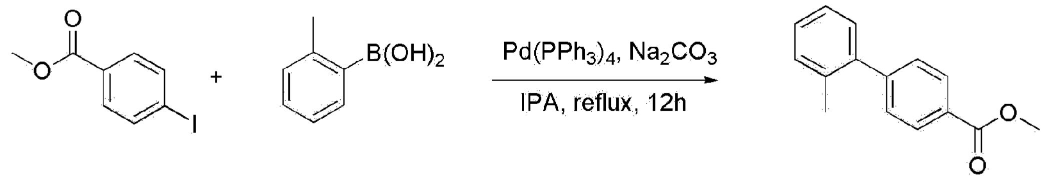


Methyl 4-iodobenzoate (5 g, 19 mmol), ortho-toluylboronic acid (3.1 g, 22.9 mmol), $Pd(PPh_3)_4$ (1.1g, 0.95 mmol), and $Na_2CO_3$ (8.5 g, 38 mmol) were dissolved in IPA (50 ml) and $H_2O$ (50 ml) in a 100 mL flask, followed by reflux-stirring for 12 hours. The reaction mixture was concentrated under reduced pressure. The organic layer was separated by using ethyl acetate and brine. The separated organic layer was dried over $MgSO_4$ and filtered. The mixture was separated by column chromatography to give methyl 2'-methyl-[1,1'-biphenyl]-4-carboxylate (4.3 g).

Step 2 : methyl 2'-(bromomethyl)-[1,1'-biphenyl]-4-carboxylate

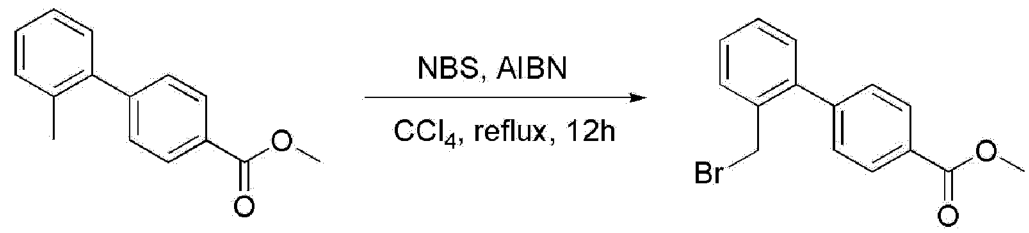


Methyl 2'-methyl-[1,1'-biphenyl]-4-carboxylate (1.1 g, 4.86 mmol), NBS (951 mg, 5.34 mmol), and AIBN (239 mg, 1.45 mmol) were dissolved in $CCl_4$ (10 ml) in a 50 mL flask, followed by reflux-stirring for 12 hours. The reaction mixture was concentrated under reduced pressure. The organic layer was separated by using dichloromethane and brine. The separated organic layer was dried over $MgSO_4$ and filtered. The mixture was separated by column chromatography to give methyl 2'-(bromomethyl)-[1,1'-biphenyl]-4-carboxylate (1 g).

Step 3 : 3,4-dihydro-2H-benzo[b][1,4]oxazine-7-carboxylic acid

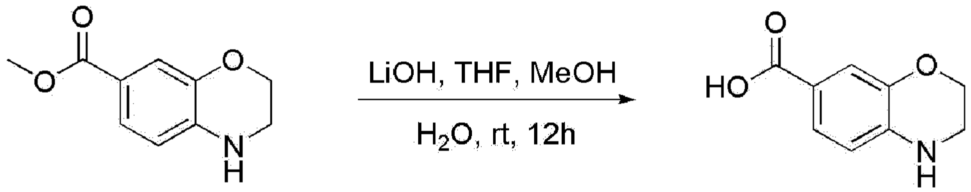


Methyl 3,4-dihydro-2H-benzo[b][1,4]oxazine-7-carboxylate (1 g, 5.2 mmol) was dissolved in MeOH (5 ml) and THF (10 ml) in a 25 mL flask, to which LiOH (2 g) dissolved in $H_2O$ (5 ml) was added, followed by stirring at room temperature for 12 hours. Upon completion of the reaction, the solvent was concentrated. PH of the reaction mixture was adjusted to 5 by using 2*N*-HCl. The mixture was extracted with EA. The organic layer was dried over $MgSO_4$ and filtered. As a result, 3,4-dihydro-2H-benzo[b][1,4]oxazine-7-carboxylic acid was obtained (1.2 g).

**<Preparative Example 8> methyl 2'-(bromomethyl)-[1,1'-biphenyl]-4-carboxylate**

Step 1 : methyl 2'-methyl-[1,1'-biphenyl]-4-carboxylate

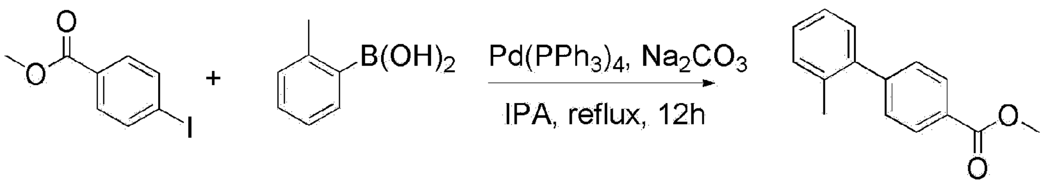


Methyl 4-iodobenzoate (5 g, 19 mmol), ortho-toluylboronic acid (3.1 g, 22.9 mmol), $Pd(PPh_3)_4$ (1.1g, 0.95 mmol), and $Na_2CO_3$ (8.5 g, 38 mmol) were dissolved in IPA (50 ml) and $H_2O$ (50 ml) in a 100 mL flask, followed by reflux-stirring for 12 hours. The reaction mixture was concentrated under reduced pressure. The organic layer was separated by using ethyl acetate and brine. The separated organic layer was dried over $MgSO_4$ and filtered. The mixture was separated by column chromatography to give methyl 2'-methyl-[1,1'-biphenyl]-4-carboxylate (4.3 g).

Step 2 : methyl 2'-(bromomethyl)-[1,1'-biphenyl]-4-carboxylate

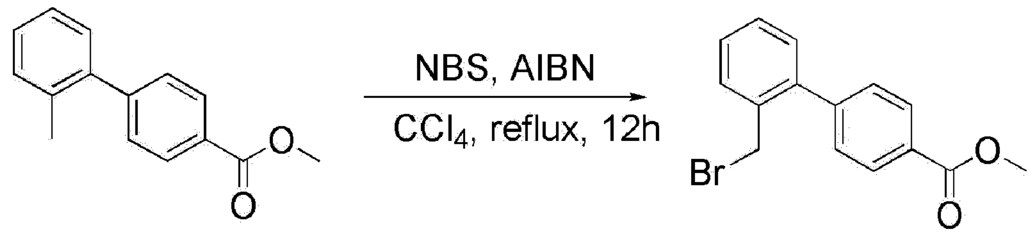


Methyl 2'-methyl-[1,1'-biphenyl]-4-carboxylate (1.1 g, 4.86 mmol), NBS (951 mg, 5.34 mmol), and AIBN (239 mg, 1.45 mmol) were dissolved in $CCl_4$ (10 ml) in a 50 mL flask, followed by reflux-stirring for 12 hours. The reaction mixture was concentrated under reduced pressure. The organic layer was separated by using dichloromethane and brine. The separated organic layer was dried over $MgSO_4$ and filtered. The mixture was separated by column chromatography to give methyl 2'-(bromomethyl)-[1,1'-biphenyl]-4-carboxylate (1 g).

**<Preparative Example 9> 4-((2'-(tert-butoxycarbonyl)-2-fluoro-[1,1'-biphenyl]-4-yl)methyl)-3,4-dihydro-2H-benzo[b][1,4]oxazine-7-carboxylic acid**

Step 1 tert-butyl 2-bromobenzoate

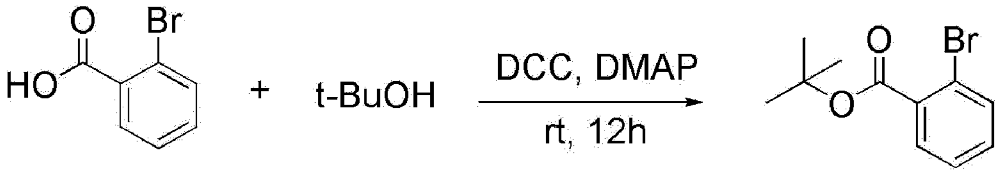


2-bromobenzoic acid (25 g, 124 mmol), DMAP (1.52 g, 12.4 mmol), and t-butanol (9.2 g, 124 mmol) were dissolved in dichloromethane (500 ml) in a 1 L flask, to which DCC (25.7 g, 124 mmol) was added, followed by stirring at room temperature for 12 hours. The organic layer was separated by using ethyl acetate and brine. The separated organic layer was dried over $MgSO_4$ and filtered. The solvent was eliminated from the mixture via vacuum distillation, followed by column chromatography to give tert-butyl 2-bromobenzoate (30 g).

Step 2 : tert-butyl 2'-fluoro-4'-methyl-[1,1'-biphenyl]-2-carboxylate

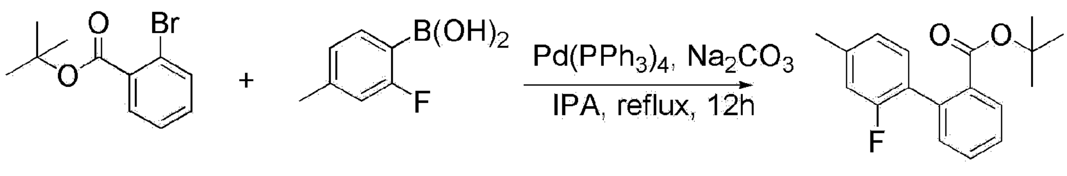


Tert-butyl 2-bromobenzoate (7 g, 27 mmol), (2-fluoro-4-methylphenyl)boronic acid (5 g, 32.5 mmol), $Pd(PPh_3)_4$ (312 mg, 0.27 mmol), and $Na_2CO_3$ (5.7 g, 54 mmol) were dissolved in IPA (30 ml) and $H_2O$ (30 ml) in a 1 L flask, followed by reflux-stirring for 12 hours. The reaction mixture was concentrated under reduced pressure. The organic layer was separated by using ethyl acetate and brine. The separated organic layer was dried over $MgSO_4$ and filtered. The mixture was separated by column chromatography to give tert-butyl 2'-fluoro-4'-methyl-[1,1'-biphenyl]-2-carboxylate (5.2 g).

Step 3 : tert-butyl 4'-(bromomethyl)-2'-fluoro-[1,1'-biphenyl]-2-carboxylate

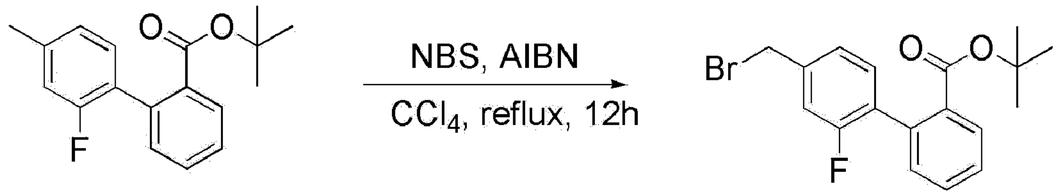


Tert-butyl 2'-fluoro-4'-methyl-[1,1'-biphenyl]-2-carboxylate (3 g, 10.5 mmol), NBS (1.9 g, 10.5 mmol), and AIBN (43 mg, 0.025 mmol) were dissolved in $CCl_4$ (10 ml) in a 1 L flask, followed by reflux-stirring for 12 hours. The reaction mixture was concentrated under reduced pressure. The organic layer was separated by using ethyl acetate and brine. The separated organic layer was dried over $MgSO_4$ and filtered. The mixture was separated by column chromatography to give tert-butyl 4'-(bromomethyl)-2'-fluoro-[1,1'-biphenyl]-2-carboxylate (2.75 g).

Step 4 : methyl 4-((2'-(tert-butoxycarbonyl)-2-fluoro-[1,1'-biphenyl]-4-yl)methyl)-3,4-dihydro-2H-benzo[b][1,4]oxazine-7-carboxylate

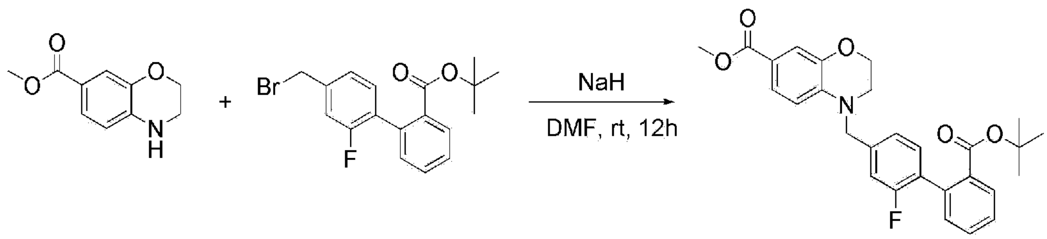


Methyl 3,4-dihydro-2H-benzo[b][1,4]oxazine-7-carboxylate (10 g, 51.7 mmol) was dissolved in DMF (50 ml) in a 250 mL flask, to which NaH (6.2 g, 155.3 mmol) and tert-butyl 4'-(bromomethyl)-2'-fluoro-[1,1'-biphenyl]-2-carboxylate (2.5 g, 67.3 mmol) were added, followed by stirring at room temperature for 12 hours. The organic layer was separated by using ethyl acetate and brine. The separated organic layer was dried over $MgSO_4$ and filtered. The solvent was eliminated from the mixture via vacuum distillation, followed by column chromatography to give methyl 4-((2'-(tert-butoxycarbonyl)-2-fluoro-[1,1'-biphenyl]-4-yl)methyl)-3,4-dihydro-2H-benzo[b][1,4]oxazine-7-carboxylate (15.4 g).

Step 5 : 4-((2'-(tert-butoxycarbonyl)-2-fluoro-[1,1'-biphenyl]-4-yl)methyl)-3,4-dihydro-2H-benzo[b][1,4]oxazine-7-carboxylic acid

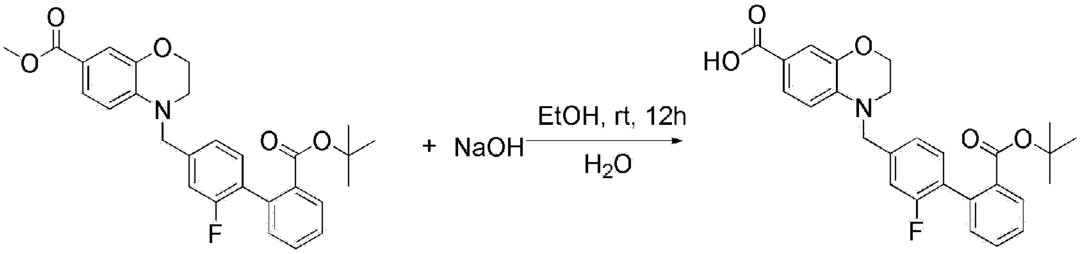


Methyl 4-((2'-(tert-butoxycarbonyl)-2-fluoro-[1,1'-biphenyl]-4-yl)methyl)-3,4-dihydro-2H-benzo[b][1,4]oxazine-7-carboxylate (15 g) was mixed with NaOH (1.48 g, 37 mmol) dissolved in EtOH (50 ml) and $H_2O$ (50 ml) in a 250 mL flask, followed by stirring at room temperature for 12 hours. Upon completion of the reaction, the solvent was concentrated. PH of the reaction mixture was adjusted to 5 by using 2*N*-HCl. The mixture was extracted with EA. The organic layer

was dried over $MgSO_4$ and filtered. As a result, 4-((2'-(tert-butoxycarbonyl)-2-fluoro-[1,1'-biphenyl]-4-yl)methyl)-3,4-dihydro-2H-benzo[b][1,4]oxazine-7-carboxylic acid was obtained (13 g).

**<Example 1> (S)-4'-((6-((1-phenylethyl)carbamoyl)-3,4-dihydroquinoline-1(2H)-yl)methyl)-[1,1'-biphenyl]-2-carboxylic acid**

Step 1 : (S)-tert-butyl 4'-((6-((1-phenylethyl)carbamoyl)-3,4-dihydroquinoline-1(2H)-yl)methyl)-[1,1'-biphenyl]-2-carboxylate

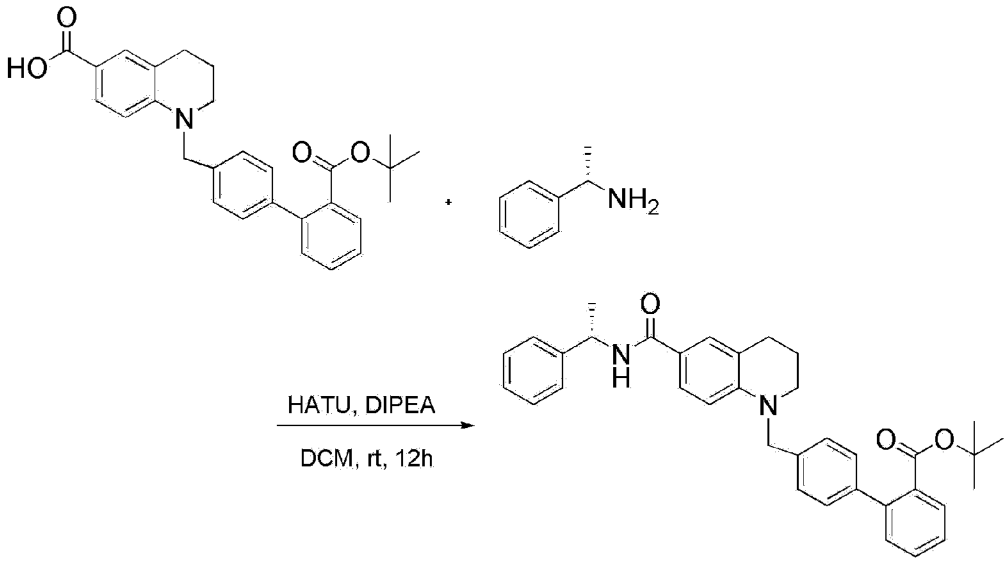


1-((2'-(tert-butoxycarbonyl)-[1,1'-biphenyl]-4-yl)methyl)-1,2,3,4-tetrahydroquinoline-6-carboxylic acid (300 mg, 0.67 mmol) obtained in Preparative Example 1 was dissolved in $CH_2Cl_2$ (10 ml) in a 100 mL flask, to which HATU (280 mg, 0.74 mmol) and DIPEA (350 ul, 2.01 mmol) were added, followed by stirring. (S)-1-phenylethylamine (90 mg, 0.74 mmol) was added thereto, followed by stirring at room temperature for 12 hours. Upon completion of the reaction, the organic layer was separated by using $CH_2Cl_2$ and $H_2O$. The separated organic layer was dried over $MgSO_4$ and filtered. The mixture was separated by column chromatography (EA/n-Hex = 1:1) to give (S)-tert-butyl 4'-((6-((1-phenylethyl)carbamoyl)-3,4-dihydroquinoline-1(2H)-yl)methyl)-[1,1'-biphenyl]-2-carboxylate (311 mg, 0.56 mmol, 85 %).
$^1H$ NMR (400 MHz, $CDCl_3$) $\delta$ 7.79 (dd, $J$ = 1.2, 8.0 Hz, 1H), 7.52-7.23 (m, 14H), 6.51 (d, $J$ = 8.8 Hz, 1H), 6.14 (d, $J$ = 8.0 Hz, 1H), 5.34 (m, 1H), 4.60 (s, 2H), 3.48 (t, $J$ = 5.6 Hz, 2H), 2.86 (t, $J$ = 6.0 Hz, 2H), 2.05 (m, 2H), 1.59 (d, $J$ = 6.8 Hz, 3H), 1.26 (s, 9H).

Step 2 : (S)-4'-((6-((1-phenylethyl)carbamoyl)-3,4-dihydroquinoline-1(2H)-yl)methyl)-[1,1'-biphenyl]-2-carboxylic acid

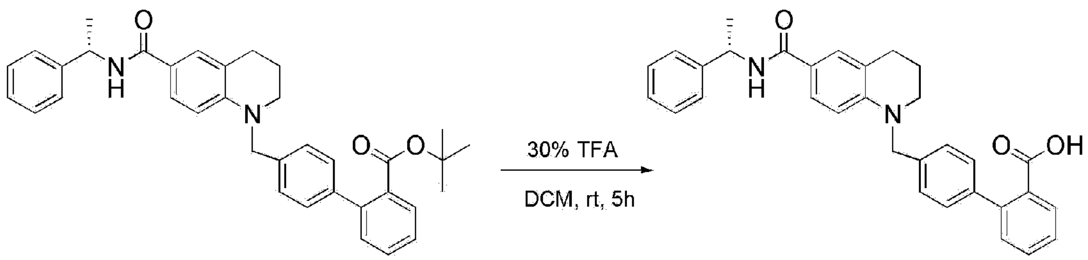


(S)-tert-butyl 4'-((6-((1-phenylethyl)carbamoyl)-3,4-dihydroquinoline-1(2H)-yl)methyl)-[1,1'-biphenyl]-2-carboxylate (311 mg, 0.57 mmol) obtained in step 1 was dissolved in $CH_2Cl_2$ (20 ml) in a 50 mL flask, to which 30% TFA (9 ml) was added, followed by stirring at room temperature for 5 hours. Upon completion of the reaction, the reaction

mixture was concentrated under reduced pressure. The mixture was separated by column chromatography (EA) to give (S)-4'-((6-((1-phenylethyl)carbamoyl)-3,4-dihydroquinoline-1(2H)-yl)methyl)-[1,1'-biphenyl]-2-carboxylic acid (176 mg, 0.53 mmol, 63 %).
$^1$H NMR (400 MHz, DMSO-$d_6$) δ 12.73 (br, OH, 1H), 8.32 (d, *J* = 8.0 Hz, 1H), 7.69 (d, *J* = 8.0 Hz, 1H), 7.60-7.48 (m, 3H), 7.43 (t, *J* = 7.6 Hz, 1H), 7.40-7.25 (m, 9H), 7.19 (t, *J* = 7.2 Hz, 1H), 6.52 (d, *J* = 8.4 Hz, 1H), 5.12 (m, 1H), 4.69 (s, 2H), 3.49 (t, *J* = 4.0 Hz, 2H), 2.81 (t, *J* = 4.0 Hz, 2H), 1.95 (m, 2H), 1.42 (d, *J* = 7.2 Hz, 3H).

**<Example 2> (S)-1-((2'-carbamoyl-[1,1'-biphenyl]-4-yl)methyl)-N-(1-phenylethyl)-1,2,3,4-tetrahydroquinoline-6-carboxamide**

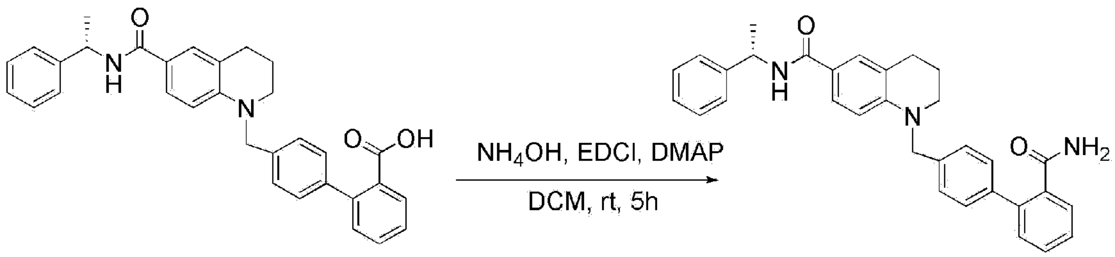


(S)-4'-((6-((1-phenylethyl)carbamoyl)-3,4-dihydroquinoline-1(2H)-yl)methyl)-[1,1'-biphenyl]-2-carboxylic acid (50 mg, 0.1 mmol) obtained in Example 1 was dissolved in $CH_2Cl_2$ (2 ml) in a 25 mL flask, to which EDCI (38 mg, 0.2 mmol) and DMAP (38 mg, 0.3 mmol) were added, followed by stirring. Ammonium hydroxide solution (2 ml) was added thereto, followed by stirring at room temperature for 5 hours. Upon completion of the reaction, the organic layer was separated by using $CH_2Cl_2$ and $H_2O$. The separated organic layer was dried over $MgSO_4$ and filtered. The mixture was separated by column chromatography (MeOH/n-Hex/$CH_2Cl_2$ = 0.5:0.5:9) to give (S)-1-((2'-carbamoyl-[1,1'-biphenyl]-4-yl)methyl)-N-(1-phenylethyl)-1,2,3,4-tetrahydroquinoline-6-carboxamide (21 mg, 0.04 mmol, 43 %).
$^1$H NMR (400 MHz, DMSO-$d_6$) δ 8.31-8.29 (m, 1H), 7.66 (m, 1H), 7.52-7.18 (m, 16H), 6.53-6.50 (m, 1H), 5.14-5.10 (m, 1H), 4.61 (s, 2H), 3.48-3.45 (m, 2H), 2.81-2.78 (m, 2H), 1.99-1.95 (m, 2H), 1.44-1.42 (m, 3H).

**<Example 3> (S)-4'-((6-((1-(4-nitrophenyl)ethyl)carbamoyl)-3,4-dihydroquinoline-1(2H)-yl)methyl)-[1,1'-biphenyl]-2-carboxylic acid**

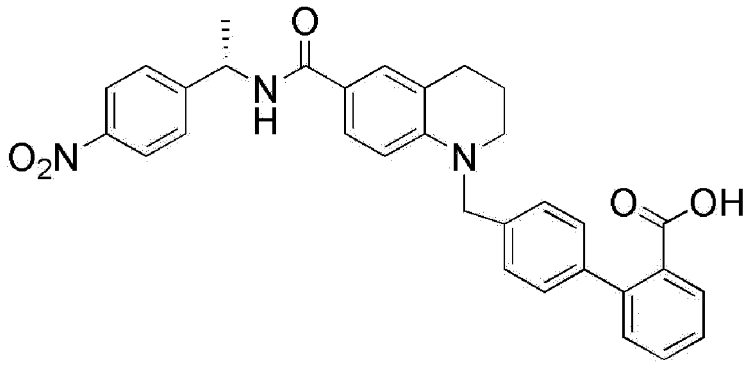

The target compound was obtained by the same manner as described in Example 1 except that (S)-1-(4-nitrophenyl)ethaneamine was used instead of (S)-1-phenylethylamine.
$^1$H NMR (400 MHz, DMSO-$d_6$) δ 12.76 (br, OH, 1H), 8.49 (d, *J* = 7.6 Hz, 1H), 8.19 (d, *J* = 8.8 Hz, 2H), 7.71 (m, 1H), 7.62 (d, *J* = 8.8 Hz, 2H), 7.57-7.25 (m, 9H), 6.52 (d, *J* = 8.0 Hz, 1H), 5.19 (m, 1H), 4.64 (s, 2H), 3.49 (t, *J* = 4.0 Hz, 2H), 2.71 (t, *J* = 4.0 Hz, 2H), 1.95 (m, 2H), 1.47 (d, *J* = 7.2 Hz, 3H).

**<Example 4> (S)-4'-((6-((1-(4-fluoro-2-methylphenyl)ethyl)carbamoyl)-3,4-dihydroquinoline-1(2H)-yl)methyl)-[1,1'-biphenyl]-2-carboxylic acid**

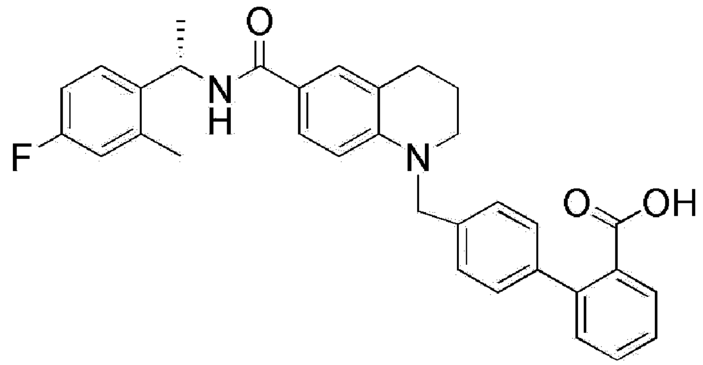

The target compound was obtained by the same manner as described in Example 1 except that (S)-1-(4-fluoro-2-methylphenyl)ethaneamine was used instead of (S)-1-phenylethylamine.
$^1$H NMR (400 MHz, DMSO-$d_6$) δ 12.73 (br, OH, 1H), 8.22 (d, *J* = 8.0 Hz, 1H), 7.79 (d, *J* = 8.0 Hz, 1H), 7.60-7.25 (m, 12H), 6.52 (d, *J* = 8.4 Hz, 1H), 5.12 (m, 1H), 4.69 (s, 2H), 3.49 (t, *J* = 4.0 Hz, 2H), 2.81 (t, *J* = 4.0 Hz, 2H), 2.28 (s, 3H), 1.95 (m, 2H), 1.42 (d, *J* = 7.2 Hz, 3H).

**<Example 5> (S)-4'-((6-((1-(3-fluoro-4-methylphenyl)ethyl)carbamoyl)-3,4-dihydroquinoline-1(2H)-yl)methyl)-[1,1'-biphenyl]-2-carboxylic acid**

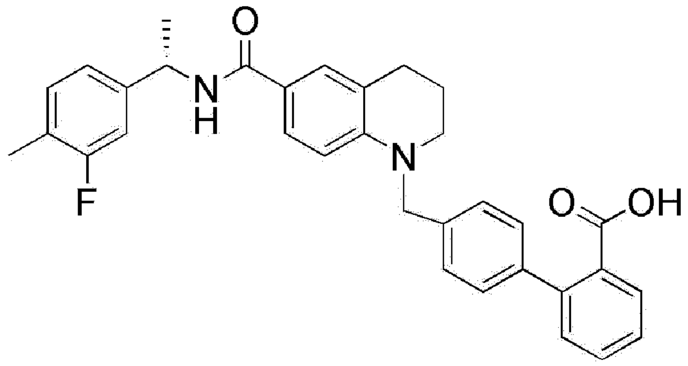

The target compound was obtained by the same manner as described in Example 1 except that (S)-1-(3-fluoro-4-methylphenyl)ethaneamine was used instead of (S)-1-phenylethylamine.
$^1$H NMR (400 MHz, DMSO-$d_6$) δ 12.63 (br, OH, 1H), 8.29 (d, *J* = 8.0 Hz, 1H), 7.70 (dd, *J* = 1.2, 7.6 Hz, 1H), 7.57-7.48 (m, 3H), 7.43 (td, *J* = 1.2, 7.6 Hz, 1H), 7.36 (d, *J* = 7.6 Hz, 1H), 7.30 (m, 3H), 7.19 (t, *J* = 7.2 Hz, 1H), 7.11-7.05 (m, 2H), 6.53 (d, *J* = 8.4 Hz, 1H), 5.09 (m, 1H), 4.62 (s, 2H), 3.47 (t, *J* = 5.2 Hz, 2H), 2.80 (t, *J* = 6.0 Hz, 2H), 2.18 (s, 3H), 1.95 (m, 2H), 1.41 (d, *J* = 7.2 Hz, 3H).

**<Example 6> (S)-4'-((6-((1-(4-fluorophenyl)ethyl)carbamoyl)-3,4-dihydroquinoline-1(2H)-yl)methyl)-[1,1'-biphenyl]-2-carboxylic acid**

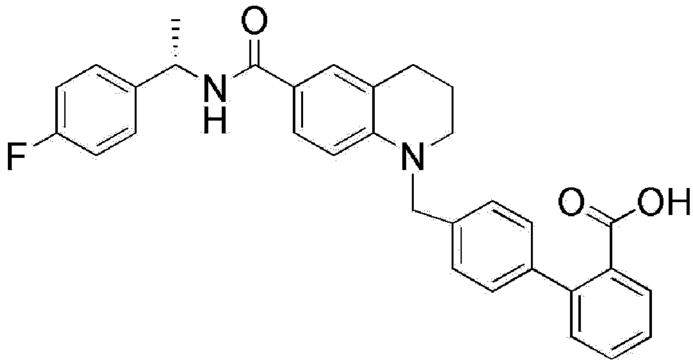

The target compound was obtained by the same manner as described in Example 1 except that (S)-1-(4-fluorophenyl)ethaneamine was used instead of (S)-1-phenylethylamine.
$^1$H NMR (400 MHz, DMSO-$d_6$) $\delta$ 12.63 (br, OH, 1H), 8.34 (d, *J* = 8.0 Hz, 1H), 7.71 (dd, *J* = 1.2, 7.6 Hz, 1H), 7.57-7.35 (m, 7H), 7.30 (m, 4H), 7.13 (m, 2H), 6.52 (d, *J* = 8.8 Hz, 1H), 5.12 (m, 1H), 4.62 (s, 2H), 3.47 (t, *J* = 5.2 Hz, 2H), 2.80 (t, *J* = 6.0 Hz, 2H), 1.95 (m, 2H), 1.41 (d, *J* = 7.2 Hz, 3H).

**<Example 7> (S)-4'-((6-((1-(4-bromophenyl)ethyl)carbamoyl)-3,4-dihydroquinoline-1(2H)-yl)methyl)-[1,1'-biphenyl]-2-carboxylic acid**

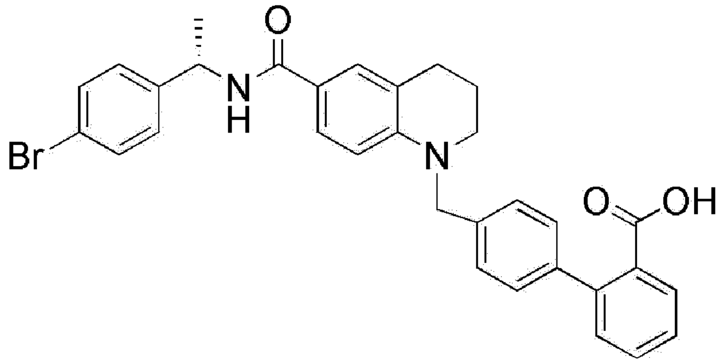

The target compound was obtained by the same manner as described in Example 1 except that (S)-1-(4-bromophenyl)ethaneamine was used instead of (S)-1-phenylethylamine.
$^1$H NMR (400 MHz, DMSO-$d_6$) $\delta$ 12.71 (br, OH, 1H), 8.34 (d, *J* = 8.0 Hz, 1H), 7.71 (m, 1H), 7.57-7.35 (m, 7H), 7.30 (m, 6H), 6.52 (d, *J* = 8.8 Hz, 1H), 5.12 (m, 1H), 4.62 (s, 2H), 3.47 (t, *J* = 5.2 Hz, 2H), 2.80 (t, *J* = 6.0 Hz, 2H), 1.95 (m, 2H), 1.41 (d, *J* = 7.2 Hz, 3H).

**<Example 8> 4'-((6-((4-nitrobenzyl)carbamoyl)-3,4-dihydroquinoline-1(2H)-yl)methyl)-[1,1'-biphenyl]-2-carboxylic acid**

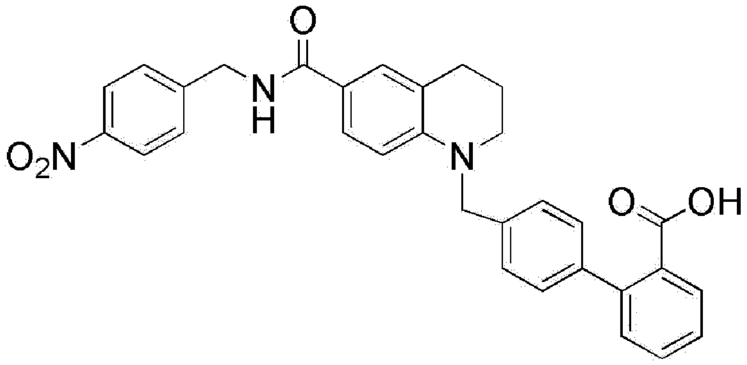

The target compound was obtained by the same manner as described in Example 1 except that (4-nitrophenyl)methaneamine was used instead of (S)-1-phenylethylamine.
$^1$H NMR (400 MHz, DMSO-$d_6$) δ 12.63 (br, OH, 1H), 8.75 (t, *J* = 6.0 Hz, 1H), 8.19 (d, *J* = 7.6 Hz, 2H), 7.70 (dd, *J* = 1.2, 8.0 Hz, 1H), 7.57-7.50 (m, 5H), 7.44 (td, *J* = 1.2, 7.6 Hz, 1H), 7.37 (d, *J* = 7.6 Hz, 1H), 7.30 (m, 4H), 6.54 (d, *J* = 7.2 Hz, 1H), 4.63 (s, 2H), 4.54 (d, *J* = 6.0 Hz, 2H), 3.48 (t, *J* = 5.6 Hz, 2H), 2.80 (t, *J* = 6.0 Hz, 2H), 1.98 (m, 2H).

**<Example 9> (S)-4'-((6-((1-(3-chlorophenyl)ethyl)carbamoyl)-3,4-dihydroquinoline-1(2H)-yl)methyl)-[1,1'-biphenyl]-2-carboxylic acid**

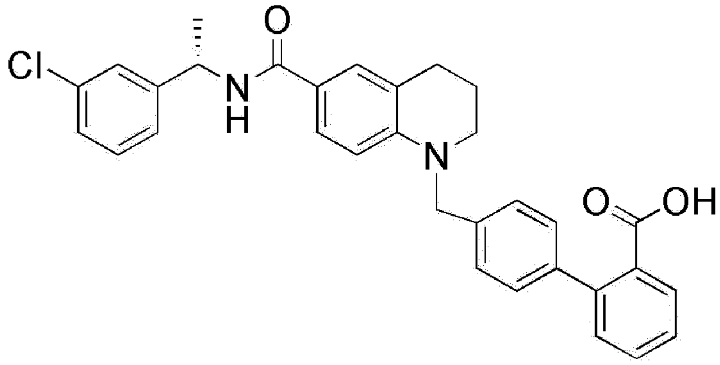

The target compound was obtained by the same manner as described in Example 1 except that (S)-1-(3-chlorophenyl)ethaneamine was used instead of (S)-1-phenylethylamine.
$^1$H NMR (400 MHz, DMSO-$d_6$) δ 12.73 (br, OH, 1H), 8.36 (d, *J* = 7.6 Hz, 1H), 7.70 (d, *J* = 6.8 Hz, 1H), 7.60-7.22 (m, 13H), 6.53 (d, *J* = 7.2 Hz, 1H), 5.11 (m, 1H), 4.62 (s, 2H), 3.47 (t, *J* = 5.2 Hz, 2H), 2.80 (t, *J* = 5.6 Hz, 2H), 1.95 (m, 2H), 1.43 (d, *J* = 7.2 Hz, 3H).

**<Example 10> (S)-4'-((6-((1-(naphthalene-1-yl)ethyl)carbamoyl)-3,4-dihydroquinoline-1(2H)-yl)methyl)-[1,1'-biphenyl]-2-carboxylic acid**

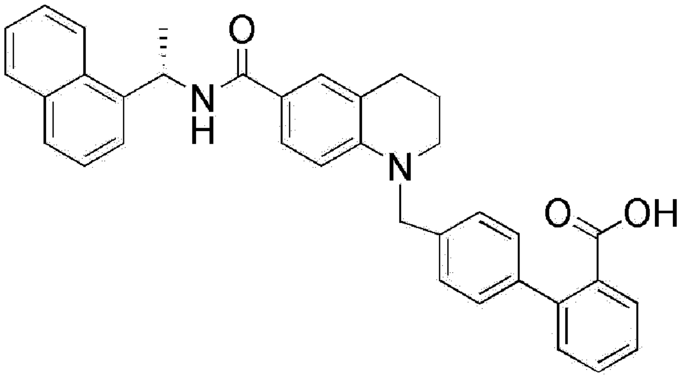

The target compound was obtained by the same manner as described in Example 1 except that (S)-1-(naphthalene-1-yl)ethaneamine was used instead of (S)-1-phenylethylamine.
$^1$H NMR (400 MHz, DMSO-$d_6$) δ 12.73 (br, OH, 1H), 8.46 (d, *J* = 7.6 Hz, 1H), 8.19 (d, *J* = 7.6 Hz, 1H), 7.81 (d, *J* = 8.0 Hz, 1H), 7.70 (d, *J* = 6.8 Hz, 1H), 7.60-7.35 (m, 10H), 7.28 (m, 4H), 6.52 (d, *J* = 8.8 Hz, 1H), 5.93 (m, 1H), 4.61 (s, 2H), 3.46 (t, *J* = 5.2 Hz, 2H), 2.78 (t, *J* = 5.6 Hz, 2H), 1.95 (m, 2H), 1.57 (d, *J* = 6.8 Hz, 3H).

**<Example 11> (S)-4'-((6-((1-(4-(trifluoromethoxy)phenyl)ethyl)carbamoyl)-3,4-dihydroquinoline-1(2H)-yl)methyl)-[1,1'-biphenyl]-2-carboxylic acid**

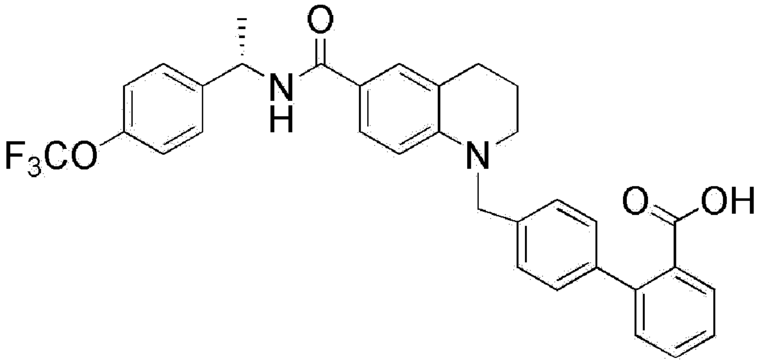

The target compound was obtained by the same manner as described in Example 1 except that (S)-1-(4-(trifluoromethoxy)phenyl)ethaneamine was used instead of (S)-1-phenylethylamine.
$^1$H NMR (400 MHz, DMSO-$d_6$) δ 12.73 (br, OH, 1H), 8.37 (d, *J* = 7.6 Hz, 1H), 7.69 (d, *J* = 8.0 Hz, 1H), 7.60-7.40 (m, 7H), 7.40-7.25 (m, 6H), 6.52 (d, *J* = 8.8 Hz, 1H), 5.14 (m, 1H), 4.62 (s, 2H), 3.58 (t, *J* = 5.2 Hz, 2H), 2.79 (t, *J* = 6.0 Hz, 2H), 1.95 (m, 2H), 1.44 (d, *J* = 6.8 Hz, 3H).

**<Example 12> (S)-4'-((6-((1-(4-(trifluoromethyl)phenyl)ethyl)carbamoyl)-3,4-dihydroquinoline-1(2H)-yl)methyl)-[1,1'-biphenyl]-2-carboxylic acid**

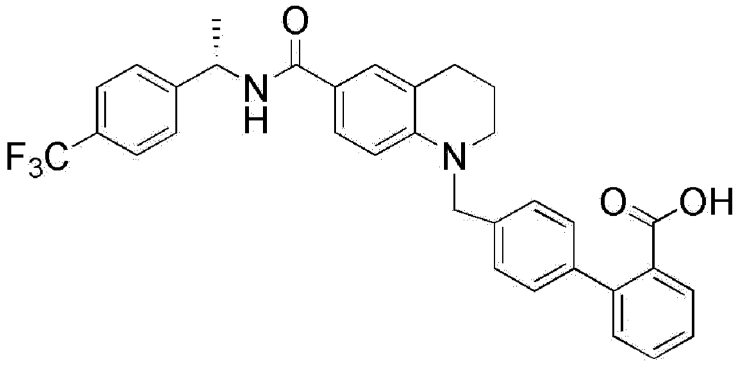

The target compound was obtained by the same manner as described in Example 1 except that (S)-1-(4-(trifluoromethyl)phenyl)ethaneamine was used instead of (S)-1-phenylethylamine.
$^1$H NMR (400 MHz, DMSO-$d_6$) δ 12.66 (br, OH, 1H), 8.43 (d, *J* = 8.0 Hz, 1H), 7.75-7.65 (m, 3H), 7.60-7.50 (m, 5H), 7.44 (t, *J* = 7.6 Hz, 1H), 7.37 (d, *J* = 7.2 Hz, 1H), 7.30-7.20 (m, 4H), 6.53 (d, *J* = 8.8 Hz, 1H), 5.16 (m, 1H), 4.62 (s, 2H), 3.47 (t, *J* = 5.2 Hz, 2H), 2.81 (t, *J* = 6.0 Hz, 2H), 1.95 (m, 2H), 1.46 (d, *J* = 6.8 Hz, 3H).

**<Example 13> (S)-4'-((6-((1-(4-((trifluoromethyl)thio)phenyl)ethyl)carbamoyl)-3,4-dihydroquinoline-1(2H)-yl)methyl)-[1,1'-biphenyl]-2-carboxylic acid**

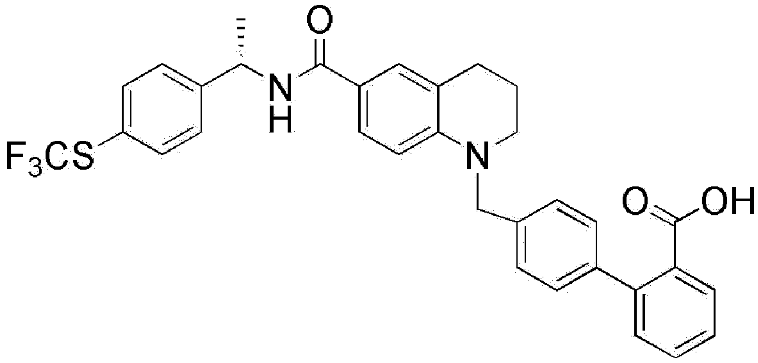

The target compound was obtained by the same manner as described in Example 1 except that (S)-1-(4-((trifluoromethyl)thio)phenyl)ethaneamine was used instead of (S)-1-phenylethylamine.
$^1$H NMR (400 MHz, DMSO-$d_6$) δ 12.83 (br, OH, 1H), 8.41 (d, *J* = 7.6 Hz, 1H), 7.94 (d, *J* = 8.0 Hz, 1H), 7.70-7.40 (m, 11H), 7.40-7.25 (m, 6H), 6.53 (d, *J* = 8.4 Hz, 1H), 5.14 (m, 1H), 4.62 (s, 2H), 3.47 (t, *J* = 5.6 Hz, 2H), 2.80 (t, *J* = 5.6 Hz, 2H), 1.95 (m, 2H), 1.45 (d, *J* = 6.8 Hz, 3H).

**<Example 14> (S)-4'-((6-((1-(4-cyanophenyl)ethyl)carbamoyl)-3,4-dihydroquinoline-1(2H)-yl)methyl)-[1,1'-biphenyl]-2-carboxylic acid**

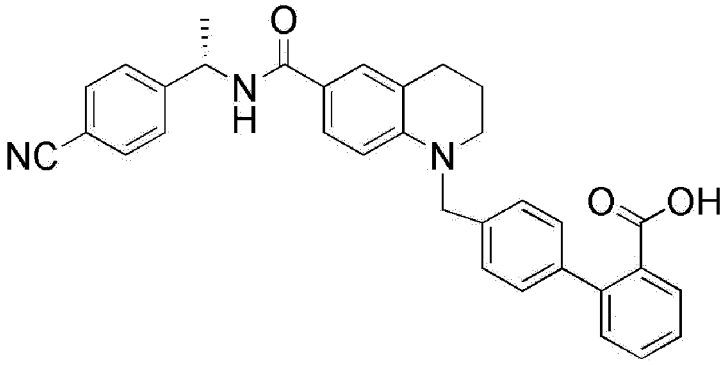

The target compound was obtained by the same manner as described in Example 1 except that (S)-1-(4-cyanophenyl)ethaneamine was used instead of (S)-1-phenylethylamine.
$^1$H NMR (400 MHz, DMSO-$d_6$) δ 12.66 (br, OH, 1H), 8.42 (d, *J* = 7.6 Hz, 1H), 7.87 (d, *J* = 7.0 Hz, 2H), 7.69 (d, *J* = 7.6 Hz, 1H), 7.58-7.49 (m, 5H), 7.43 (t, *J* = 7.6 Hz, 1H), 7.38 (d, *J* = 7.2 Hz, 1H), 7.32-7.25 (m, 4H), 6.53 (d, *J* = 8.4 Hz, 1H), 5.15 (m, 1H), 4.62 (s, 2H), 3.47 (t, *J* = 5.2 Hz, 2H), 2.80 (t, *J* = 6.0 Hz, 2H), 1.96 (m, 2H), 1.44 (d, *J* = 7.2 Hz, 3H).

**<Example 15> 4'-((6-(cromene-3-ylcarbamoyl)-3,4-dihydroquinoline-1(2H)-yl)methyl)-[1,1'-biphenyl]-2-carboxylic acid**

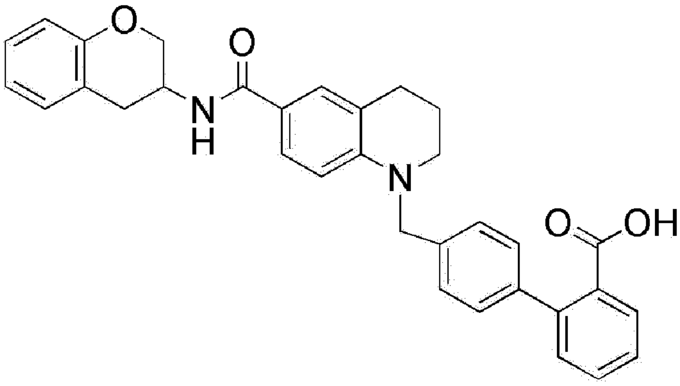

The target compound was obtained by the same manner as described in Example 1 except that cromene-3-amine was used instead of (S)-1-phenylethylamine.
$^1$H NMR (400 MHz, DMSO-$d_6$) δ 12.62 (br, OH, 1H), 7.97 (m, 1H), 7.71 (m, 1H), 7.60-7.40 (m, 4H), 7.40-7.33 (m, 1H), 7.26 (m, 4H), 7.09 (m, 2H), 6.84 (t, *J* = 8.0 Hz, 1H), 6.79 (d, *J* = 8.0 Hz, 1H), 6.54 (d, *J* = 8.4 Hz, 1H), 4.62 (s, 2H), 4.30 (m, 1H), 4.18 (m, 1H), 3.82 (m, 1H), 3.47 (t, *J* = 5.6 Hz, 2H), 3.05-2.89 (m, 2H), 2.80 (t, *J* = 5.6 Hz, 2H), 1.97 (m, 2H).

**<Example 16> (S)-4'-((6-((1-(2,6-difluorophenyl)ethyl)carbamoyl)-3,4-dihydroquinoline-1(2H)-yl)methyl)-[1,1'-biphenyl]-2-carboxylic acid**

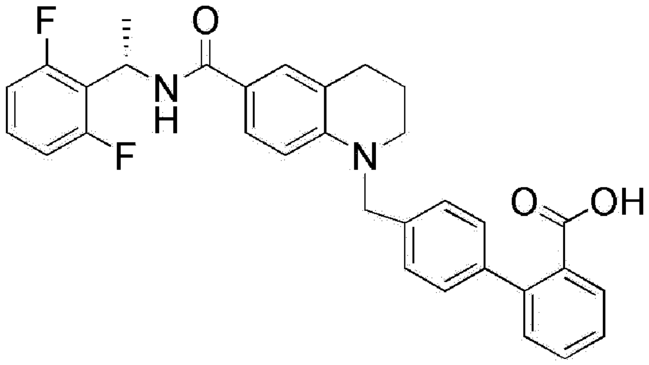

The target compound was obtained by the same manner as described in Example 1 except that (S)-1-(2,6-difluorophenyl)ethaneamine was used instead of (S)-1-phenylethylamine.
$^1$H NMR (400 MHz, DMSO-$d_6$) δ 12.73 (br, OH, 1H), 8.26 (d, *J* = 6.8 Hz, 1H), 7.69 (d, *J* = 7.6 Hz, 1H), 7.58-7.40 (m, 4H), 7.36 (d, *J* = 7.6 Hz, 1H), 7.31-7.23 (m, 5H), 7.00 (t, *J* = 8.0 Hz, 1H), 6.51 (d, *J* = 8.8 Hz, 1H), 5.35 (m, 1H), 4.61 (s, 2H), 3.47 (t, *J* = 5.2 Hz, 2H), 2.78 (t, *J* = 5.6 Hz, 2H), 1.97 (m, 2H), 1.53 (d, *J* = 6.8 Hz, 3H).

**<Example 17> (S)-4'-((6-((1-(3-fluorophenyl)ethyl)carbamoyl)-3,4-dihydroquinoline-1(2H)-yl)methyl)-[1,1'-biphenyl]-2-carboxylic acid**

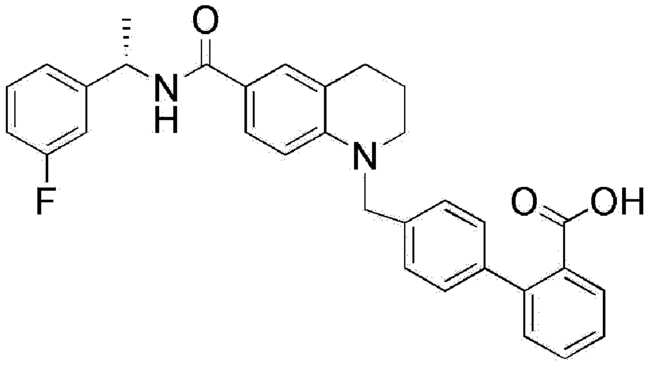

The target compound was obtained by the same manner as described in Example 1 except that (S)-1-(3-fluorophenyl)ethaneamine was used instead of (S)-1-phenylethylamine.
$^1$H NMR (400 MHz, DMSO-$d_6$) δ 12.81 (br, OH, 1H), 8.32 (d, *J* = 7.6 Hz, 1H), 7.70 (d, *J* = 7.6 Hz, 1H), 7.58-7.50 (m, 3H), 7.44 (t, *J* = 7.2 Hz, 1H), 7.38-7.25 (m, 6H), 7.18 (m, 1H), 7.02 (m, 1H), 6.54 (d, *J* = 8.8 Hz, 1H), 5.13 (m, 1H), 4.62 (s, 2H), 3.45 (t, *J* = 5.2 Hz, 2H), 2.80 (t, *J* = 5.6 Hz, 2H), 1.97 (m, 2H), 1.44 (d, *J* = 6.8 Hz, 3H).

**<Example 18> (S)-4'-((6-((1-(3-(trifluoromethoxy)phenyl)ethyl)carbamoyl)-3,4-dihydroquinoline-1(2H)-yl)methyl)-[1,1'-biphenyl]-2-carboxylic acid**

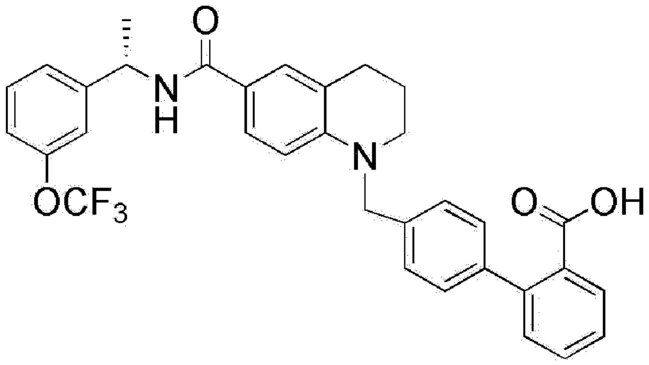

The target compound was obtained by the same manner as described in Example 1 except that (S)-1-(3-(trifluoromethoxy)phenyl)ethaneamine was used instead of (S)-1-phenylethylamine.
$^1$H NMR (400 MHz, DMSO-$d_6$) δ 12.73 (br, OH, 1H), 8.37 (d, *J* = 8.0 Hz, 1H), 7.70 (d, *J* = 7.6 Hz, 1H), 7.58-7.46 (m, 3H), 7.44 (t, *J* = 7.2 Hz, 2H), 7.38 (t, *J* = 7.6 Hz, 2H), 7.35-7.25 (m, 5H), 7.19 (d, *J* = 7.6 Hz, 1H), 6.54 (d, *J* = 8.4 Hz, 1H), 5.15 (m, 1H), 4.62 (s, 2H), 3.47 (t, *J* = 5.2 Hz, 2H), 2.80 (t, *J* = 5.6 Hz, 2H), 1.97 (m, 2H), 1.44 (d, *J* = 6.8 Hz, 3H).

**<Example 19> (S)-4'-((6-((1-(3-(trifluoromethyl)phenyl)ethyl)carbamoyl)-3,4-dihydroquinoline-1(2H)-yl)methyl)-[1,1'-biphenyl]-2-carboxylic acid**

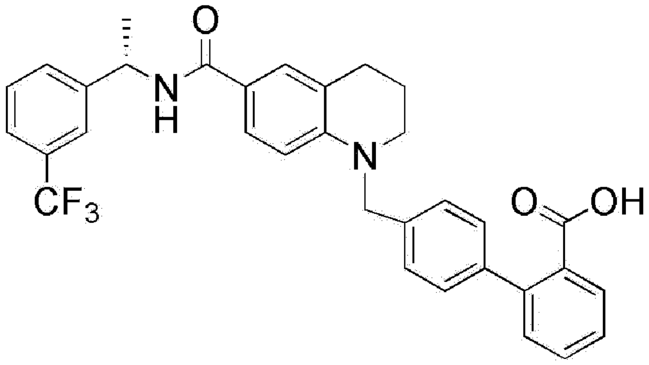

The target compound was obtained by the same manner as described in Example 1 except that S)-1-(3-(trifluoromethyl)phenyl)ethaneamine was used instead of (S)-1-phenylethylamine.
$^1$H NMR (400 MHz, DMSO-$d_6$) δ 12.81 (br, OH, 1H), 8.42 (d, *J* = 8.0 Hz, 1H), 7.73-7.65 (m, 3H), 7.59-7.50 (m, 5H), 7.4 (td, *J* = 1.2, 7.6 Hz, 1H), 7.39 (d, *J* = 7.2 Hz, 1H), 7.31-7.25 (m, 4H), 6.54 (d, *J* = 7.6 Hz, 1H), 5.19 (m, 1H), 4.62 (s, 2H), 3.47 (t, *J* = 5.2 Hz, 2H), 2.80 (t, *J* = 6.0 Hz, 2H), 1.97 (m, 2H), 1.47 (d, *J* = 6.8 Hz, 3H).

**<Example 20> (S)-4'-((6-((1-(naphthalene-2-yl)ethyl)carbamoyl)-3,4-dihydroquinoline-1(2H)-yl)methyl)-[1,1'-biphenyl]-2-carboxylic acid**

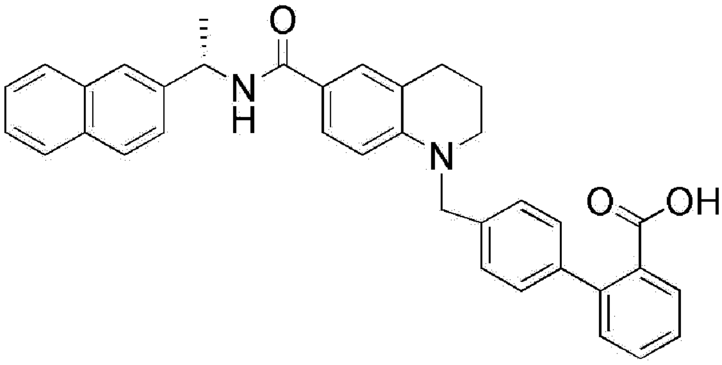

The target compound was obtained by the same manner as described in Example 1 except that (S)-1-(naphthalene-2-yl)ethaneamine was used instead of (S)-1-phenylethylamine.
$^1$H NMR (400 MHz, DMSO-$d_6$) δ 12.73 (br, OH, 1H), 8.41 (d, *J* = 8.0 Hz, 1H), 7.89-7.81 (m, 4H), 7.70 (d, *J* = 7.6 Hz, 1H), 7.60-7.42 (m, 7H), 7.37 (d, *J* = 7.6 Hz, 1H), 7.31-7.25 (m, 4H), 6.54 (d, *J* = 8.8 Hz, 1H), 5.28 (m, 1H), 4.62 (s, 2H), 3.47 (t, *J* = 5.2 Hz, 2H), 2.80 (t, *J* = 5.6 Hz, 2H), 1.97 (m, 2H), 1.54 (d, *J* = 6.8 Hz, 3H).

**<Example 21> (S)-4'-((6-((1-(3,4-difluorophenyl)ethyl)carbamoyl)-3,4-dihydroquinoline-1(2H)-yl)methyl)-[1,1'-biphenyl]-2-carboxylic acid**

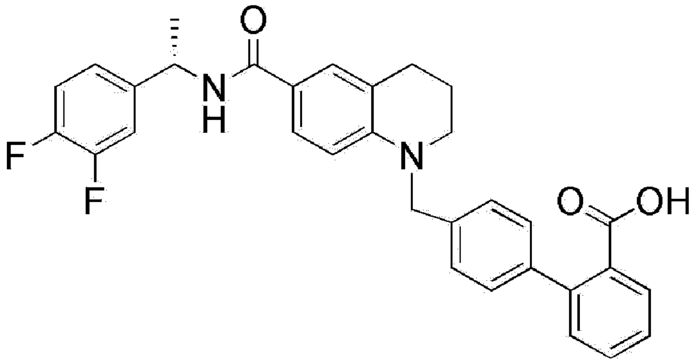

The target compound was obtained by the same manner as described in Example 1 except that (S)-1-(3,4-difluorophenyl)ethaneamine was used instead of (S)-1-phenylethylamine.
$^1$H NMR (400 MHz, DMSO-$d_6$) δ 12.73 (br, OH, 1H), 8.33 (d, *J* = 8.0 Hz, 1H), 7.70 (d, *J* = 7.6 Hz, 1H), 7.60-7.15 (m, 12H), 6.53 (d, *J* = 8.4 Hz, 1H), 5.10 (m, 1H), 4.62 (s, 2H), 3.47 (t, *J* = 5.2 Hz, 2H), 2.80 (t, *J* = 5.6 Hz, 2H), 1.97 (m, 2H), 1.54 (d, *J* = 6.8 Hz, 3H).

**<Example 22> (S)-4'-((6-((1-(3-methoxyphenyl)ethyl)carbamoyl)-3,4-dihydroquinoline-1(2H)-yl)methyl)-[1,1'-biphenyl]-2-carboxylic acid**

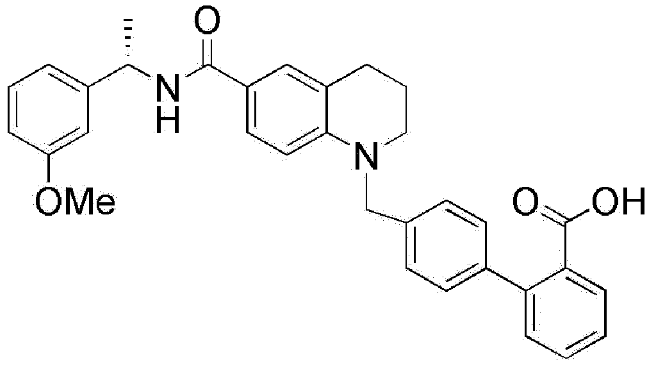

The target compound was obtained by the same manner as described in Example 1 except that (S)-1-(3-methoxyphenyl)ethaneamine was used instead of (S)-1-phenylethylamine.
$^1$H NMR (400 MHz, DMSO-$d_6$) $\delta$ 12.73 (br, OH, 1H), 8.28 (d, *J* = 8.4 Hz, 1H), 7.70 (dd, *J* = 1.2, 7.6 Hz, 1H), 7.60-7.46 (m, 3H), 7.45 (m, 1H), 7.38 (m, 1H), 7.34-7.19 (m, 5H), 6.95 (m, 2H), 6.78 (m, 1H), 6.52 (d, *J* = 8.4 Hz, 1H), 5.10 (m, 1H), 4.62 (s, 2H), 3.74 (s, 3H), 3.47 (t, *J* = 5.2 Hz, 2H), 2.80 (t, *J* = 5.6 Hz, 2H), 1.97 (m, 2H), 1.42 (d, *J* = 6.8 Hz, 3H).

**<Example 23> (S)-4'-((6-((1-(2-methoxyphenyl)ethyl)carbamoyl)-3,4-dihydroquinoline-1(2H)-yl)methyl)-[1,1'-biphenyl]-2-carboxylic acid**

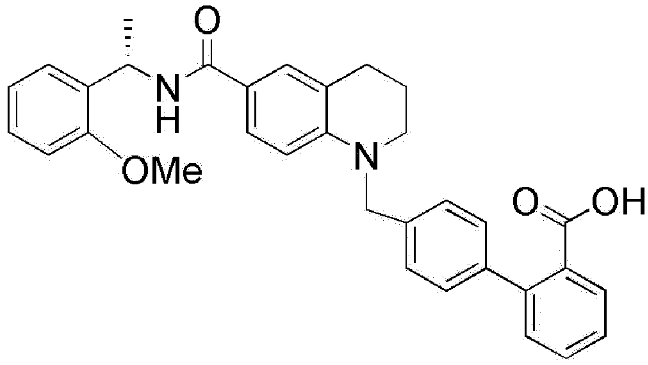

The target compound was obtained by the same manner as described in Example 1 except that (S)-1-(2-methoxyphenyl)ethaneamine was used instead of (S)-1-phenylethylamine.
$^1$H NMR (400 MHz, DMSO-$d_6$) $\delta$ 12.73 (br, OH, 1H), 8.23 (d, *J* = 8.8 Hz, 1H), 7.70 (dd, *J* = 1.2, 7.6 Hz, 1H), 7.60-7.15 (m, 11H), 7.0-6.86 (m, 2H), 6.52 (d, *J* = 8.4 Hz, 1H), 5.39 (m, 1H), 4.62 (s, 2H), 3.82 (s, 3H), 3.47 (t, *J* = 5.2 Hz, 2H), 2.80 (t, *J* = 5.6 Hz, 2H), 1.97 (m, 2H), 1.34 (d, *J* = 7.2 Hz, 3H).

**<Example 24> (S)-4'-((6-((1-(2-fluorophenyl)ethyl)carbamoyl)-3,4-dihydroquinoline-1(2H)-yl)methyl)-[1,1'-biphenyl]-2-carboxylic acid**

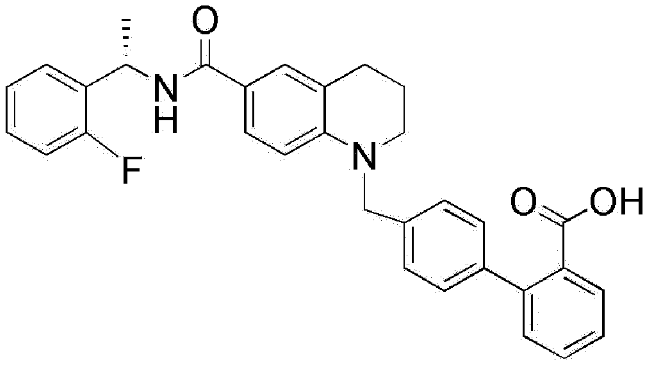

The target compound was obtained by the same manner as described in Example 1 except that (S)-1-(2-fluorophenyl)ethaneamine was used instead of (S)-1-phenylethylamine.
$^1$H NMR (400 MHz, DMSO-$d_6$) δ 12.73 (br, OH, 1H), 8.31 (d, *J* = 8.0 Hz, 1H), 7.70 (d, *J* = 7.2 Hz, 1H), 7.60-7.10 (m, 13H), 6.53 (d, *J* = 8.8 Hz, 1H), 5.35 (m, 1H), 4.62 (s, 2H), 3.47 (t, *J* = 5.2 Hz, 2H), 2.80 (t, *J* = 5.6 Hz, 2H), 1.97 (m, 2H), 1.42 (d, *J* = 6.8 Hz, 3H).

**<Example 25> (S)-4'-((6-((1-(2-(trifluoromethyl)phenyl)ethyl)carbamoyl)-3,4-dihydroquinoline-1(2H)-yl)methyl)-[1,1'-biphenyl]-2-carboxylic acid**

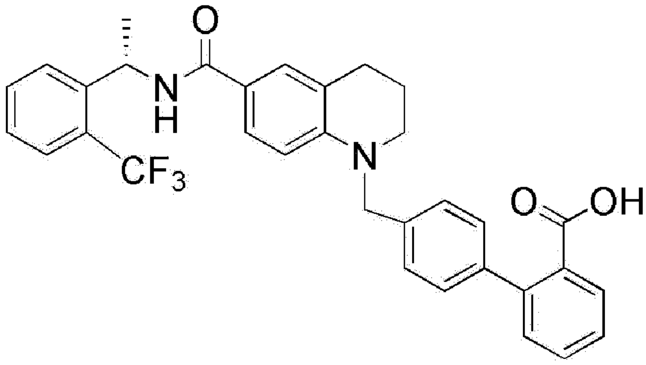

The target compound was obtained by the same manner as described in Example 1 except that (S)-1-(2-(trifluoromethyl)phenyl)ethaneamine was used instead of (S)-1-phenylethylamine.
$^1$H NMR (400 MHz, DMSO-$d_6$) δ 12.81 (br, OH, 1H), 8.48 (d, *J* = 7.2 Hz, 1H), 7.92 (d, *J* = 7.6 Hz, 1H), 7.71-7.60 (m, 3H), 7.59-7.35 (m, 6H), 7.32-7.20 (m, 4H), 6.52 (d, *J* = 8.4 Hz, 1H), 5.41 (m, 1H), 4.62 (s, 2H), 3.46 (t, *J* = 5.2 Hz, 2H), 2.80 (t, *J* = 5.6 Hz, 2H), 1.97 (m, 2H), 1.42 (d, *J* = 6.8 Hz, 3H).

**<Example 26> 4'-((6-((pyridine-2-ylmethyl)carbamoyl)-3,4-dihydroquinoline-1(2H)-yl)methyl)-[1,1'-biphenyl]-2-carboxylic acid**

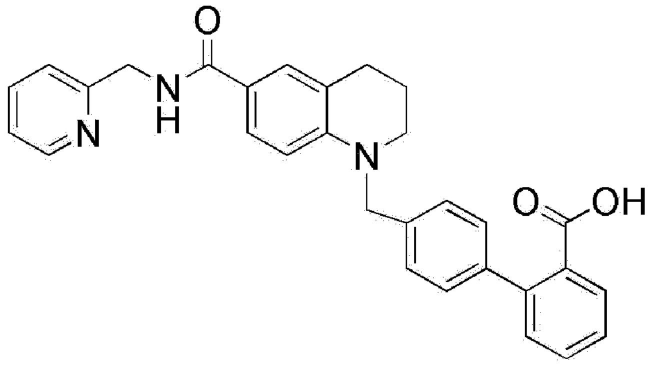

The target compound was obtained by the same manner as described in Example 1 except that pyridine-2-ylmethaneamine was used instead of (S)-1-phenylethylamine.
$^1H$ NMR (400 MHz, DMSO-$d_6$) δ 12.75 (br, OH, 1H), 8.68-8.67 (m, 1H), 8.52-8.51 (m, 1H), 7.81-7.77 (m, 1H), 7.70-7.69 (m, 1H), 7.57-7.54 (m, 3H), 7.46-7.42 (m, 1H), 7.38-7.36 (m, 1H), 7.31-7.26 (m, 6H), 4.63 (s, 2H), 4.53-4.52 (m, 2H), 3.34 (m, 2H), 2.80 (m, 2H), 1.97 (m, 2H).

**<Example 27> 4'-((6-((pyridine-4-ylmethyl)carbamoyl)-3,4-dihydroquinoline-1(2H)-yl)methyl)-[1,1'-biphenyl]-2-carboxylic acid**

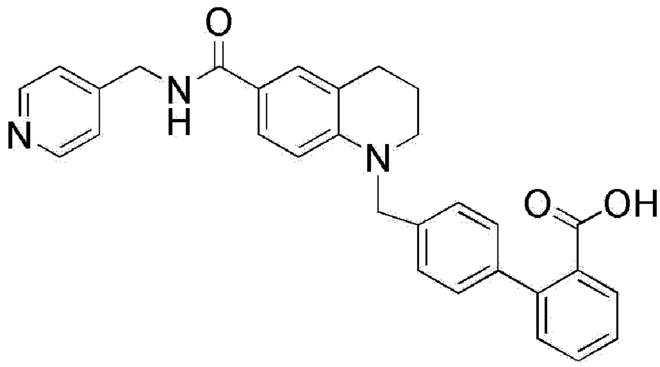

The target compound was obtained by the same manner as described in Example 1 except that pyridine-4-ylmethaneamine was used instead of (S)-1-phenylethylamine.
$^1H$ NMR (400 MHz, DMSO-$d_6$) δ 12.75 (br, OH, 1H), 8.74-8.72 (m, 1H), 8.58-8.56 (m, 2H), 7.71-7.69 (m, 1H), 7.57-7.51 (m, 3H), 7.46-7.42 (m, 3H), 7.38-7.36 (m, 1H), 7.31-7.26 (m, 4H), 6.56-6.53 (m, 1H), 4.63 (s, 2H), 4.50-4.48 (m, 2H), 3.49-3.46 (m, 2H), 2.81-2.79 (m, 2H), 1.98-1.95 (m, 2H).

**<Example 28> 4'-((6-(benzylcarbamoyl)-3,4-dihydroquinoline-1(2H)-yl)methyl)-[1,1'-biphenyl]-2-carboxylic acid**

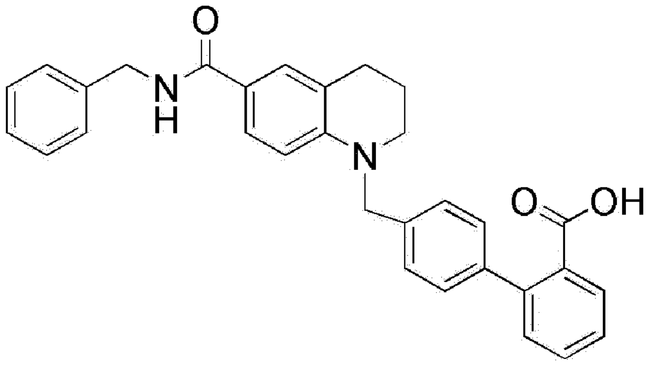

The target compound was obtained by the same manner as described in Example 1 except that phenylmethaneamine was used instead of (S)-1-phenylethylamine.
$^1$H NMR (400 MHz, DMSO-$d_6$) δ 12.75 (br, OH, 1H), 7.71-7.68 (m, 1H), 7.57-7.49 (m, 3H), 7.46-7.42 (m, 1H), 7.38-7.36 (m, 1H), 7.32-7.25 (m, 8H), 7.23-7.19 (m, 1H), 6.53-6.51 (m, 1H), 4.62 (s, 2H), 4.43-4.41 (m, 2H), 3.48-3.45 (m, 2H), 2.81-2.78 (m, 2H), 1.99-1.91 (m, 2H).

**<Example 29> 4'-((6-((2-bromobenzyl)carbamoyl)-3,4-dihydroquinoline-1(2H)-yl)methyl)-[1,1'-biphenyl]-2-carboxylic acid**

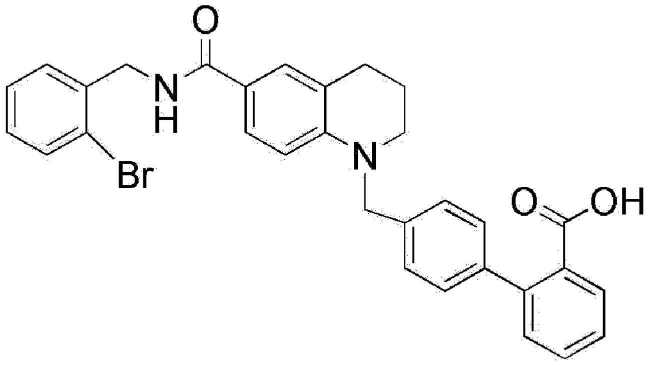

The target compound was obtained by the same manner as described in Example 1 except that (2-bromophenyl)methaneamine was used instead of (S)-1-phenylethylamine.
$^1$H NMR (400 MHz, DMSO-$d_6$) δ 8.63-8.60 (m, 1H), 7.65-7.59 (m, 2H), 7.56-7.53 (m, 2H), 7.50-7.47 (m, 1H), 7.41-7.32 (m, 5H), 7.26-7.24 (m, 3H), 7.21-7.18 (m, 1H), 6.56-6.53 (m, 1H), 4.62 (s, 2H), 4.44-4.42 (m, 2H), 3.49-3.47 (m, 2H), 2.82-2.79 (m, 2H), 1.97 (m, 2H).

**<Example 30> 4'-((6-(((2,3-dihydrobenzo[b][1,4]dioxin-6-yl)methyl)carbamoyl)-3,4-dihydroquinoline-1(2H)-yl)methyl)-[1,1'-biphenyl]-2-carboxylic acid**

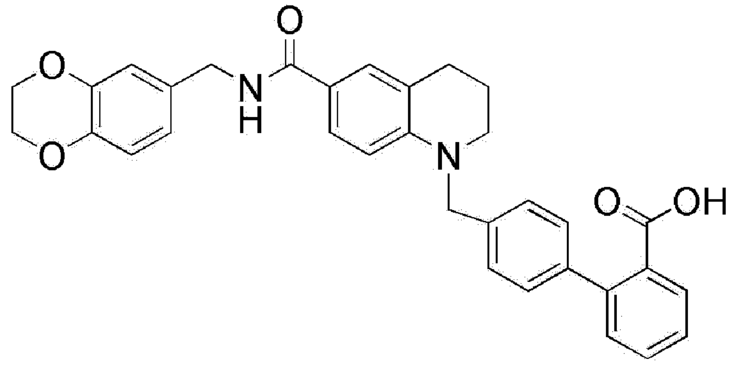

The target compound was obtained by the same manner as described in Example 1 except that (2,3-dihydrobenzo[b][1,4]dioxin-6-yl)methaneamine was used instead of (S)-1-phenylethylamine.
$^1$H NMR (400 MHz, DMSO-$d_6$) δ 12.75 (br, OH, 1H), 8.50-8.47 (m, 1H), 7.56-7.41 (m, 4H), 7.38-7.36 (m, 1H), 7.31-7.25 (m, 4H), 6.83-6.71 (m, 3H), 6.53-6.51 (m, 1H), 4.61 (s, 2H), 4.29-4.28 (m, 2H), 4.19 (s, 4H),3.47-3.45 (m, 2H), 2.80-2.77 (m, 2H), 1.97-1.94 (m, 2H).

**<Example 31> 4'-((6-((4-bromobenzyl)carbamoyl)-3,4-dihydroquinoline-1(2H)-yl)methyl)-[1,1'-biphenyl]-2-carboxylic acid**

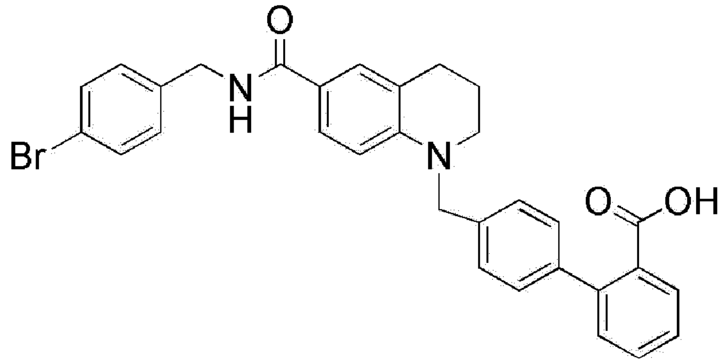

The target compound was obtained by the same manner as described in Example 1 except that (4-bromophenyl)methaneamine was used instead of (S)-1-phenylethylamine.
$^1$H NMR (400 MHz, $CDCl_3$) δ 7.94 (d, $J$ = 7.2 Hz, 1H), 7.95-7.20 (m, 13H), 6.49 (d, $J$ = 8.8 Hz, 1H), 6.25 (s, 1H), 4.59-4.55 (m, 4H), 3.50 (t, $J$ = 5.6 Hz, 2H), 2.86 (t, $J$ = 6.0 Hz, 2H), 2.07-1.98 (m, 2H).

**<Example 32> 4'-((6-((3-(trifluoromethoxy)benzyl)carbamoyl)-3,4-dihydroquinoline-1(2H)-yl)methyl)-[1,1'-biphenyl]-2-carboxylic acid**

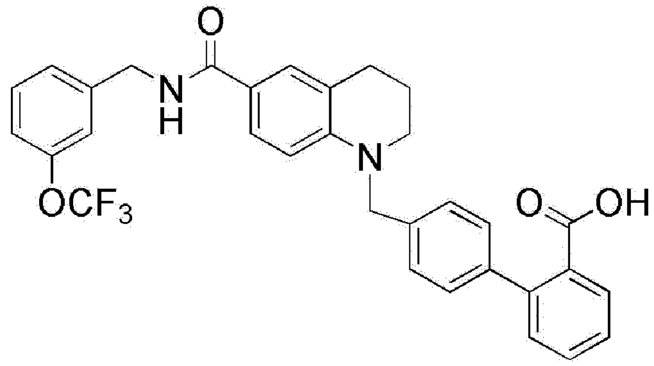

The target compound was obtained by the same manner as described in Example 1 except that (3-(trifluoromethoxy)phenyl)methaneamine was used instead of (S)-1-phenylethylamine.
$^1$H NMR (400 MHz, $CDCl_3$) $\delta$ 7.91 (s, 1H), 7.60-7.02 (m, 13H), 6.47 (d, $J$ = 8.4 Hz, 1H), 6.37 (s, 1H), 4.60-4.55 (m, 4H), 3.51-3.45 (m, 2H), 2.84 (m, 2H), 1.94-1.90 (m, 2H).

**<Example 33> (S)-1-((2'-carbamoyl-[1,1'-biphenyl]-4-yl)methyl)-N-(1-(4-(trifluoromethyl)phenyl)ethyl)-1,2,3,4-tetrahydroquinoline-6-carboxamide**

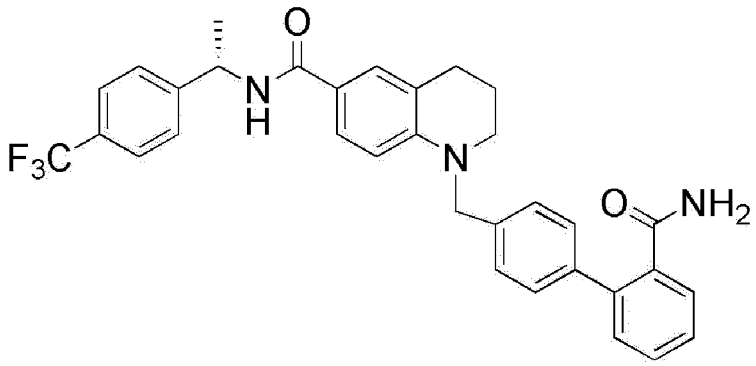

The target compound was obtained by the same manner as described in Example 2 except that (S)-4'-((6-((1-(4-(trifluoromethyl)phenyl)ethyl)carbamoyl)-3,4-dihydroquinoline-1(2H)-yl)methyl)-[1,1'-biphenyl]-2-carboxylic acid obtained in Example 12 was used instead of (S)-4'-((6-((1-phenylethyl)carbamoyl)-3,4-dihydroquinoline-1(2H)-yl)methyl)-[1,1'-biphenyl]-2-carboxylic acid.
$^1$H NMR (400 MHz, DMSO-$d_6$) $\delta$ 8.42 (d, $J$ = 7.6 Hz, 1H), 7.68-7.73 (m, 3H), 7.60-7.35 (m, 10H), 7.28-2.22 (m, 3H), 6.53 (d, $J$ = 8.8 Hz, 1H), 5.16 (m, 1H), 4.61 (s, 2H), 3.47 (t, $J$ = 5.2 Hz, 2H), 2.80 (t, $J$ = 5.6 Hz, 2H), 1.97 (m, 2H), 1.46 (d, $J$ = 7.2 Hz, 3H).

**<Example 34> (S)-1-((2'-carbamoyl-[1,1'-biphenyl]-4-yl)methyl)-N-(1-(4-(trifluoromethoxy)phenyl)ethyl)-1,2,3,4-tetrahydroquinoline-6-carboxamide**

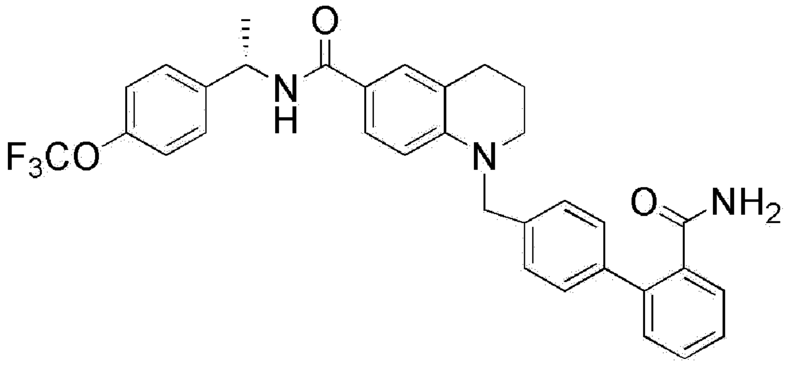

The target compound was obtained by the same manner as described in Example 2 except that (S)-4'-((6-((1-(4-(trifluoromethoxy)phenyl)ethyl)carbamoyl)-3,4-dihydroquinoline-1(2H)-yl)methyl)-[1,1'-biphenyl]-2-carboxylic acid obtained in Example 11 was used instead of (S)-4'-((6-((1-phenylethyl)carbamoyl)-3,4-dihydroquinoline-1(2H)-yl)methyl)-[1,1'-biphenyl]-2-carboxylic acid.
$^1$H NMR (400 MHz, DMSO-$d_6$) δ 8.36 (d, *J* = 8.0 Hz, 1H), 7.65 (s, 1H), 7.58-7.23 (m, 15H), 6.52 (d, *J* = 8.4 Hz, 1H), 5.14 (m, 1H), 4.61 (s, 2H), 3.47 (t, *J* = 5.2 Hz, 2H), 2.80 (t, *J* = 5.6 Hz, 2H), 1.97 (m, 2H), 1.44 (d, *J* = 7.2 Hz, 3H).

**<Example 35> (S)-1-((2'-carbamoyl-[1,1'-biphenyl]-4-yl)methyl)-N-(1-(3-chlorophenyl)ethyl)-1,2,3,4-tetrahydroquinoline-6-carboxamide**

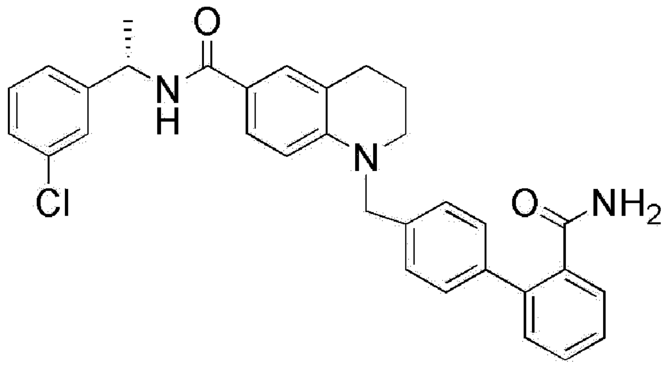

The target compound was obtained by the same manner as described in Example 2 except that (S)-4'-((6-((1-(3-chlorophenyl)ethyl)carbamoyl)-3,4-dihydroquinoline-1(2H)-yl)methyl)-[1,1'-biphenyl]-2-carboxylic acid obtained in Example 9 was used instead of (S)-4'-((6-((1-phenylethyl)carbamoyl)-3,4-dihydroquinoline-1(2H)-yl)methyl)-[1,1'-biphenyl]-2-carboxylic acid.
$^1$H NMR (400 MHz, DMSO-$d_6$) δ 8.33 (d, *J* = 7.6 Hz, 1H), 7.63 (m, 1H), 7.55-7.25 (m, 15H), 6.54 (d, *J* = 8.4 Hz, 1H), 5.10 (m, 1H), 4.61 (s, 2H), 3.47 (t, *J* = 5.2 Hz, 2H), 2.80 (t, *J* = 5.6 Hz, 2H), 1.97 (m, 2H), 1.43 (d, *J* = 6.8 Hz, 3H).

**<Example 36> (S)-1-((2'-carbamoyl-[1,1'-biphenyl]-4-yl)methyl)-N-(1-(4-fluorophenyl)ethyl)-1,2,3,4-tetrahydroquinoline-6-carboxamide**

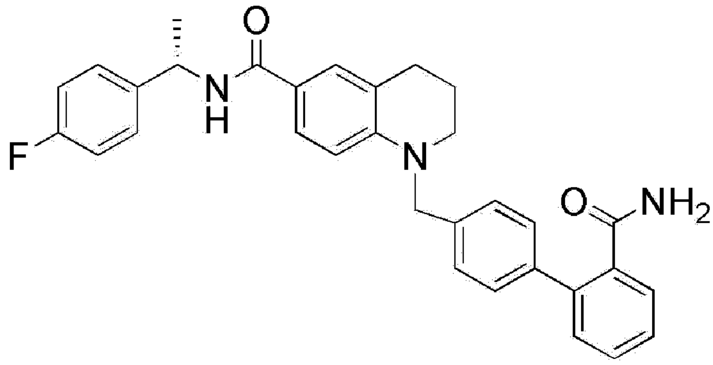

The target compound was obtained by the same manner as described in Example 2 except that (S)-4'-((6-((1-(4-fluorophenyl)ethyl)carbamoyl)-3,4-dihydroquinoline-1(2H)-yl)methyl)-[1,1'-biphenyl]-2-carboxylic acid obtained in Example 6 was used instead of (S)-4'-((6-((1-phenylethyl)carbamoyl)-3,4-dihydroquinoline-1(2H)-yl)methyl)-[1,1'-biphenyl]-2-carboxylic acid.

$^1$H NMR (400 MHz, DMSO-$d_6$) δ 8.39 (d, *J* = 7.6 Hz, 1H), 7.64 (m, 1H), 7.56-7.35 (m, 10H), 7.28-2.22 (m, 3H), 7.15-1.08 (m, 2H), 6.52 (d, *J* = 8.8 Hz, 1H), 5.12 (m, 1H), 4.60 (s, 2H), 3.47 (t, *J* = 5.2 Hz, 2H), 2.79 (t, *J* = 5.6 Hz, 2H), 1.97 (m, 2H), 1.44 (d, *J* = 7.2 Hz, 3H).

**<Example 37> (S)-N-(1-(4-bromophenyl)ethyl)-1-((2'-carbamoyl-[1,1'-biphenyl]-4-yl)methyl)-1,2,3,4-tetrahydroquinoline-6-carboxamide**

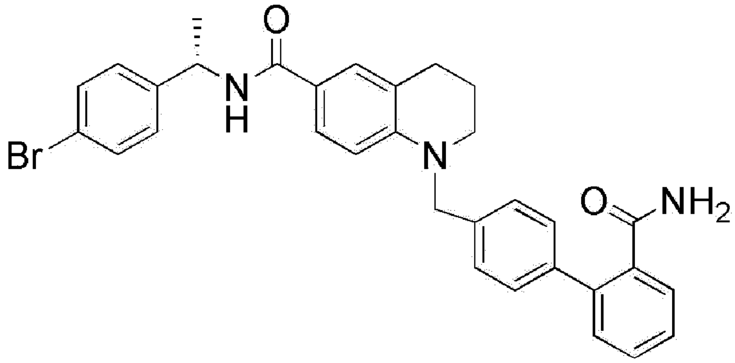

The target compound was obtained by the same manner as described in Example 2 except that (S)-4'-((6-((1-(4-bromophenyl)ethyl)carbamoyl)-3,4-dihydroquinoline-1(2H)-yl)methyl)-[1,1'-biphenyl]-2-carboxylic acid obtained in Example 7 was used instead of (S)-4'-((6-((1-phenylethyl)carbamoyl)-3,4-dihydroquinoline-1(2H)-yl)methyl)-[1,1'-biphenyl]-2-carboxylic acid.

$^1$H NMR (400 MHz, DMSO-$d_6$) δ 8.31 (d, *J* = 8.0 Hz, 1H), 7.63 (m, 1H), 7.55-7.20 (m, 15H), 6.52 (d, *J* = 8.4 Hz, 1H), 5.08 (m, 1H), 4.61 (s, 2H), 3.47 (t, *J* = 5.2 Hz, 2H), 2.79 (t, *J* = 6.0 Hz, 2H), 1.97 (m, 2H), 1.42 (d, *J* = 7.2 Hz, 3H).

**<Example 38> (S)-1-((2'-carbamoyl-[1,1'-biphenyl]-4-yl)methyl)-N-(1-(naphthalene-1-yl)ethyl)-1,2,3,4-tetrahydroquinoline-6-carboxamide**

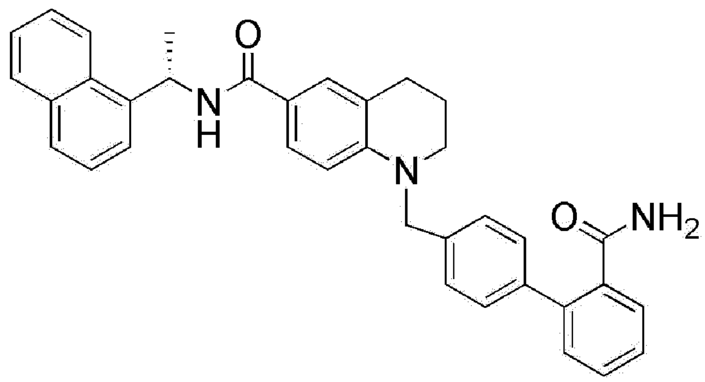

The target compound was obtained by the same manner as described in Example 2 except that (S)-4'-((6-((1-(naphthalene-1-yl)ethyl)carbamoyl)-3,4-dihydroquinoline-1(2H)-yl)methyl)-[1,1'-biphenyl]-2-carboxylic acid obtained in Example 10 was used instead of (S)-4'-((6-((1-phenylethyl)carbamoyl)-3,4-dihydroquinoline-1(2H)-yl)methyl)-[1,1'-biphenyl]-2-carboxylic acid.
$^1H$ NMR (400 MHz, DMSO-$d_6$) δ 8.44 (d, *J* = 8.0 Hz, 1H), 8.19 (d, *J* = 8.0 Hz, 1H), 7.93 (m, 1H), 7.68-7.32 (m, 12H), 7.28-7.22 (m, 3H), 6.53 (d, *J* = 8.8 Hz, 1H), 5.92 (m, 1H), 4.60 (s, 2H), 3.46 (t, *J* = 5.2 Hz, 2H), 2.79 (t, *J* = 5.6 Hz, 2H), 1.95 (m, 2H), 1.59 (d, *J* = 6.8 Hz, 3H).

**<Example 39> (S)-1-((2'-carbamoyl-[1,1'-biphenyl]-4-yl)methyl)-N-(1-(3,4-difluorophenyl)ethyl)-1,2,3,4-tetrahydroquinoline-6-carboxamide**

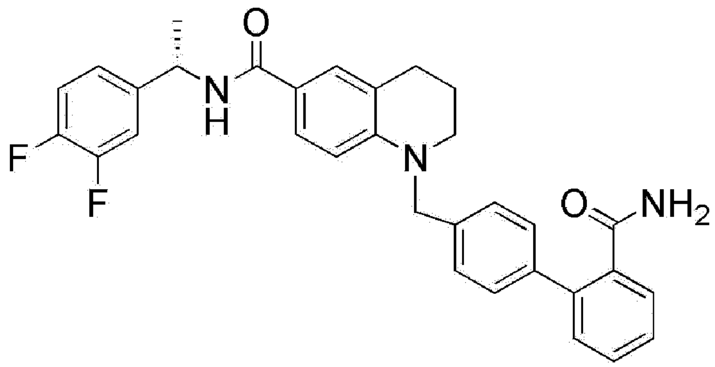

The target compound was obtained by the same manner as described in Example 2 except that (S)-4'-((6-((1-(3,4-difluorophenyl)ethyl)carbamoyl)-3,4-dihydroquinoline-1(2H)-yl)methyl)-[1,1'-biphenyl]-2-carboxylic acid obtained in Example 21 was used instead of (S)-4'-((6-((1-phenylethyl)carbamoyl)-3,4-dihydroquinoline-1(2H)-yl)methyl)-[1,1'-biphenyl]-2-carboxylic acid.
$^1H$ NMR (400 MHz, DMSO-$d_6$) δ 8.33 (d, *J* = 8.0 Hz, 1H), 7.66 (m, 1H), 7.55-7.15 (m, 13H), 6.52 (d, *J* = 8.8 Hz, 1H), 5.08 (m, 1H), 4.61 (s, 2H), 3.47 (t, *J* = 5.2 Hz, 2H), 2.80 (t, *J* = 6.0 Hz, 2H), 1.97 (m, 2H), 1.42 (d, *J* = 7.2 Hz, 3H).

**<Example 40> (S)-1-((2'-carbamoyl-[1,1'-biphenyl]-4-yl)methyl)-N-(1-(3-methoxyphenyl)ethyl)-1,2,3,4-tetrahydroquinoline-6-carboxamide**

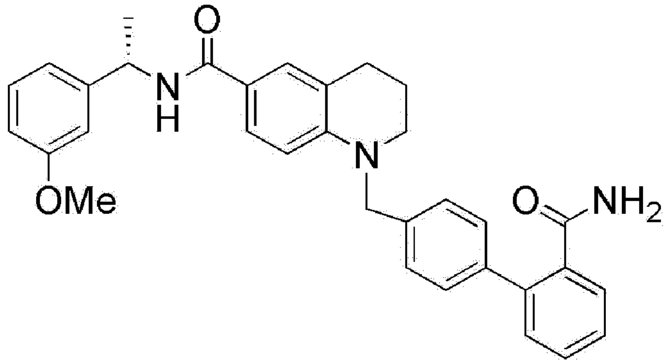

The target compound was obtained by the same manner as described in Example 2 except that (S)-4'-((6-((1-(3-methoxyphenyl)ethyl)carbamoyl)-3,4-dihydroquinoline-1(2H)-yl)methyl)-[1,1'-biphenyl]-2-carboxylic acid obtained in Example 22 was used instead of (S)-4'-((6-((1-phenylethyl)carbamoyl)-3,4-dihydroquinoline-1(2H)-yl)methyl)-[1,1'-biphenyl]-2-carboxylic acid.
$^1$H NMR (400 MHz, DMSO-$d_6$) δ 8.28 (d, *J* = 7.6 Hz, 1H), 7.65 (m, 1H), 7.55-7.20 (m, 12H), 6.93 (m, 1H), 6.77 (d, *J* = 6.8 Hz, 1H), 6.52 (d, *J* = 8.4 Hz, 1H), 5.09 (m, 1H), 4.61 (s, 2H), 3.73 (s, 3H), 3.47 (t, *J* = 5.2 Hz, 2H), 2.80 (t, *J* = 6.0 Hz, 2H), 1.97 (m, 2H), 1.42 (d, *J* = 6.0 Hz, 3H).

**<Example 41> (S)-1-((2'-carbamoyl-[1,1'-biphenyl]-4-yl)methyl)-N-(1-(2-methoxyphenyl)ethyl)-1,2,3,4-tetrahydroquinoline-6-carboxamide**

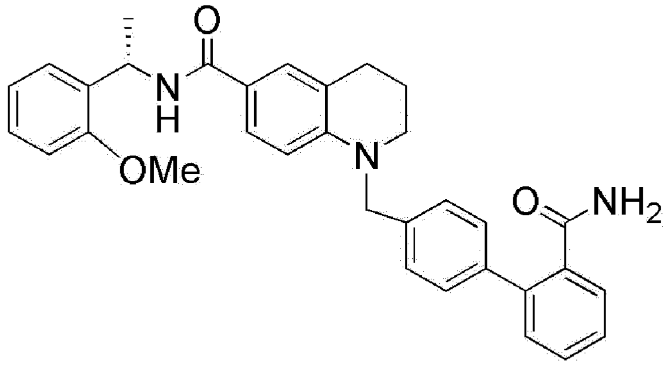

The target compound was obtained by the same manner as described in Example 2 except that (S)-4'-((6-((1-(2-methoxyphenyl)ethyl)carbamoyl)-3,4-dihydroquinoline-1(2H)-yl)methyl)-[1,1'-biphenyl]-2-carboxylic acid obtained in Example 23 was used instead of (S)-4'-((6-((1-phenylethyl)carbamoyl)-3,4-dihydroquinoline-1(2H)-yl)methyl)-[1,1'-biphenyl]-2-carboxylic acid.
$^1$H NMR (400 MHz, DMSO-$d_6$) δ 8.22 (d, *J* = 8.0 Hz, 1H), 7.60 (m, 1H), 7.55-7.25 (m, 13H), 6.95 (d, *J* = 7.6 Hz, 1H), 6.88 (t, *J* = 7.6 Hz, 1H), 6.52 (d, *J* = 8.4 Hz, 1H), 5.39 (m, 1H), 4.61 (s, 2H), 3.82 (s, 3H), 3.47 (t, *J* = 5.2 Hz, 2H), 2.80 (t, *J* = 6.0 Hz, 2H), 1.97 (m, 2H), 1.34 (d, *J* = 7.2 Hz, 3H).

**<Example 42> (S)-1-((2'-carbamoyl-[1,1'-biphenyl]-4-yl)methyl)-N-(1-(2-fluorophenyl)ethyl)-1,2,3,4-tetrahydroquinoline-6-carboxamide**

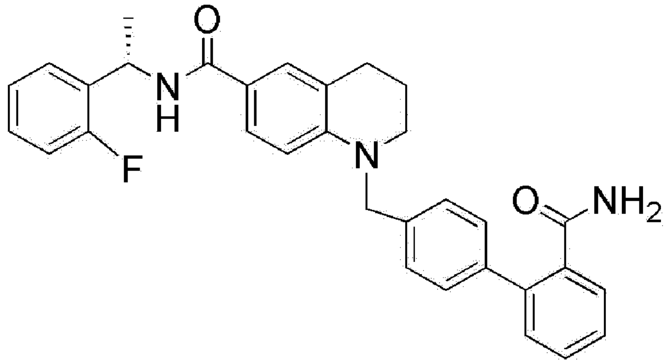

The target compound was obtained by the same manner as described in Example 2 except that (S)-4'-((6-((1-(2-fluorophenyl)ethyl)carbamoyl)-3,4-dihydroquinoline-1(2H)-yl)methyl)-[1,1'-biphenyl]-2-carboxylic acid obtained in Example 24 was used instead of (S)-4'-((6-((1-phenylethyl)carbamoyl)-3,4-dihydroquinoline-1(2H)-yl)methyl)-[1,1'-biphenyl]-2-carboxylic acid.

$^1$H NMR (400 MHz, DMSO-$d_6$) δ 8.37 (d, *J* = 8.0 Hz, 1H), 7.63 (s, 1H), 7.55-7.10 (m, 15H), 6.52 (d, *J* = 8.8 Hz, 1H), 5.35 (m, 1H), 4.61 (s, 2H), 3.47 (t, *J* = 5.2 Hz, 2H), 2.79 (t, *J* = 6.0 Hz, 2H), 1.97 (m, 2H), 1.42 (d, *J* = 7.2 Hz, 3H).

**<Example 43> (S)-1-((2'-carbamoyl-[1,1'-biphenyl]-4-yl)methyl)-N-(1-(2-(trifluoromethyl)phenyl)ethyl)-1,2,3,4-tetrahydroquinoline-6-carboxamide**

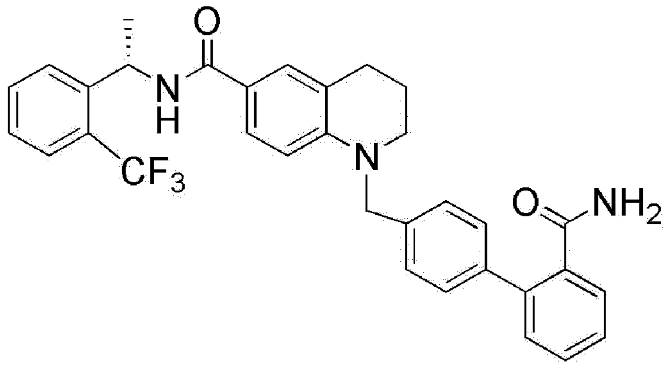

The target compound was obtained by the same manner as described in Example 2 except that (S)-4'-((6-((1-(2-(trifluoromethyl)phenyl)ethyl)carbamoyl)-3,4-dihydroquinoline-1(2H)-yl)methyl)-[1,1'-biphenyl]-2-carboxylic acid obtained in Example 25 was used instead of (S)-4'-((6-((1-phenylethyl)carbamoyl)-3,4-dihydroquinoline-1(2H)-yl)methyl)-[1,1'-biphenyl]-2-carboxylic acid.

$^1$H NMR (400 MHz, DMSO-$d_6$) δ 8.47 (d, *J* = 7.2 Hz, 1H), 7.81 (d, *J* = 8.0 Hz, 1H), 7.68-7.60 (m, 3H), 7.55-7.35 (m, 9H), 7.30-7.20 (m, 3H), 6.52 (d, *J* = 8.4 Hz, 1H), 5.41 (m, 1H), 4.61 (s, 2H), 3.47 (t, *J* = 5.2 Hz, 2H), 2.80 (t, *J* = 6.4 Hz, 2H), 1.97 (m, 2H), 1.42 (d, *J* = 6.8 Hz, 3H).

**<Example 44> (S)-1-((2'-carbamoyl-[1,1'-biphenyl]-4-yl)methyl)-N-(1-(naphthalene-2-yl)ethyl)-1,2,3,4-tetrahydroquinoline-6-carboxamide**

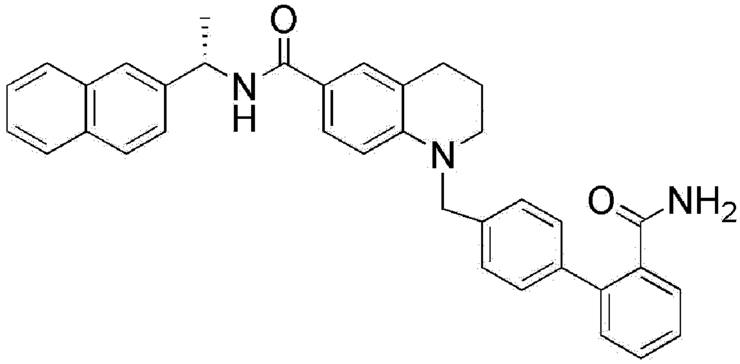

The target compound was obtained by the same manner as described in Example 2 except that (S)-4'-((6-((1-(naphthalene-2-yl)ethyl)carbamoyl)-3,4-dihydroquinoline-1(2H)-yl)methyl)-[1,1'-biphenyl]-2-carboxylic acid obtained in Example 20 was used instead of (S)-4'-((6-((1-phenylethyl)carbamoyl)-3,4-dihydroquinoline-1(2H)-yl)methyl)-[1,1'-biphenyl]-2-carboxylic acid.

$^1$H NMR (400 MHz, $CDCl_3$) δ 7.84-7.82 (m, 4H), 7.78-7.76 (m, 1H), 7.53-7.40 (m, 9H), 7.37-7.35 (m, 1H), 7.30-7.28 (m, 3H), 6.50-6.48 (m, 1H), 6.22-6.20 (m, NH, 1H), 5.53-5.48 (m, 1H), 5.44 (br, NH, 1H), 5.30 (br, NH, 1H), 4.59 (s, 2H), 3.51-3.47 (m, 2H), 2.89-2.86 (m, 2H), 2.09-2.04 (m, 2H), 1.74-1.68 (m, 3H).

**<Example 45> (S)-1-((2'-carbamoyl-[1,1'-biphenyl]-4-yl)methyl)-N-(1-(3-fluorophenyl)ethyl)-1,2,3,4-tetrahydroquinoline-6-carboxamide**

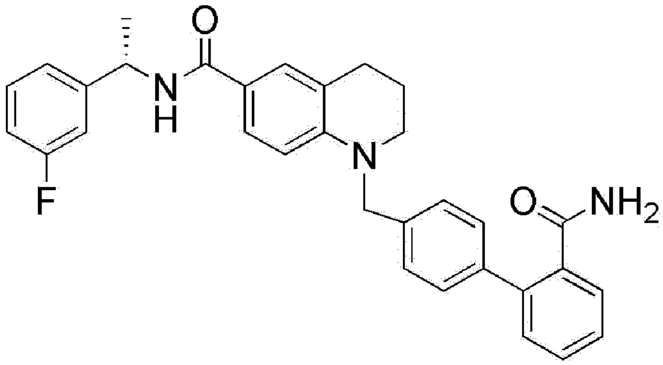

The target compound was obtained by the same manner as described in Example 2 except that (S)-4'-((6-((1-(3-fluorophenyl)ethyl)carbamoyl)-3,4-dihydroquinoline-1(2H)-yl)methyl)-[1,1'-biphenyl]-2-carboxylic acid obtained in Example 17 was used instead of (S)-4'-((6-((1-phenylethyl)carbamoyl)-3,4-dihydroquinoline-1(2H)-yl)methyl)-[1,1'-biphenyl]-2-carboxylic acid.

$^1$H NMR (400 MHz, DMSO-$d_6$) δ 8.34-8.32 (m, 1H), 7.66 (m, 1H), 7.52-7.31 (m, 9H), 7.27-7.24 (m, 3H), 7.19-7.15 (m, 2H), 7.05-7.00 (m, 1H), 6.54-6.51 (m, 1H), 5.14-5.11 (m, 1H), 4.61 (s, 2H), 3.47-3.43 (m, 2H), 2.80-2.79 (m, 2H), 1.99-1.96 (m, 2H), 1.44-1.42 (m, 3H).

**<Example 46> (S)-1-((2'-carbamoyl-[1,1'-biphenyl]-4-yl)methyl)-N-(1-(3-(trifluoromethoxy)phenyl)ethyl)-1,2,3,4-tetrahydroquinoline-6-carboxamide**

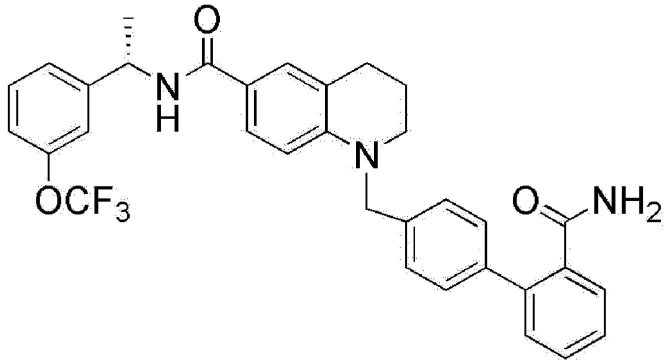

The target compound was obtained by the same manner as described in Example 2 except that (S)-4'-((6-((1-(3-(trifluoromethoxy)phenyl)ethyl)carbamoyl)-3,4-dihydroquinoline-1(2H)-yl)methyl)-[1,1'-biphenyl]-2-carboxylic acid obtained in Example 18 was used instead of (S)-4'-((6-((1-phenylethyl)carbamoyl)-3,4-dihydroquinoline-1(2H)-yl)methyl)-[1,1'-biphenyl]-2-carboxylic acid.
$^1$H NMR (400 MHz, DMSO-$d_6$) $\delta$ 8.40 (d, $J$ = 8.0 Hz, 1H), 7.67 (s, 1H), 7.55-7.19 (m, 15H), 6.53 (d, $J$ = 8.4 Hz, 1H), 5.15 (m, 1H), 4.61 (s, 2H), 3.47 (t, $J$ = 5.2 Hz, 2H), 2.80 (t, $J$ = 5.6 Hz, 2H), 1.96 (m, 2H), 1.46 6 (d, $J$ = 6.8 Hz, 3H).

**<Example 47> (S)-1-((2'-carbamoyl-[1,1'-biphenyl]-4-yl)methyl)-N-(1-(3-(trifluoromethyl)phenyl)ethyl)-1,2,3,4-tetrahydroquinoline-6-carboxamide**

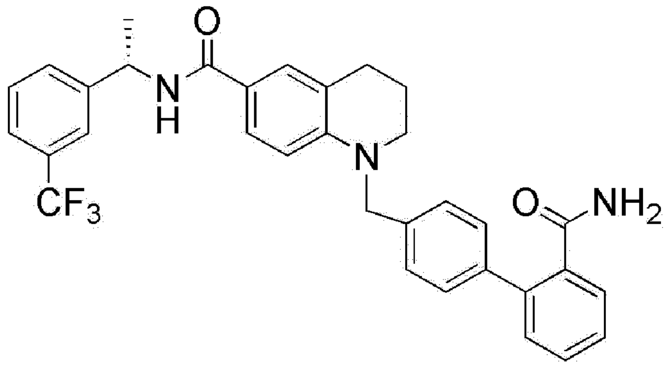

The target compound was obtained by the same manner as described in Example 2 except that (S)-4'-((6-((1-(3-(trifluoromethyl)phenyl)ethyl)carbamoyl)-3,4-dihydroquinoline-1(2H)-yl)methyl)-[1,1'-biphenyl]-2-carboxylic acid obtained in Example 19 was used instead of (S)-4'-((6-((1-phenylethyl)carbamoyl)-3,4-dihydroquinoline-1(2H)-yl)methyl)-[1,1'-biphenyl]-2-carboxylic acid.
$^1$H NMR (400 MHz, DMSO-$d_6$) $\delta$ 8.44 (d, $J$ = 8.0 Hz, 1H), 7.75-7.24 (m, 16H), 6.53 (d, $J$ = 8.8 Hz, 1H), 5.18 (m, 1H), 4.61 (s, 2H), 3.47 (t, $J$ = 5.2 Hz, 2H), 2.79 (t, $J$ = 5.6 Hz, 2H), 1.95 (m, 2H), 1.46 (d, $J$ = 7.2 Hz, 3H).

**<Example 48> 1-((2'-carbamoyl-[1,1'-biphenyl]-4-yl)methyl)-N-(pyridine-4-ylmethyl)-1,2,3,4-tetrahydroquinoline-6-carboxamide**

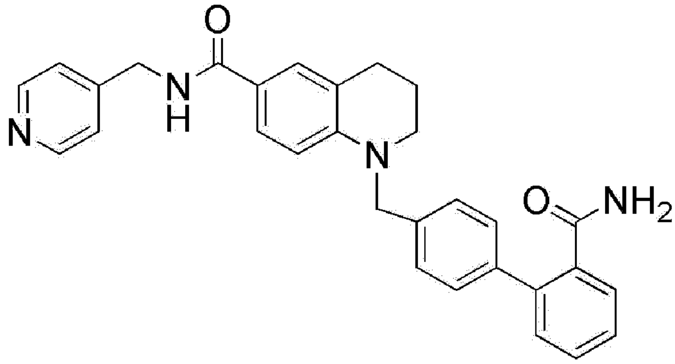

The target compound was obtained by the same manner as described in Example 2 except that 4'-((6-((pyridine-4-ylmethyl)carbamoyl)-3,4-dihydroquinoline-1(2H)-yl)methyl)-[1,1'-biphenyl]-2-carboxylic acid obtained in Example 27 was used instead of (S)-4'-((6-((1-phenylethyl)carbamoyl)-3,4-dihydroquinoline-1(2H)-yl)methyl)-[1,1'-biphenyll-2-carboxylic acid.
$^1$H NMR (400 MHz, DMSO-$d_6$) δ 8.67-8.64 (m, 1H), 8.48-8.47 (m, 2H), 7.65 (m, 1H), 7.53-7.35 (m, 8H), 7.27-7.25 (m, 5H), 6.55-6.53 (m, 1H), 4.62 (s, 2H), 4.44-4.42 (m, 2H), 3.49-3.46 (m, 2H), 2.82-2.79 (m, 2H), 1.98-1.97 (m, 2H).

**<Example 49> (S)-1-((2'-carbamoyl-[1,1'-biphenyl]-4-yl)methyl)-N-(1-(3-fluoro-4-methylphenyl)ethyl)-1,2,3,4-tetrahydroquinoline-6-carboxamide**

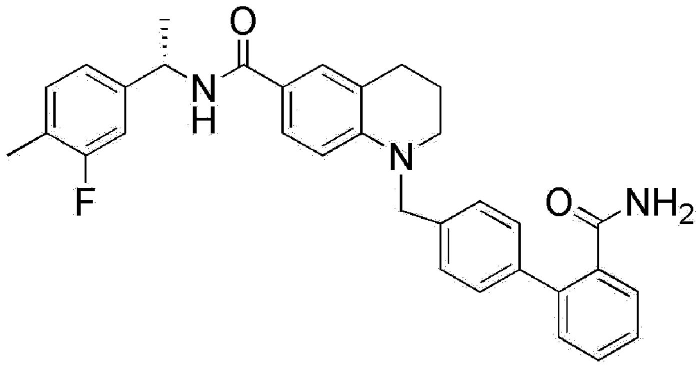

The target compound was obtained by the same manner as described in Example 2 except that (S)-4'-((6-((1-(3-fluoro-4-methyl)phenyl)ethyl)carbamoyl)-3,4-dihydroquinoline-1(2H)-yl)methyl)-[1,1'-biphenyl]-2-carboxylic acid obtained in Example 5 was used instead of (S)-4'-((6-((1-phenylethyl)carbamoyl)-3,4-dihydroquinoline-1(2H)-yl)methyl)-[1,1'-biphenyl]-2-carboxylic acid.
$^1$H NMR (400 MHz, $CDCl_3$) δ 7.74 (d, $J$ = 8.0 Hz, 1H), 7.59-7.25 (m, 9H), 7.13 (d, $J$ = 8.0 Hz, 1H), 7.03 (t, $J$ = 8.0 Hz, 1H), 6.46 (d, $J$ = 7.6 Hz, 1H), 6.19 (d, $J$ = 7.6 Hz, 1H), 5.66 (s, 1H), 5.39 (s, 1H), 5.26 (m, 1H), 4.58 (s, 2H), 3.49 (t, $J$ = 5.2 Hz, 2H), 2.86 (t, $J$ = 6.0 Hz, 2H), 2.24 (s, 3H), 2.05 (m, 2H), 1.54 (d, $J$ = 6.8 Hz, 3H).

**<Example 50> N-benzyl-1-((2'-carbamoyl-[1,1'-biphenyl]-4-yl)methyl)-1,2,3,4-tetrahydroquinoline-6-carboxamide**

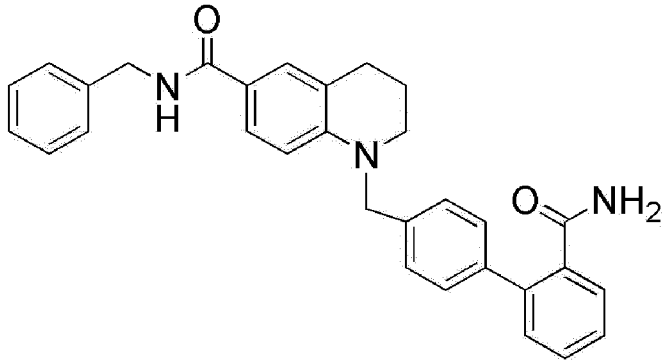

The target compound was obtained by the same manner as described in Example 2 except that 4'-((6-(benzylcarbamoyl)-3,4-dihydroquinoline-1(2H)-yl)methyl)-[1,1'-biphenyl]-2-carboxylic acid obtained in Example 28 was used instead of (S)-4'-((6-((1-phenylethyl)carbamoyl)-3,4-dihydroquinoline-1(2H)-yl)methyl)-[1,1'-biphenyll-2-carboxylic acid.
$^1$H NMR (400 MHz, $CDCl_3$) δ 7.53-7.49 (m, 2H), 7.45-7.41 (m, 4H), 7.37-7.35 (m, 5H), 7.33-7.28 (m, 3H), 6.49-6.47 (m, 1H), 6.23-6.21 (m, NH, 1H), 5.49 (br, NH, 1H), 5.32 (br, NH, 1H), 4.64-4.63 (m, 2H), 4.59 (s, 2H), 3.59-3.47 (m, 2H), 2.89-2.85 (m, 2H), 2.09-2.03 (m, 2H).

**<Example 51> N-(2-bromobenzyl)-1-((2'-carbamoyl-[1,1'-biphenyl]-4-yl)methyl)-1,2,3,4-tetrahydroquinoline-6-carboxamide**

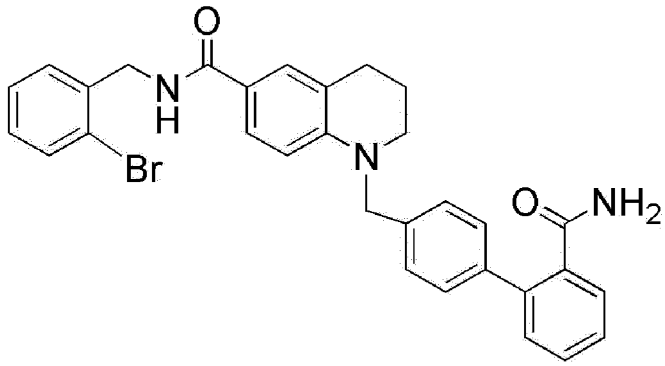

The target compound was obtained by the same manner as described in Example 2 except that 4'-((7-((2-bromobenzyl)carbamoyl)-2H-benzo[b][1,4]oxazine-4(3H)-yl)methyl)-[1,1'-biphenyl]-2-carboxylic acid obtained in Example 29 was used instead of (S)-4'-((6-((1-phenylethyl)carbamoyl)-3,4-dihydroquinoline-1(2H)-yl)methyl)-[1,1'-biphenyll-2-carboxylic acid.
$^1$H NMR (400 MHz, $CDCl_3$) δ 7.78-7.76 (m, 1H), 7.57-7.55 (m, 1H), 7.54-7.35 (m, 8H), 7.31-7.29 (m, 3H), 7.17-7.13 (m, 1H), 6.49-6.47 (m, 1H), 6.47-6.46 (m, NH, 1H), 5.50 (br, NH, 1H), 5.32 (br, NH, 1H), 4.70-4.69 (m, 2H), 4.60 (s, 2H), 3.50-3.48 (m, 2H), 2.89-2.86 (m, 2H), 2.09-2.03 (m, 2H).

**<Example 52> 1-((2'-carbamoyl-[1,1'-biphenyl]-4-yl)methyl)-N-((2,3-dihydrobenzo[b][1,4]dioxin-6-yl)methyl)-1,2,3,4-tetrahydroquinoline-6-carboxamide**

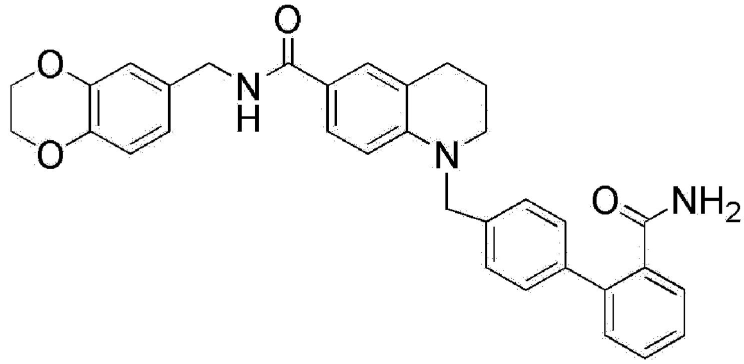

The target compound was obtained by the same manner as described in Example 2 except that 4'-((6-(((2,3-dihydrobenzo[b][1,4]dioxin-6-yl)methyl)carbamoyl)-3,4-dihydroquinoline-1(2H)-yl)methyl)-[1,1'-biphenyl]-2-carboxylic acid obtained in Example 30 was used instead of (S)-4'-((6-((1-phenylethyl)carbamoyl)-3,4-dihydroquinoline-1(2H)-yl)methyl)-[1,1'-biphenyll-2-carboxylic acid.
$^1$H NMR (400 MHz, $CDCl_3$) δ 7.78-7.76 (m, 1H), 7.53-7.48 (m, 2H), 7.46-7.41 (m, 4H), 7.37-7.35 (m, 1H), 7.30-7.28 (m, 2H), 6.86 (m, 1H), 6.83 (m, 2H), 6.14-6.12 (m, NH, 1H), 5.46 (br, NH, 1H), 5.31 (br, NH, 1H), 4.59 (s, 2H), 4.52-4.51 (m, 2H), 4.26 (s, 4H), 3.50-3.47 (m, 2H), 2.88-2.85 (m, 2H), 2.09-2.03 (m, 2H).

**<Example 53> N-(4-bromobenzyl)-1-((2'-carbamoyl-[1,1'-biphenyl]-4-yl)methyl)-1,2,3,4-tetrahydroquinoline-6-carboxamide**

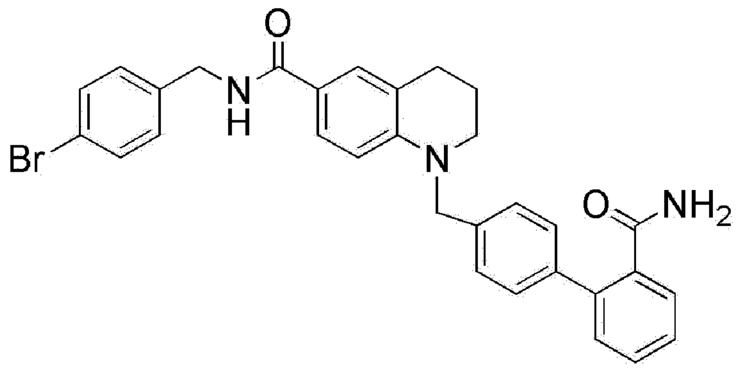

The target compound was obtained by the same manner as described in Example 2 except that 4'-((6-((4-bromobenzyl)carbamoyl)-3,4-dihydroquinoline-1(2H)-**yl)methyl)-[1,1'-biphenyl]-2-carboxylic** acid obtained in Example 31 was used instead of (S)-4'-((6-((1-phenylethyl)carbamoyl)-3,4-dihydroquinoline-1(2H)-yl)methyl)-[1,1'-biphenyll-2-carboxylic acid.
$^1$H NMR (400 MHz, $CDCl_3$) δ 7.78-7.76 (m, 1H), 7.54-7.36 (m, 10H), 7.30-7.22 (m, 3H), 6.49 (d, *J* = 8.8 Hz, 1H), 6.23 (t, *J* = 5.6 Hz, 1H), 5.44 (s, 1H), 5.32 (s, 1H), 4.60-4.58 (m, 4H) 4.50 (t, *J* = 5.6 Hz, 2H), 2.88 (t, *J* = 6.0 Hz, 2H), 2.10-2.04 (m, 2H).

**<Example 54> 1-((2'-carbamoyl-[1,1'-biphenyl]-4-yl)methyl)-N-(3-(trifluoromethoxy)benzyl)-1,2,3,4-tetrahydroquinoline-6-carboxamide**

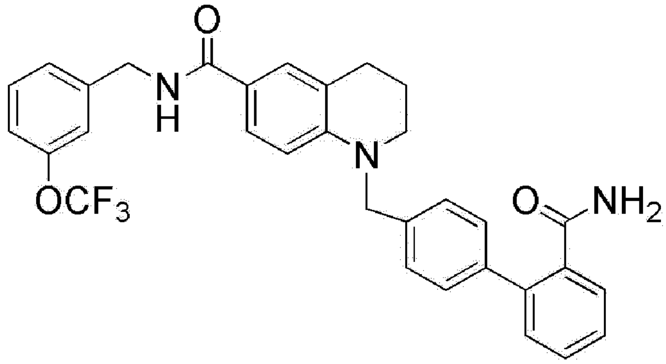

The target compound was obtained by the same manner as described in Example 2 except that 4'-((6-((3-(trifluoromethoxy)benzyl)carbamoyl)-3,4-dihydroquinoline-1(2H)-yl)methyl)-[1,1'-biphenyl]-2-carboxylic acid obtained in Example 32 was used instead of (S)-4'-((6-((1-phenylethyl)carbamoyl)-3,4-dihydroquinoline-1(2H)-yl)methyl)-[1,1'-biphenyl]-2-carboxylic acid.
$^1$H NMR (400 MHz, $CDCl_3$) δ 7.78-7.76 (m, 1H), 7.54-7.30 (m, 11H), 7.20 (s, 1H), 7.15-7.13 (m, 1H), 6.50 (d, *J* = 8.4 Hz, 1H), 6.28 (t, *J* = 5.6 Hz, 1H), 5.42 (s, 1H), 5.31 (s, 1H), 4.66 (d, *J* = 5.6 Hz, 1H), 4.61 (s, 2H), 3.50 (t, *J* = 5.6 Hz, 2H), 2.89 (t, *J* = 6.0 Hz, 2H), 2.11-2.06 (m, 2H).

**<Example 55> sodium (S)-4'-((6-((1-phenylethyl)carbamoyl)-3,4-dihydroquinoline-1(2H)-yl)methyl)-[1,1'-biphenyl]-2-carboxylate**

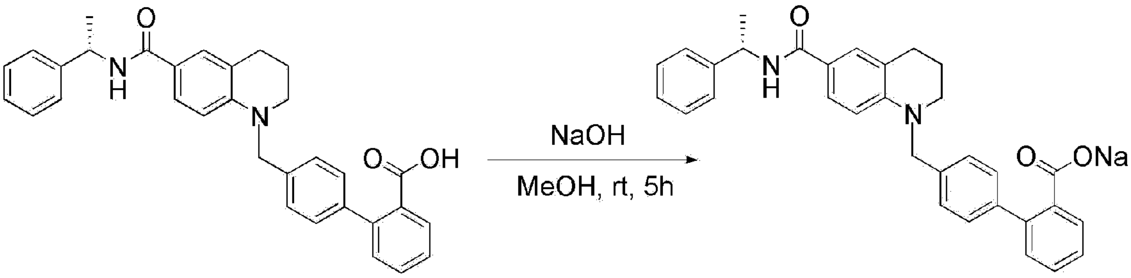


(S)-4'-((6-((1-phenylethyl)carbamoyl)-3,4-dihydroquinoline-1(2H)-yl)methyl)-[1,1'-biphenyl]-2-carboxylic acid (300 0 mg, 0.61 mmol) obtained in Example 2 was dissolved in MeOH (10 ml) in a 25 mL flask, to which NaOH (24.4 mg, 0.61 mmol) was added, followed by stirring at room temperature for 5 hours. Upon completion of the reaction, the reaction mixture was concentrated under reduced pressure. As a result, sodium (S)-4'-((6-((1-phenylethyl)carbamoyl)-3,4-dihydroquinoline-1(2H)-yl)methyl)-[1,1'-biphenyl]-2-carboxylate was obtained (300 mg).
$^1$H NMR (400 MHz, DMSO-$d_6$) δ 8.33 (d, *J* = 8.0 Hz, 1H), 7.68 (d, *J* = 8.0 Hz, 1H), 7.60-7.46 (m, 3H), 7.43 (m, 1H), 7.42-7.25 (m, 9H), 7.19 (m, 1H), 6.52 (m, 1H), 5.12 (m, 1H), 4.69 (s, 2H), 3.49 (t, *J* = 4.0 Hz, 2H), 2.81 (t, *J* = 4.0 Hz, 2H), 1.95 (m, 2H), 1.42 (d, *J* = 7.2 Hz, 3H).

**<Example 56> sodium (S)-4'-((6-((1-(3-fluoro-4-methylphenyl)ethyl)carbamoyl)-3,4-dihydroquinoline-1(2H)-yl)methyl)-[1,1'-biphenyl]-2-carboxylate**

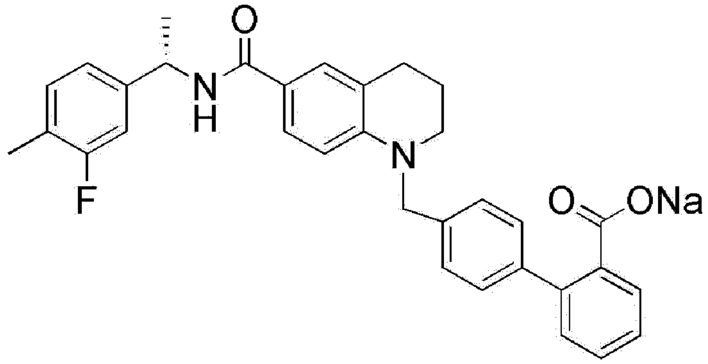

The target compound was obtained by the same manner as described in Example 55 except that (S)-4'-((6-((1-(3-fluoro-4-methylphenyl)ethyl)carbamoyl)-3,4-dihydroquinoline-1(2H)-yl)methyl)-[1,1'-biphenyl]-2-carboxylic acid obtained in Example 5 was used instead of (S)-4'-((6-((1-phenylethyl)carbamoyl)-3,4-dihydroquinoline-1(2H)-yl)methyl)-[1,1'-biphenyl]-2-carboxylic acid.
$^1$H NMR (400 MHz, DMSO-$d_6$) δ 8.33 (d, *J* = 8.0 Hz, 1H), 7.50-7.42 (m, 4H), 7.30(m, 1H) 7.35-7.05 (m, 8H), 6.53 (d, *J* = 8.8 Hz, 1H), 5.08 (m, 1H), 4.57 (s, 2H), 3.46 (t, *J* = 5.2 Hz, 2H), 2.79 (t, *J* = 6.0 Hz, 2H), 2.18 (s, 3H), 1.95 (m, 2H), 1.40 (d, *J* = 7.2 Hz, 3H).

**<Example 57> sodium (S)-4'-((6-((1-(4-cyanophenyl)ethyl)carbamoyl)-3,4-dihydroquinoline-1(2H)-yl)methyl)-[1,1'-biphenyl]-2-carboxylate**

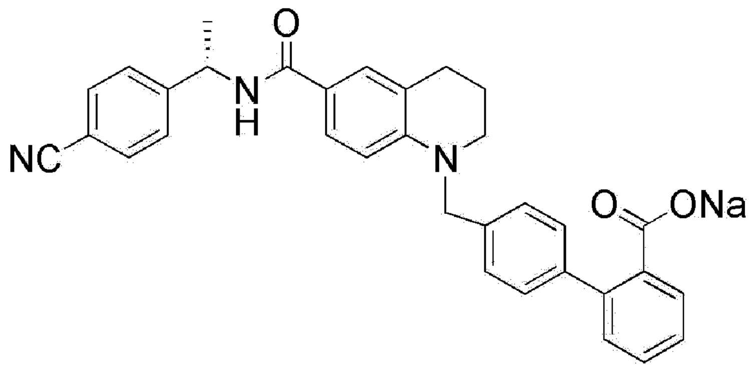

The target compound was obtained by the same manner as described in Example 55 except that (S)-4'-((6-((1-(4-cyanophenyl)ethyl)carbamoyl)-3,4-dihydroquinoline-1(2H)-yl)methyl)-[1,1'-biphenyl]-2-carboxylic acid obtained in Example 14 was used instead of (S)-4'-((6-((1-phenylethyl)carbamoyl)-3,4-dihydroquinoline-1(2H)-yl)methyl)-[1,1'-biphenyl]-2-carboxylic acid.
$^1$H NMR (400 MHz, DMSO-$d_6$) δ 8.47 (d, *J* = 6.8 Hz, 1H), 7.77 (d, *J* = 8.0 Hz, 2H), 7.60-7.42 (m, 6H), 7.30-7.13 (m, 6H), 6.53 (d, *J* = 9.2 Hz, 1H), 5.14 (m, 1H), 4.58 (s, 2H), 3.47 (t, *J* = 5.2 Hz, 2H), 2.79 (t, *J* = 5.6 Hz, 2H), 1.95 (m, 2H), 1.43 (d, *J* = 6.8 Hz, 3H).

**<Example 58> (S)-4'-((7-((1-phenylethyl)carbamoyl)-2H-benzo[b][1,4]oxazine-4(3H)-yl)methyl)-[1,1'-biphenyl]-2-carboxylic** acid

Step 1 : (S) -tert-butyl 4'-((7-((1-phenylethyl)carbamoyl)-2H-benzo[b][1,4]oxazine-4(3H)-yl)methyl)-[1,1'-biphenyl]-2-carboxylate

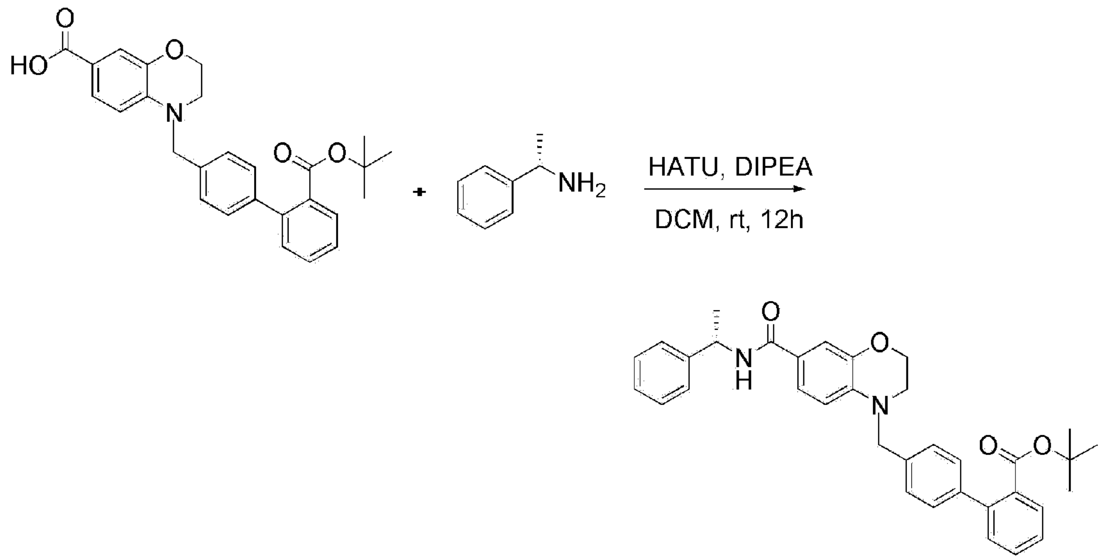


4-((2'-(tert-butoxycarbonyl)-[1,1'-biphenyl]-4-yl)methyl)-3,4-dihydro-2H-benzo[b][1,4]oxazine-7-carboxylic acid (300 mg, 0.67 mmol) obtained in Preparative Example 2 was dissolved in $CH_2Cl_2$ (10 ml) in a 100 mL flask, to which HATU (280 mg, 0.74 mmol) and DIPEA (350 ul, 2.01 mmol) were added, followed by stirring. (S)-1-phenylethylamine (90 mg, 0.74 mmol) was added thereto, followed by stirring at room temperature for 12 hours. Upon completion of the reaction, the organic layer was separated by using $CH_2Cl_2$ and $H_2O$. The separated organic layer was dried over $MgSO_4$ and filtered. The mixture was separated by column chromatography (EA/*n*-Hex = 1:1) to give(S)-tert-butyl 4'-((7-((1-phenylethyl)carbamoyl)-2H-benzo[b][1,4]oxazine-4(3H)-yl)methyl)-[1,1'-biphenyl]-2-carboxylate (341 mg, 0.62 mmol, 93 %).
$^1$H NMR (400 MHz, $CDCl_3$) $\delta$ 7.80 (d, *J* = 8.0 Hz, 1H), 7.61-7.23 (m, 15H), 6.68 (d, *J* = 8.4 Hz, 1H), 6.14 (d, *J* = 7.6 Hz, 1H), 5.34 (m, 1H), 4.59 (s, 2H), 4.30 (t, *J* = 4.4 Hz, 2H), 3.52 (t, *J* = 4.4 Hz, 2H), 1.54 (d, *J* = 7.2 Hz, 3H).

Step 2 : (S)-4'-((7-((1-phenylethyl)carbamoyl)-2H-benzo[b][1,4]oxazine-4(3H)-yl)methyl)-[1,1'-biphenyl]-2-carboxylic acid

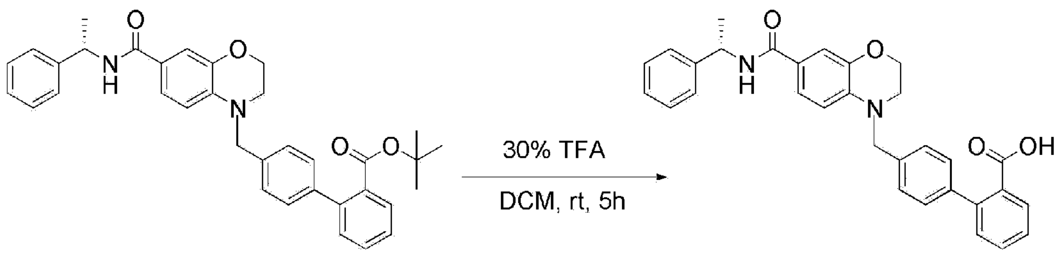


(S)-tert-butyl 4'-((7-((1-phenylethyl)carbamoyl)-2H-benzo[b][1,4]oxazine-4(3H)-yl)methyl)-[1,1'-biphenyl]-2-carboxylate (341 mg, 0.62 mmol) obtained in step 1 was dissolved in $CH_2Cl_2$ (20 ml) in a 50 mL flask, to which 30% TFA (9 ml) was added, followed by stirring at room temperature for 5 hours. Upon completion of the reaction, the reaction mixture was concentrated under reduced pressure. The mixture was separated by column chromatography (EA) to give (S)-4'-((7-((1-phenylethyl)carbamoyl)-2H-benzo[b][1,4]oxazine-4(3H)-yl)methyl)-[1,1'-biphenyl]-2-carboxylic acid (156 mg, 0.31 mmol, 51 %).
$^1$H NMR (400 MHz, DMSO-$d_6$) $\delta$ 12.66 (br, OH, 1H), 8.39 (d, *J* = 8.0 Hz, 1H), 7.70 (d, *J* = 7.2 Hz, 1H), 7.61-7.18 (m, 14H), 6.73 (d, *J* = 8.4 Hz, 1H), 5.11 (m, 1H), 4.63 (s, 2H), 4.25 (t, *J* = 4.0 Hz, 2H), 3.51 (t, *J* = 4.0 Hz, 2H), 1.43 (d, *J* = 7.2 Hz, 3H).

**<Example 59> (S)-4-((2'-carbamoyl-[1,1'-biphenyl]-4-yl)methyl)-N-(1-phenylethyl)-3,4-dihydro-2H-benzo[b][1,4]oxazine-7-carboxamide**

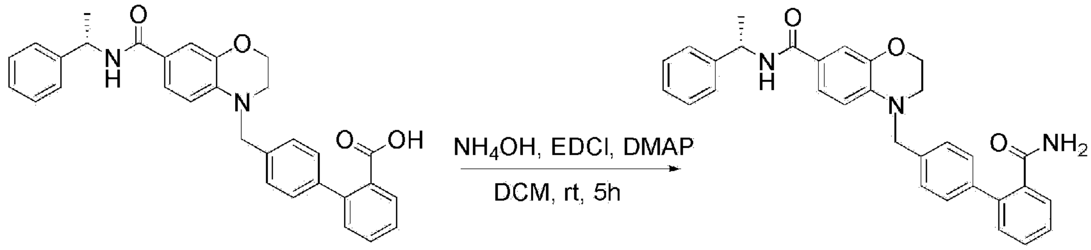


1-((2'-(tert-butoxycarbonyl)-[1,1'-biphenyl]-4-yl)methyl)-1,2,3,4-tetrahydroquinoline-6-carboxylic acid (300 mg, 0.67 mmol) obtained in Example 57 was dissolved in $CH_2Cl_2$ (2 ml) in a 25 mL flask, to which EDCI (38 mg, 0.2 mmol) and DMAP (38 mg, 0.3 mmol) were added, followed by stirring. Ammonium hydroxide solution (2 ml) was added thereto, followed by stirring at room temperature for 5 hours. Upon completion of the reaction, the organic layer was separated by using $CH_2Cl_2$ and $H_2O$. The separated organic layer was dried over $MgSO_4$ and filtered. The mixture was separated by column chromatography (MeOH/*n*-Hex/$CH_2Cl_2$ = 0.5:0.5:9) to give (S)-4-((2'-carbamoyl-[1,1'-biphenyl]-4-yl)methyl)-N-(1-phenylethyl)-3,4-dihydro-2H-benzo[b][1,4]oxazine-7-carboxamide (23 mg, 0.04 mmol, 44 %).
$^1$H NMR (400 MHz, $CDCl_3$) δ 7.74 (d, *J* = 7.6 Hz, 1H), 7.51 (td, *J* = 1.2, 7.6 Hz, 1H), 7.47-7.24 (m, 13H), 6.63 (d, *J* = 9.2 Hz, 1H), 6.20 (d, *J* = 8.0 Hz, 1H), 5.61 (s, 1H), 5.39 (s, 1H), 5.32 (m, 1H), 4.57 (s, 2H), 4.30 (t, *J* = 4.4 Hz, 2H), 3.50 (t, *J* = 4.4 Hz, 2H), 1.48 (d, *J* = 6.8 Hz, 3H).

**<Example 60> (S)-4'-((7-((1-(4-nitrophenyl)ethyl)carbamoyl)-2H-benzo[b][1,4]oxazine-4(3H)-yl)methyl)-[1,1'-biphenyl]-2-carboxylic acid**

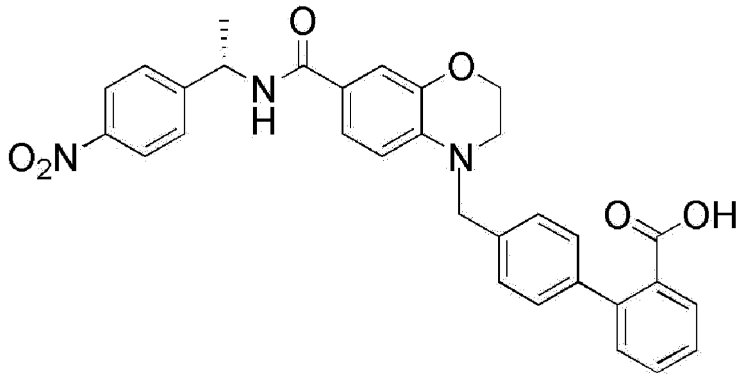

The target compound was obtained by the same manner as described in Example 58 except that (S)-1-(4-nitrophenyl)ethaneamine was used instead of (S)-1-phenylethylamine.
$^1$H NMR (400 MHz, DMSO-$d_6$) δ 12.76 (br, OH, 1H), 8.57 (d, *J* = 7.6 Hz, 1H), 8.16 (d, *J* = 8.8 Hz, 2H), 7.70 (m, 1H), 7.69-7.52 (m, 3H), 7.47-7.28 (m, 8H), 6.74 (d, *J* = 8.0 Hz, 1H), 5.19 (m, 1H), 4.64 (s, 2H), 4.26 (t, *J* = 4.0 Hz, 2H), 3.52 (t, *J* = 4.0 Hz, 2H), 1.47 (d, *J* = 7.2 Hz, 3H).

**<Example 61> (S)-4'-((7-((1-(4-bromophenyl)ethyl)carbamoyl)-2H-benzo[b][1,4]oxazine-4(3H)-yl)methyl)-[1,1'-biphenyl]-2-carboxylic acid**

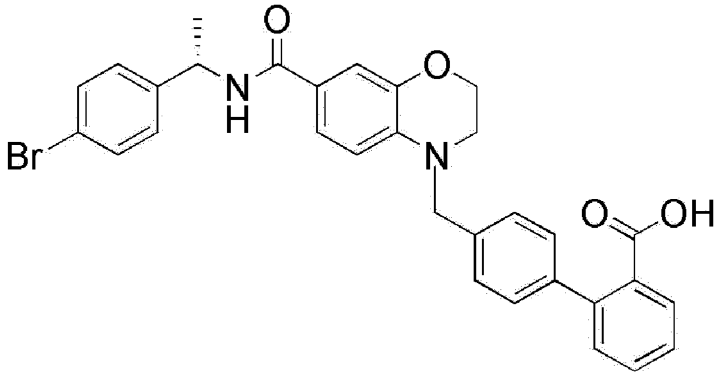

The target compound was obtained by the same manner as described in Example 58 except that (S)-1-(4-bromophenyl)ethaneamine was used instead of (S)-1-phenylethylamine.
$^1$H NMR (400 MHz, DMSO-$d_6$) δ 12.76 (br, OH, 1H), 8.43 (d, *J* = 7.6 Hz, 1H), 7.70 (d, *J* = 7.6 Hz, 1H), 7.58-7.41 (m, 4H), 7.39-7.28 (m, 9H), 6.73 (d, *J* = 8.4 Hz, 1H), 5.08 (m, 1H), 4.63 (s, 2H), 4.25 (t, *J* = 4.0 Hz, 2H), 3.52 (t, *J* = 4.0 Hz, 2H), 1.42 (d, *J* = 7.2 Hz, 3H).

**<Example 62> (S)-4'-((7-((1-phenylpropyl)carbamoyl)-2H-benzo[b][1,4]oxazine-4(3H)-yl)methyl)-[1,1'-biphenyl]-2-carboxylic acid**

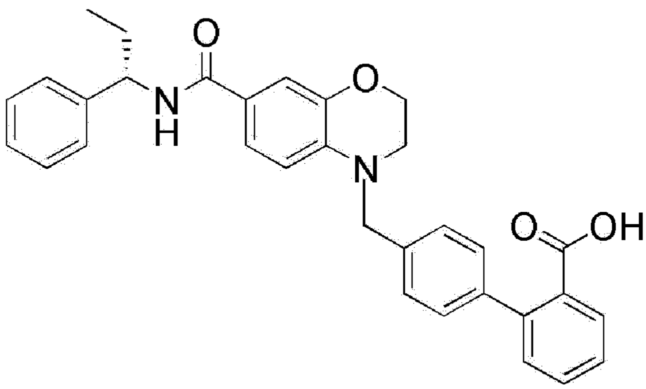

The target compound was obtained by the same manner as described in Example 58 except that (S)-1-phenylpropane-1-amine was used instead of (S)-1-phenylethylamine.
$^1$H NMR (400 MHz, DMSO-$d_6$) δ 12.76 (br, OH, 1H), 8.34 (d, *J* = 8.4 Hz, 1H), 7.70 (dd, *J* = 1.2, 8.0 Hz, 1H), 7.54 (m, 1H), 7.47-7.19 (m, 12H), 6.53 (d, *J* = 8.4 Hz, 1H), 4.85 (q, *J* = 8.4 Hz, 1H), 4.63 (s, 2H), 4.25 (t, *J* = 4.0 Hz, 2H), 3.53 (t, *J* = 4.0 Hz, 2H), 1.78 (m, 2H), 0.87 (t, *J* = 7.2 Hz, 3H).

**<Example 63> (S)-4'-((7-((1-(4-fluorophenyl)ethyl)carbamoyl)-2H-benzo[b][1,4]oxazine-4(3H)-yl)methyl)-[1,1'-biphenyl]-2-carboxylic acid**

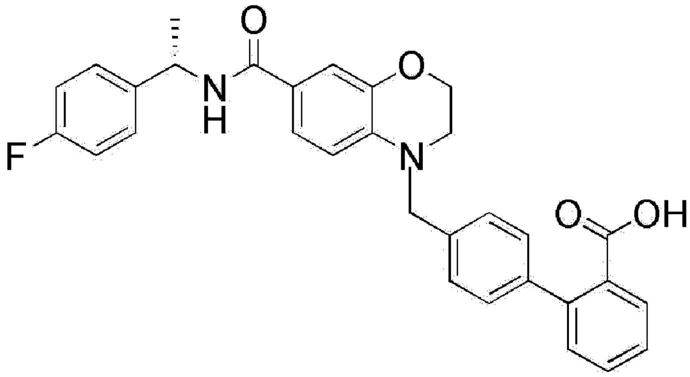

The target compound was obtained by the same manner as described in Example 58 except that (S)-1-(4-fluorophenyl)ethaneamine was used instead of (S)-1-phenylethylamine.
$^1$H NMR (400 MHz, DMSO-$d_6$) δ 12.86 (br, OH, 1H), 8.42 (d, *J* = 8.0 Hz, 1H), 7.72 (dd, *J* = 1.2, 8.0 Hz, 1H), 7.65-7.28 (m, 13H), 6.74 (d, *J* = 8.4 Hz, 1H), 5.13 (m, 1H), 4.63 (s, 2H), 4.28 (t, *J* = 4.0 Hz, 2H), 3.58 (t, *J* = 4.0 Hz, 2H), 1.42 (d, *J* = 7.2 Hz, 3H).

**<Example 64> (S)-4'-((7-((1-(4-chlorophenyl)ethyl)carbamoyl)-2H-benzo[b][1,4]oxazine-4(3H)-yl)methyl)-[1,1'-biphenyl]-2-carboxylic acid**

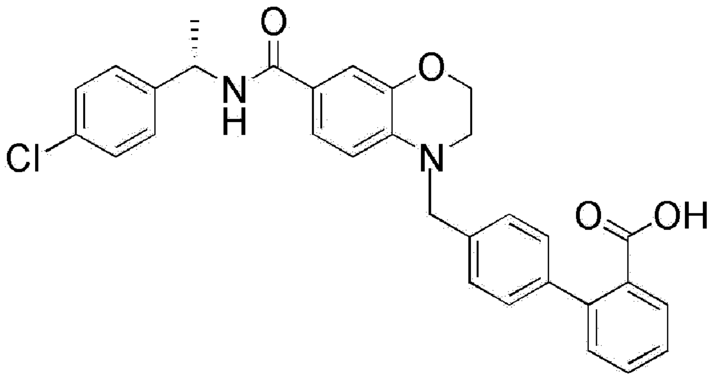

The target compound was obtained by the same manner as described in Example 58 except that (S)-1-(4-chlorophenyl)ethaneamine was used instead of (S)-1-phenylethylamine.
$^1$H NMR (400 MHz, DMSO-$d_6$) δ 12.76 (br, OH, 1H), 8.46 (d, *J* = 7.6 Hz, 1H), 7.72 (m, 1H), 7.55 (m, 1H), 7.47-7.28 (m, 12H), 6.74 (d, *J* = 8.0 Hz, 1H), 5.09 (m, 1H), 4.63 (s, 2H), 4.26 (t, *J* = 4.0 Hz, 2H), 3.52 (t, *J* = 4.0 Hz, 2H), 1.42 (d, *J* = 7.2 Hz, 3H).

**<Example 65> (S)-4'-((7-((1-(3-chlorophenyl)ethyl)carbamoyl)-2H-benzo[b][1,4]oxazine-4(3H)-yl)methyl)-[1,1'-biphenyl]-2-carboxylic acid**

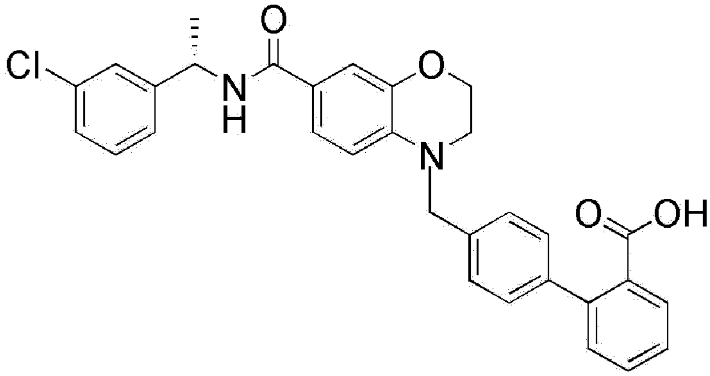

The target compound was obtained by the same manner as described in Example 58 except that (S)-1-(3-chlorophenyl)ethaneamine was used instead of (S)-1-phenylethylamine.
$^1$H NMR (400 MHz, DMSO-$d_6$) δ 12.86 (br, OH, 1H), 8.45 (d, *J* = 8.0 Hz, 1H), 7.72 (m, 1H), 7.56 (m, 1H), 7.47-7.28 (m, 12H), 6.74 (d, *J* = 8.0 Hz, 1H), 5.09 (m, 1H), 4.63 (s, 2H), 4.26 (t, *J* = 4.0 Hz, 2H), 3.52 (t, *J* = 4.0 Hz, 2H), 1.42 (d, *J* = 7.2 Hz, 3H).

**<Example 66> 4'-((7-((4-nitrobenzyl)carbamoyl)-2H-benzo[b][1,4]oxazine-4(3H)-yl)methyl)-[1,1'-biphenyl]-2-carboxylic acid**

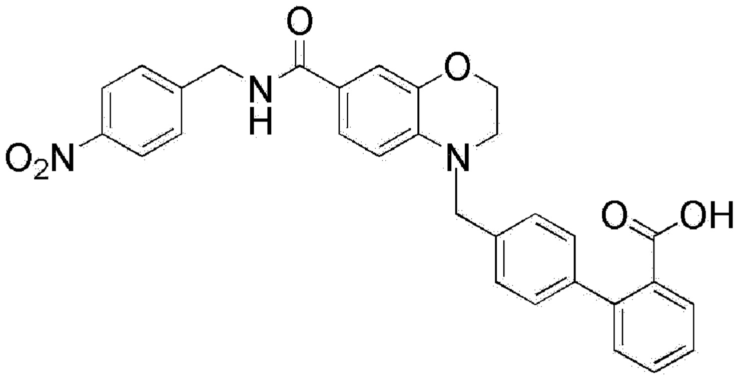

The target compound was obtained by the same manner as described in Example 58 except that (4-nitrophenyl)methaneamine was used instead of (S)-1-phenylethylamine.
$^1$H NMR (400 MHz, DMSO-$d_6$) δ 12.83 (br, OH, 1H), 8.75 (m,1H), 8.19 (dd, *J* = 1.2, 8.8 Hz, 1H), 7.71-7.19 (m, 12H), 6.73 (d, *J* = 8.4 Hz, 1H), 4.63 (s, 2H), 4.52 (d, *J* = 6.0 Hz, 1H), 4.25 (t, *J* = 4.0 Hz, 2H), 3.53 (t, *J* = 4.0 Hz, 2H).

**<Example 67> 4'-((7-(benzylcarbamoyl)-2H-benzo[b][1,4]oxazine-4(3H)-yl)methyl)-[1,1'-biphenyl]-2-carboxylic acid**

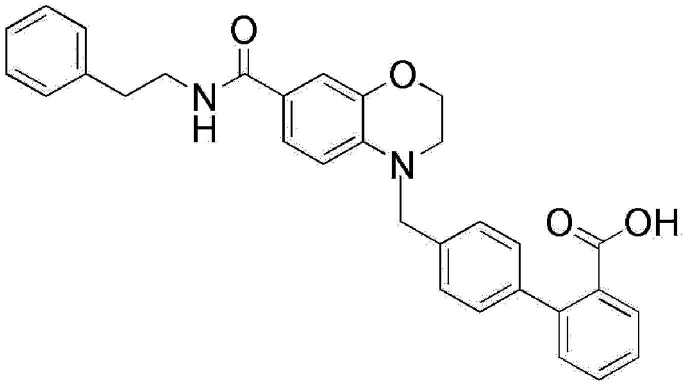

The target compound was obtained by the same manner as described in Example 58 except that 2-phenylethaneamine was used instead of (S)-1-phenylethylamine.
$^1$H NMR (400 MHz, DMSO-$d_6$) δ 12.63 (br, OH, 1H), 8.17 (t, *J* = 5.6 Hz, 1H), 7.71 (dd, *J* = 1.2, 8.8 Hz, 1H), 7.58-7.19 (m, 14H), 6.72 (d, *J* = 8.4 Hz, 1H), 4.62 (s, 2H), 4.25 (t, *J* = 4.0 Hz, 1H), 4.52 (t, *J* = 4.0 Hz, 2H), 3.40 (m, 2H), 2.74 (t, *J* = 7.6 Hz, 2H).

**<Example 69> 4'-((7-((furan-2-ylmethyl)carbamoyl)-2H-benzo[b][1,4]oxazine-4(3H)-yl)methyl)-[1,1'-biphenyl]-2-carboxylic acid**

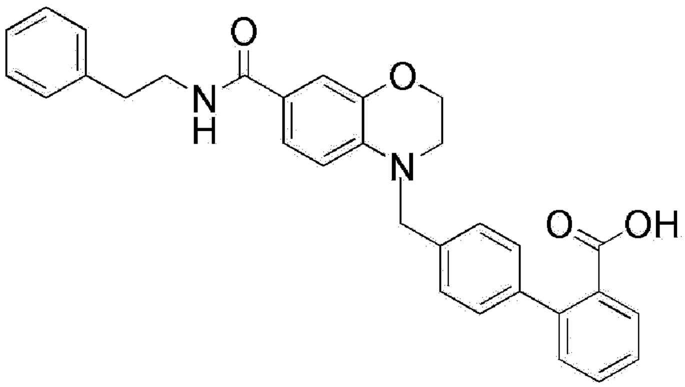

The target compound was obtained by the same manner as described in Example 58 except that 2-phenylethaneamine was used instead of (S)-1-phenylethylamine.
$^1$H NMR (400 MHz, DMSO-$d_6$) δ 12.63 (br, OH, 1H), 8.17 (t, *J* = 5.6 Hz, 1H), 7.71 (dd, *J* = 1.2, 8.8 Hz, 1H), 7.58-7.19 (m, 14H), 6.72 (d, *J* = 8.4 Hz, 1H), 4.62 (s, 2H), 4.25 (t, *J* = 4.0 Hz, 1H), 4.52 (t, *J* = 4.0 Hz, 2H), 3.40 (m, 2H), 2.74 (t, *J* = 7.6 Hz, 2H).

**<Example 69> 4'-((7-((furan-2-ylmethyl)carbamoyl)-2H-benzo[b][1,4]oxazine-4(3H)-yl)methyl)-[1,1'-biphenyl]-2-carboxylic acid의 제조**

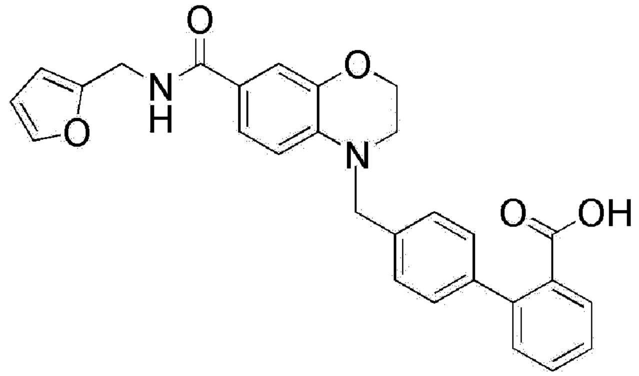

The target compound was obtained by the same manner as described in Example 58 except that furan-2-ylmethaneamine was used instead of (S)-1-phenylethylamine.
$^1$H NMR (400 MHz, DMSO-$d_6$) δ 12.88 (br, OH, 1H), 8.56 (t, *J* = 5.6 Hz, 1H), 7.73 (dd, *J* = 1.2, 8.8 Hz, 1H), 7.58-7.19 (m, 10H), 6.72 (d, *J* = 8.8 Hz, 1H), 6.37 (m, 1H), 6.21 (m, 1H), 4.62 (s, 2H), 4.41 (d, *J* = 6.0 Hz, 1H), 4.25 (t, *J* = 4.0 Hz, 2H), 3.51 (t, *J* = 4.0 Hz, 2H).

**<Example 70> (S)-4'-((7-((1-(naphthalene-1-yl)ethyl)carbamoyl)-2H-benzo[b][1,4]oxazine-4(3H)-yl)methyl)-[1,1'-biphenyl]-2-carboxylic acid**

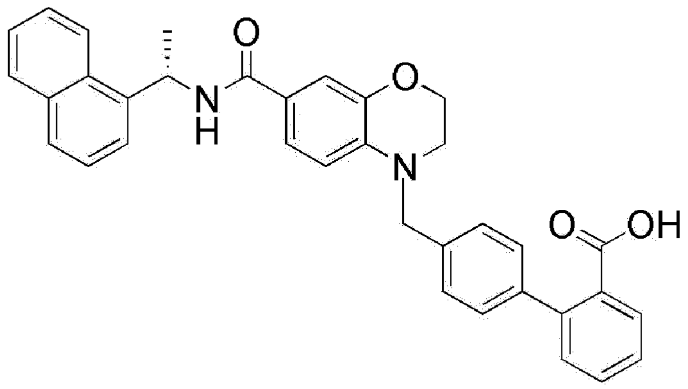

The target compound was obtained by the same manner as described in Example 58 except that (S)-1-(naphthalene-1-yl)ethaneamine was used instead of (S)-1-phenylethylamine.
$^1$H NMR (400 MHz, DMSO-$d_6$) δ 12.76 (br, OH, 1H), 8.57 (d, *J* = 8.4 Hz, 1H), 8.18 (d, *J* = 8.4 Hz, 1H), 7.93 (m, 1H), 7.81 (d, *J* = 8.4 Hz, 1H), 7.70 (dd, *J* = 1.2, 7.6 Hz, 1H), 7.62-7.25 (m, 13H), 6.70 (d, *J* = 8.8 Hz, 1H), 5.91 (m, 1H), 4.62 (s, 2H), 4.25 (t, *J* = 4.0 Hz, 2H), 3.51 (t, *J* = 4.0 Hz, 2H), 1.57 (d, *J* = 6.8 Hz, 1H).

**<Example 71> 4'-((7-((4-(methoxycarbonyl)benzyl)carbamoyl)-2H-benzo[b][1,4]oxazine-4(3H)-yl)methyl)-[1,1'-biphenyl]-2-carboxylic acid**

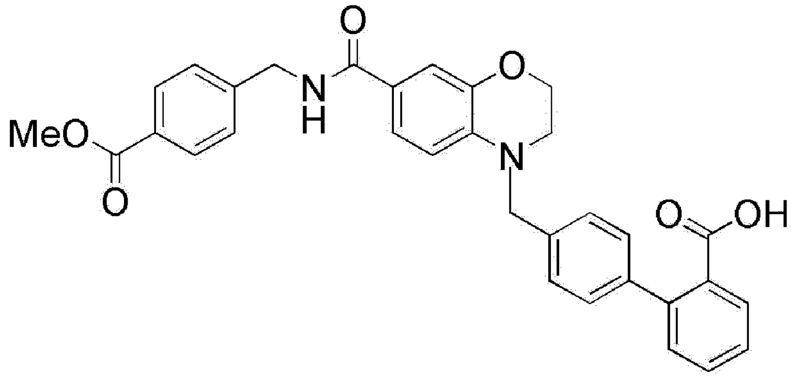

The target compound was obtained by the same manner as described in Example 58 except that methyl 4-(aminomethyl)benzoate was used instead of (S)-1-phenylethylamine.
$^1$H NMR (400 MHz, DMSO-$d_6$) δ12.86 (br, OH, 1H), 8.75 (t, *J* = 6.0 Hz, 1H), 7.91 (d, *J* = 8.0 Hz, 1H), 7.91 (m, 3H), 7.70 (d, *J* = 7.6 Hz, 1H), 7.56 (t, *J* = 7.6 Hz, 1H), 7.47-7.19 (m, 11H), 6.74 (d, *J* = 8.8 Hz, 1H), 4.63 (s, 2H), 4.49 (d, *J* = 5.2 Hz, 1H), 4.25 (t, *J* = 4.0 Hz, 2H), 3.83 (s, 3H), 3.51 (t, *J* = 4.0 Hz, 2H).

**<Example 72> 4'-((7-((3-methoxybenzyl)carbamoyl)-2H-benzo[b][1,4]oxazine-4(3H)-yl)methyl)-[1,1'-biphenyl]-2-carboxylic acid**

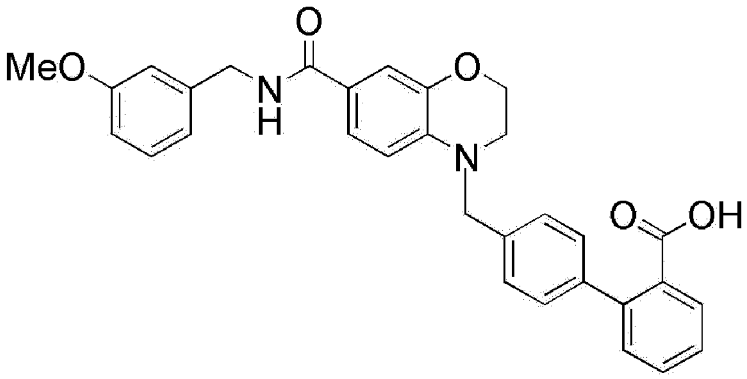

The target compound was obtained by the same manner as described in Example 58 except that (3-methoxyphenyl)methaneamine was used instead of (S)-1-phenylethylamine.
$^1$H NMR (400 MHz, DMSO-$d_6$) δ 12.76 (br, OH, 1H), 7.70 (dd, *J* = 1.2, 8.0 Hz, 1H), 7.56 (m, 1H), 7.46 (m, 1H), 7.40-7.19 (m, 8H), 6.85-6.82 (m, 2H), 6.79-6.74 (m, 1H), 6.73 (d, *J* = 8.8 Hz, 1H), 4.63 (s, 2H), 4.39 (d, *J* = 6.0 Hz, 1H), 4.25 (t, *J* = 4.0 Hz, 2H), 3.72 (s, 3H), 3.51 (t, *J* = 4.0 Hz, 2H).

**<Example 73> 4'-((7-((4-bromobenzyl)carbamoyl)-2H-benzo[b][1,4]oxazine-4(3H)-yl)methyl)-[1,1'-biphenyl]-2-carboxylic acid**

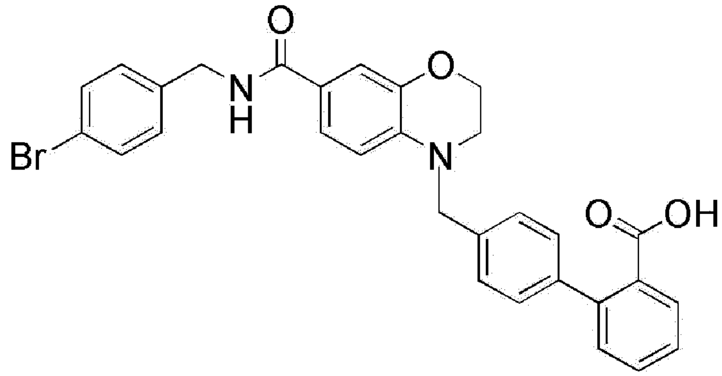

The target compound was obtained by the same manner as described in Example 58 except that (4-bromophenyl)methaneamine was used instead of (S)-1-phenylethylamine.
$^1$H NMR (400 MHz, DMSO-$d_6$) δ 12.76 (br, OH, 1H), 8.69 (t, *J* = 6.0 Hz, 1H), 7.70 (dd, *J* = 1.2, 8.4 Hz, 1H), 7.62-7.20 (m, 13H), 6.73 (d, *J* = 8.4 Hz, 1H), 4.63 (s, 2H), 4.42 (d, *J* = 8.0 Hz, 1H), 4.25 (t, *J* = 4.0 Hz, 2H), 3.51 (t, *J* = 4.0 Hz, 2H).

**<Example 74> (S)-4'-((7-((1-(4-cyanophenyl)ethyl)carbamoyl)-2H-benzo[b][1,4]oxazine-4(3H)-yl)methyl)-[1,1'-biphenyl]-2-carboxylic acid**

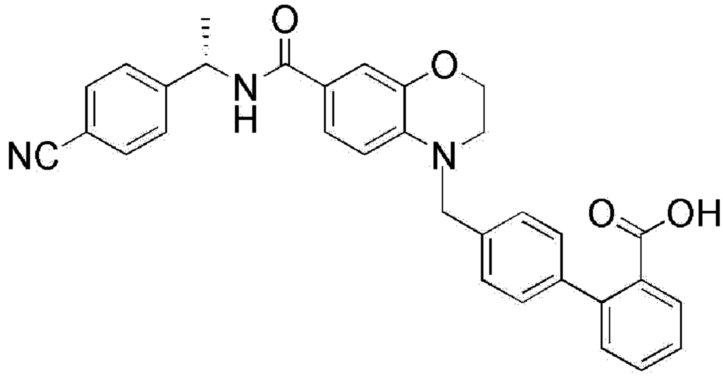

The target compound was obtained by the same manner as described in Example 58 except that (S)-1-(4-cyanophenyl)ethaneamine was used instead of (S)-1-phenylethylamine.
$^1$H NMR (400 MHz, DMSO-$d_6$) δ 12.74 (br, OH, 1H), 8.51 (d, *J* = 7.6 Hz, 1H), 7.80 (m, 2H), 7.61-7.18 (m, 10H), 6.74 (d, *J* = 8.0 Hz, 1H), 5.12 (m, 1H), 4.63 (s, 2H), 4.25 (t, *J* = 4.0 Hz, 2H), 3.51 (t, *J* = 4.0 Hz, 2H), 1.44 (d, *J* = 7.2 Hz, 3H).

**<Example 75> (S)-4'-((7-((1-(4-(trifluoromethoxy)phenyl)ethyl)carbamoyl)-2H-benzo[b][1,4]oxazine-4(3H)-yl)methyl)-[1,1'-biphenyl]-2-carboxylic acid**

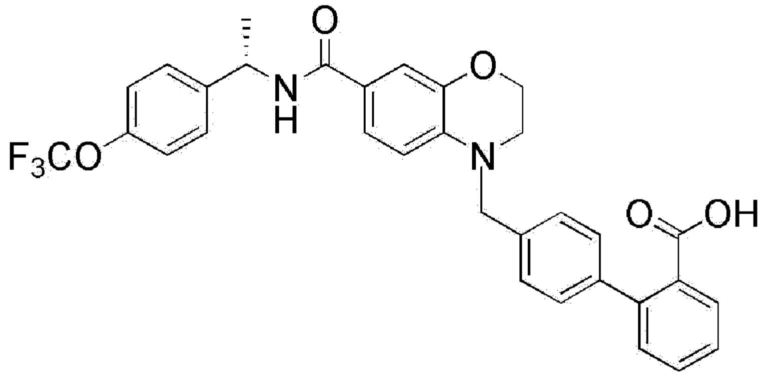

The target compound was obtained by the same manner as described in Example 58 except that (S)-1-(4-(trifluoromethoxy)phenyl)ethaneamine was used instead of (S)-1-phenylethylamine.
$^1$H NMR (400 MHz, DMSO-$d_6$) δ 12.90 (br, OH, 1H), 8.44 (d, *J* = 8.0 Hz, 1H), 7.95 (d, *J* = 8.0 Hz, 1H), 7.71-7.18 (m, 13H), 6.74 (d, *J* = 8.4 Hz, 1H), 5.13 (m, 1H), 4.63 (s, 2H), 4.25 (t, *J* = 4.0 Hz, 2H), 3.51 (t, *J* = 4.0 Hz, 2H), 1.44 (d, *J* = 7.2 Hz, 3H).

**<Example 76> (S)-4'-((7-((1-(4-(trifluoromethyl)phenyl)ethyl)carbamoyl)-2H-benzo[b][1,4]oxazine-4(3H)-yl)methyl)-[1,1'-biphenyl]-2-carboxylic acid**

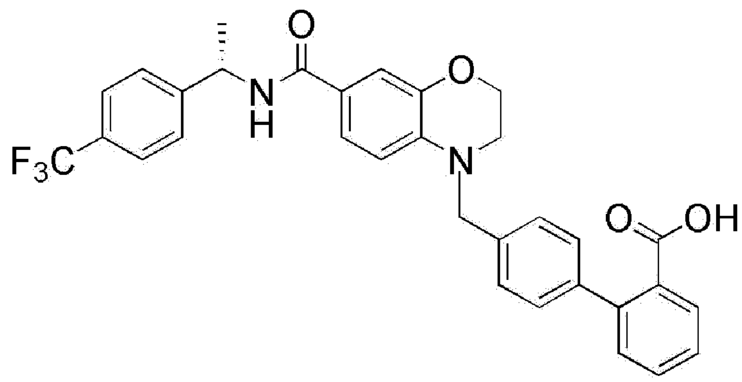

The target compound was obtained by the same manner as described in Example 58 except that (S)-1-(4-(trifluoromethyl)phenyl)ethaneamine used instead of (S)-1-phenylethylamine.
$^1$H NMR (400 MHz, DMSO-$d_6$) δ 12.73 (br, OH, 1H), 8.50 (d, *J* = 8.0 Hz, 1H), 7.94 (d, *J* = 8.4 Hz, 1H), 7.75-7.43 (m, 6H), 7.48-7.40 (m, 1H), 7.38-7.25 (m, 6H), 6.74 (d, *J* = 8.4 Hz, 1H), 5.16 (m, 1H), 4.63 (s, 2H), 4.25 (t, *J* = 4.0 Hz, 2H), 3.52 (t, *J* = 4.0 Hz, 2H), 1.46 (d, *J* = 7.2 Hz, 3H).

**<Example 77> (S)-4'-((7-((1-(4-((trifluoromethyl)thio)phenyl)ethyl)carbamoyl)-2H-benzo[b][1,4]oxazine-4(3H)-yl)methyl)-[1,1'-biphenyl]-2-carboxylic acid**

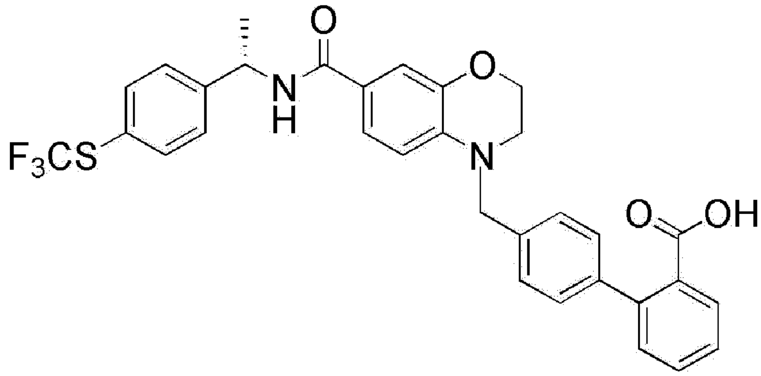

The target compound was obtained by the same manner as described in Example 58 except that (S)-1-(4-((trifluoromethyl)thio)phenyl)ethaneamine was used instead of (S)-1-phenylethylamine.
$^1$H NMR (400 MHz, DMSO-$d_6$) δ 12.63 (br, OH, 1H), 8.49 (d, *J* = 8.0 Hz, 1H), 7.74 (d, *J* = 8.0 Hz, 1H), 7.65 (m, 2H), 7.60-7.50 (m, 3H), 7.45 (m, 1H), 7.40-7.15 (m, 5H), 6.74 (d, *J* = 8.4 Hz, 1H), 5.15 (m, 1H), 4.63 (s, 2H), 4.25 (t, *J* = 4.0 Hz, 2H), 3.52 (t, *J* = 4.0 Hz, 2H), 1.44 (d, *J* = 6.8 Hz, 3H).

**<Example 78> (S)-4'-((7-((1-(4-(tert-butyl)phenyl)ethyl)carbamoyl)-2H-benzo[b][1,4]oxazine-4(3H)-yl)methyl)-[1,1'-biphenyl]-2-carboxylic acid**

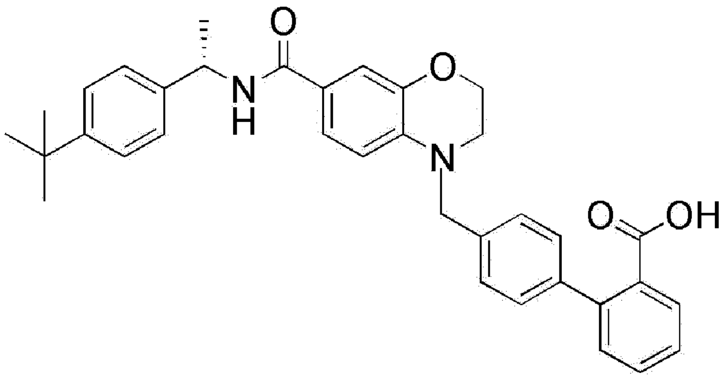

The target compound was obtained by the same manner as described in Example 58 except that (S)-1-(4-(tert-butyl)phenyl)ethaneamine was used instead of (S)-1-phenylethylamine.
$^1$H NMR (400 MHz, DMSO-$d_6$) δ 12.63 (br, OH, 1H), 8.35 (d, *J* = 8.0 Hz, 1H), 8.00 (d, *J* = 8.0 Hz, 1H), 7.65-7.15 (m, 13H), 6.73 (d, *J* = 8.4 Hz, 1H), 5.10 (m, 1H), 4.63 (s, 2H), 4.25 (t, *J* = 4.0 Hz, 2H), 3.51 (t, *J* = 4.0 Hz, 2H), 1.42 (d, *J* = 7.2 Hz, 3H), 1.25 (s, 9H).

**<Example 79> (S)-4'-((7-((1-(4-fluoro-2-methylphenyl)ethyl)carbamoyl)-2H-benzo[b][1,4]oxazine-4(3H)-yl)methyl)-[1,1'-biphenyl]-2-carboxylic acid**

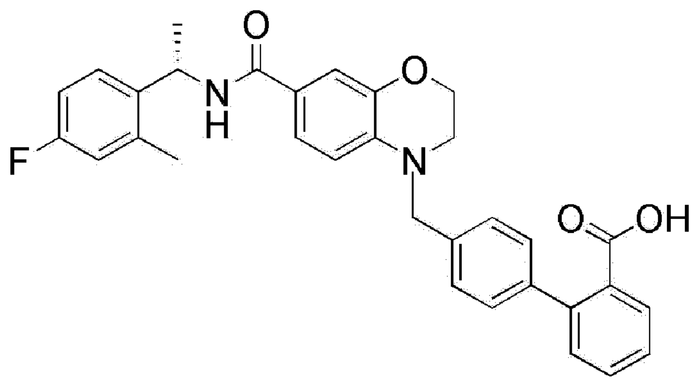

The target compound was obtained by the same manner as described in Example 58 except that (S)-1-(4-fluoro-2-methylphenyl)ethaneamine was used instead of (S)-1-phenylethylamine.
$^1H$ NMR (400 MHz, DMSO-$d_6$) δ 12.63 (br, OH, 1H), 8.41 (d, *J* = 8.0 Hz, 1H), 7.71 (d, *J* = 8.0 Hz, 1H), 7.60-7.52 (m, 1H), 7.47-7.10 (m, 6H), 6.98 (m, 2H), 6.72 (d, *J* = 8.0 Hz, 1H), 5.23 (m, 1H), 4.62 (s, 2H), 4.25 (t, *J* = 4.0 Hz, 2H), 3.52 (t, *J* = 4.0 Hz, 2H), 2.36 (s, 3H), 1.38 (d, *J* = 6.8 Hz, 3H).

**<Example 80> (S)-4'-((7-((1-(3-fluoro-4-methylphenyl)ethyl)carbamoyl)-2H-benzo[b][1,4]oxazine-4(3H)-yl)methyl)-[1,1'-biphenyl]-2-carboxylic acid**

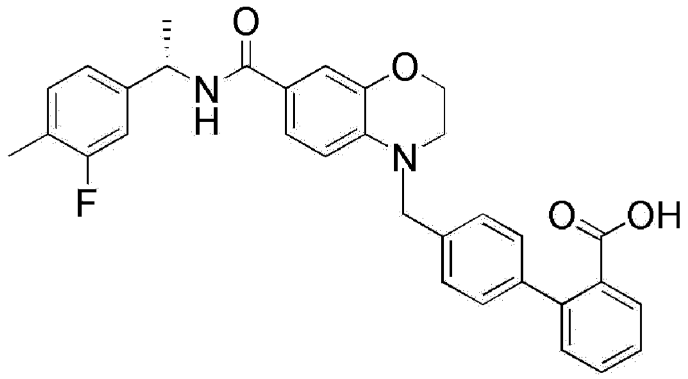

The target compound was obtained by the same manner as described in Example 58 except that (S)-1-(3-fluoro-4-methylphenyl)ethaneamine was used instead of (S)-1-phenylethylamine.
$^1H$ NMR (400 MHz, DMSO-$d_6$) δ 12.71 (br, OH, 1H), 8.39 (d, *J* = 8.0 Hz, 1H), 7.71 (d, *J* = 8.0 Hz, 1H), 7.60-7.00 (m, 12H), 6.74 (d, *J* = 8.4 Hz, 1H), 5.08 (m, 1H), 4.63 (s, 2H), 4.25 (t, *J* = 4.0 Hz, 2H), 3.52 (t, *J* = 4.0 Hz, 2H), 2.18 (s, 3H), 1.41 (d, *J* = 7.2 Hz, 3H).

**<Example 81> (R)-4'-((7-((1-(4-cyanophenyl)ethyl)carbamoyl)-2H-benzo[b][1,4]oxazine-4(3H)-yl)methyl)-[1,1'-biphenyl]-2-carboxylic acid**

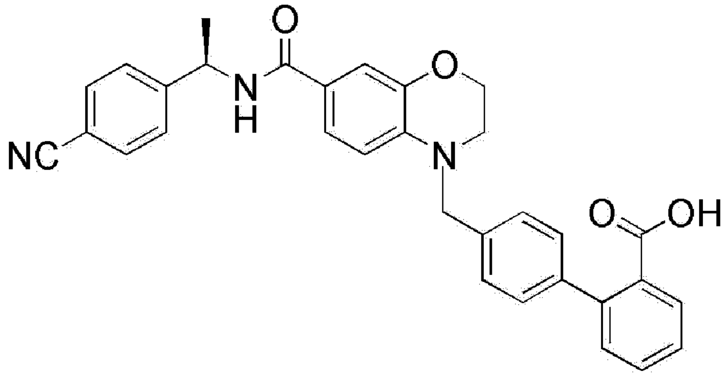

The target compound was obtained by the same manner as described in Example 58 except that (R)-1-(4-cyanophenyl)ethaneamine was used instead of (S)-1-phenylethylamine.
$^1$H NMR (400 MHz, DMSO-d6) δ 8.51 (d, J = 8.0 Hz, 1H), 7.78 (d, J = 8.0 Hz, 2H), 7.66 (s, 1H), 7.54 (d, J = 8.0 Hz, 2H), 7.50-7.20 (m, 11H), 6.74 (d, J = 8.4 Hz, 1H), 5.14 (m, 1H), 4.62 (s, 2H), 4.26 (t, J = 4.0 Hz, 2H), 3.52 (t, J = 4.0 Hz, 2H), 1.42 (d, J = 7.2 Hz, 3H).

**<Example 82> 4'-((7-(cromene-3-ylcarbamoyl)-2H-benzo[b][1,4]oxazine-4(3H)-yl)methyl)-[1,1'-biphenyl]-2-carboxylic acid**

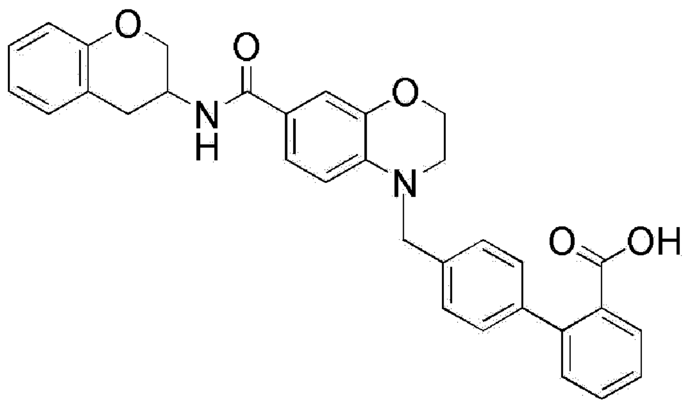

The target compound was obtained by the same manner as described in Example 58 except that cromene-3-amine was used instead of (S)-1-phenylethylamine.
$^1$H NMR (400 MHz, DMSO-$d_6$) δ 12.74 (br, OH, 1H), 8.08 (m, 1H), 7.95 (m, 1H), 7.72 (m, 1H), 7.45 (m, 1H), 7.40-7.28 (m, 6H), 7.13-7.05 (m, 2H), 6.88 (m, 1H), 6.80-6.71 (m, 2H), 4.63 (s, 2H), 4.31-4.13 (m, 4H), 3.83 (m, 1H), 3.51 (t, *J* = 4.0 Hz, 2H), 3.03-2.86 (m, 2H).

**<Example 83> (S)-4'-((7-((1-(2,6-difluorophenyl)ethyl)carbamoyl)-2H-benzo[b][1,4]oxazine-4(3H)-yl)methyl)-[1,1'-biphenyl]-2-carboxylic acid**

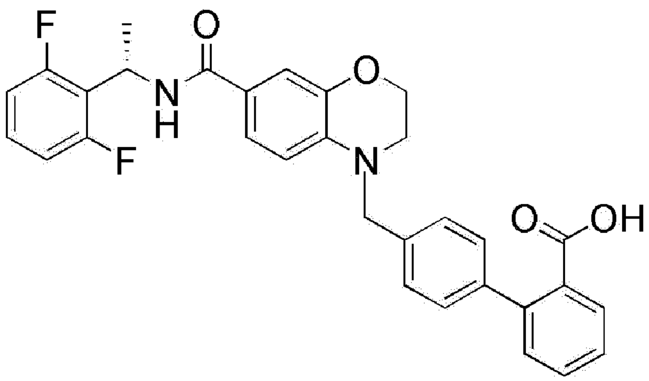

The target compound was obtained by the same manner as described in Example 58 except that (S)-1-(2,6-difluorophenyl)ethaneamine was used instead of (S)-1-phenylethylamine.

$^1$H NMR (400 MHz, DMSO-$d_6$) δ 12.78 (br, OH, 1H), 8.40 (d, *J* = 6.8 Hz, 1H), 7.70 (dd, *J* = 0.8, 7.6 Hz, 1H), 7.56 (td, *J* = 1.2, 7.2 Hz, 1H), 7.44 (td, *J* = 0.8, 7.2 Hz, 1H), 7.38-7.25 (m, 8H), 7.01 (t, *J* = 8.0 Hz, 1H), 6.72 (d, *J* = 8.8 Hz, 1H), 5.34 (m, 1H), 4.62 (s, 2H), 4.24 (t, *J* = 4.0 Hz, 2H), 3.51 (t, *J* = 4.0 Hz, 2H), 1.53 (d, *J* = 7.2 Hz, 3H).

**<Example 84> (S)-4'-((7-((1-(3-fluorophenyl)ethyl)carbamoyl)-2H-benzo[b][1,4]oxazine-4(3H)-yl)methyl)-[1,1'-biphenyl]-2-carboxylic acid**

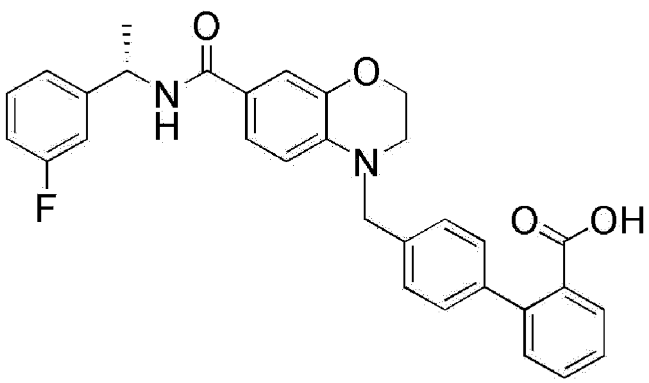

The target compound was obtained by the same manner as described in Example 58 except that (S)-1-(3-fluorophenyl)ethaneamine was used instead of (S)-1-phenylethylamine.

$^1$H NMR (400 MHz, DMSO-$d_6$) δ 12.68 (br, OH, 1H), 8.42 (d, *J* = 8.0 Hz, 1H), 7.71 (d, *J* = 6.8 Hz, 1H), 7.56 (td, *J* = 1.2, 7.6 Hz, 1H), 7.45 (m, 1H), 7.40-7.28 (m, 8H), 7.23-7.15 (m, 2H), 7.02 (td, *J* = 2.0, 8.4 Hz, 1H), 6.74 (d, *J* = 8.4 Hz, 1H), 5.12 (m, 1H), 4.63 (s, 2H), 4.25 (t, *J* = 4.0 Hz, 2H), 3.51 (t, *J* = 4.0 Hz, 2H), 1.44 (d, *J* = 6.8 Hz, 3H).

**<Example 85> (S)-4'-((7-((1-(3-(trifluoromethoxy)phenyl)ethyl)carbamoyl)-2H-benzo[b][1,4]oxazine-4(3H)-yl)methyl)-[1,1'-biphenyl]-2-carboxylic acid**

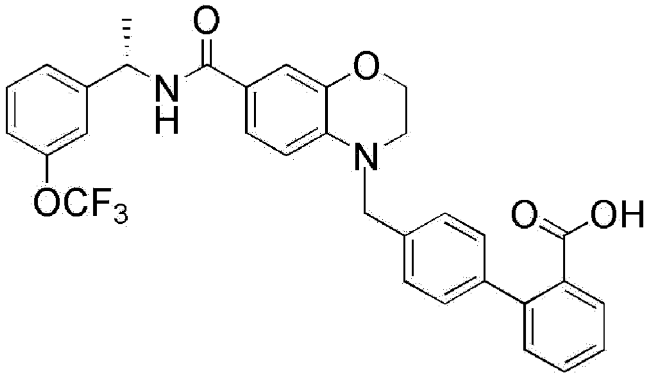

The target compound was obtained by the same manner as described in Example 58 except that (S)-1-(3-(trifluoromethoxy)phenyl)ethaneamine was used instead of (S)-1-phenylethylamine.
$^1H$ NMR (400 MHz, DMSO-$d_6$) δ 12.72 (br, OH, 1H), 8.47 (d, *J* = 8.0 Hz, 1H), 7.71 (dd, *J* = 0.8, 7.6 Hz, 1H), 7.55 (td, *J* = 1.2, 7.6 Hz, 1H), 7.49-7.28 (m, 11H), 7.20 (m, 1H), 6.75 (d, *J* = 7.6 Hz, 1H), 5.15 (m, 1H), 4.63 (s, 2H), 4.26 (t, *J* = 4.0 Hz, 2H), 3.52 (t, *J* = 4.0 Hz, 2H), 1.46 (d, *J* = 6.8 Hz, 3H).

**<Example 86> (S)-4'-((7-((1-(3-(trifluoromethyl)phenyl)ethyl)carbamoyl)-2H-benzo[b][1,4]oxazine-4(3H)-yl)methyl)-[1,1'-biphenyl]-2-carboxylic acid**

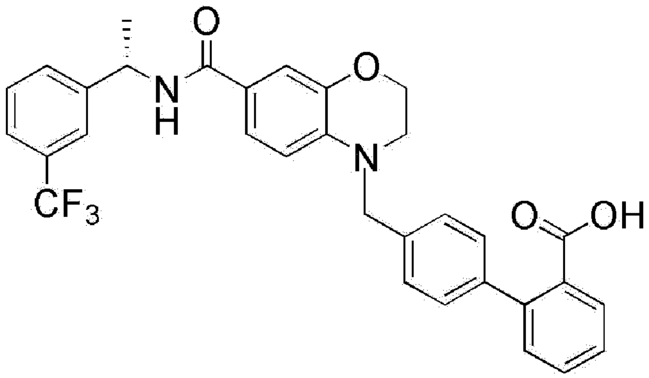

The target compound was obtained by the same manner as described in Example 58 except that (S)-1-(3-(trifluoromethyl)phenyl)ethaneamine was used instead of (S)-1-phenylethylamine.
$^1H$ NMR (400 MHz, DMSO-$d_6$) δ 12.74 (br, OH, 1H), 8.51 (d, *J* = 7.6 Hz, 1H), 7.80-7.20 (m, 14H), 6.75 (d, *J* = 8.4 Hz, 1H), 5.19 (m, 1H), 4.63 (s, 2H), 4.25 (t, *J* = 4.0 Hz, 2H), 3.51 (t, *J* = 4.0 Hz, 2H), 1.46 (d, *J* = 7.2 Hz, 3H).

**<Example 87> (S)-4'-((7-((1-(naphthalene-2-yl)ethyl)carbamoyl)-2H-benzo[b][1,4]oxazine-4(3H)-yl)methyl)-[1,1'-biphenyl]-2-carboxylic acid**

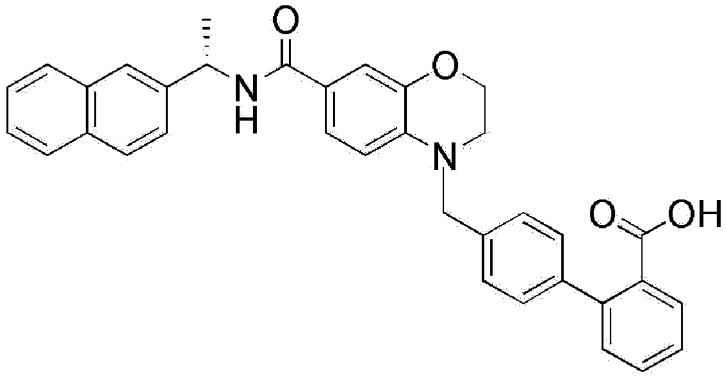

The target compound was obtained by the same manner as described in Example 58 except that (S)-1-(naphthalene-2-yl)ethaneamine was used instead of (S)-1-phenylethylamine.
$^1$H NMR (400 MHz, DMSO-$d_6$) δ 12.72 (br, OH, 1H), 8.50 (d, *J* = 8.0 Hz, 1H), 7.90-7.80 (m, 4H), 7.71 (d, *J* = 7.6 Hz, 1H), 7.58-7.28 (m, 12H), 6.74 (d, *J* = 8.4 Hz, 1H), 5.29 (m, 1H), 4.63 (s, 2H), 4.25 (t, *J* = 4.0 Hz, 2H), 3.51 (t, *J* = 4.0 Hz, 2H), 1.54 (d, *J* = 6.8 Hz, 3H).

**<Example 88> 4'-((7-((pyridine-2-ylmethyl)carbamoyl)-2H-benzo[b][1,4]oxazine-4(3H)-yl)methyl)-[1,1'-biphenyl]-2-carboxylic acid**

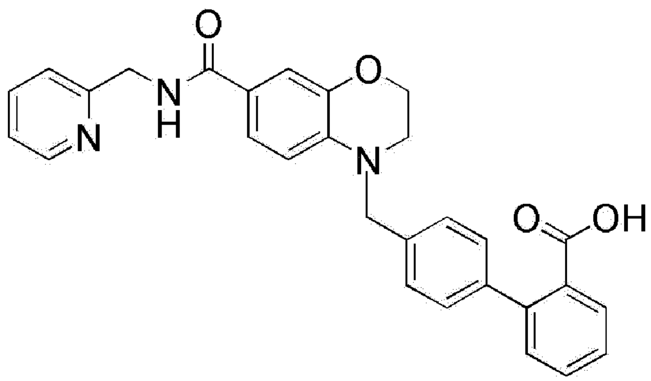

The target compound was obtained by the same manner as described in Example 58 except that pyridine-2-ylmethaneamine was used instead of (S)-1-phenylethylamine.
$^1$H NMR (400 MHz, DMSO-$d_6$) δ12.75 (br, OH, 1H), 8.76-8.73 (m, 1H), 8.53-8.52 (m, 1H), 7.83-7.80 (m, 1H), 7.71-7.69 (m, 1H), 7.57-7.54 (m, 1H), 7.46-7.42 (m, 1H), 7.38-7.30 (m, 9H), 6.76-6.74 (m, 1H), 4.64 (s, 2H), 4.54-4.52 (m, 2H), 4.26-4.25 (m, 2H), 3.52-3.38 (m, 2H).

**<Example 89> 4'-((7-((pyridine-3-ylmethyl)carbamoyl)-2H-benzo[b][1,4]oxazine-4(3H)-yl)methyl)-[1,1'-biphenyl]-2-carboxylic acid**

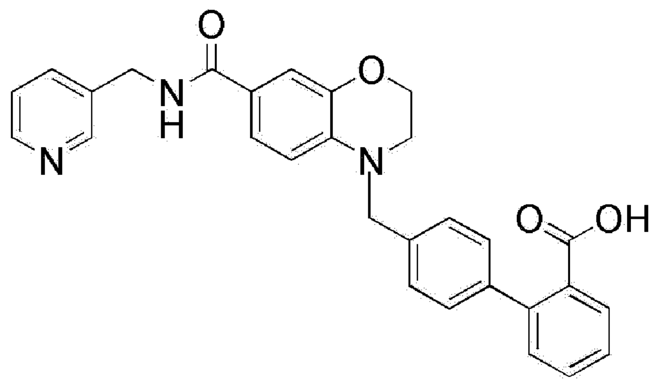

The target compound was obtained by the same manner as described in Example 58 except that pyridine-3-ylmethaneamine was used instead of (S)-1-phenylethylamine.
$^1$H NMR (400 MHz, DMSO-$d_6$) δ 12.75 (br, OH, 1H), 8.82-8.76 (m, 1H), 8.60 (m, 1H), 8.54-8.53 (m, 1H), 7.89-7.87 (m, 1H), 7.71-7.69 (m, 1H), 7.57-7.50 (m, 2H), 7.46-7.42 (m, 1H), 7.38-7.32 (m. 7H), 6.75-6.73 (m, 1H), 4.64 (s, 2H), 4.48-4.46 (m, 2H), 4.25-4.24 (m, 2H), 3.52 (m, 2H).

**<Example 90> 4'-((7-((pyridine-4-ylmethyl)carbamoyl)-2H-benzo[b][1,4]oxazine-4(3H)-yl)methyl)-[1,1'-biphenyl]-2-carboxylic acid**

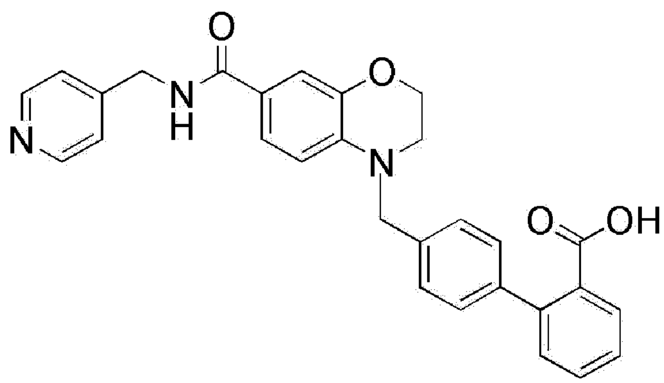

The target compound was obtained by the same manner as described in Example 58 except that pyridine-4-ylmethaneamine was used instead of (S)-1-phenylethylamine.
$^1$H NMR (400 MHz, DMSO-$d_6$) δ 12.75 (br, OH, 1H), 8.84-8.81 (m, 1H), 8.60 (m, 2H), 7.71-7.69 (m, 1H), 7.58-7.54 (m, 1H), 7.49-7.42 (m, 3H), 7.38-7.35 (m, 2H), 7.33-7.31 (m, 5H), 6.77-6.75 (m, 1H), 4.65 (s, 2H), 4.52-4.50 (m, 2H), 4.26-4.25 (m, 2H), 3.54-3.53 (m, 2H).

**<Example 91> 4'-((7-(benzylcarbamoyl)-2H-benzo[b][1,4]oxazine-4(3H)-yl)methyl)-[1,1'-biphenyl]-2-carboxylic acid**

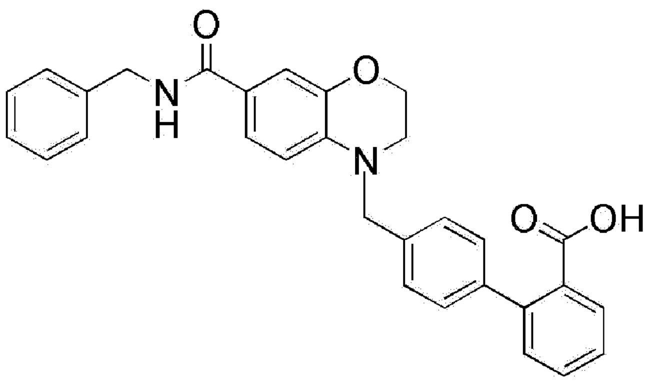

The target compound was obtained by the same manner as described in Example 58 except that phenylmethaneamine was used instead of (S)-1-phenylethylamine.
$^1H$ NMR (400 MHz, DMSO-$d_6$) δ 12.75 (br, OH, 1H), 8.69-8.66 (m, 1H), 7.71-7.69 (m, 1H), 7.58-7.54 (m, 1H), 7.46-7.42 (m, 1H), 7.38-7.26 (m, 1H), 7.23-7.20 (m, 1H), 6.75-6.72 (m, 1H), 4.63 (s, 2H), 4.43-4.41 (m, 2H), 4.26-4.24 (m, 2H), 3.52-3.50 (m, 2H).

**<Example 92> 4'-((7-((2-bromobenzyl)carbamoyl)-2H-benzo[b][1,4]oxazine-4(3H)-yl)methyl)-[1,1'-biphenyl]-2-carboxylic acid**

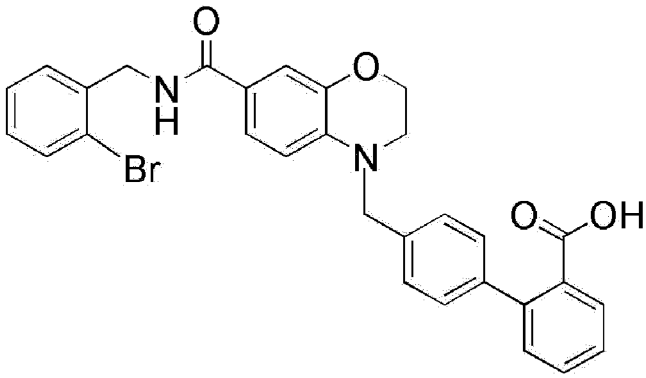

The target compound was obtained by the same manner as described in Example 58 except that (2-bromophenyl)methaneamine was used instead of (S)-1-phenylethylamine.
$^1H$ NMR (400 MHz, DMSO-$d_6$) δ 12.75 (br, OH, 1H), 8.71-8.64 (m, 1H), 7.71-7.69 (m, 1H), 7.62-7.59 (m, 1H), 7.58-7.54 (m, 1H), 7.46-7.42 (m, 1H), 7.39-7.33 (m, 8H), 7.28-7.24 (m, 1H), 7.22-7.18 (m, 1H), 6.77-6.75 (m, 1H), 4.65 (s, 2H), 4.44-4.43 (m, 2H), 4.28-4.25 (m, 2H), 3.54-3.52 (m, 2H).

**<Example 93> 4'-((7-(((2,3-dihydrobenzo[b][1,4]dioxin-6-yl)methyl)carbamoyl)-2H-benzo[b][1,4]oxazine-4(3H)-yl)methyl)-[1,1'-biphenyl]-2-carboxylic acid**

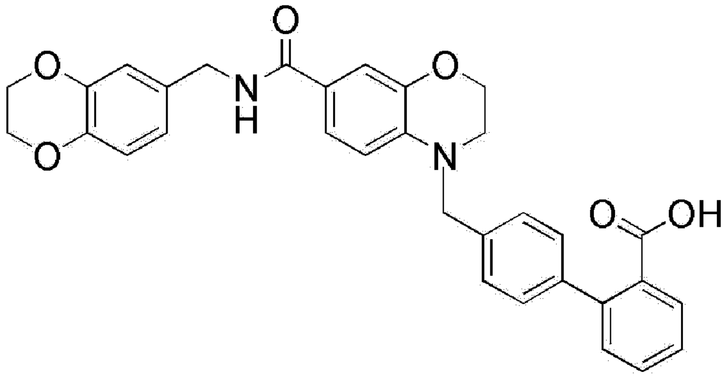

The target compound was obtained by the same manner as described in Example 58 except that (2,3-dihydrobenzo[b][1,4]dioxin-6-yl)methylamine was used instead of (S)-1-phenylethylamine.
$^1$H NMR (400 MHz, DMSO-$d_6$) δ 8.62-8.59 (m, 1H), 7.56-7.54 (m, 1H), 7.41-7.39 (m, 2H), 7.35-7.30 (m, 4H), 7.25-7.23 (m, 3H), 6.78-6.71 (m, 4H), 4.60 (s, 2H), 4.29-4.27 (m, 2H), 4.26-4.25 (m, 2H), 4.19 (s, 4H), 3.51 (m, 2H).

**<Example 94> (R)-4-((2'-carbamoyl-[1,1'-biphenyl]-4-yl)methyl)-N-(1-(4-cyanophenyl)ethyl)-3,4-dihydro-2H-benzo[b][1,4]oxazine-7-carboxamide**

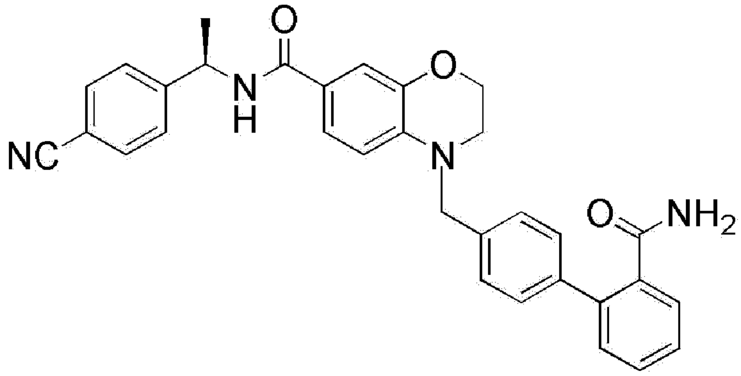

The target compound was obtained by the same manner as described in Example 59 except that (R)-4'-((7-((1-(4-cyanophenyl)ethyl)carbamoyl)-2H-benzo[b][1,4]oxazine-4(3H)-yl)methyl)-[1,1'-biphenyl]-2-carboxylic acid obtained in Example 81 was used instead of (S)-4'-((7-((1-phenylethyl)carbamoyl)-2H-benzo[b][1,4]oxazine-4(3H)-yl)methyl)-[1,1'-biphenyl]-2-carboxylic acid (50 mg, 0.1 mmol).
$^1$H NMR (400 MHz, DMSO-$d_6$) δ 8.51 (d, *J* = 8.0 Hz, 1H), 7.78 (d, *J* = 8.0 Hz, 2H), 7.66 (s, 1H), 7.54 (d, *J* = 8.0 Hz, 2H), 7.50-7.20 (m, 11H), 6.74 (d, *J* = 8.4 Hz, 1H), 5.14 (m, 1H), 4.62 (s, 2H), 4.26 (t, *J* = 4.0 Hz, 2H), 3.52 (t, *J* = 4.0 Hz, 2H), 1.42 (d, *J* = 7.2 Hz, 3H).

**<Example 95> (S)-4-((2'-carbamoyl-[1,1'-biphenyl]-4-yl)methyl)-N-(1-(3-fluorophenyl)ethyl)-3,4-dihydro-2H-benzo[b][1,4]oxazine-7-carboxamide**

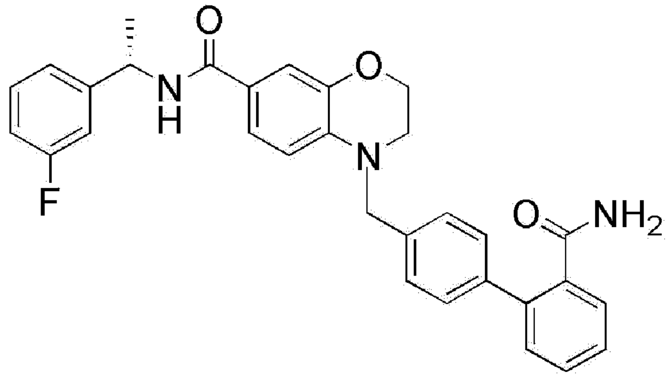

The target compound was obtained by the same manner as described in Example 59 except that (S)-4'-((7-((1-(3-fluorophenyl)ethyl)carbamoyl)-2H-benzo[b][1,4]oxazine-4(3H)-yl)methyl)-[1,1'-biphenyl]-2-carboxylic acid obtained in Example 84 was used instead of (S)-4'-((7-((1-phenylethyl)carbamoyl)-2H-benzo[b][1,4]oxazine-4(3H)-yl)methyl)-[1,1'-biphenyl]-2-carboxylic acid (50 mg, 0.1 mmol).
$^1$H NMR (400 MHz, $CDCl_3$) $\delta$ 7.77-7.75 (m, 1H), 7.54-7.50 (m, 1H), 7.46-7.43 (m, 3H), 7.38-7.28 (m, 6H), 7.17-7.15 (m, 1H), 7.09-7.06 (m, 1H), 6.98-6.94 (m, 1H), 6.67-6.65 (m, 1H), 6.15-6.13 (m, NH, 1H), 5.46 (br, NH, 1H), 5.34-5.27 (m, 2H), 4.59 (s, 2H), 4.33-4.31 (m, 2H), 3.54-3.47 (m, 2H), 1.58-1.56 (m, 3H).

**<Example 96> (S)-4-((2'-carbamoyl-[1,1'-biphenyl]-4-yl)methyl)-N-(1-(naphthalene-2-yl)ethyl)-3,4-dihydro-2H-benzo[b][1,4]oxazine-7-carboxamide**

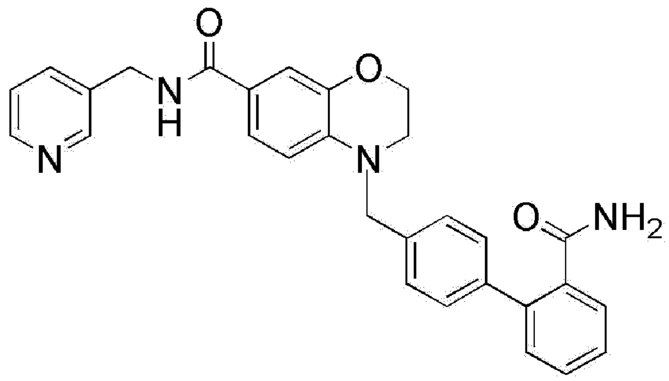

The target compound was obtained by the same manner as described in Example 59 except that 4'-((7-((pyridine-3-ylmethyl)carbamoyl)-2H-benzo[b][1,4]oxazine-4(3H)-yl)methyl)-[1,1'-biphenyl]-2-carboxylic acid obtained in Example 89 was used instead of (S)-4'-((7-((1-phenylethyl)carbamoyl)-2H-benzo[b][1,4]oxazine-4(3H)-yl)methyl)-[1,1'-biphenyl]-2-carboxylic acid (50 mg, 0.1 mmol).
$^1$H NMR (400 MHz, DMSO-$d_6$) $\delta$ 8.72-8.70 (m, 1H), 8.51 (m, H), 8.44-8.43 (m, 1H), 7.68-7.64 (m, 2H), 7.48-7.26 (m, 12H), 6.75-6.72 (m, 1H), 4.62 (s, 2H), 4.44-4.42 (m, 2H), 4.26-4.24 (m, 2H), 3.53-3.50 (m, 2H).

**<Example 98> 4-((2'-carbamoyl-[1,1'-biphenyl]-4-yl)methyl)-N-(pyridine-4-ylmethyl)-3,4-dihydro-2H-benzo[b][1,4]oxazine-7-carboxamide**

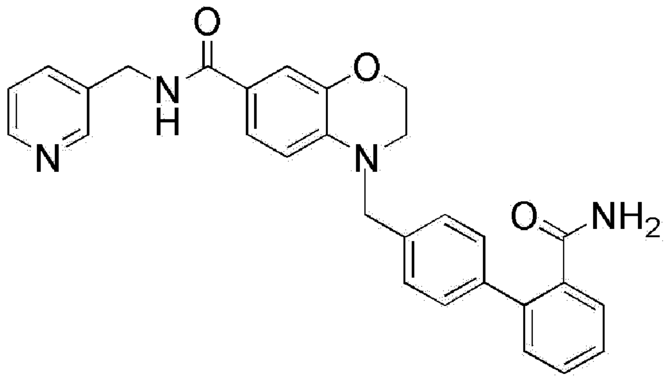

The target compound was obtained by the same manner as described in Example 59 except that 4'-((7-((pyridine-3-ylmethyl)carbamoyl)-2H-benzo[b][1,4]oxazine-4(3H)-yl)methyl)-[1,1'-biphenyl]-2-carboxylic acid obtained in Example 89 was used instead of (S)-4'-((7-((1-phenylethyl)carbamoyl)-2H-benzo[b][1,4]oxazine-4(3H)-yl)methyl)-[1,1'-biphenyl]-2-carboxylic acid (50 mg, 0.1 mmol).

$^1$H NMR (400 MHz, DMSO-$d_6$) 8.72-8.70 (m, 1H), 8.51 (m, H), 8.44-8.43 (m, 1H), 7.68-7.64 (m, 2H), 7.48-7.26 (m, 12H), 6.75-6.72 (m, 1H), 4.62 (s, 2H), 4.44-4.42 (m, 2H), 4.26-4.24 (m, 2H), 3.53-3.50 (m, 2H).

**<Example 98> 4-((2'-carbamoyl-[1,1'-biphenyl]-4-yl)methyl)-N-(pyridine-4-ylmethyl)-3,4-dihydro-2H-benzo[b][1,4]oxazine-7-carboxamide의 제조**

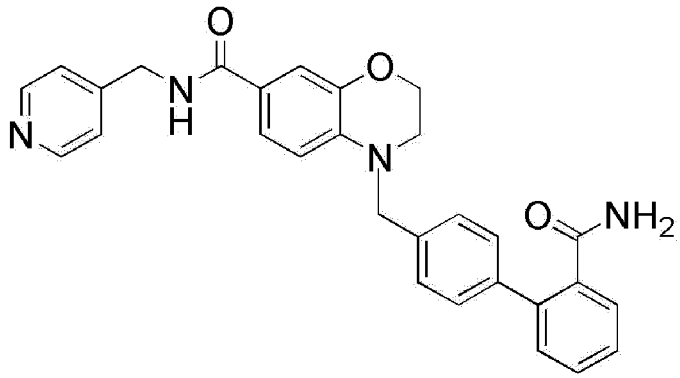

The target compound was obtained by the same manner as described in Example 59 except that 4'-((7-((pyridine-4-ylmethyl)carbamoyl)-2H-benzo[b][1,4]oxazine-4(3H)-yl)methyl)-[1,1'-biphenyl]-2-carboxylic acid obtained in Example 90 was used instead of (S)-4'-((7-((1-phenylethyl)carbamoyl)-2H-benzo[b][1,4]oxazine-4(3H)-yl)methyl)-[1,1'-biphenyl]-2-carboxylic acid (50 mg, 0.1 mmol).

$^1$H NMR (400 MHz, DMSO-$d_6$) 8.76-8.73 (m, 1H), 8.49-8.47 (m, 2H), 7.65 (m, 1H), 7.49-7.43 (m, 1H), 7.41-7.35 (m, 6H), 7.32-7.25 (m, 6H), 6.76-6.74 (m, 1H), 4.63 (s, 2H), 4.44-4.42 (m, 2H), 4.27-4.25 (m, 2H), 3.54-3.52 (m, 2H).

**<Example 99> (S)-4-((2'-carbamoyl-[1,1'-biphenyl]-4-yl)methyl)-N-(1-(4-((trifluoromethyl)thio)phenyl)ethyl)-3,4-dihydro-2H-benzo[b][1,4]oxazine-7-carboxamide**

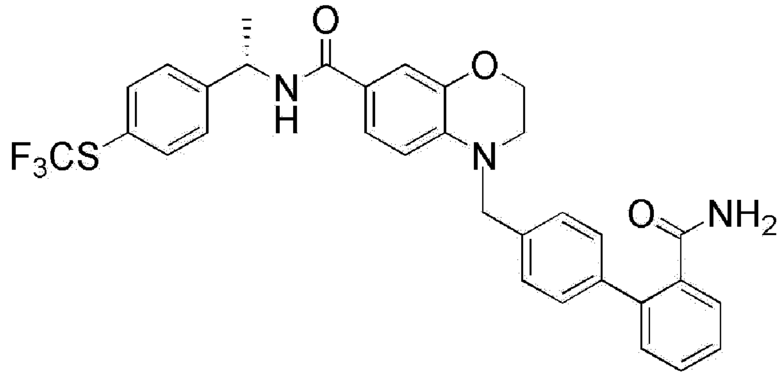

The target compound was obtained by the same manner as described in Example 59 except that (S)-4'-((7-((1-(4-((trifluoromethyl)thio)phenyl)ethyl)carbamoyl)-2H-benzo[b][1,4]oxazine-4(3H)-yl)methyl)-[1,1'-biphenyl]-2-carboxylic acid obtained in Example 77 was used instead of (S)-4'-((7-((1-phenylethyl)carbamoyl)-2H-benzo[b][1,4]oxazine-4(3H)-yl)methyl)-[1,1'-biphenyl]-2-carboxylic acid (50 mg, 0.1 mmol).
$^1$H NMR (400 MHz, DMSO-$d_6$) 8.50-8.48 (m, 1H), 7.67-7.65 (m, 3H), 7.51-7.27 (m, 13H), 6.74-6.72 (m, 1H), 5.16-5.13 (m, 1H), 4.62 (s, 2H), 4.26-4.25 (m, 2H), 3.52-3.51 (m, 2H), 1.46-4.11 (m, 3H).

**<Example 100> N-benzyl-4-((2'-carbamoyl-[1,1'-biphenyl]-4-yl)methyl)-3,4-dihydro-2H-benzo[b][1,4]oxazine-7-carboxamide**

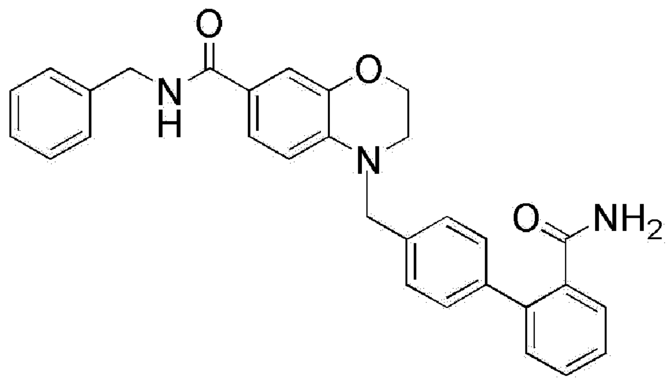

The target compound was obtained by the same manner as described in Example 59 except that 4'-((7-(benzylcarbamoyl)-2H-benzo[b][1,4]oxazine-4(3H)-yl)methyl)-[1,1'-biphenyl]-2-carboxylic acid obtained in Example 91 was used instead of (S)-4'-((7-((1-phenylethyl)carbamoyl)-2H-benzo[b][1,4]oxazine-4(3H)-yl)methyl)-[1,1'-biphenyl]-2-carboxylic acid (50 mg, 0.1 mmol).
$^1$H NMR (400 MHz, $CDCl_3$) δ 7.77-7.74 (m, 1H), 7.54-7.50 (m, 1H), 7.49-7.42 (m, 3H), 7.38-7.28 (m, 10H), 6.66-6.64 (m, 1H), 6.24-6.21 (m, NH, 1H), 5.59 (br, NH, 1H), 5.34 (br, NH, 1H), 4.64-4.63 (m, 2H), 4.59 (s, 2H), 4.32-4.30 (m, 2H), 3.53-3.51 (m, 2H).

**<Example 101> N-(2-bromobenzyl)-4-((2'-carbamoyl-[1,1'-biphenyl]-4-yl)methyl)-3,4-dihydro-2H-benzo[b][1,4]oxazine-7-carboxamide**

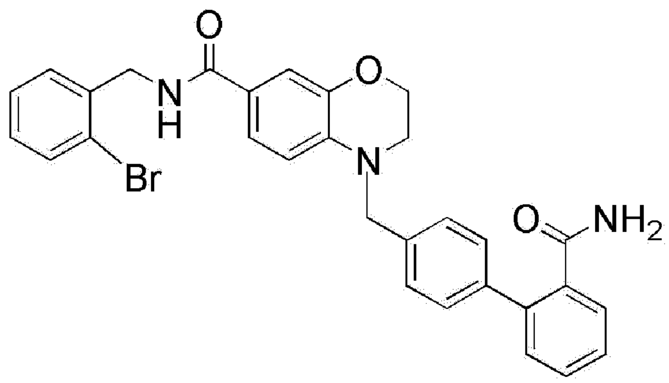

The target compound was obtained by the same manner as described in Example 59 except that 4'-((7-((2-bromobenzyl)carbamoyl)-2H-benzo[b][1,4]oxazine-4(3H)-yl)methyl)-[1,1'-biphenyl]-2-carboxylic acid obtained in Example 92 was used instead of (S)-4'-((7-((1-phenylethyl)carbamoyl)-2H-benzo[b][1,4]oxazine-4(3H)-yl)methyl)-[1,1'-biphenyl]-2-carboxylic acid (50 mg, 0.1 mmol).
$^1$H NMR (400 MHz, $CDCl_3$) $\delta$ 7.70-7.75 (m, 1H), 7.58-7.56 (m, 1H), 7.54-7.48 (m, 2H), 7.46-7.42 (m, 4H), 7.38-7.36 (m, 1H), 7.33-7.28 (m, 4H), 7.18-7.14 (m, 1H), 6.66-6.64 (m, 1H), 6.49-6.46 (m, NH, 1H), 5.48 (br, NH, 1H), 5.32 (br, NH, 1H), 4.70-4.69 (m, 2H), 4.59 (s, 2H), 4.33-4.30 (m, 2H), 3.54-3.51 (m, 2H).

**<Example 102> 4-((2'-carbamoyl-[1,1'-biphenyl]-4-yl)methyl)-N-((2,3-dihydrobenzo[b][1,4]dioxin-6-yl)methyl)-3,4-dihydro-2H-benzo[b][1,4]oxazine-7-carboxamide**

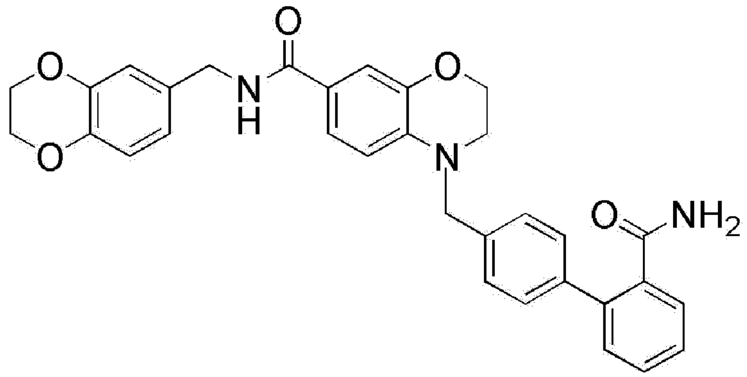

The target compound was obtained by the same manner as described in Example 59 except that 4'-((7-(((2,3-dihydrobenzo[b][1,4]dioxin-6-yl)methyl)carbamoyl)-2H-benzo[b][1,4]oxazine-4(3H)-yl)methyl)-[1,1'-biphenyl]-2-carboxylic acid obtained in Example 93 was used instead of (S)-4'-((7-((1-phenylethyl)carbamoyl)-2H-benzo[b][1,4]oxazine-4(3H)-yl)methyl)-[1,1'-biphenyl]-2-carboxylic acid (50 mg, 0.1 mmol).
$^1$H NMR (400 MHz, $CDCl_3$) $\delta$ 7.77-7.75 (m, 1H), 7.53-7.50 (m, 1H), 7.48-7.42 (m, 3H), 7.38-7.36 (m, 1H), 7.33-7.31 (m, 2H), 7.28-7.27 (m, 2H), 6.86-6.81 (m, 3H), 6.65-6.63 (m, 1H), 6.16-6.14 (m, NH, 1H), 5.50 (br, NH, 1H), 5.34 (br, NH, 1H), 4.58 (s, 2H), 4.51-4.50 (m, 2H), 4.32-4.30 (m, 2H), 4.26 (s, 4H), 3.53-3.51 (m, 2H).

**<Example 103> sodium (S)-4'-((7-((1-phenylethyl)carbamoyl)-2H-benzo[b][1,4]oxazine-4(3H)-yl)methyl)-[1,1'-biphenyl]-2-carboxylate**

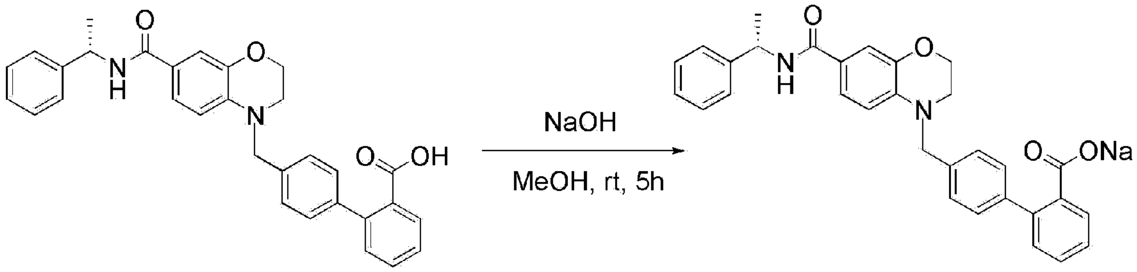


(S)-4'-((7-((1-phenylethyl)carbamoyl)-2H-benzo[b][1,4]oxazine-4(3H)-yl)methyl)-[1,1'-biphenyl]-2-carboxylic acid (300 mg, 0.61 mmol) obtained in Example 58 was dissolved in MeOH (10 ml) in a 25 mL flask, to which NaOH (24.4 mg, 0.61 mmol) was added, followed by stirring at room temperature for 5 hours. Upon completion of the reaction, the reaction mixture was concentrated under reduced pressure. As a result, sodium (S)-4'-((7-((1-phenylethyl)carbamoyl)-2H-benzo[b][1,4]oxazine-4(3H)-yl)methyl)-[1,1'-biphenyl]-2-carboxylate was obtained (300 mg).
$^1$H NMR (400 MHz, DMSO-$d_6$) δ 8.41 (d, *J* = 8.0 Hz, 1H), 7.48 (m, 2H), 7.39-7.25 (m, 7H), 7.23-7.15 (m, 6H), 6.74 (d, *J* = 8.4 Hz, 1H), 5.12 (m, 1H), 4.58 (s, 2H), 4.24 (t, *J* = 4.0 Hz, 2H), 3.51 (t, *J* = 4.0 Hz, 2H), 1.44 (d, *J* = 7.2 Hz, 3H).

**<Example 104> sodium (S)-4'-((7-((1-(4-cyanophenyl)ethyl)carbamoyl)-2H-benzo[b][1,4]oxazine-4(3H)-yl)methyl)-[1,1'-biphenyl]-2-carboxylate**

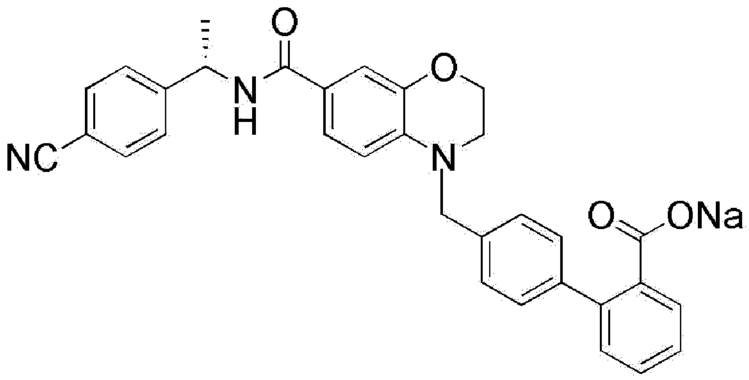

The target compound was obtained by the same manner as described in Example 103 except that (S)-4'-((7-((1-(4-cyanophenyl)ethyl)carbamoyl)-2H-benzo[b][1,4]oxazine-4(3H)-yl)methyl)-[1,1'-biphenyl]-2-carboxylic acid obtained in Example 74 was used instead of (S)-4'-((7-((1-phenylethyl)carbamoyl)-2H-benzo[b][1,4]oxazine-4(3H)-yl)methyl)-[1,1'-biphenyl]-2-carboxylic acid.
$^1$H NMR (400 MHz, DMSO-$d_6$) δ 8.55 (d, *J* = 7.6 Hz, 1H), 7.76 (d, *J* = 8.4 Hz, 2H), 7.54, (d, *J* = 8.4 Hz, 2H), 7.49-7.42 (m, 3H), 7.39-7.30 (m, 2H), 7.28-7.15 (m, 5H), 6.73 (d, *J* = 8.4 Hz, 1H), 5.14 (m, 1H), 4.56 (s, 2H), 4.22 (t, *J* = 4.0 Hz, 2H), 3.48 (t, *J* = 4.0 Hz, 2H), 1.44 (d, *J* = 7.2 Hz, 3H).

**<Example 105> sodium (S)-4'-((7-((1-(4-nitrophenyl)ethyl)carbamoyl)-2H-benzo[b][1,4]oxazine-4(3H)-yl)methyl)-[1,1'-biphenyl]-2-carboxylate**

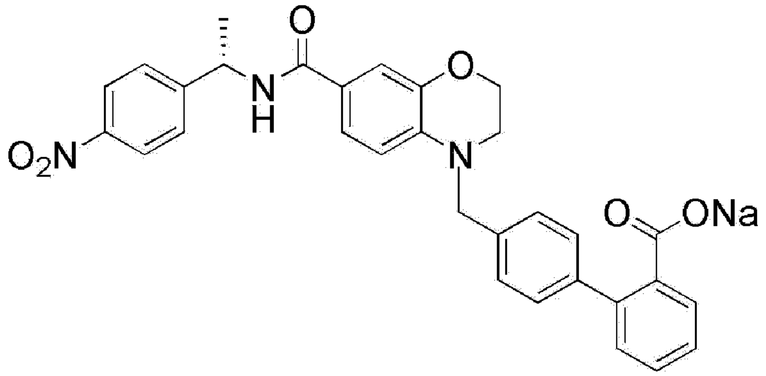

The target compound was obtained by the same manner as described in Example 103 except that (S)-4'-((7-((1-(4-nitrophenyl)ethyl)carbamoyl)-2H-benzo[b][1,4]oxazine-4(3H)-yl)methyl)-[1,1'-biphenyl]-2-carboxylic acid obtained in Example 60 was used instead of (S)-4'-((7-((1-phenylethyl)carbamoyl)-2H-benzo[b][1,4]oxazine-4(3H)-yl)methyl)-[1,1'-biphenyl]-2-carboxylic acid.
$^1$H NMR (400 MHz, DMSO-$d_6$) δ 8.61 (d, *J* = 7.6 Hz, 1H), 8.16 (d, *J* = 8.4 Hz, 2H), 7.61 (d, *J* = 8.8 Hz, 2H), 7.46 (d, *J* = 8.4 Hz, 2H), 7.37-7.16 (m, 8H), 6.75 (d, *J* = 8.8 Hz, 1H), 5.18 (m, 1H), 4.59 (s, 2H), 4.25 (t, *J* = 4.0 Hz, 2H), 3.52 (t, *J* = 4.0 Hz, 2H), 1.46 (d, *J* = 7.2 Hz, 3H).

**<Example 106> (S)-1-((2'-carbamoyl-3-fluoro-[1,1'-biphenyl]-4-yl)methyl)-N-(1-phenylethyl)-1,2,3,4-tetrahydroquinoline-6-carboxylic acid**

Step 1 : (S)-tert-butyl 3'-fluoro-4'-((6-((1-phenylethyl)carbamoyl)-3,4-dihydroquinoline-1(2H)-yl)methyl)-[1,1'-biphenyl]-2-carboxylate

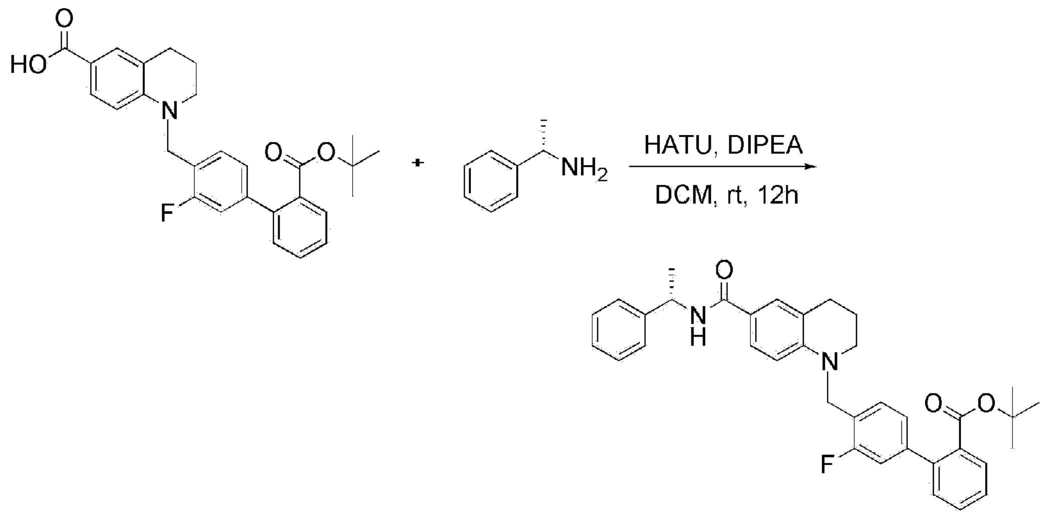


1-((2'-(tert-butoxycarbonyl)-3-fluoro-[1,1'-biphenyl]-4-yl)methyl)-1,2,3,4-tetrahydroquinoline-6-carboxylic acid (300 mg, 0.67 mmol) obtained in Preparative Example 3 was dissolved in $CH_2Cl_2$ (10 ml) in a 100 mL flask, to which HATU (386 mg, 1.01 mmol) and DIPEA (353 ul, 2.02 mmol) were added, followed by stirring. (S)-1-phenylethylamine (90 mg, 0.74 mmol) was added thereto, followed by stirring at room temperature for 12 hours. Upon completion of the reaction, the organic layer was separated by using $CH_2Cl_2$ and $H_2O$. The separated organic layer was dried over $MgSO_4$ and filtered. The mixture was separated by column chromatography (EA/*n*-Hex = 1:1) to give (S)-tert-butyl 3'-fluoro-4'-((6-((1-phenylethyl)carbamoyl)-3,4-dihydroquinoline-1(2H)-yl)methyl)-[1,1'-biphenyl]-2-carboxylate (169 mg, 0.29 mmol, 44 %).

Step 2 : (S)-1-((2'-carbamoyl-3-fluoro-[1,1'-biphenyl]-4-yl)methyl)-N-(1-phenylethyl)-1,2,3,4-tetrahydroquinoline-6-carboxylic acid

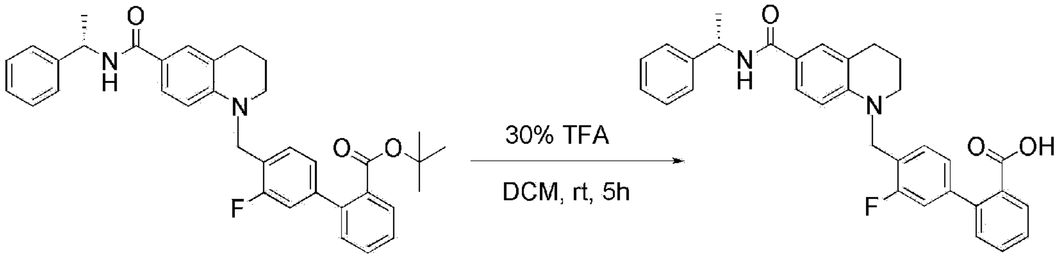


(S)-tert-butyl 3'-fluoro-4'-((6-((1-phenylethyl)carbamoyl)-3,4-dihydroquinoline-1(2H)-yl)methyl)-[1,1'-biphenyl]-2-carboxylate (168 mg, 0.3 mmol) obtained in step 1 was dissolved in $CH_2Cl_2$ (10 ml) in a 50 mL flask, to which 30% TFA (3 ml) was added, followed by stirring at room temperature for 5 hours. Upon completion of the reaction the reaction mixture was concentrated under reduced pressure. The mixture was separated by column chromatography (EA) to give (S)-1-((2'-carbamoyl-3-fluoro-[1,1'-biphenyl]-4-yl)methyl)-N-(1-phenylethyl)-1,2,3,4-tetrahydroquinoline-6-carboxylic acid (83 mg, 0.16 mmol, 54 %).
$^1$H NMR (400 MHz, CDCl3) δ 7.93 (d, *J* = 7.2 Hz, 1H), 7.56-7.25 (m, 10H), 7.10-7.07 (m, 2H), 7.02-7.00 (m, 1H), 6.45 (d, *J* = 7.6 Hz, 1H) , 6.16 (d, *J* = 7.6 Hz, 1H), 5.38-5.32 (m, 1H), 4.60 (s, 2H), 3.47 (t, *J* = 4.0 Hz, 2H), 2.85 (t, *J* = 4.0 Hz, 2H), 2.03 (t, *J* = 4.0 Hz, 2H), 1.57 (d, *J* = 6.8 Hz, 3H).

**<Example 107> (S)-1-((2'-carbamoyl-3-fluoro-[1,1'-biphenyl]-4-yl)methyl)-N-(1-phenylethyl)-1,2,3,4-tetrahydroquinoline-6-carboxamide**

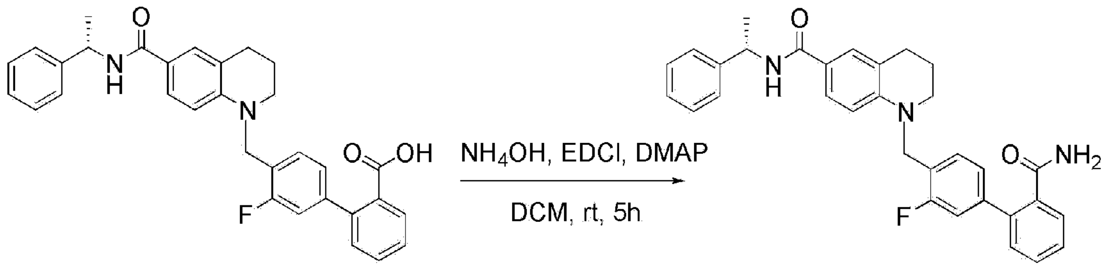


(S)-1-((2'-carbamoyl-3-fluoro-[1,1'-biphenyl]-4-yl)methyl)-N-(1-phenylethyl)-1,2,3,4-tetrahydroquinoline-6-carboxylic acid (78 mg, 0.15 mmol) obtained in Example 106 was dissolved in $CH_2Cl_2$ (2 ml) in a 25 mL flask, to which EDCI (45 mg, 0.23 mmol) and DMAP (24 mg, 0.18 mmol) were added, followed by stirring. Ammonium hydroxide solution (3 ml) was added thereto, followed by stirring at room temperature for 5 hours. Upon completion of the reaction, the organic layer was separated by using $CH_2Cl_2$ and $H_2O$. The separated organic layer was dried over $MgSO_4$ and filtered. The mixture was separated by column chromatography (MeOH/*n*-Hex/$CH_2Cl_2$ = 0.5:0.5:9) to give (S)-1-((2'-carbamoyl-3-fluoro-[1,1'-biphenyl]-4-yl)methyl)-N-(1-phenylethyl)-1,2,3,4-tetrahydroquinoline-6-carboxamide (76 mg, 0.14 mmol, 97.6 %) .
$^1$H NMR (400 MHz, $CDCl_3$) δ 7.73-7.71 (m, 1H), 7.54-7.25 (m, 10H), 7.23-7.14 (m, 3H), 6.46 (d, *J* = 8.8 Hz, 1H), 6.14 (d, *J* = 8.0 Hz, 1H), 5.51 (s, 1H), 5.40 (s, 1H), 5.38-5.31 (m, 1H), 4.62 (s, 2H), 3.49 (t, *J* = 5.6 Hz, 2H), 2.87 (t, *J* = 6.4 Hz, 2H), 2.10-2.04 (m, 2H), 1.59 (d, *J* = 6.8 Hz, 3H).

**<Example 108> (S)-3'-fluoro-4'-((6-((1-(naphthalene-2-yl)ethyl)carbamoyl)-3,4-dihydroquinoline-1(2H)-yl)methyl)-[1,1'-biphenyl]-2-carboxylic acid**

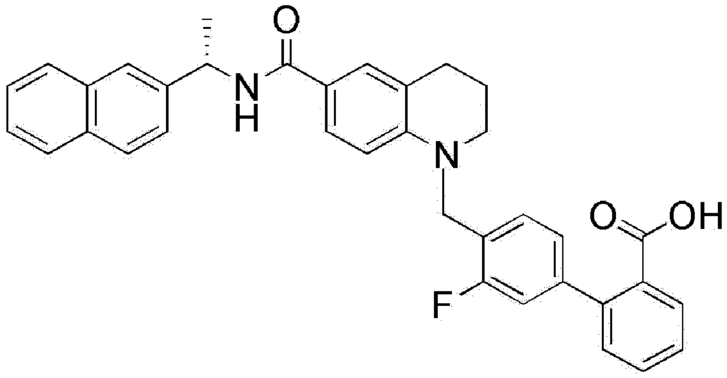

The target compound was obtained by the same manner as described in Example 106 except that (S)-1-(naphthalene-2-yl)ethaneamine was used instead of (S)-1-phenylethylamine.
$^1$H NMR (400 MHz, $CDCl_3$) δ 7.91 (s, 1H), 7.82-7.80 (m, 4H), 7.54-7.36 (m, 8H), 7.32-7.30 (m, 1H), 7.09-7.07 (m, 2H), 7.02-6.98 (m, 1H), 6.43 (d, *J* = 8.4 Hz, 1H), 6.26 (d, *J* = 7.6 Hz, 1H), 5.51-5.47 (m, 1H), 4.58 (s, 2H), 3.44 (t, *J* = 4.0 Hz, 2H), 2.83 (t, *J* = 4.0 Hz, 2H), 2.01 (t, *J* = 4.0 Hz, 2H), 1.65 (d, *J* = 6.8 Hz, 3H).

**<Example 109> (S)-3'-fluoro-4'-((6-((1-(4-nitrophenyl)ethyl)carbamoyl)-3,4-dihydroquinoline-1(2H)-yl)methyl)-[1,1'-biphenyl]-2-carboxylic acid**

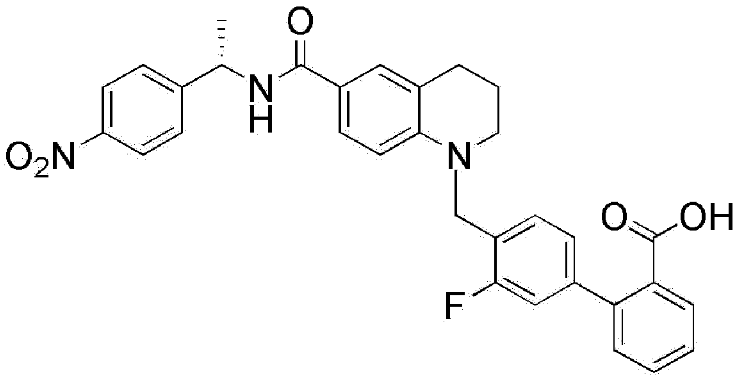

The target compound was obtained by the same manner as described in Example 106 except that (S)-1-(4-nitrophenyl)ethaneamine was used instead of (S)-1-phenylethylamine.
$^1$H NMR (400 MHz, $CDCl_3$) δ 8.17 (d, *J* = 8.8 Hz, 2H), 7.97 (d, *J* = 7.6 Hz, 1H), 7.61-7.57 (m, 1H), 7.53-7.42 (m, 5H), 7.35 (d, *J* = 7.6 Hz, 1H), 7.16-7.09 (m, 2H), 7.03 (d, *J* = 8.0 Hz, 1H), 6.48 (d, *J* = 8.4 Hz, 1H), 6.27 (d, *J* = 7.2 Hz, 1H), 5.38-5.31 (m, 1H), 4.63 (s, 2H), 3.53-3.48 (m, 2H), 2.86 (t, *J* = 6.0 Hz, 2H), 2.07-2.04 (m, 2H), 1.58 (d, *J* = 6.8 Hz, 3H).

**<Example 110> (S)-4'-((6-((1-(4-cyanophenyl)ethyl)carbamoyl)-3,4-dihydroquinoline-1(2H)-yl)methyl)-3'-fluoro-[1,1'-biphenyl]-2-carboxylic acid**

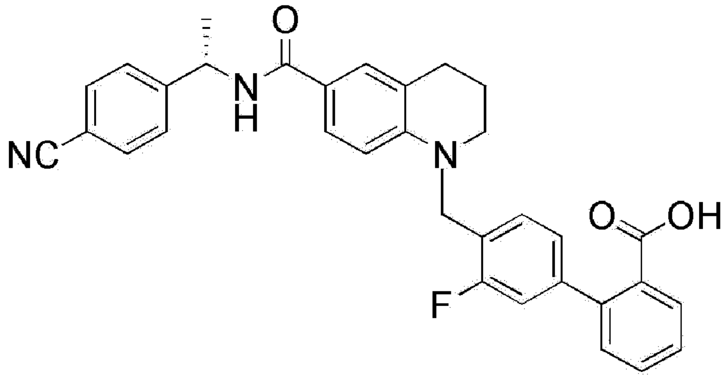

The target compound was obtained by the same manner as described in Example 106 except that (S)-1-(4-cyanophenyl)ethaneamine was used instead of (S)-1-phenylethylamine.
$^1$H NMR (400 MHz, $CDCl_3$) δ 7.98 (d, *J* = 6.4 Hz, 1H), 7.68-7.58 (m, 3H), 7.49-7.42 (m, 6H), 7.36-7.33 (m, 2H), 7.16-7.09 (m, 2H), 7.05-7.02 (m, 1H), 6.49 (d, *J* = 8.8 Hz, 1H), 6.19 (d, *J* = 8.0 Hz, 1H), 5.35-5.30 (m, 1H), 4.64 (s, 2H), 3.51-3.48 (m, 2H), 2.87 (t, *J* = 6.8 Hz, 2H), 2.07-2.00 (m, 2H), 1.57 (d, *J* = 6.8 Hz, 3H).

**<Example 111> (S)-3'-fluoro-4'-((6-((1-(3-fluoro-4-methylphenyl)ethyl)carbamoyl)-3,4-dihydroquinoline-1(2H)-yl)methyl)-[1,1'-biphenyl]-2-carboxylic acid**

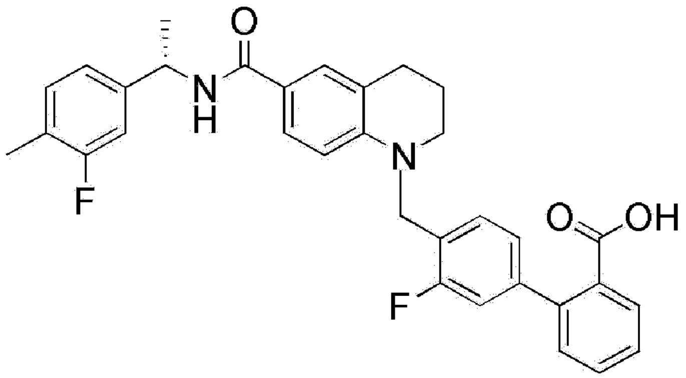

The target compound was obtained by the same manner as described in Example 106 except that (S)-1-(3-fluoro-4-methylphenyl)ethaneamine was used instead of (S)-1-phenylethylamine.
$^1$H NMR (400 MHz, $CDCl_3$) δ 7.97 (d, *J* = 8.0 Hz, 1H), 7.61-7.57 (m, 1H), 7.49-7.35 (m, 5H), 7.16-7.00 (m, 6H), 6.48 (d, *J* = 8.8 Hz, 1H), 6.13 (d, *J* = 7.2 Hz, 1H), 5.32-5.26 (m, 1H), 4.63 (s, 2H), 3.50 (t, *J* = 6.4 Hz, 2H), 2.86 (t, *J* = 5.6 Hz, 2H), 2.26 (s, 3H) 2.12-2.06 (m, 2H), 1.55 (d, *J* = 6.8 Hz, 3H).

**<Example 112> (S)-1-((2'-carbamoyl-3-fluoro-[1,1'-biphenyl]-4-yl)methyl)-N-(1-(4-cyanophenyl)ethyl)-1,2,3,4-tetrahydroquinoline-6-carboxamide**

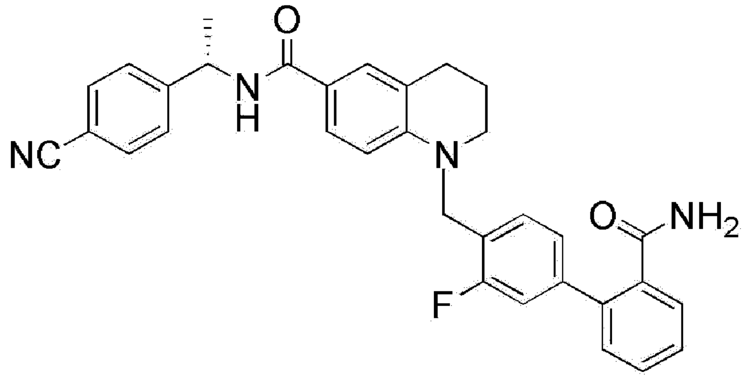

The target compound was obtained by the same manner as described in Example 107 except that (S)-4'-((6-((1-(4-cyanophenyl)ethyl)carbamoyl)-3,4-dihydroquinoline-1(2H)-yl) methyl)-3'-fluoro-[1,1'-biphenyl]-2-carboxylic acid obtained in Example 110 was used instead of (S)-1-((2'-carbamoyl-3-fluoro-[1,1'-biphenyl]-4-yl)methyl)-N-(1-phenylethyl)-1,2,3,4-tetrahydroquinoline-6-carboxylic acid.
$^1$H NMR (400 MHz, $CDCl_3$) δ 7.72 (d, *J* = 8.0 Hz, 1H), 7.63 (d, *J* = 8.0 Hz, 1H), 7.54-7.43 (m, 6H), 7.36 (d, *J* = 8.4 Hz, 1H), 7.23-7.15 (m, 3H), 6.48 (d, *J* = 8.8 Hz, 1H), 6.17 (d, *J* = 7.2 Hz, 1H), 5.48 (s, 1H), 5.41 (s, 1H), 5.36-5.29 (m, 1H), 4.64 (s, 2H), 3.51 (t, *J* = 5.6 Hz, 2H), 2.88 (t, *J* = 5.6 Hz, 2H), 2.09-2.05 (m, 2H), 1.58 (d, *J* = 7.2 Hz, 3H).

**<Example 113> (S)-1-((2'-carbamoyl-3-fluoro-[1,1'-biphenyl]-4-yl)methyl)-N-(1-(4-nitrophenyl)ethyl)-1,2,3,4-tetrahydroquinoline-6-carboxamide**

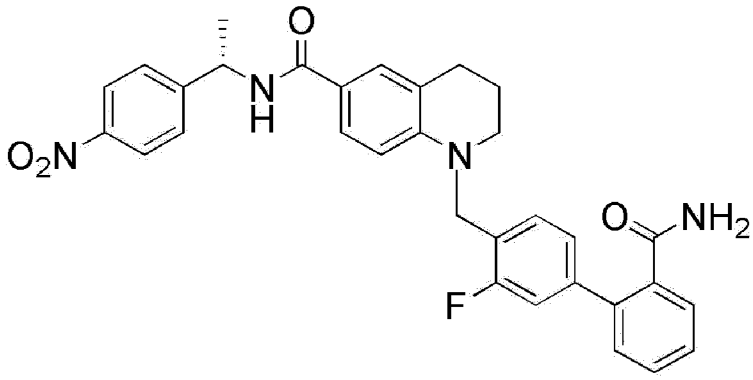

The target compound was obtained by the same manner as described in Example 107 except that (S)-3'-fluoro-4'-((6-((1-(4-nitrophenyl)ethyl)carbamoyl)-3,4-dihydroquinoline-1(2H)-yl)methyl)-[1,1'-biphenyl]-2-carboxylic acid obtained in Example 109 was used instead of (S)-1-((2'-carbamoyl-3-fluoro-[1,1'-biphenyl]-4-yl)methyl)-N-(1-phenylethyl)-1,2,3,4-tetrahydroquinoline-6-carboxylic acid.
$^1$H NMR (400 MHz, $CDCl_3$) δ 8.20 (d, *J* = 8.8 Hz, 2H), 7.72 (d, *J* = 6.8 Hz, 1H), 7.55-7.44 (m, 6H), 7.36 (d, *J* = 5.6 Hz, 1H), 7.23-7.15 (m, 3H), 6.49 (d, *J* = 8.8 Hz, 1H), 6.20 (d, *J* = 7.2 Hz, 1H), 5.47 (s, 1H), 5.41 (s, 1H), 5.38-5.35 (m, 1H), 4.64 (s, 2H), 3.51 (t, *J* = 5.6 Hz, 2H), 2.89 (t, *J* = 6.0 Hz, 2H), 2.09-2.07 (m, 2H), 1.61 (d, *J* = 7.2 Hz, 3H).

**<Example 114> (S)-1-((2'-carbamoyl-3-fluoro-[1,1'-biphenyl]-4-yl)methyl)-N-(1-(3-fluoro-4-methylphenyl)ethyl)-1,2,3,4-tetrahydroquinoline-6-carboxamide**

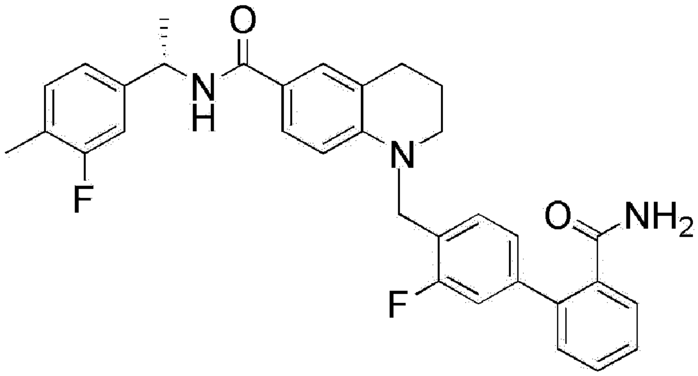

The target compound was obtained by the same manner as described in Example 107 except that (S)-3'-fluoro-4'-((6-((1-(3-fluoro-4-methylphenyl)ethyl)carbamoyl)-3,4-dihydroquinoline-1(2H)-yl)methyl)-[1,1'-biphenyl]-2-carboxylic acid obtained in Example 111 was used instead of (S)-1-((2'-carbamoyl-3-fluoro-[1,1'-biphenyl]-4-yl)methyl)-N-(1-phenylethyl)-1,2,3,4-tetrahydroquinoline-6-carboxylic acid.

$^1$H NMR (400 MHz, $CDCl_3$) δ 7.74-7.71 (m, 1H), 7.54-7.33 (m, 5H), 7.23-7.13 (m, 4H), 7.07-7.02 (m, 2H), 6.47 (d, *J* = 8.4 Hz, 1H), 6.09 (d, *J* = 7.6 Hz, 1H), 5.49 (s, 1H), 5.40 (s, 1H), 5.32-5.25 (m, 1H), 4.63 (s, 2H), 3.50 (t, *J* = 5.2 Hz, 2H), 2.88 (t, *J* = 6.0 Hz, 2H), 2.26 (s, 3H), 2.10-2.04 (m, 2H), 1.56 (d, *J* = 6.8 Hz, 3H).

**<Example 115> (S)-1-((2'-carbamoyl-3-fluoro-[1,1'-biphenyl]-4-yl)methyl)-N-(1-(naphthalene-2-yl)ethyl)-1,2,3,4-tetrahydroquinoline-6-carboxamide**

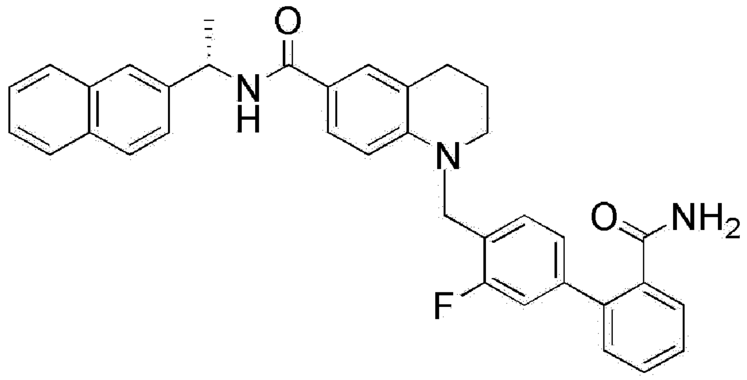

The target compound was obtained by the same manner as described in Example 107 except that (S)-3'-fluoro-4'-((6-((1-(naphthalene-2-yl)ethyl)carbamoyl)-3,4-dihydroquinoline-1(2H)-yl)methyl)-[1,1'-biphenyl]-2-carboxylic acid obtained in Example 108 was used instead of (S)-1-((2'-carbamoyl-3-fluoro-[1,1'-biphenyl]-4-yl)methyl)-N-(1-phenylethyl)-1,2,3,4-tetrahydroquinoline-6-carboxylic acid.

$^1$H NMR (400 MHz, $CDCl_3$) δ 7.85-7.83 (m, 4H), 7.73-7.71 (m, 1H), 7.53-7.43 (m, 7H), 7.37-7.35 (m, 1H), 7.22-7.14 (m 3H), 6.47 (d, *J* = 8.8 Hz, 1H), 6.22 (d, *J* = 7.6 Hz, 1H), 5.55-5.48 (m, 2H), 5.40 (s, 1H), 4.63 (s, 2H), 3.50 (t, *J* = 5.6 Hz, 2H), 2.88 (t, *J* = 6.0 Hz, 2H), 2.09-2.04 (m, 2H), 1.69 (d, *J* = 6.8 Hz, 3H).

**<Example 116> (S)-3'-((6-((1-phenylethyl)carbamoyl)-3,4-dihydroquinoline-1(2H)-yl)methyl)-[1,1'-biphenyl]-2-carboxylic** acid

<u>Step 1 : (S)-tert-butyl 3'-((6-((1-phenylethyl)carbamoyl)-3,4-dihydroquinoline-1(2H)-yl)methyl)-[1,1'-biphenyl]-2-carboxylate</u>

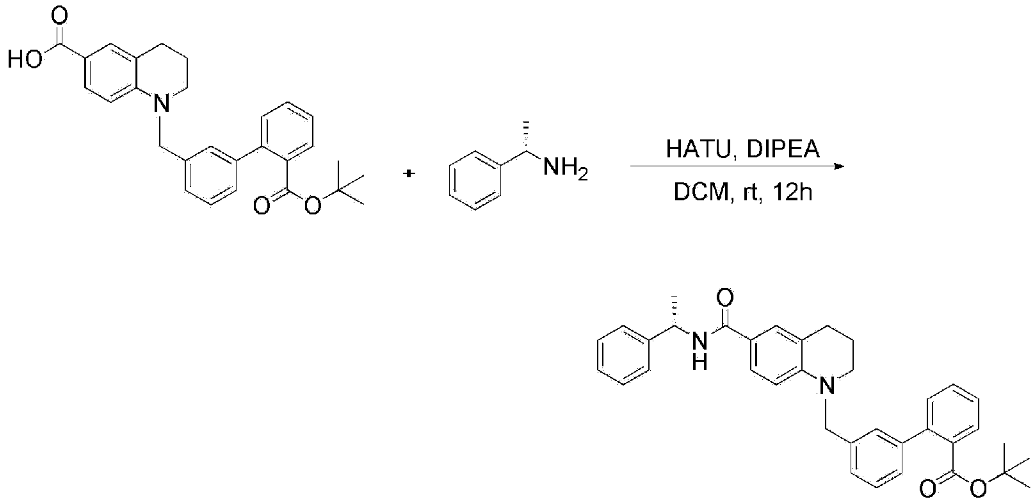


1-((2'-(tert-butoxycarbonyl)-[1,1'-biphenyl]-3-yl)methyl)-1,2,3,4-tetrahydroquinoline-6-carboxylic acid (300 mg, 0.67 mmol) obtained in Preparative Example 4 was dissolved in $CH_2Cl_2$ (10 ml) in a 100 mL flask, to which HATU (386 mg, 1.01 mmol) and DIPEA (353 ul, 2.02 mmol) were added, followed by stirring. (S)-1-phenylethylamine (90 mg, 0.74 mmol) was added thereto, followed by stirring at room temperature for 12 hours. Upon completion of the reaction, the organic layer was separated by using $CH_2Cl_2$ and $H_2O$. The separated organic layer was dried over $MgSO_4$ and filtered. The mixture was separated by column chromatography (EA/*n*-Hex = 1:1) to give (S)-tert-butyl 3'-((6-((1-phenylethyl)carbamoyl)-3,4-dihydroquinoline-1(2H)-yl)methyl)-[1,1'-biphenyl]-2-carboxylate (169 mg, 0.29 mmol, 44 %).

<u>Step 2 : (S)-3'-((6-((1-phenylethyl)carbamoyl)-3,4-dihydroquinoline-1(2H)-yl)methyl)-[1,1'-biphenyl]-2-carboxylic acid</u>

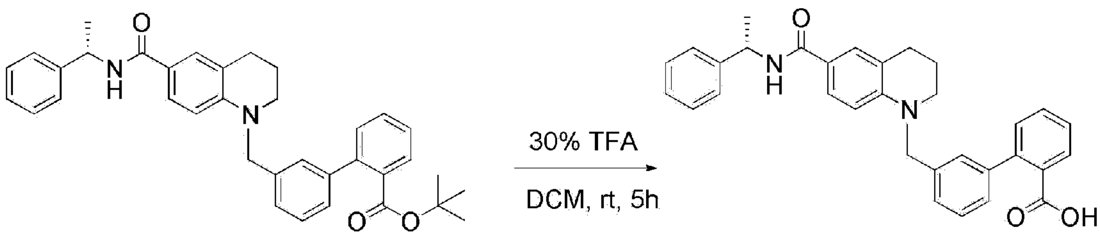


(S)-tert-butyl 3'-((6-((1-phenylethyl)carbamoyl)-3,4-dihydroquinoline-1(2H)-yl)methyl)-[1,1'-biphenyl]-2-carboxylate (168 mg, 0.3 mmol) obtained in step 1 was dissolved in $CH_2Cl_2$ (10 ml) in a 50 mL flask, to which 30% TFA (3 ml) was added, followed by stirring at room temperature for 5 hours. Upon completion of the reaction the reaction mixture was concentrated under reduced pressure. The mixture was separated by column chromatography (EA) to give (S)-3'-((6-((1-phenylethyl)carbamoyl)-3,4-dihydroquinoline-1(2H)-yl)methyl)-[1,1'-biphenyl]-2-carboxylic acid (83 mg, 0.16 mmol, 54 %).
$^1H$ NMR (400 MHz, $CDCl_3$) $\delta$ 7.89 (d, *J* = 8.0 Hz, 1H), 7.56-7.52 (m, 1H), 7.43-7.32 (m, 10H), 7.27-7.21 (m, 2H), 7.14 (s, 1H), 6.46 (d, *J* = 9.2 Hz, 1H), 6.17 (d, *J* = 8.0 Hz, 1H), 5.35-5.28 (m, 1H), 4.56 (s, 2H), 3.44 (t, *J* = 5.6 Hz, 2H), 2.81 (t, *J* = 6.0 Hz, 2H), 2.02-1.96 (m, 2H), 1.56 (d, *J* = 6.4 Hz, 3H).

**<Example 117> (S)-1-((2'-carbamoyl-[1,1'-biphenyl]-3-yl)methyl)-N-(1-phenylethyl)-1,2,3,4-tetrahydroquinoline-6-carboxamide**

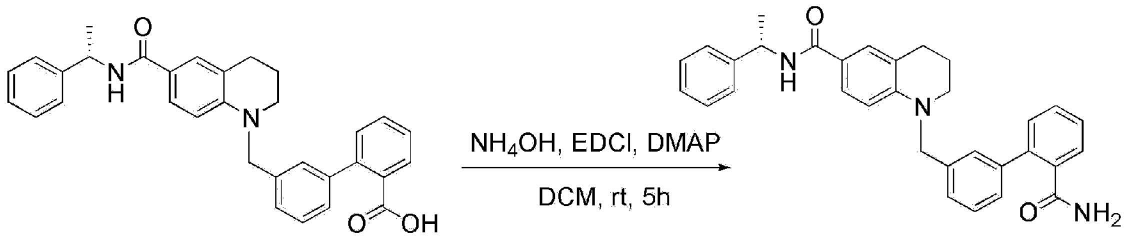


(S)-3'-((6-((1-phenylethyl)carbamoyl)-3,4-dihydroquinoline-1(2H)-yl)methyl)-[1,1'-biphenyl]-2-carboxylic acid (78 mg, 0.15 mmol) obtained in Example 116 was dissolved in $CH_2Cl_2$ (2 ml) in a 25 mL flask, to which EDCI (45 mg, 0.23 mmol) and DMAP (24 mg, 0.18 mmol) were added, followed by stirring at room temperature for 5 hours. Upon completion of the reaction, the organic layer was separated by using $CH_2Cl_2$ and $H_2O$. The separated organic layer was dried over $MgSO_4$ and filtered. The mixture was separated by column chromatography (MeOH/*n*-Hex/$CH_2Cl_2$ = 0.5:0.5:9) to give (S)-1-((2'-carbamoyl-[1,1'-biphenyl]-3-yl)methyl)-N-(1-phenylethyl)-1,2,3,4-tetrahydroquinoline-6-carboxamide (76 mg, 0.14 mmol, 97.6 %) .
$^1$H NMR (400 MHz, $CDCl_3$) δ 7.75-7.73 (m, 1H), 7.54-7.02 (m, 14H), 6.45 (d, *J* = 8.8 Hz, 1H), 6.17 (d, *J* = 8.0 Hz, 1H), 5.37-5.30 (m, 1H), 5.13 (s, 2H), 4.59 (s, 2H), 3.53-3.48 (m, 2H), 2.87 (t, *J* = 6.4 Hz, 2H), 2.07-2.03 (m, 2H), 1.59 (d, *J* = 6.8 Hz, 3H).

**<Example 118> (S)-3'-((6-((1-(4-cyanophenyl)ethyl)carbamoyl)-3,4-dihydroquinoline-1(2H)-yl)methyl)-[1,1'-biphenyl]-2-carboxylic acid**

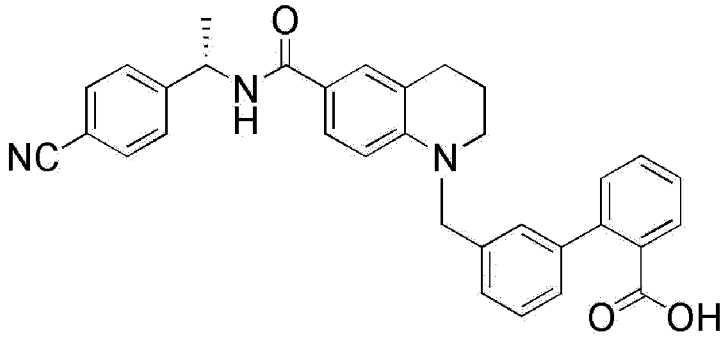

The target compound was obtained by the same manner as described in Example 116 except that (S)-1-(4-cyanophenyl)ethaneamine was used instead of (S)-1-phenylethylamine.
$^1$H NMR (400 MHz, $CDCl_3$) δ 7.89 (d, *J* = 7.6 Hz, 1H), 7.60-7.53 (m, 3H), 7.45-7.33 (m, 8H), 7.26-7.23 (m, 1H), 7.14 (s, 1H), 6.44 (d, *J* = 8.8 Hz, 1H), 6.28 (d, *J* = 7.6 Hz, 1H), 5.31-5.24 (m, 1H), 4.58 (s, 2H), 3.47 (t, *J* = 5.2 Hz, 2H), 2.82 (t, *J* = 6.0 Hz, 2H), 2.07-1.99 (m, 2H), 1.51 (d, *J* = 6.8 Hz, 3H).

**<Example 119> (S)-3'-((6-((1-(4-nitrophenyl)ethyl)carbamoyl)-3,4-dihydroquinoline-1(2H)-yl)methyl)-[1,1'-biphenyl]-2-carboxylic acid**

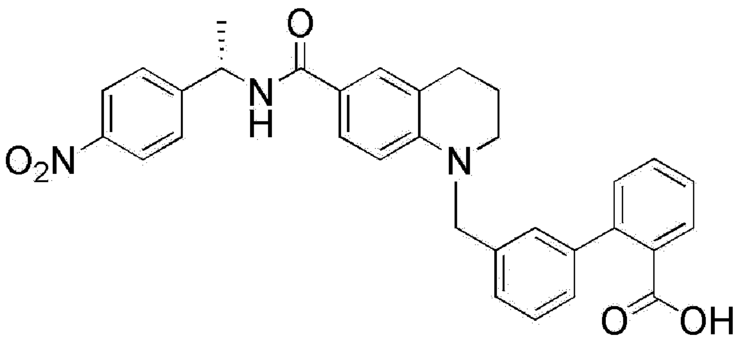

The target compound was obtained by the same manner as described in Example 116 except that (S)-1-(4-nitrophenyl)ethaneamine was used instead of (S)-1-phenylethylamine.
$^1$H NMR (400 MHz, $CDCl_3$) δ 8.14 (d, *J* = 8.8 Hz, 2H), 7.57-7.53 (m, 1H), 7.48-7.34 (m, 8H), 7.27-7.23 (m, 2H), 7.14 (s, 1H), 6.42 (d, *J* = 8.8 Hz, 1H), 6.35 (d, *J* = 7.2 Hz, 1H), 5.34-5.28 (m, 1H), 4.58 (s, 2H), 3.46 (t, *J* = 6.0 Hz, 2H), 2.81 (t, *J* = 6.0 Hz, 2H), 2.07-1.97 (m, 2H), 1.52 (d, *J* = 7.2 Hz, 3H).

**<Example 120> (S)-3'-((6-((1-(naphthalene-2-yl)ethyl)carbamoyl)-3,4-dihydroquinoline-1(2H)-yl)methyl)-[1,1'-biphenyl]-2-carboxylic acid**

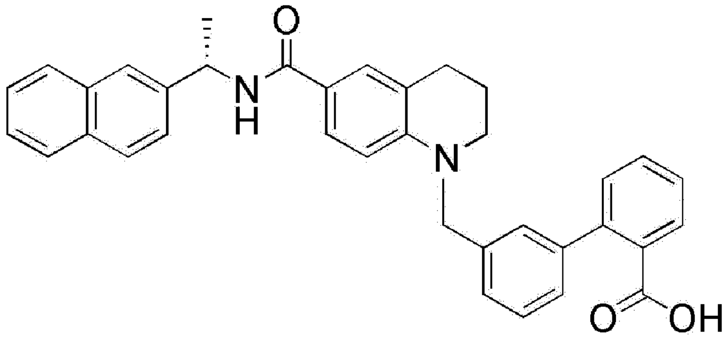

The target compound was obtained by the same manner as described in Example 116 except that (S)-1-(naphthalene-2-yl)ethaneamine was used instead of (S)-1-phenylethylamine.
$^1$H NMR (400 MHz, $CDCl_3$) δ 7.89-7.81 (m, 5H), 7.55-7.33 (m, 9H), 7.28-7.21 (m, 2H), 7.14 (s, 1H), 6.46 (d, *J* = 9.2 Hz, 1H), 6.27 (d, *J* = 8.0 Hz, 1H), 5.52-5.45 (m, 1H), 4.56 (s, 2H), 3.43 (t, *J* = 5.6 Hz, 2H), 2.80 (t, *J* = 6.0 Hz, 2H), 2.07-1.95 (m, 2H), 1.54 (d, *J* = 6.8 Hz, 3H).

**<Example 121> (S)-3'-((6-((1-(3-fluoro-4-methylphenyl)ethyl)carbamoyl)-3,4-dihydroquinoline-1(2H)-yl)methyl)-[1,1'-biphenyl]-2-carboxylic acid**

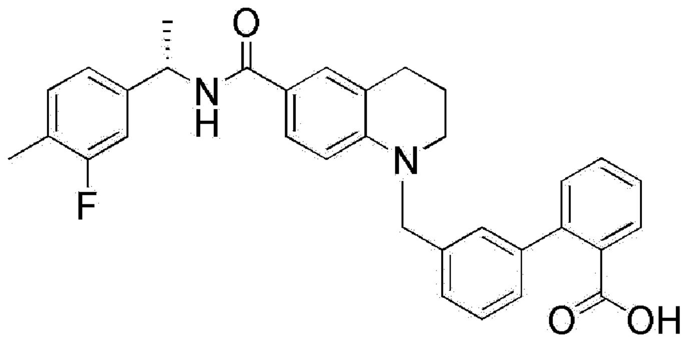

The target compound was obtained by the same manner as described in Example 116 except that (S)-1-(3-fluoro-4-methylphenyl)ethaneamine was used instead of (S)-1-phenylethylamine.
$^1$H NMR (400 MHz, $CDCl_3$) δ 7.88 (d, *J* = 7.2 Hz, 1H), 7.56-7.52 (m, 1H), 7.42-7.34 (m, 5H), 7.25-7.21 (m, 2H), 7.14-7.11 (m, 2H), 7.04-6.99 (m, 2H), 6.46 (d, *J* = 9.2 Hz, 1H), 6.15 (d, *J* = 7.6 Hz, 1H), 5.29-5.21 (m, 1H), 4.57 (s, 2H), 3.44 (t, *J* = 6.0 Hz, 2H), 2.81 (t, *J* = 6.0 Hz, 2H), 2.25 (s, 3H), 2.01-1.98 (m. 2H), 1.52 (d, *J* = 6.8 Hz, 3H).

**<Example 122> (S)-1-((2'-carbamoyl-[1,1'-biphenyl]-3-yl)methyl)-N-(1-(4-cyanophenyl)ethyl)-1,2,3,4-tetrahydroquinoline-6-carboxamide**

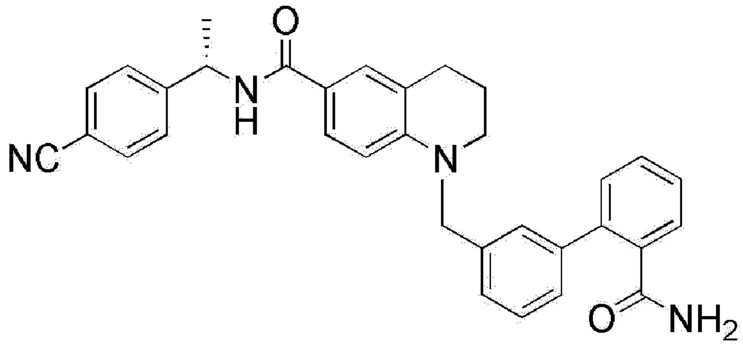

The target compound was obtained by the same manner as described in Example 117 except that (S)-3'-((6-((1-(4-cyanophenyl)ethyl)carbamoyl)-3,4-dihydroquinoline-1(2H)-yl)methyl)-[1,1'-biphenyl]-2-carboxylic acid obtained in Example 118 was used instead of (S)-3'-((6-((1-phenylethyl)carbamoyl)-3,4-dihydroquinoline-1(2H)-yl)methyl)-[1,1'-biphenyl]-2-carboxylic acid.
$^1$H NMR (400 MHz, $CDCl_3$) δ 7.75-7.73 (m, 1H), 7.63 (d, *J* = 8.4 Hz, 2H), 7.54-7.31 (m, 11H), 6.46 (d, *J* = 8.8 Hz, 2H), 6.29 (d, *J* = 6.8 Hz, 2H), 5.34-5.27 (m, 1H), 5.14 (s, 2H), 4.60 (s, 2H), 3.53-3.47 (m, 2H), 2.87 (t, *J* = 6.8 Hz, 2H), 2.09-2.03 (m, 2H), 1.57 (d, *J* = 3.6 Hz, 3H).

**<Example 123> (S)-1-((2'-carbamoyl-[1,1'-biphenyl]-3-yl)methyl)-N-(1-(4-nitrophenyl)ethyl)-1,2,3,4-tetrahydroquinoline-6-carboxamide**

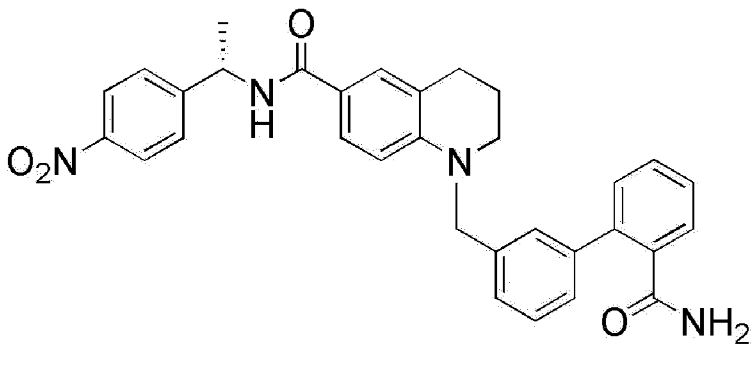

The target compound was obtained by the same manner as described in Example 117 except that (S)-3'-((6-((1-(4-nitrophenyl)ethyl)carbamoyl)-3,4-dihydroquinoline-1(2H)-yl)methyl)-[1,1'-biphenyl]-2-carboxylic acid obtained in Example 119 was used instead of (S)-3'-((6-((1-phenylethyl)carbamoyl)-3,4-dihydroquinoline-1(2H)-yl)methyl)-[1,1'-biphenyl]-2-carboxylic acid.
$^1$H NMR (400 MHz, $CDCl_3$) δ 8.20-8.18 (m, 2H), 7.75-7.73 (m, 1H), 7.55-7.49 (m, 3H), 7.46-7.41 (m, 4H), 7.38-7.36 (m, 2H), 7.31-7.28 (m, 2H), 6.46 (d, *J* = 8.8 Hz, 1H), 6.34 (d, *J* = 7.2 Hz, 1H), 5.36-5.32 (m, 1H), 5.14 (s, 2H), 4.60 (s, 2H), 3.54-3.48 (m, 2H), 2.87 (t, *J* = 6.0 Hz, 2H), 2.09-2.03 (m, 2H), 1.59 (d, *J* = 7.2 Hz, 3H).

**<Example 124> (S)-1-((2'-carbamoyl-[1,1'-biphenyl]-3-yl)methyl)-N-(1-(naphthalene-2-yl)ethyl)-1,2,3,4-tetrahydroquinoline-6-carboxamide**

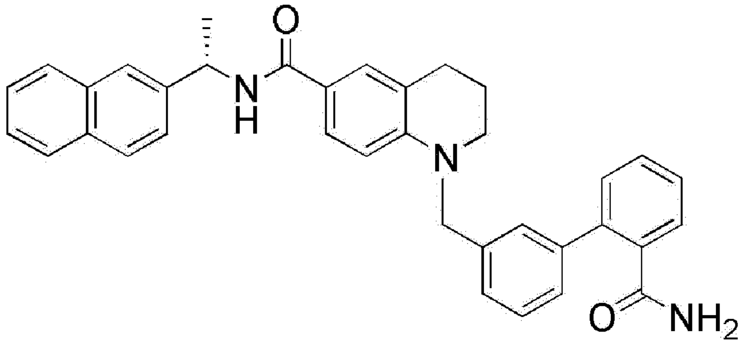

The target compound was obtained by the same manner as described in Example 117 except that (S)-3'-((6-((1-(naphthalene-2-yl)ethyl)carbamoyl)-3,4-dihydroquinoline-1(2H)-yl)methyl)-[1,1'-biphenyl]-2-carboxylic acid obtained in Example 120 was used instead of (S)-3'-((6-((1-phenylethyl)carbamoyl)-3,4-dihydroquinoline-1(2H)-yl)methyl)-[1,1'-biphenyl]-2-carboxylic acid.

$^1$H NMR (400 MHz, $CDCl_3$) δ 7.98-7.81 (m, 4H), 7.74-7.73 (m, 1H), 7.54-7.28 (m, 12H), 6.46 (d, *J* = 8.8 Hz, 1H), 6.27 (d, *J* = 7.2 Hz, 1H), 5.52-5.49 (m, 1H),5.13 (s, 2H), 4.59 (s, 2H), 3.51-3.48 (m, 2H), 2.87 (t, *J* = 5.6 Hz, 2H), 2.09-2.05 (m, 2H), 1.67 (d, *J* = 6.8 Hz, 3H).

**<Example 125> (S)-1-((2'-carbamoyl-[1,1'-biphenyl]-3-yl)methyl)-N-(1-(3-fluoro-4-methylphenyl)ethyl)-1,2,3,4-tetrahydroquinoline-6-carboxamide**

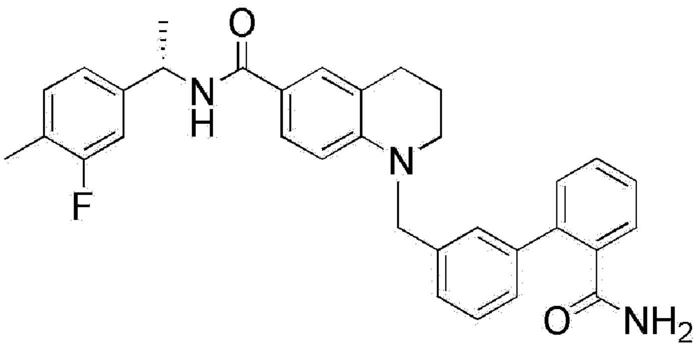

The target compound was obtained by the same manner as described in Example 117 except that (S)-3'-((6-((1-(3-fluoro-4-methylphenyl)ethyl)carbamoyl)-3,4-dihydroquinoline-1(2H)-yl)methyl)-[1,1'-biphenyl]-2-carboxylic acid obtained in Example 121 was used instead of (S)-3'-((6-((1-phenylethyl)carbamoyl)-3,4-dihydroquinoline-1(2H)-yl)methyl)-[1,1'-biphenyl]-2-carboxylic acid.

$^1$H NMR (400 MHz, $CDCl_3$) δ 7.75-7.30 (m, 1H), 7.54-7.23 (m, 10H), 7.16-7.13 (m, 1H), 7.07-7.01 (m, 2H), 6.46 (d, *J* = 8.8 Hz, 1H), 6.15 (d, *J* = 8.0 Hz, 1H), 5.30-5.23 (m, 1H), 5.09 (s, 2H), 4.59 (s, 2H), 3.53-3.48 (m, 2H), 2.87 (t, *J* = 6.0 Hz, 2H), 2.26 (s, 2H), 2.09-2.04 (m, 3H), 1.55 (d, *J* = 6.8 Hz, 3H).

**<Example 126> (S)-2'-fluoro-3'-((6-((1-phenylethyl)carbamoyl)-3,4-dihydroquinoline-1(2H)-yl)methyl)-[1,1'-biphenyl]-2-carboxylic acid**

Step 1 : (S)-tert-butyl 2'-fluoro-3'-((6-((1-phenylethyl)carbamoyl)-3,4-dihydroquinoline-1(2H)-yl)methyl)-[1,1'-biphenyl]-2-carboxylate

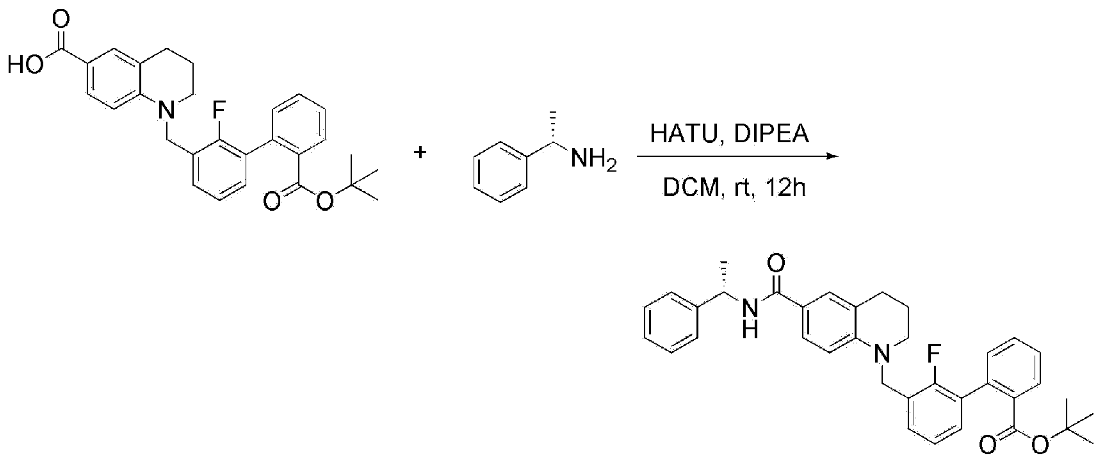


1-((2'-(tert-butoxycarbonyl)-2-fluoro-[1,1'-biphenyl]-3-yl)methyl)-1,2,3,4-tetrahydroquinoline-6-carboxylic acid (300 mg, 0.67 mmol) obtained in Preparative Example 5 was dissolved in $CH_2Cl_2$ (10 ml) in a 50 mL flask, to which HATU (386 mg, 1.01 mmol) and DIPEA (353 ul, 2.02 mmol) were added, followed by stirring. (S)-1-phenylethylamine (105 ul, 0.81 mmol) was added, followed by stirring at room temperature for 12 hours. Upon completion of the reaction, the organic layer was separated by using $CH_2Cl_2$ and $H_2O$. The separated organic layer was dried over $MgSO_4$ and filtered. The mixture was separated by column chromatography (EA/*n*-Hex = 1:1) to give (S)-tert-butyl 2'-fluoro-3'-((6-((1-phenylethyl)carbamoyl)-3,4-dihydroquinoline-1(2H)-yl)methyl)-[1,1'-biphenyl]-2-carboxylate (169 mg, 0.29 mmol, 44 %).

Step 2 : (S)-2'-fluoro-3'-((6-((1-phenylethyl)carbamoyl)-3,4-dihydroquinoline-1(2H)-yl)methyl)-[1,1'-biphenyl]-2-carboxylic acid

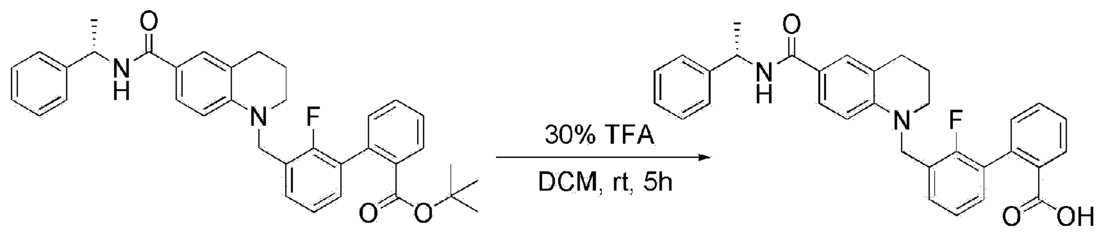


(S)-tert-butyl 2'-fluoro-3'-((6-((1-phenylethyl)carbamoyl)-3,4-dihydroquinoline-1(2H)-yl)methyl)-[1,1'-biphenyl]-2-carboxylate (168 mg, 0.3 mmol) obtained in step 1 was dissolved in $CH_2Cl_2$ (10 ml) in a 50 mL flask, to which 30% TFA (3 ml) was added, followed by stirring at room temperature for 5 hours. Upon completion of the reaction the reaction mixture was concentrated under reduced pressure. The mixture was separated by column chromatography (EA) to give (S)-2'-fluoro-3'-((6-((1-phenylethyl)carbamoyl)-3,4-dihydroquinoline-1(2H)-yl)methyl)-[1,1'-biphenyl]-2-carboxylic acid (83 mg, 0.16 mmol, 54 %).
$^1$H NMR (400 MHz, $CDCl_3$) $\delta$ 8.08 (d, *J* = 7.2 Hz, 1H), 7.66-7.62 (m, 1H), 7.54-7.33 (m, 9H), 7.25-7.21 (m, 2H), 7.17-7.02 (m, 1H), 6.47 (d, *J* = 8.4 Hz, 1H), 6.14 (d, *J* = 7.6 Hz, 1H), 5.36-5.32 (m, 1H), 4.57 (s, 2H), 3.46 (d, *J* = 5.6 Hz, 1H), 2.84 (t, *J* = 6.0 Hz, 2H), 2.07-2.00 (m, 2H), 1.58 (d, *J* = 6.8 Hz, 3H).

**<Example 127> (S)-1-((2'-carbamoyl-2-fluoro-[1,1'-biphenyl]-3-yl)methyl)-N-(1-phenylethyl)-1,2,3,4-tetrahydroquinoline-6-carboxamide**

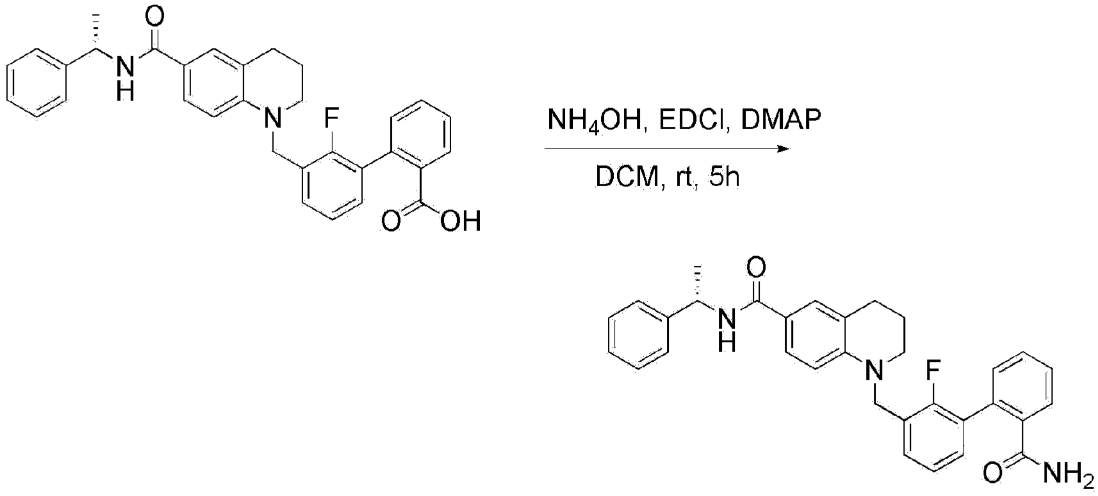


(S)-2'-fluoro-3'-((6-((1-phenylethyl)carbamoyl)-3,4-dihydroquinoline-1(2H)-yl)methyl)-[1,1'-biphenyl]-2-carboxylic acid (78 mg, 0.15 mmol) obtained in Example 126 was dissolved in $CH_2Cl_2$ (2 ml) in a 25 mL flask, to which EDCI (45 mg, 0.23 mmol) and DMAP (24 mg, 0.18 mmol) were added, followed by stirring. Ammonium hydroxide solution (3 ml) was added thereto, followed by stirring at room temperature for 5 hours. Upon completion of the reaction, the organic layer was separated by using $CH_2Cl_2$ and $H_2O$. The separated organic layer was dried over $MgSO_4$ and filtered. The mixture was separated by column chromatography (MeOH/*n*-Hex/$CH_2Cl_2$ = 0.5:0.5:9) to give (S)-1-((2'-carbamoyl-2-fluoro-[1,1'-biphenyl]-3-yl)methyl)-N-(1-phenylethyl)-1,2,3,4-tetrahydroquinoline-6-carboxamide (76 mg, 0.14 mmol, 97.6 %) .
$^1$H NMR (400 MHz, $CDCl_3$) δ 7.83-7.81 (m, 1H), 7.59-7.54 (m, 1H), 7.52-7.48 (m, 2H), 7.45-7.26 (m, 8H), 7.21-7.13 (m, 2H), 7.31-7.28 (m, 2H), 6.46 (d, *J* = 8.8 Hz, 1H), 6.15 (d, *J* = 8.4 Hz, 1H), 5.45-5.32 (m, 3H), 4.62 (s, 2H), 3.49 (d, *J* = 6.0 Hz, 1H), 2.87 (t, *J* = 6.8 Hz, 2H), 2.09-2.03 (m, 2H), 1.59 (d, *J* = 6.8 Hz, 3H).

**<Example 128> (S)-2'-fluoro-3'-((6-((1-(4-nitrophenyl)ethyl)carbamoyl)-3,4-dihydroquinoline-1(2H)-yl)methyl)-[1,1'-biphenyl]-2-carboxylic acid**

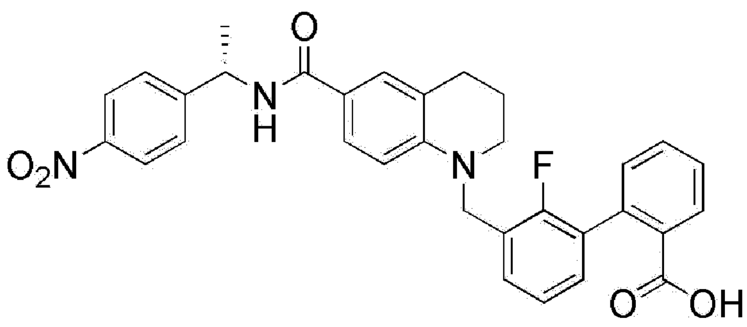

The target compound was obtained by the same manner as described in Example 126 except that (S)-1-(4-nitrophenyl)ethaneamine was used instead of (S)-1-phenylethylamine.
$^1$H NMR (400 MHz, $CDCl_3$) 8.19-8.17 (m, 2H), 7.10-7.08 (m, 1H), 7.78-7.63 (m, 1H), 7.53-7.39 (m, 6H), 7.26-7.24 (m, 1H), 7.16-7.13 (m, 2H), 6.49 (d, *J* = 8.4 Hz, 1H), 6.22 (d, *J* = 6.8 Hz, 1H), 5.37-5.32 (m, 1H), 4.59 (s, 2H), 3.49 (t, *J* = 6.0 Hz, 2H), 2.85 (t, *J* = 6.4 Hz, 2H), 2.07-2.03 (m, 2H), 1.57 (d, *J* = 6.8 Hz, 3H).

**<Example 129> (S)-2'-fluoro-3'-((6-((1-(naphthalene-2-yl)ethyl)carbamoyl)-3,4-dihydroquinoline-1(2H)-yl)methyl)-[1,1'-biphenyl]-2-carboxylic acid**

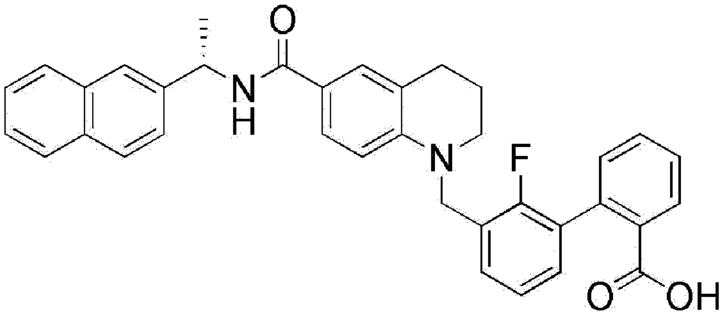

The target compound was obtained by the same manner as described in Example 126 except that (S)-1-(naphthalene-2-yl)ethaneamine was used instead of (S)-1-phenylethylamine.
$^1$H NMR (400 MHz, $CDCl_3$) δ 8.08-8.06 (m, 1H), 7.84-7.81 (m, 4H), 7.63-7.59 (m, 1H), 7.54-7.43 (m, 6H), 7.39-7.37 (m, 1H), 7.23-7.20 (m, 1H), 7.16-7.09 (m, 2H), 6.46 (d, *J* = 8.4 Hz, 1H), 6.23 (d, *J* = 8.4 Hz, 1H), 5.52-5.49 (m, 1H), 4.55 (s, 2H), 3.45 (t, *J* = 6.0 Hz, 2H), 2.83 (t, *J* = 6.0 Hz, 2H), 2.07-2.00 (m, 2H), 1.66 (d, *J* = 6.8 Hz, 3H).

**<Example 130> (S)-2'-fluoro-3'-((6-((1-(3-fluoro-4-methylphenyl)ethyl)carbamoyl)-3,4-dihydroquinoline-1(2H)-yl)methyl)-[1,1'-biphenyl]-2-carboxylic acid**

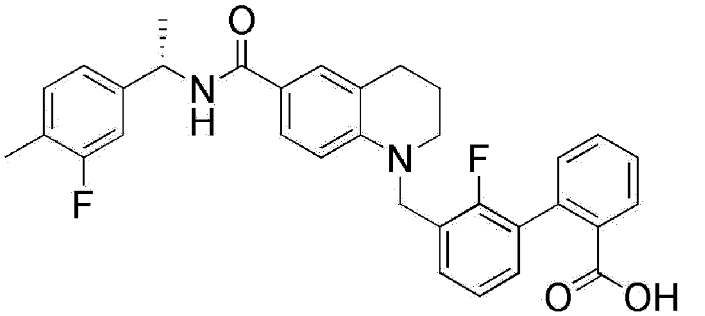

The target compound was obtained by the same manner as described in Example 126 except that (S)-1-(3-fluoro-4-methylphenyl)ethaneamine was used instead of (S)-1-phenylethylamine.
$^1$H NMR (400 MHz, $CDCl_3$) δ 8.10-8.08 (m, 1H), 7.66-7.62 (m, 1H), 7.54-7.39 (m, 4H), 7.25-7.21 (m, 1H), 7.16-7.10 (m, 3H), 7.06-7.00 (m, 2H), 6.48 (d, *J* = 8.8 Hz, 1H), 6.10 (d, *J* = 7.2 Hz, 1H), 5.29-5.26 (m, 1H), 4.58 (s, 2H), 3.47 (t, *J* = 6.0 Hz, 2H), 2.85 (t, *J* = 5.6 Hz, 2H), 2.25 (s, 3H), 2.07-2.01 (m, 2H), 1.54 (d, *J* = 6.8 Hz, 3H).

**<Example 131> (S)-1-((2'-carbamoyl-2-fluoro-[1,1'-biphenyl]-3-yl)methyl)-N-(1-(4-nitrophenyl)ethyl)-1,2,3,4-tetrahydroquinoline-6-carboxamide**

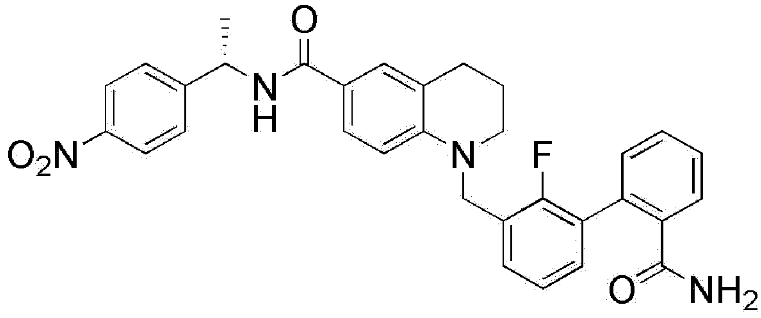

The target compound was obtained by the same manner as described in Example 127 except that (S)-2'-fluoro-3'-((6-((1-(4-nitrophenyl)ethyl)carbamoyl)-3,4-dihydroquinoline-1(2H)-yl)methyl)-[1,1'-biphenyl]-2-carboxylic acid obtained in Example 128 was used instead of (S)-2'-fluoro-3'-((6-((1-phenylethyl)carbamoyl)-3,4-dihydroquinoline-1(2H)-yl)methyl)-[1,1'-biphenyl]-2-carboxylic acid.
$^1$H NMR (400 MHz, $CDCl_3$) δ 8.21-8.19 (m, 2H), 7.82-7.80 (m, 1H), 7.60-7.49 (m, 5H), 7.46-7.45 (m, 1H), 7.44-7.43 (m, 1H), 7.41-7.39 (m, 1H), 7.21-7.14 (m, 2H), 6.49 (d, *J* = 8.8 Hz, 1H), 6.26 (d, *J* = 6.8 Hz, 1H), 5.46-5.32 (m, 3H), 4.63 (s, 2H), 3.51 (t, *J* = 5.6 Hz, 2H), 2.87 (t, *J* = 6.0 Hz, 2H), 2.10-2.04 (m, 2H), 1.60 (d, *J* = 7.2 Hz, 3H).

**<Example 132> (S)-1-((2'-carbamoyl-2-fluoro-[1,1'-biphenyl]-3-yl)methyl)-N-(1-(naphthalene-2-yl)ethyl)-1,2,3,4-tetrahydroquinoline-6-carboxamide**

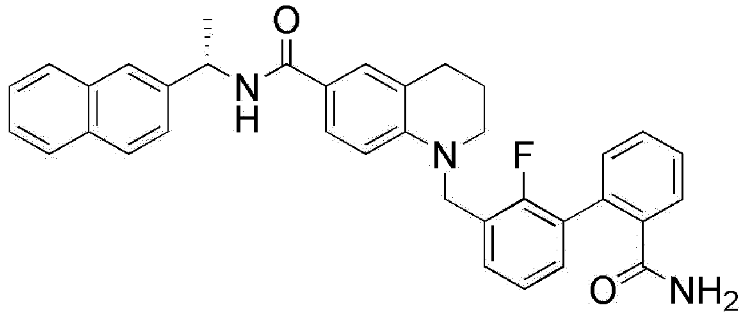

The target compound was obtained by the same manner as described in Example 127 except that (S)-2'-fluoro-3'-((6-((1-(naphthalene-2-yl)ethyl)carbamoyl)-3,4-dihydroquinoline-1(2H)-yl)methyl)-[1,1'-biphenyl]-2-carboxylic acid obtained in Example 129 was used instead of (S)-2'-fluoro-3'-((6-((1-phenylethyl)carbamoyl)-3,4-dihydroquinoline-1(2H)-yl)methyl)-[1,1'-biphenyl]-2-carboxylic acid.
$^1$H NMR (400 MHz, $CDCl_3$) δ 7.85-7.80 (m, 5H), 7.59-7.45 (m, 7H), 7.40-7.38 (m, 1H), 7.31-7.28 (m, 1H), 7.21-7.13 (m, 2H), 6.47 (d, *J* = 8.8 Hz, 1H), 6.24 (d, *J* = 8.0 Hz, 1H), 5.53-5.40 (m, 3H), 4.62 (s, 2H), 3.49 (t, *J* = 5.6 Hz, 2H), 2.87 (t, *J* = 6.0 Hz, 2H), 2.07-2.04 (m, 2H), 1.69 (d, *J* = 6.8 Hz, 3H).

**<Example 133> (S)-1-((2'-carbamoyl-2-fluoro-[1,1'-biphenyl]-3-yl)methyl)-N-(1-(3-fluoro-4-methylphenyl)ethyl)-1,2,3,4-tetrahydroquinoline-6-carboxamide**

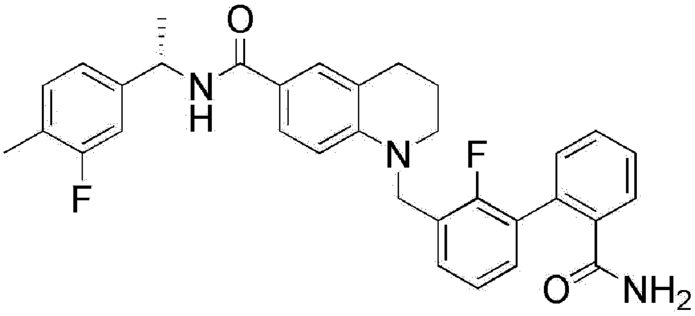

The target compound was obtained by the same manner as described in Example 127 except that (S)-2'-fluoro-3'-((6-((1-(3-fluoro-4-methylphenyl)ethyl)carbamoyl)-3,4-dihydroquinoline-1(2H)-yl)methyl)-[1,1'-biphenyl]-2-carboxylic acid obtained in Example 130 was used instead of (S)-2'-fluoro-3'-((6-((1-phenylethyl)carbamoyl)-3,4-dihydroquinoline-1(2H)-yl)methyl)-[1,1'-biphenyl]-2-carboxylic acid.
$^1H$ NMR (400 MHz, $CDCl_3$) δ 7.83-7.81 (m, 1H), 7.59-7.54 (m, 1H), 7.52-7.48 (m, 2H), 7.44-7.39 (m, 2H), 7.32-7.28 (m, 2H), 7.21-7.13 (m, 3H), 7.07-7.02 (m, 2H), 6.47 (d, *J* = 8.4 Hz, 1H), 6.11 (d, *J* = 7.6 Hz, 1H), 5.45-5.40 (m, 2H), 5.32-5.25 (m, 1H), 4.63 (s, 2H), 3.49 (t, *J* = 5.2 Hz, 2H), 2.87 (t, *J* = 6.4 Hz, 2H), 2.26 (s, 3H), 2.09-2.03 (m, 2H), 1.56 (d, *J* = 8.4 Hz, 3H).

**<Example 134> (S)-4'-fluoro-3'-((6-((1-phenylethyl)carbamoyl)-3,4-dihydroquinoline-1(2H)-yl)methyl)-[1,1'-biphenyl]-2-carboxylic acid**

Step 1 : (S)-tert-butyl 4'-fluoro-3'-((6-((1-phenylethyl)carbamoyl)-3,4-dihydroquinoline-1(2H)-yl)methyl)-[1,1'-biphenyl]-2-carboxylate

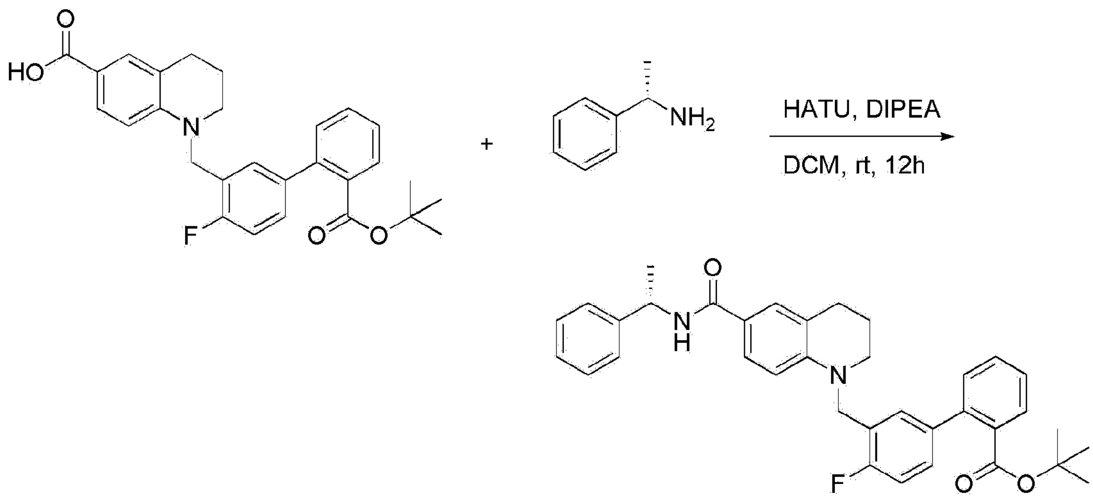


1-((2'-(tert-butoxycarbonyl)-4-fluoro-[1,1'-biphenyl]-3-yl)methyl)-1,2,3,4-tetrahydroquinoline-6-carboxylic acid (300 mg, 0.67 mmol) obtained in Preparative Example 6 was dissolved in $CH_2Cl_2$ (10 ml) in a 100 mL flask, to which HATU (386 mg, 1.01 mmol) and DIPEA (353 ul, 2.02 mmol) were added, followed by stirring at room temperature for 12 hours. Upon completion of the reaction, the organic layer was separated by using $CH_2Cl_2$ and $H_2O$. The separated organic layer was dried over $MgSO_4$ and filtered. The mixture was separated by column chromatography (EA/*n*-Hex = 1:1) to give (S)-tert-butyl 4'-fluoro-3'-((6-((1-phenylethyl)carbamoyl)-3,4-dihydroquinoline-1(2H)-yl)methyl)-[1,1'-biphenyl]-2-carboxylate (169 mg, 0.29 mmol, 44 %).

Step 2 : (S)-4'-fluoro-3'-((6-((1-phenylethyl)carbamoyl)-3,4-dihydroquinoline-1(2H)-yl)methyl)-[1,1'-biphenyl]-2-carboxylic acid

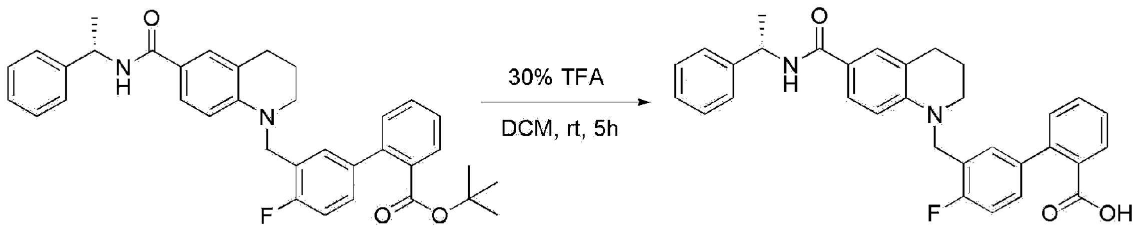


(S)-tert-butyl 4'-fluoro-3'-((6-((1-phenylethyl)carbamoyl)-3,4-dihydroquinoline-1(2H)-yl)methyl)-[1,1'-biphenyl]-2-carboxylate (168 mg, 0.3 mmol) obtained in step 1 was dissolved in $CH_2Cl_2$ (10 ml) in a 50 mL flask, to which 30% TFA (3 ml) was added, followed by stirring at room temperature for 5 hours. Upon completion of the reaction the reaction mixture was concentrated under reduced pressure. The mixture was separated by column chromatography (EA) to give (S)-4'-fluoro-3'-((6-((1-phenylethyl)carbamoyl)-3,4-dihydroquinoline-1(2H)-yl)methyl)-[1,1'-biphenyl]-2-carboxylic acid (83 mg, 0.16 mmol, 54 %).
$^1H$ NMR (400 MHz, $CDCl_3$) $\delta$ 7.85-7.83 (d, *J* = 7.2 Hz, 1H), 7.54-7.44 (m, 2H), 7.39-7.34 (m, 7H), 7.25-7.23 (m, 2H), 7.15-7.10 (m, 1H), 6.44 (d, *J* = 8.8 Hz, 1H), 6.21 (d, *J* = 8.0 Hz, 1H), 5.32-5.28 (m, 1H), 4.61 (s, 2H), 3.45 (d, *J* = 5.6 Hz, 1H), 2.79 (t, *J* = 6.4 Hz, 2H), 2.01-1.95 (m, 2H), 1.53 (d, *J* = 5.6 Hz, 3H).

**<Example 135> (S)-1-((2'-carbamoyl-4-fluoro-[1,1'-biphenyl]-3-yl)methyl)-N-(1-phenylethyl)-1,2,3,4-tetrahydroquinoline-6-carboxamide**

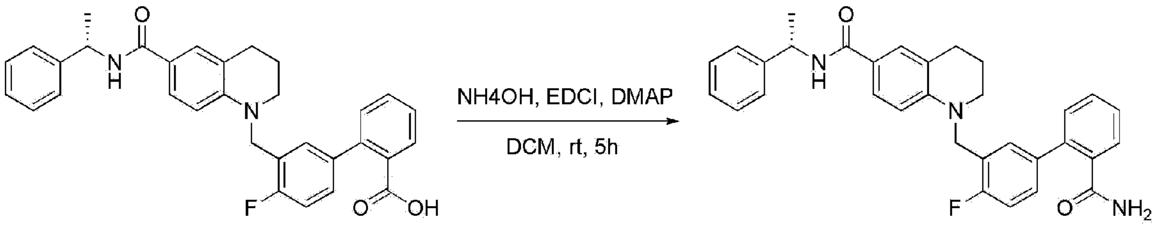


(S)-4'-fluoro-3'-((6-((1-phenylethyl)carbamoyl)-3,4-dihydroquinoline-1(2H)-yl)methyl)-[1,1'-biphenyl]-2-carboxylic acid (78 mg, 0.15 mmol) obtained in Example 134 was dissolved in $CH_2Cl_2$ (2 ml) in a 25 mL flask, to which EDCI (45 mg, 0.23 mmol) and DMAP (24 mg, 0.18 mmol) were added, followed by stirring at room temperature for 5 hours. Upon completion of the reaction, the organic layer was separated by using $CH_2Cl_2$ and $H_2O$. The separated organic layer was dried over $MgSO_4$ and filtered. The mixture was separated by column chromatography (MeOH/*n*-Hex/$CH_2Cl_2$ = 0.5:0.5:9) to give (S)-1-((2'-carbamoyl-4-fluoro-[1,1'-biphenyl]-3-yl)methyl)-N-(1-phenylethyl)-1,2,3,4-tetrahydroquinoline-6-carboxamide (76 mg, 0.14 mmol, 97.6 %) .
$^1H$ NMR (400 MHz, $CDCl_3$) $\delta$ 7.64-7.62 (m, 1H), 7.49-7.33 (m, 11H), 7.25-7.23 (m, 1H), 7.19-7.15 (m, 1H), 6.43 (d, *J* = 7.2 Hz, 1H), 6.20 (d, *J* = 7.6 Hz, 1H), 5.34-5.30 (m, 1H), 5.10 (s, 1H), 5.01 (s, 1H), 4.62 (s, 2H), 3.52(d, *J* = 6.0 Hz, 1H), 2.87 (t, *J* = 5.6 Hz, 2H), 2.07-2.04 (m, 2H), 1.59 (d, *J* = 6.8 Hz, 3H).

**<Example 136> (S)-4'-fluoro-3'-((6-((1-(4-nitrophenyl)ethyl)carbamoyl)-3,4-dihydroquinoline-1(2H)-yl)methyl)-[1,1'-biphenyl]-2-carboxylic acid**

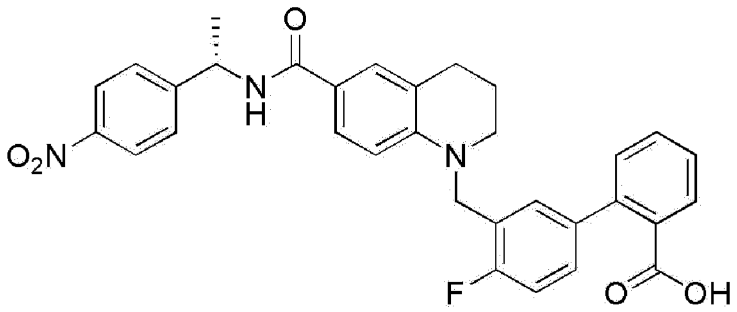

The target compound was obtained by the same manner as described in Example 134 except that (S)-1-(4-nitrophenyl)ethaneamine was used instead of (S)-1-phenylethylamine.
$^1$H NMR (400 MHz, $CDCl_3$) δ 8.10-8.08 (m, 2H), 7.84-7.82 (m, 1H), 7.53-7.50 (m, 1H), 7.41-7.37 (m, 4H), 7.28-7.21 (m, 3H), 7.14-7.09 (m, 1H), 7.01-6.99 (m, 1H), 6.52 (d, *J* = 7.6 Hz, 1H), 6.29 (d, *J* = 8.4 Hz, 1H), 5.27-5.23 (m, 1H), 4.62 (s, 2H), 3.48 (t, *J* = 5.2 Hz, 2H), 2.80 (t, J = 6.4 Hz, 2H), 2.07-2.00 (m, 2H), 1.47 (d, *J* = 6.8 Hz, 3H).

**<Example 137> (S)-4'-fluoro-3'-((6-((1-(naphthalene-2-yl)ethyl)carbamoyl)-3,4-dihydroquinoline-1(2H)-yl)methyl)-[1,1'-biphenyl]-2-carboxylic acid**

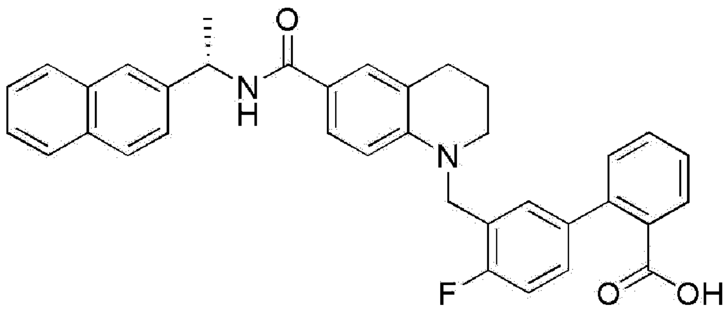

The target compound was obtained by the same manner as described in Example 134 except that (S)-1-(naphthalene-2-yl)ethaneamine was used instead of (S)-1-phenylethylamine.
$^1$H NMR (400 MHz, $CDCl_3$) δ 7.84-7.80 (m, 5H), 7.54-7.44 (m, 5H), 7.38-7.35 (m, 2H), 7.27-7.22 (m, 2H), 7.15-7.10 (m, 1H), 7.00-6.98 (m, 1H), 6.43 (d, *J* = 8.8 Hz, 1H), 6.33 (d, *J* = 8.0 Hz, 1H), 5.48-5.44 (m, 1H), 4.60 (s, 2H), 3.44 (t, *J* = 6.0 Hz, 2H), 2.77 (t, *J* = 6.0 Hz, 2H), 2.00-1.94 (m, 2H), 1.63 (d, *J* = 7.2 Hz, 3H).

**<Example 138> (S)-4'-fluoro-3'-((6-((1-(3-fluoro-4-methylphenyl)ethyl)carbamoyl)-3,4-dihydroquinoline-1(2H)-yl)methyl)-[1,1'-biphenyl]-2-carboxylic acid**

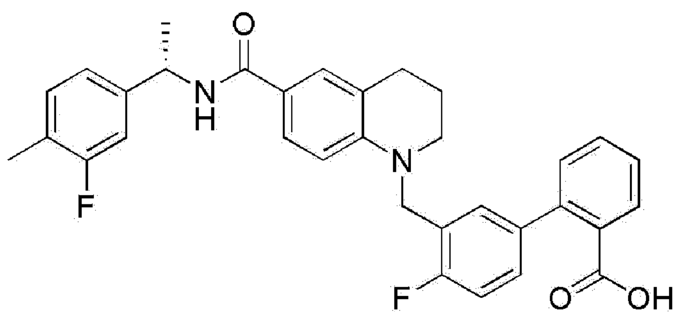

The target compound was obtained by the same manner as described in Example 134 except that (S)-1-(3-fluoro-4-methylphenyl)ethaneamine was used instead of (S)-1-phenylethylamine.
$^1$H NMR (400 MHz, $CDCl_3$) δ 7.84 (t, *J* = 7.6 Hz, 2H), 7.54-7.49 (m, 1H), 7.44-7.34 (m, 4H), 7.26-7.23 (m, 2H), 7.15-7.11

(m, 2H), 7.04-6.99 (m, 3H), 6.43 (d, *J* = 8.8 Hz, 1H), 6.20 (d, *J* = 8.0 Hz, 1H), 5.25-5.22 (m, 1H), 4.62 (s, 2H), 3.46 (t, *J* = 5.6 Hz, 2H), 2.80 (t, *J* = 6.0 Hz, 2H), 2.25 (s, 3H), 2.07-1.98 (m, 2H), 1.51 (d, *J* = 7.2 Hz, 3H).

**<Example 139> (S)-1-((2'-carbamoyl-4-fluoro-[1,1'-biphenyl]-3-yl)methyl)-N-(1-(4-nitrophenyl)ethyl)-1,2,3,4-tetrahydroquinoline-6-carboxamide**

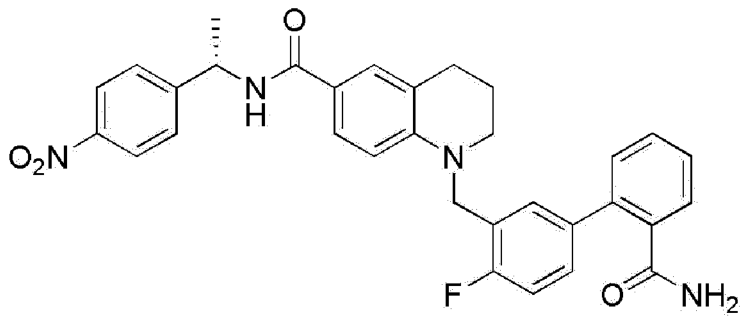

The target compound was obtained by the same manner as described in Example 135 except that (S)-4'-fluoro-3'-((6-((1-(4-nitrophenyl)ethyl)carbamoyl)-3,4-dihydroquinoline-1(2H)-yl)methyl)-[1,1'-biphenyl]-2-carboxylic acid obtained in Example 136 was used instead of (S)-4'-fluoro-3'-((6-((1-phenylethyl)carbamoyl)-3,4-dihydroquinoline-1(2H)-yl)methyl)-[1,1'-biphenyl]-2-carboxylic acid.
$^1$H NMR (400 MHz, $CDCl_3$) δ 8.18-8.16 (m, 2H), 7.65-7.62 (m, 1H), 7.54-7.28 (m, 8H), 7.23-7.15 (m, 2H), 6.49 (d, *J* = 7.2 Hz, 1H), 6.40 (d, *J* = 8.4 Hz, 1H), 5.34-5.28 (m, 1H), 5.21 (s, 1H), 5.11 (s, 1H), 4.64 (s, 2H), 3.53 (t, *J* = 5.6 Hz, 2H), 2.86 (t, *J* = 6.0 Hz, 2H), 2.08-2.03 (m, 2H), 1.58 (d, *J* = 6.8 Hz, 3H).

**<Example 140> (S)-1-((2'-carbamoyl-4-fluoro-[1,1'-biphenyl]-3-yl)methyl)-N-(1-(naphthalene-2-yl)ethyl)-1,2,3,4-tetrahydroquinoline-6-carboxamide**

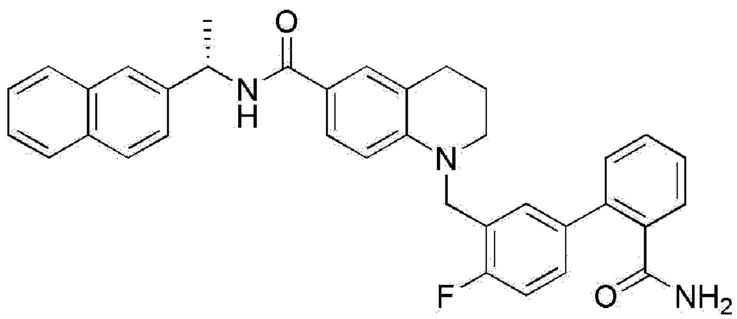

The target compound was obtained by the same manner as described in Example 135 except that (S)-4'-fluoro-3'-((6-((1-(naphthalene-2-yl)ethyl)carbamoyl)-3,4-dihydroquinoline-1(2H)-yl)methyl)-[1,1'-biphenyl]-2-carboxylic acid obtained in Example 137 was used instead of (S)-4'-fluoro-3'-((6-((1-phenylethyl)carbamoyl)-3,4-dihydroquinoline-1(2H)-yl)methyl)-[1,1'-biphenyl]-2-carboxylic acid.
$^1$H NMR (400 MHz, $CDCl_3$) δ 7.84-7.82 (m, 4H), 7.63-7.61 (m, 1H), 7.54-7.45 (m, 7H), 7.41-7.34 (m, 2H), 7.25-7.23 (m, 1H), 7.17-7.14 (m, 1H), 6.43 (d, *J* = 9.2 Hz, 1H), 6.31 (d, *J* = 7.6 Hz, 1H), 5.51-5.47 (m, 1H), 5.10 (s, 1H), 5.03 (s, 1H), 4.62 (s, 2H), 3.52 (t, *J* = 6.0 Hz, 2H), 2.86 (t, *J* = 6.4 Hz, 2H), 2.07-2.04 (m, 2H), 1.68 (d, *J* = 6.8 Hz, 3H).

**<Example 141> (S)-1-((2'-carbamoyl-4-fluoro-[1,1'-biphenyl]-3-yl)methyl)-N-(1-(3-fluoro-4-methylphenyl)ethyl)-1,2,3,4-tetrahydroquinoline-6-carboxamide**

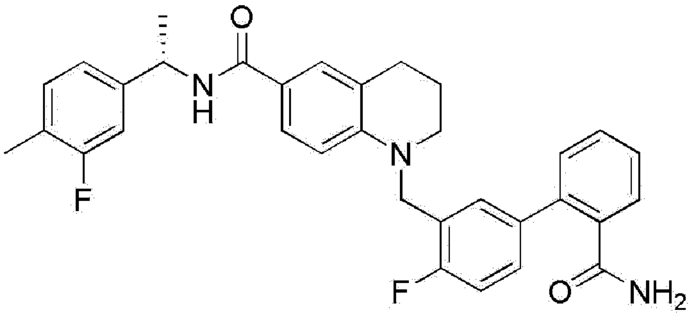

The target compound was obtained by the same manner as described in Example 135 except that (S)-4'-fluoro-3'-((6-((1-(3-fluoro-4-methylphenyl)ethyl)carbamoyl)-3,4-dihydroquinoline-1(2H)-yl)methyl)-[1,1'-biphenyl]-2-carboxylic acid obtained in Example 138 was used instead of (S)-4'-fluoro-3'-((6-((1-phenylethyl)carbamoyl)-3,4-dihydroquinoline-1(2H)-yl)methyl)-[1,1'-biphenyl]-2-carboxylic acid.
$^1H$ NMR (400 MHz, $CDCl_3$) δ 7.64-7.62 (m, 1H), 7.54-7.34 (m, 7H), 7.30-7.28 (m, 1H), 7.25-7.22 (m, 1H), 7.19-7.12 (m, 2H), 7.07-7.01 (m, 2H), 6.43 (d, *J* = 9.2 Hz, 1H), 6.21 (d, *J* = 7.2 Hz, 1H), 5.27-5.24 (m, 1H), 5.11 (s, 1H), 5.03 (s, 1H), 4.63 (s, 2H), 3.52 (t, *J* = 6.0 Hz, 2H), 2.86 (t, *J* = 6.4 Hz, 2H), 2.25 (s, 3H), 2.07-2.03 (m, 2H), 1.55 (d, *J* = 6.8 Hz, 3H).

**<Example 142> (S)-2'-((7-((1-phenylethyl)carbamoyl)-2H-benzo[b][1,4]oxazine-4(3H)-yl)methyl)-[1,1'-biphenyl]-4-carboxylic** acid

Step 1 : (S)-N-(1-phenylethyl)-3,4-dihydro-2H-benzo[b][1,4]oxazine-7-carboxamide

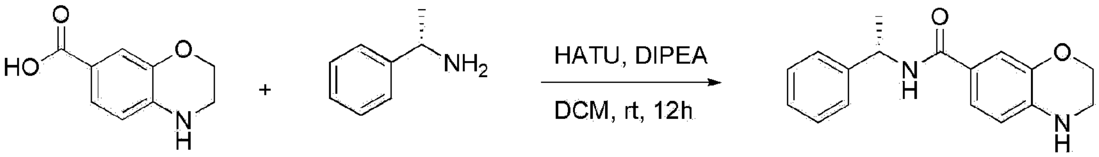


3,4-dihydro-2H-benzo[b][1,4]oxazine-7-carboxylic acid (314 mg, 1.69 mmol) obtained in Preparative Example 7 was dissolved in $CH_2Cl_2$ (10 ml) in a 100 mL flask, to which HATU (969 mg, 1.54 mmol) and DIPEA (888 ul, 5.09 mmol) were added, followed by stirring. (S)-1-phenylethylamine (547 ul, 4.24 mmol) was added, followed by stirring at room temperature for 12 hours. Upon completion of the reaction, the organic layer was separated by using $CH_2Cl_2$ and $H_2O$. The separated organic layer was dried over $MgSO_4$ and filtered. The mixture was separated by column chromatography (EA/*n*-Hex = 1:1) to give (S)-N-(1-phenylethyl)-3,4-dihydro-2H-benzo[b][1,4]oxazine-7-carboxamide (210 mg, 1.34 mmol, 79 %).

Step 2 : (S)-methyl 2'-((7-((1-phenylethyl)carbamoyl)-2H-benzo[b][1,4]oxazine-4(3H)-yl)methyl)-[1,1'-biphenyl]-4-carboxylate

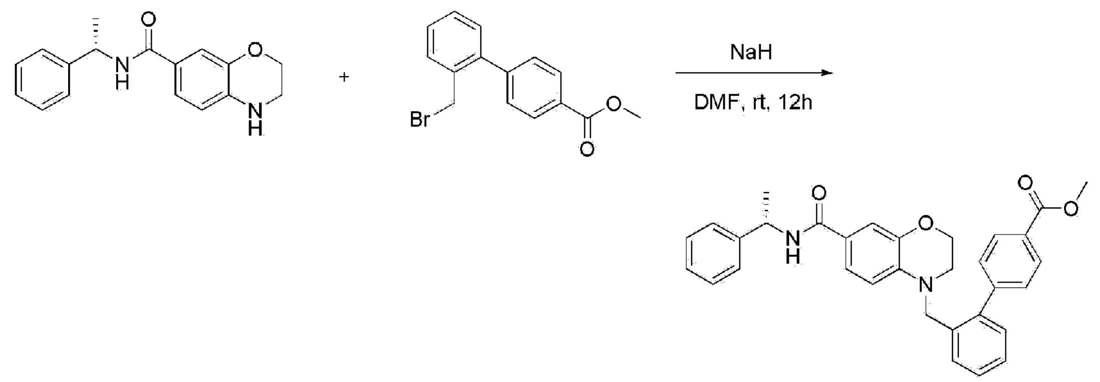


(S)-N-(1-phenylethyl)-3,4-dihydro-2H-benzo[b][1,4]oxazine-7-carboxamide (210 mg, 0.83 mmol) was dissolved in DMF (3 ml) in a 25 mL flask, to which NaH (33 mg, 0.83 mmol) and methyl 2'-(bromomethyl)-[1,1'-biphenyl]-4-carboxylate (254 mg, 0.83 mmol) were added, followed by stirring at room temperature for 12 hours. The organic layer was separated by using ethyl acetate and brine. The separated organic layer was dried over $MgSO_4$ and filtered. The solvent was eliminated from the mixture via vacuum distillation, followed by column chromatography to give (S)-methyl 2'-((7-((1-phenylethyl)carbamoyl)-2H-benzo[b][1,4]oxazine-4(3H)-yl)methyl)-[1,1'-biphenyl]-4-carboxylate (160 mg).

Step 3 : (S)-2'-((7-((1-phenylethyl)carbamoyl)-2H-benzo[b][1,4]oxazine-4(3H)-yl)methyl)-[1,1'-biphenyl]-4-carboxylic acid

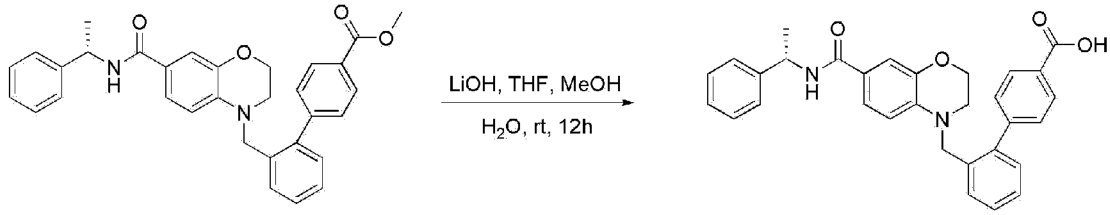


(S)-methyl 2'-((7-((1-phenylethyl)carbamoyl)-2H-benzo[b][1,4]oxazine-4(3H)-yl)methyl)-[1,1'-biphenyl]-4-carboxylate (160 mg, 0.31 mmol) was dissolved in MeOH (5 ml) and THF (10ml) in a 25 mL flask, to which LiOH (2 g) dissolved in $H_2O$ (5 ml) was added, followed by stirring at room temperature for 12 hours. Upon completion of the reaction, the solvent was concentrated. PH of the reaction mixture was adjusted to 5 by using 2*N*-HCl. The mixture was extracted with EA. The organic layer was dried over $MgSO_4$ and filtered. As a result, (S)-2'-((7-((1-phenylethyl)carbamoyl)-2H-benzo[b][1,4]oxazine-4(3H)-yl)methyl)-[1,1'-biphenyl]-4-carboxylic acid (180 mg) was obtained (3 g).
$^1H$ NMR (400 MHz, $CDCl_3$) $\delta$ 8.18 (d, *J* = 8.4 Hz, 2H), 7.46 (d, *J* = 8.4 Hz, 2H), 7.40-7.20 (m, 11H), 6.43 (d, *J* = 8.8 Hz, 1H), 6.13 (d, *J* = 7.6 Hz, 1H), 5.34-5.30 (m, 1H), 4.43 (s, 2H), 4.19 (t, *J* = 4.0 Hz, 2H), 3.35 (t, *J* = 4.4 Hz, 2H), 1.58 (d, *J* = 6.8 Hz, 3H).

**<Example 143> (S)-4-((4'-carbamoyl-[1,1'-biphenyl]-2-yl)methyl)-N-(1-phenylethyl)-3,4-dihydro-2H-benzo[b][1,4]oxazine-7-carboxamide**

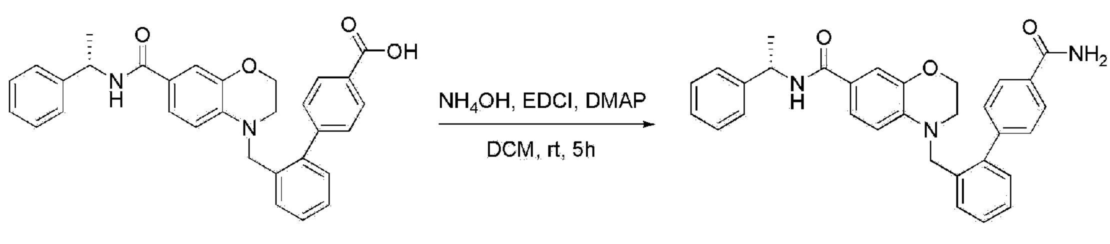


(S)-2'-((7-((1-phenylethyl)carbamoyl)-2H-benzo[b][1,4]oxazine-4(3H)-yl)methyl)-[1,1'-biphenyl]-4-carboxylic

acid (70 mg, 0.14 mmol) obtained in Example 142 was dissolved in $CH_2Cl_2$ (2 ml) in a 25 mL flask, to which EDCI (41 mg, 0.21 mmol) and DMAP (23 mg, 0.17 mmol) were added, followed by stirring at room temperature for 5 hours. Upon completion of the reaction, the organic layer was separated by using $CH_2Cl_2$ and $H_2O$. The separated organic layer was dried over $MgSO_4$ and filtered. The mixture was separated by column chromatography (MeOH/*n*-Hex/$CH_2Cl_2$ = 0.5:0.5:9) to give (S)-4-((4'-carbamoyl-[1,1'-biphenyl]-2-yl)methyl)-N-(1-phenylethyl)-3,4-dihydro-2H-benzo[b][1,4]oxazine-7-carboxamide (30 mg, 0.06 mmol, 42.9 %) .

$^1$H NMR (400 MHz, $CDCl_3$) $\delta$ 7.85 (d, *J* = 8.4 Hz, 2H), 7.50-7.28 (m, 11H), 7.20-7.15 (m, 2H), 6.37 (d, *J* = 8.4 Hz, 1H), 6.16-6.14 (m, 2H), 5.55 (s, 1H), 5.35-5.27 (m, 1H), 4.42 (s, 2H), 4.16 (t, *J* = 4.0 Hz, 2H), 3.32 (t, *J* = 4.4 Hz, 2H), 1.59 (d, *J* = 7.2 Hz, 3H).

**<Example 144> (S)-2'-((7-((1-(4-cyanophenyl)ethyl)carbamoyl)-2H-benzo[b][1,4]oxazine-4(3H)-yl)methyl)-[1,1'-biphenyl]-4-carboxylic acid**

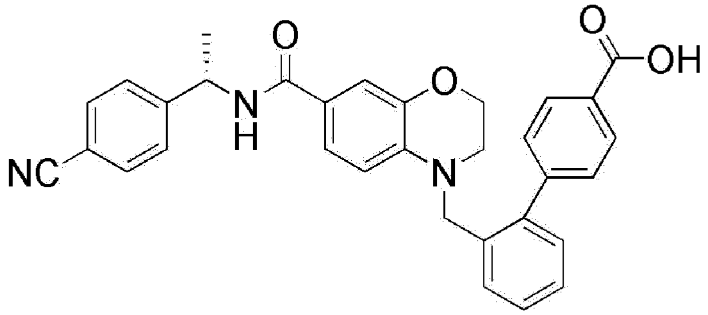

The target compound was obtained by the same manner as described in Example 142 except that (S)-1-(4-cyanophenyl)ethaneamine was used instead of (S)-1-phenylethylamine.

$^1$H NMR (400 MHz, $CDCl_3$) $\delta$ 8.17 (d, *J* = 8.4 Hz, 2H), 7.62 (d, *J* = 8.4 Hz, 2H), 7.48-7.45 (m, 4H), 7.42-7.24 (m, 4H), 7.22-7.02 (m, 2H), 6.42 (d, *J* = 9.2 Hz, 1H), 6.16 (d, *J* = 7.2 Hz, 1H), 5.34-5.27 (m, 1H), 4.44 (s, 2H), 4.19 (t, *J* = 4.4 Hz, 2H), 3.35 (t, *J* = 4.4 Hz, 2H), 1.57 (d, *J* = 7.2 Hz, 3H).

**<Example 145> (S)-2'-((7-((1-(3-fluoro-4-methylphenyl)ethyl)carbamoyl)-2H-benzo[b][1,4]oxazine-4(3H)-yl)methyl)-[1,1'-biphenyl]-4-carboxylic acid**

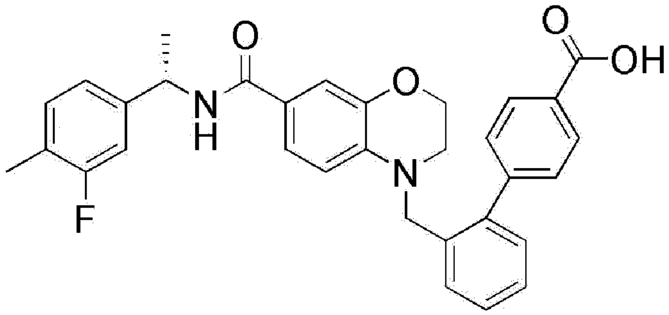

The target compound was obtained by the same manner as described in Example 142 except that (S)-1-(3-fluoro-4-methylphenyl)ethaneamine was used instead of (S)-1-phenylethylamine.

$^1$H NMR (400 MHz, $CDCl_3$) $\delta$ 8.18 (d, *J* = 8.4 Hz, 2H), 7.45 (d, *J* = 8.0 Hz, 2H), 7.41-7.34 (m, 3H), 7.32-7.31 (m, 1H), 7.22-7.20 (m, 2H), 7.16-7.12 (m, 1H), 7.05-7.00 (m, 2H), 6.42 (d, *J* = 9.2 Hz, 1H), 6.09 (d, *J* = 7.6 Hz, 1H), 5.28-5.24 (m, 1H), 4.43 (s, 2H), 4.19 (t, *J* = 4.0 Hz, 2H), 3.34 (t, *J* = 4.0 Hz, 2H), 2.25 (s, 3H), 1.54 (d, *J* = 6.8 Hz, 3H).

**<Example 146> (S)-2'-((7-((1-(4-nitrophenyl)ethyl)carbamoyl)-2H-benzo[b][1,4]oxazine-4(3H)-yl)methyl)-[1,1'-biphenyl]-4-carboxylic acid**

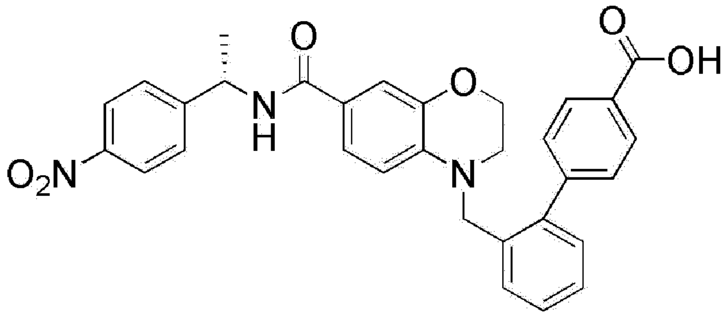

The target compound was obtained by the same manner as described in Example 142 except that (S)-1-(4-nitrophenyl)ethaneamine was used instead of (S)-1-phenylethylamine.
$^1$H NMR (400 MHz, $CDCl_3$) δ 8.21-8.15 (m, 4H), 7.54 (d, *J* = 1.6 Hz, 2H), 7.45 (d, *J* = 8.0 Hz, 2H), 7.40-7.30 (m, 4H), 7.23-7.21 (m, 2H), 6.42 (d, *J* = 9.2 Hz, 1H), 6.19 (d, *J* = 6.8 Hz, 1H), 5.34-5.32 (m, 1H), 4.44 (s, 2H), 4.18 (t, *J* = 4.0 Hz, 2H), 3.35 (t, *J* = 4.0 Hz, 2H), 1.59 (d, *J* = 6.8 Hz, 3H).

**<Example 147> (S)-2'-((7-((1-(naphthalene-2-yl)ethyl)carbamoyl)-2H-benzo[b][1,4]oxazine-4(3H)-yl)methyl)-[1,1'-biphenyl]-4-carboxylic acid**

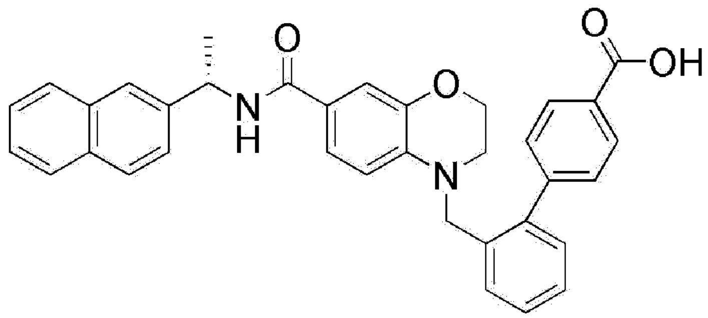

The target compound was obtained by the same manner as described in Example 142 except that (S)-1-(naphthalene-2-yl)ethaneamine was used instead of (S)-1-phenylethylamine.
$^1$H NMR (400 MHz, $CDCl_3$) δ 8.17 (d, *J* = 8.4 Hz, 2H), 7.84-7.82 (m, 4H), 7.54-7.46 (m, 6H), 7.38-7.34 (m, 3H), 7.31-7.02 (m, 7H), 6.42 (d, *J* = 8.0 Hz, 1H), 6.21 (d, *J* = 7.2 Hz, 1H), 5.51-5.47 (m, 1H), 4.42 (s, 2H), 4.19 (t, *J* = 4.0 Hz, 2H), 3.34 (t, *J* = 4.8 Hz, 2H), 1.67 (d, *J* = 6.8 Hz, 3H).

**<Example 148> (S)-4-((4'-carbamoyl-[1,1'-biphenyl]-2-yl)methyl)-N-(1-(3-fluoro-4-methylphenyl)ethyl)-3,4-dihydro-2H-benzo[b][1,4]oxazine-7-carboxamide**

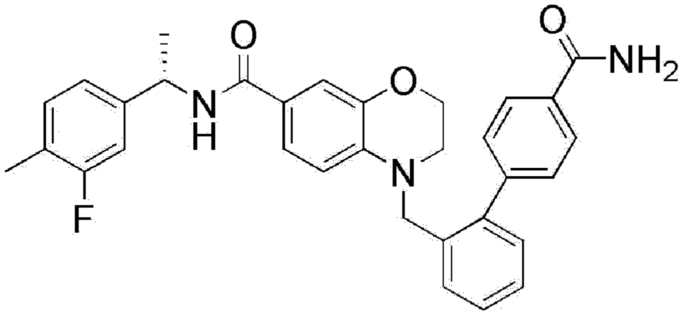

The target compound was obtained by the same manner as described in Example 143 except that (S)-2'-((7-((1-(3-fluoro-4-methylphenyl)ethyl)carbamoyl)-2H-benzo[b][1,4]oxazine-4(3H)-yl)methyl)-[1,1'-biphenyl]-4-carboxylic acid obtained in Example 145 was used instead of (S)-2'-((7-((1-phenylethyl)carbamoyl)-2H-benzo[b][1,4]oxazine-4(3H)-yl)methyl)-[1,1'-biphenyl]-4-carboxylic acid.
$^1$H NMR (400 MHz, $CDCl_3$) δ 7.86 (d, *J* = 7.6 Hz, 2H), 7.54-7.28 (m, 6H), 7.17-7.13 (m, 3H), 7.06-7.01 (m, 2H), 6.38 (d, *J* = 8.4 Hz, 1H), 6.25-6.12 (m, 2H), 5.61 (s, 1H), 5.32-5.21 (m, 1H), 4.42 (s, 2H), 4.16 (t, *J* = 4.0 Hz, 2H), 3.32 (t, *J* = 4.0 Hz, 2H), 2.26 (s, 1H), 1.55 (d, *J* = 6.4 Hz, 3H).

**<Example 149> (S)-4-((4'-carbamoyl-[1,1'-biphenyl]-2-yl)methyl)-N-(l-(4-cyanophenyl)ethyl)-3,4-dihydro-2H-benzo[b][1,4]oxazine-7-carboxamide**

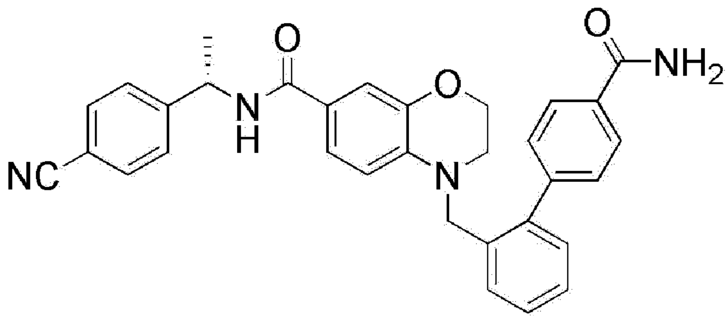

The target compound was obtained by the same manner as described in Example 143 except that (S)-2'-((7-((1-(4-cyanophenyl)ethyl)carbamoyl)-2H-benzo[b][1,4]oxazine-4(3H)-yl)methyl)-[1,1'-biphenyl]-4-carboxylic acid obtained in Example 144 was used instead of (S)-2'-((7-((1-phenylethyl)carbamoyl)-2H-benzo[b][1,4]oxazine-4(3H)-yl)methyl)-[1,1'-biphenyl]-4-carboxylic acid.
$^1$H NMR (400 MHz, $CDCl_3$) δ 7.86 (d, *J* = 8.4 Hz, 2H), 7.64 (d, *J* = 8.0 Hz, 2H), 7.52-7.47 (m, 2H), 7.40-7.30 (m, 6H), 7.21-7.16 (m, 2H), 6.38 (d, *J* = 8.4 Hz, 1H), 6.23 (d, *J* = 6.8 Hz, 1H), 6.12 (s, 1H), 5.64 (s, 1H), 5.32-5.26 (m, 1H), 4.43 (s, 2H), 4.16 (t, *J* = 4.0 Hz, 2H), 3.33 (t, *J* = 4.4 Hz, 2H), 1.57 (d, *J* = 7.2 Hz, 3H).

**<Example 150> (S)-4-((4'-carbamoyl-[1,1'-biphenyl]-2-yl)methyl)-N-(1-(4-nitrophenyl)ethyl)-3,4-dihydro-2H-benzo[b][1,4]oxazine-7-carboxamide**

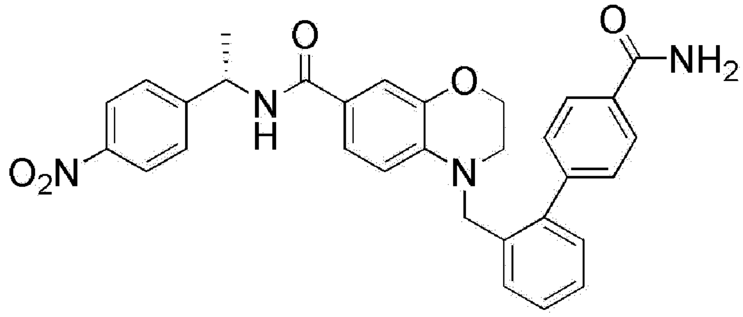

The target compound was obtained by the same manner as described in Example 143 except that (S)-2'-((7-((1-(4-nitrophenyl)ethyl)carbamoyl)-2H-benzo[b][1,4]oxazine-4(3H)-yl)methyl)-[1,1'-biphenyl]-4-carboxylic acid obtained in Example 146 was used instead of (S)-2'-((7-((1-phenylethyl)carbamoyl)-2H-benzo[b][1,4]oxazine-4(3H)-yl)methyl)-[1,1'-biphenyl]-4-carboxylic acid.
$^1$H NMR (400 MHz, $CDCl_3$) δ 8.20 (d, *J* = 8.4 Hz, 2H), 7.85 (d, *J* = 8.0 Hz, 2H), 7.54 (d, *J* = 8.8 Hz, 2H), 7.42-7.30 (m, 6H), 7.21-7.16 (m, 2H), 6.38 (d, *J* = 8.4 Hz, 1H), 6.27 (d, *J* = 7.2 Hz, 1H), 6.11 (s, 1H), 5.60 (s, 1H), 5.37-5.28 (m, 1H), 4.43 (s, 2H), 4.17 (t, *J* = 4.0 Hz, 2H), 3.33 (t, *J* = 4.4 Hz, 2H), 1.62 (d, *J* = 8.8 Hz, 3H).

**<Example 151> (S)-4-((4'-carbamoyl-[1,1'-biphenyl]-2-yl)methyl)-N-(1-(naphthalene-2-yl)ethyl)-3,4-dihydro-2H-benzo[b][1,4]oxazine-7-carboxamide**

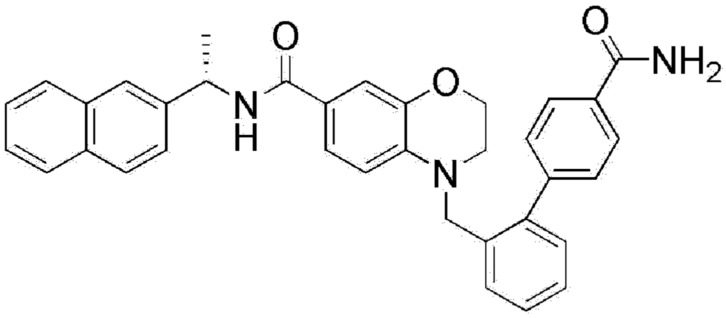

The target compound was obtained by the same manner as described in Example 143 except that (S)-2'-((7-((1-(naphthalene-2-yl)ethyl)carbamoyl)-2H-benzo[b][1,4]oxazine-4(3H)-yl)methyl)-[1,1'-biphenyl]-4-carboxylic acid obtained in Example 147 was used instead of (S)-2'-((7-((1-phenylethyl)carbamoyl)-2H-benzo[b][1,4]oxazine-4(3H)-yl)methyl)-[1,1'-biphenyl]-4-carboxylic acid.
$^1$H NMR (400 MHz, $CDCl_3$) δ 7.86-7.82 (m, 6H), 7.53-7.45 (m, 3H), 7.41-7.27 (m, 6H), 7.22-7.17 (m, 2H), 7.21-7.16 (m, 2H), 6.39 (d, *J* = 8.8 Hz, 1H), 6.25 (d, *J* = 8.0 Hz, 1H), 6.17 (s, 1H), 5.62 (s, 1H), 5.54-5.45 (m, 1H), 4.41 (s, 2H), 4.16 (t, *J* = 4.4 Hz, 2H), 3.31 (t, *J* = 4.4 Hz, 2H), 1.68 (d, *J* = 6.8 Hz, 3H).

**<Example 152> (S)-2'-((6-((1-phenylethyl)carbamoyl)-3,4-dihydroquinoline-1(2H)-yl)methyl)-[1,1'-biphenyl]-4-carboxylic acid**

Step 1 : (S)-N-(1-phenylethyl)-1,2,3,4-tetrahydroquinoline-6-carboxamide

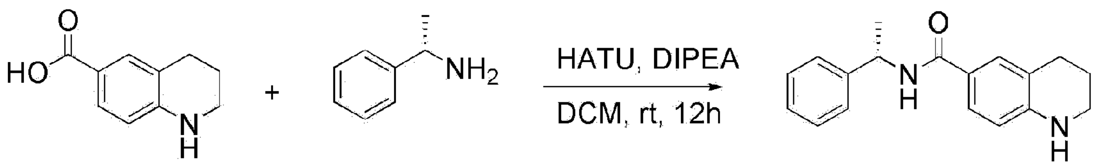

1,2,3,4-tetrahydroquinoline-6-carboxylic acid (300 mg, 1.63 mmol) obtained in Preparative Example 8 was dissolved in $CH_2Cl_2$ (10 ml) in a 100 mL flask, to which HATU (934 mg, 2.45 mmol) and DIPEA (856 ul, 4.91 mmol) were added, followed by stirring at room temperature for 12 hours. Upon completion of the reaction, the organic layer was separated by using $CH_2Cl_2$ and $H_2O$. The separated organic layer was dried over $MgSO_4$ and filtered. The mixture was separated by column chromatography (EA/*n*-Hex = 1:1) to give (S)-N-(1-phenylethyl)-1,2,3,4-tetrahydroquinoline-6-carboxamide (210 mg).

Step 2 : (S)-methyl 2'-((6-((1-phenylethyl)carbamoyl)-3,4-dihydroquinoline-1(2H)-yl)methyl)-[1,1'-biphenyll-4-carboxylate

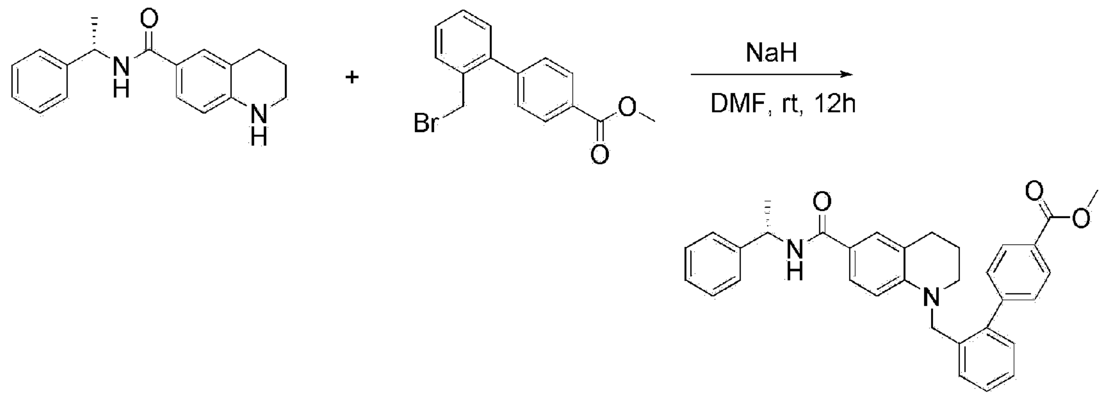


(S)-N-(1-phenylethyl)-1,2,3,4-tetrahydroquinoline-6-carboxamide (182 mg, 0.65 mmol) was dissolved in DMF (3 ml) in a 25 mL flask, to which NaH (26 mg, 0.65 mmol) and methyl 2'-(bromomethyl)-[1,1'-biphenyl]-4-carboxylate (200 mg, 0.65 mmol) were added, followed by stirring at room temperature for 12 hours. The organic layer was separated by using ethyl acetate and brine. The separated organic layer was dried over $MgSO_4$ and filtered. The solvent was eliminated from the mixture via vacuum distillation, followed by column chromatography to give (S)-methyl 2'-((6-((1-phenylethyl)carbamoyl)-3,4-dihydroquinoline-1(2H)-yl)methyl)-[1,1'-biphenyl]-4-carboxylate (140 mg).

Step 3 : (S)-2'-((6-((1-phenylethyl)carbamoyl)-3,4-dihydroquinoline-1(2H)-yl)methyl)-[1,1'-biphenyl]-4-carboxylic acid

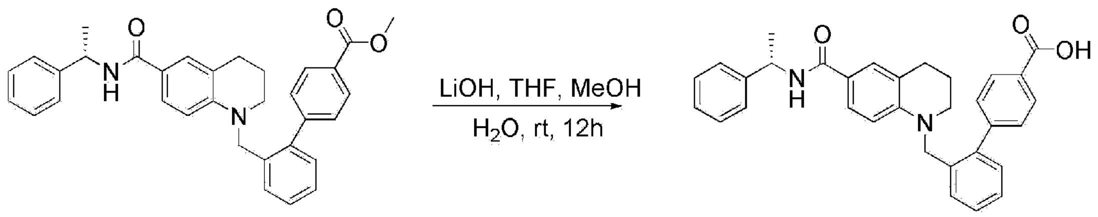


(S)-methyl 2'-((6-((1-phenylethyl)carbamoyl)-3,4-dihydroquinoline-1(2H)-yl)methyl)-[1,1'-biphenyl]-4-carboxylate (140 mg, 0.27 mmol) was dissolved in MeOH (5 ml) and THF (10 ml) in a 25 mL flask, to which LiOH (2 g) dissolved in $H_2O$ (5 ml) was added, followed by stirring at room temperature for 12 hours. Upon completion of the reaction, the solvent was concentrated. PH of the reaction mixture was adjusted to 5 by using 2*N*-HCl. The mixture was extracted with EA. The organic layer was dried over $MgSO_4$ and filtered. As a result, (S)-2'-((6-((1-phenylethyl)carbamoyl)-3,4-dihydroquinoline-1(2H)-yl)methyl)-[1,1'-biphenyl]-4-carboxylic acid (99.4 mg) was obtained.
$^1$H NMR (400 MHz, $CDCl_3$) $\delta$ 8.19 (d, *J* = 8.0 Hz, 2H), 7.48-7.44 (m, 4H), 7.41-7.25 (m, 9H), 7.58-7.27 (m, 8H), 6.28 (d, *J* = 8.8 Hz, 1H), 6.12 (d, *J* = 8.0 Hz, 1H), 5.36-5.32 (m, 1H), 4.42 (s, 2H), 3.34 (t, *J* = 5.6 Hz, 2H), 2.80 (t, *J* = 6.0 Hz, 2H), 2.01-1.95 (m, 2H), 1.59 (d, *J* = 7.2 Hz, 3H).

**<Example 153> (S)-1-((4'-carbamoyl-[1,1'-biphenyl]-2-yl)methyl)-N-(1-phenylethyl)-1,2,3,4-tetrahydroquinoline-6-carboxamide**

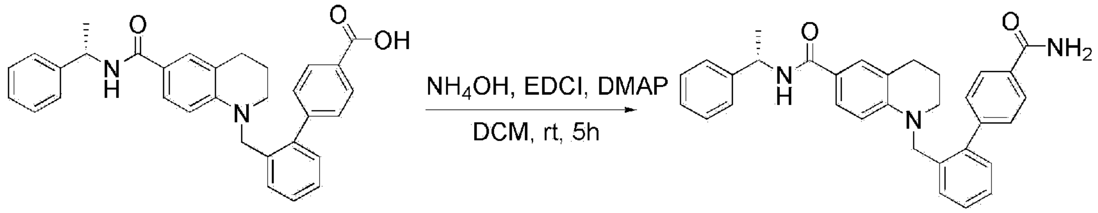


(S)-2'-((6-((1-phenylethyl)carbamoyl)-3,4-dihydroquinoline-1(2H)-yl)methyl)-[1,1'-biphenyl]-4-carboxylic acid (66.4 mg, 0.14 mmol) obtained in Example 152 was dissolved in $CH_2Cl_2$ (2 ml) in a 25 mL flask, to which EDCI (41 mg, 0.21 mmol) and DMAP (23 mg, 0.17 mmol) were added, followed by stirring. Ammonium hydroxide solution (3 ml) was added thereto, followed by stirring at room temperature for 5 hours. Upon completion of the reaction, the organic layer was separated by using $CH_2Cl_2$ and $H_2O$. The separated organic layer was dried over $MgSO_4$ and filtered. The mixture was separated by column chromatography (MeOH/*n*-Hex/$CH_2Cl_2$ = 0.5:0.5:9) to give (S)-1-((4'-carbamoyl-[1,1'-biphenyl]-2-yl)methyl)-N-(1-phenylethyl)-1,2,3,4-tetrahydroquinoline-6-carboxamide (54.7 mg).
$^1$H NMR (400 MHz, $CDCl_3$) δ 7.86 (d, *J* = 8.0 Hz, 2H), 7.43-7.26 (m, 12H), 6.26 (d, *J* = 8.8 Hz, 2H), 6.20-6.11 (m, 2H), 6.64 (s, 1H), 5.37-5.30 (m, 1H), 4.41 (s, 2H), 3.31 (t, *J* = 5.6 Hz, 2H), 2.78 (t, *J* = 6.0 Hz, 2H), 1.99-1.93 (m, 2H), 1.57 (d, *J* = 6.4 Hz, 3H).

**<Example 154> (S)-2'-((6-((1-(3-fluoro-4-methylphenyl)ethyl)carbamoyl)-3,4-dihydroquinoline-1(2H)-yl)methyl)-[1,1'-biphenyl]-4-carboxylic acid**

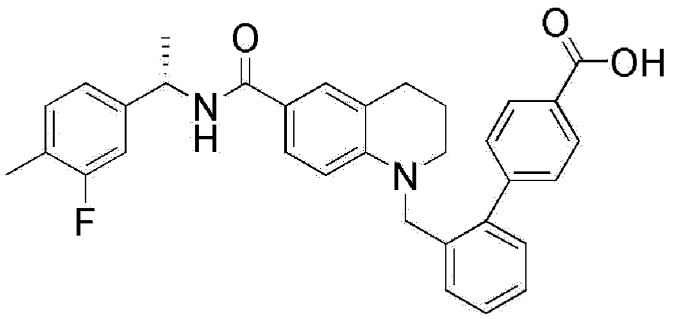

The target compound was obtained by the same manner as described in Example 152 except that (S)-1-(3-fluoro-4-methylphenyl)ethaneamine was used instead of (S)-1-phenylethylamine.
$^1$H NMR (400 MHz, $CDCl_3$) δ 8.18 (d, *J* = 8.0 Hz, 2H), 7.47-7.42 (m, 3H), 7.38-7.28 (m, 5H), 7.16-7.12 (m, 1H), 7.06-7.00 (m, 2H), 6.27 (d, *J* = 8.8 Hz, 1H), 6.08 (d, *J* = 8.0 Hz, 1H), 5.32-5.26 (m, 1H), 4.42 (s, 2H), 3.33 (t, *J* = 4.0 Hz, 2H), 2.78 (t, *J* = 4.0 Hz, 2H), 2.25 (s, 3H), 1.99-1.97 (m, 2H), 1.54 (d, *J* = 6.8 Hz, 3H).

**<Example 155> (S)-2'-((6-((1-(4-nitrophenyl)ethyl)carbamoyl)-3,4-dihydroquinoline-1(2H)-yl)methyl)-[1,1'-biphenyl]-4-carboxylic acid**

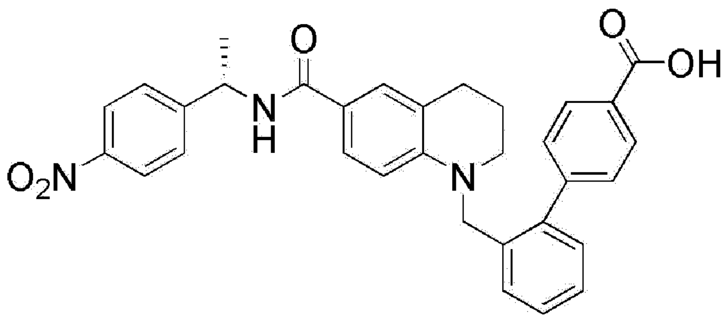

The target compound was obtained by the same manner as described in Example 152 except that (S)-1-(4-nitrophenyl)ethaneamine was used instead of (S)-1-phenylethylamine.
$^1H$ NMR (400 MHz, $CDCl_3$) δ 8.18 (d, *J* = 8.4 Hz, 4H), 7.54-7.28 (m, 10H), 6.28 (d, *J* = 8.4 Hz, 1H), 6.20 (d, *J* = 6.8 Hz, 1H), 5.38-5.32 (m, 1H), 4.43 (s, 2H), 3.35 (t, *J* = 4.4 Hz, 2H), 2.80 (t, *J* = 5.6 Hz, 2H), 1.98 (t, *J* = 4.8 Hz, 2H), 1.59 (d, *J* = 6.8 Hz, 3H).

**<Example 156> (S)-2'-((6-((1-(naphthalene-2-yl)ethyl)carbamoyl)-3,4-dihydroquinoline-1(2H)-yl)methyl)-[1,1'-biphenyl]-4-carboxylic acid**

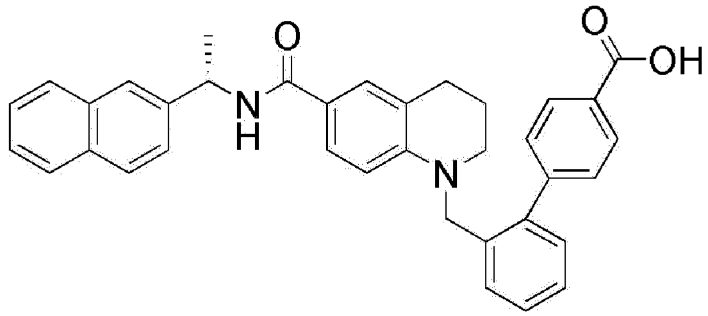

The target compound was obtained by the same manner as described in Example 152 except that (S)-1-(naphthalene-2-yl)ethaneamine was used instead of (S)-1-phenylethylamine.
$^1H$ NMR (400 MHz, $CDCl_3$) δ 8.18 (d, *J* = 7.6 Hz, 2H), 7.84-7.81 (m, 4H), 7.53-7.28 (m, 11H), 6.28 (d, *J* = 8.8 Hz, 1H), 6.22 (d, *J* = 7.6 Hz, 1H), 5.53-5.48 (m, 1H), 4.41 (s, 2H), 3.32 (t, *J* = 4.4 Hz, 2H), 2.79 (t, *J* = 5.6 Hz, 2H), 1.97 (t, *J* = 4.8 Hz, 2H), 1.68 (d, *J* = 6.8 Hz, 3H).

**<Example 157> (S)-2'-((6-((1-(4-cyanophenyl)ethyl)carbamoyl)-3,4-dihydroquinoline-1(2H)-yl)methyl)-[1,1'-biphenyl]-4-carboxylic acid**

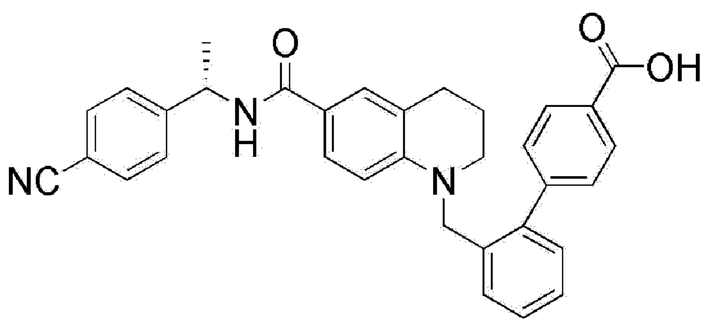

The target compound was obtained by the same manner as described in Example 152 except that (S)-1-(4-cyanophenyl)ethaneamine was used instead of (S)-1-phenylethylamine.
$^1$H NMR 400 MHz, $CDCl_3$) δ 8.18 (d, *J* = 8.0 Hz, 2H), 7.62 (d, *J* = 8.0 Hz, 2H), 7.48-7.28 (m, 10H), 6.27 (d, *J* = 8.4 Hz, 1H), 6.16 (d, *J* = 7.6 Hz, 1H), 5.34-5.30 (m, 1H), 4.43 (s, 2H), 3.34 (t, *J* = 5.6 Hz, 2H), 2.80 (t, *J* = 6.4 Hz, 2H), 2.00-1.97 (m, 2H), 1.57 (d, *J* = 6.8 Hz, 3H).

**<Example 158> (S)-1-((4'-carbamoyl-[1,1'-biphenyl]-2-yl)methyl)-N-(1-(3-fluoro-4-methylphenyl)ethyl)-1,2,3,4-tetrahydroquinoline-6-carboxamide**

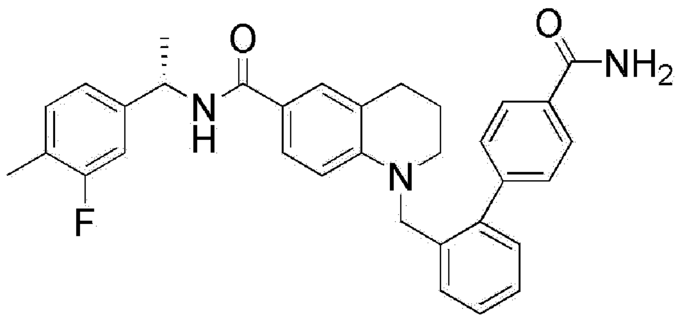

The target compound was obtained by the same manner as described in Example 153 except that (S)-2'-((6-((1-(3-fluoro-4-methylphenyl)ethyl)carbamoyl)-3,4-dihydroquinoline-1(2H)-yl)methyl)-[1,1'-biphenyl]-4-carboxylic acid obtained in Example 154 was used instead of (S)-2'-((6-((1-phenylethyl)carbamoyl)-3,4-dihydroquinoline-1(2H)-yl)methyl)-[1,1'-biphenyl]-4-carboxylic acid.
$^1$H NMR (400 MHz, $CDCl_3$) δ 7.87 (d, *J* = 7.6 Hz, 2H), 7.44-7.32 (m, 8H), 7.17-7.13 (m, 1H), 7.07-7.02 (m, 2H), 6.26 (d, *J* = 8.4 Hz, 1H), 6.10-6.08 (m, 2H), 5.57 (s, 1H), 5.33-5.25 (m, 1H), 4.42 (s, 2H), 3.32 (t, *J* = 5.2 Hz, 2H), 2.79 (t, *J* = 6.0 Hz, 2H), 2.26 (s, 3H), 1.98-1.95 (m, 2H), 1.55 (d, *J* = 6.8 Hz, 3H).

**<Example 159> (S)-1-((4'-carbamoyl-[1,1'-biphenyl]-2-yl)methyl)-N-(l-(4-nitrophenyl)ethyl)-1,2,3,4-tetrahydroquinoline-6-carboxamide**

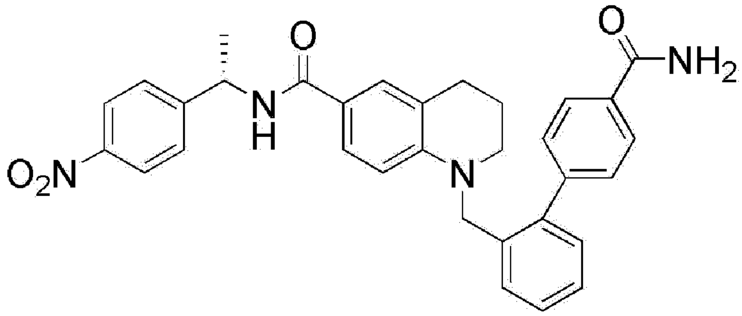

The target compound was obtained by the same manner as described in Example 153 except that (S)-2'-((6-((1-(4-nitrophenyl)ethyl)carbamoyl)-3,4-dihydroquinoline-1(2H)-yl)methyl)-[1,1'-biphenyl]-4-carboxylic acid obtained in Example 155 was used instead of (S)-2'-((6-((1-phenylethyl)carbamoyl)-3,4-dihydroquinoline-1(2H)-yl)methyl)-[1,1'-biphenyl]-4-carboxylic acid.
$^1$H NMR (400 MHz, $CDCl_3$) δ 8.20 (d, *J* = 8.8 Hz, 2H), 7.87(d, *J* = 8.0 Hz, 2H), 7.54(d, *J* = 8.8 Hz, 2H), 7.50-7.33 (m, 8H), 6.26 (d, *J* = 8.4 Hz, 1H), 6.21 (d, *J* = 7.2 Hz, 1H), 6.01 (s, 1H), 5.58 (s, 1H), 5.38-5.32 (m, 1H), 4.42 (s, 2H), 3.33 (t, *J* = 5.6 Hz, 2H), 2.79 (t, *J* = 5.6 Hz, 2H), 1.99-1.96 (m, 2H), 1.60 (d, *J* = 6.8 Hz, 3H).

**<Example 160> (S)-1-((4'-carbamoy1-[1,1'-biphenyl]-2-yl)methyl)-N-(1-(naphthalene-2-yl)ethyl)-1,2,3,4-tetrahydroquinoline-6-carboxamide**

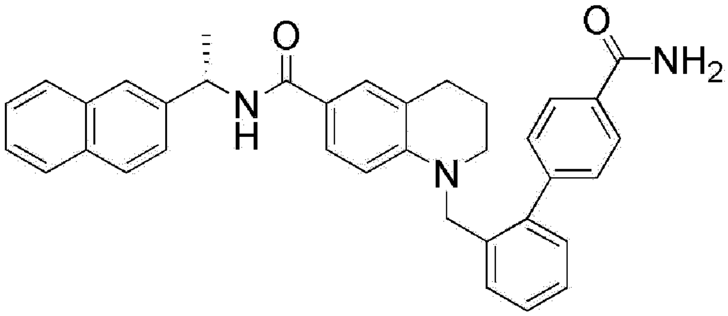

The target compound was obtained by the same manner as described in Example 153 except that (S)-2'-((6-((1-(naphthalene-2-yl)ethyl)carbamoyl)-3,4-dihydroquinoline-1(2H)-yl)methyl)-[1,1'-biphenyl]-4-carboxylic acid obtained in Example 156 was used instead of (S)-2'-((6-((1-phenylethyl)carbamoyl)-3,4-dihydroquinoline-1(2H)-yl)methyl)-[1,1'-biphenyl]-4-carboxylic acid.
$^1$H NMR (400 MHz, $CDCl_3$) δ 7.87-7.83 (m, 6H), 7.54-7.26 (d, 11H), 6.27 (d, $J$ = 8.4 Hz, 1H), 6.21 (d, $J$ = 8.0 Hz, 1H), 6.13 (s, 1H), 5.55-5.48 (m,2 H), 4.41 (s, 2H), 3.31 (t, $J$ = 5.2 Hz, 2H), 2.78 (t, $J$ = 5.6 Hz, 2H), 2.01-1.96 (m, 2H), 1.68 (d, $J$ = 6.8 Hz, 3H).

**<Example 161> (S)-1-((4'-carbamoyl-[1,1'-biphenyl]-2-yl)methyl)-N-(1-(4-cyanophenyl)ethyl)-1,2,3,4-tetrahydroquinoline-6-carboxamide**

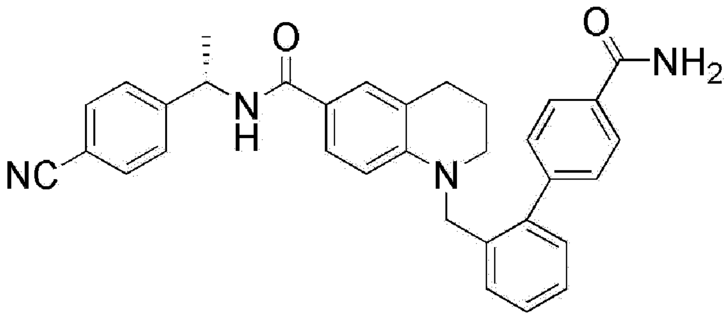

The target compound was obtained by the same manner as described in Example 153 except that (S)-2'-((6-((1-(4-cyanophenyl)ethyl)carbamoyl)-3,4-dihydroquinoline-1(2H)-yl)methyl)-[1,1'-biphenyl]-4-carboxylic acid obtained in Example 157 was used instead of (S)-2'-((6-((1-phenylethyl)carbamoyl)-3,4-dihydroquinoline-1(2H)-yl)methyl)-[1,1'-biphenyl]-4-carboxylic acid.
$^1$H NMR (400 MHz, $CDCl_3$) δ 7.97 (d, $J$ = 8.8 Hz, 2H), 7.87(d, $J$ = 8.0 Hz, 2H), 7.63(d, $J$ = 8.0 Hz, 2H), 7.58-7.27 (m, 8H), 6.26 (d, $J$ = 8.8 Hz, 1H), 6.19-6.04 (m, 2H), 5.57 (s, 1H), 5.34-5.27 (m, 1H), 4.42 (s, 2H), 3.33 (t, $J$ = 5.6 Hz, 2H), 2.79 (t, $J$ = 6.0 Hz, 2H), 2.07-1.95 (m, 2H), 1.57 (d, $J$ = 6.4 Hz, 3H).

**<Example 162> (S)-2'-fluoro-4'-((7-((1-phenylethyl)carbamoyl)-2H-benzo[b][1,4]oxazine-4(3H)-yl)methyl)-[1,1'-biphenyl]-2-carboxylic acid**

<u>Step 1 : (S)-tert-butyl 2'-fluoro-4'-((7-((1-phenylethyl)carbamoyl)-2H-benzo[b][1,4]oxazine-4(3H)-yl)methyl)-[1,1'-biphenyl]-2-carboxylate</u>

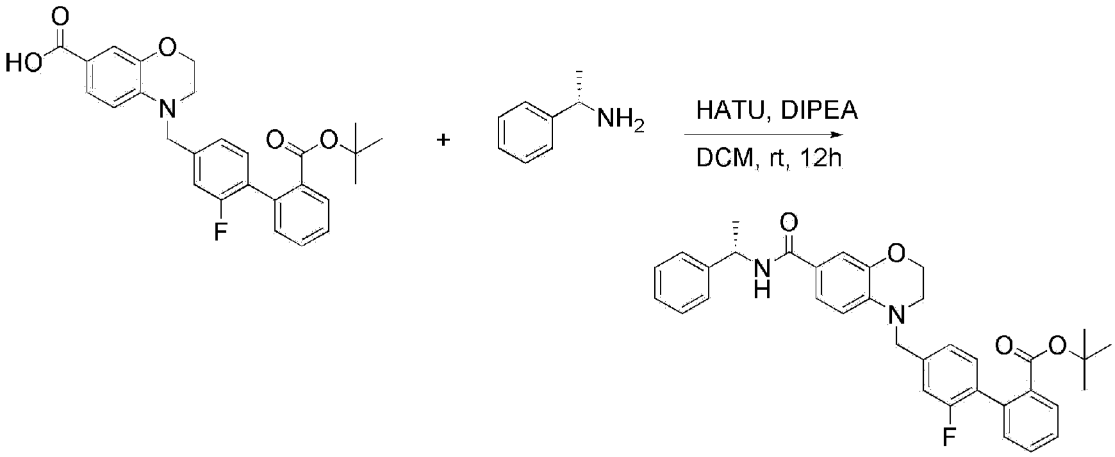


4-((2'-(tert-butoxycarbonyl)-2-fluoro-[1,1'-biphenyl]-4-yl)methyl)-3,4-dihydro-2H-benzo[b][1,4]oxazine-7-carboxylic acid (300 mg, 0.65 mmol) obtained in Preparative Example 9 was dissolved in $CH_2Cl_2$ (10 ml) in a 100 mL flask, to which HATU (386 mg, 1.01 mmol) and DIPEA (353 ul, 2.02 mmol) were added, followed by stirring. (S)-1-phenylethylamine (105 ul, 0.81 mmol) was added thereto, followed by stirring at room temperature for 12 hours. Upon completion of the reaction, the organic layer was separated by using $CH_2Cl_2$ and $H_2O$ The separated organic layer was dried over $MgSO_4$ and filtered. The mixture was separated by column chromatography (EA/n-Hex = 1:1) to give (S)-tert-butyl 2'-fluoro-4'-((7-((1-phenylethyl)carbamoyl)-2H-benzo[b][1,4]oxazine-4(3H)-yl)methyl)-[1,1'-biphenyl]-2-carboxylate (180 mg, 0.31 mmol, 47 %).

Step 2 : (S)-2'-fluoro-4'-((7-((1-phenylethyl)carbamoyl)-2H-benzo[b][1,4]oxazine-4(3H)-yl)methyl)-[1,1'-biphenyl]-2-carboxylic acid

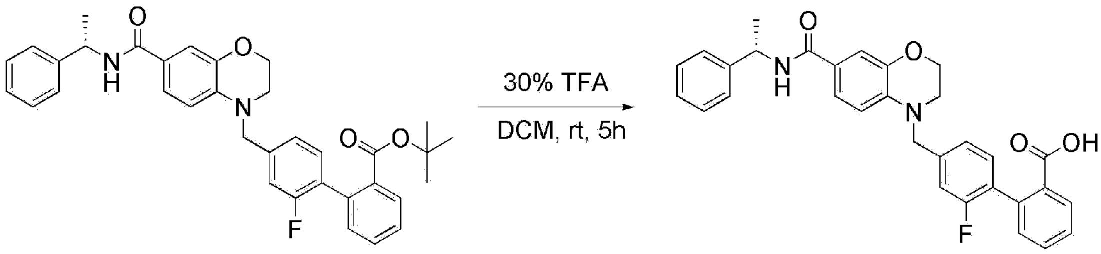


(S)-tert-butyl 2'-fluoro-4'-((7-((1-phenylethyl)carbamoyl)-2H-benzo[b][1,4]oxazine-4(3H)-yl)methyl)-[1,1'-biphenyl]-2-carboxylate(180 mg, 0.31 mmol) obtained in step 1 was dissolved in $CH_2Cl_2$ (10 ml) in a 50 mL flask, to which 30% TFA (3 ml) was added, followed by stirring at room temperature for 5 hours. Upon completion of the reaction the reaction mixture was concentrated under reduced pressure. The mixture was separated by column chromatography (EA) to give (S)-2'-fluoro-4'-((7-((1-phenylethyl)carbamoyl)-2H-benzo[b][1,4]oxazine-4(3H)-yl)methyl)-[1,1'-biphenyl]-2-carboxylic acid (100 mg, 0.19 mmol, 61 %).

$^1$H NMR (400 MHz, $CDCl_3$) $\delta$ 8.07-8.05 (m, 1H), 7.65-7.61 (m, 1H), 7.51-7.47 (m, 1H), 7.40-7.33 (m, 6H), 7.30-7.11 (m, 3H), 7.11-7.09 (m, 1H), 7.05-6.96 (m, 3H), 6.62 (d, $J$ = 8.4 Hz, 1H), 6.19 (d, $J$ = 7.6 Hz, 1H), 5.29-5.22 (m, 1H), 4.55 (s, 2H), 4.29 (t, $J$ = 4.4 Hz, 2H), 3.50 (t, $J$ = 4.4 Hz, 2H), 2.25 (s, 3H), 1.53 (d, $J$ = 7.2 Hz, 3H).

**<Example 163> (S)-2'-fluoro-4'-((7-((1-(naphthalene-2-yl)ethyl)carbamoyl)-2H-benzo[b][1,4]oxazine-4(3H)-yl)methyl)-[1,1'-biphenyl]-2-carboxylic acid**

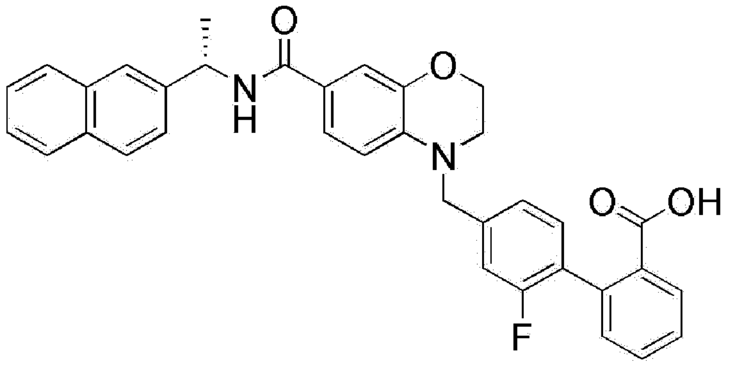

The target compound was obtained by the same manner as described in Example 162 except that (S)-1-(naphthalene-2-yl)ethaneamine was used instead of (S)-1-phenylethylamine.
$^1$H NMR (400 MHz, DMSO-$d_6$) δ 12.67 (br, OH, 1H), 8.56-8.53 (m, 1H), 7.87-7.81 (m, 5H), 7.63-7.48 (m, 5H), 7.38-7.35 (m, 4H), 7.17-7.08 (m, 2H), 6.75-7.71 (m, 1H), 5.30-5.28 (m, 1H), 4.65 (s, 2H), 4.27-4.21 (m, 2H), 3.54 (s, 2H), 1.56-1.47 (m, 3H).

**<Example 164> (S)-2'-fluoro-4'-((7-(((3-fluoro-4-methylphenyl)ethyl)carbamoyl)-2H-benzo[b][1,4]oxazine-4(3H)-yl)methyl)-[1,1'-biphenyl]-2-carboxylic acid**

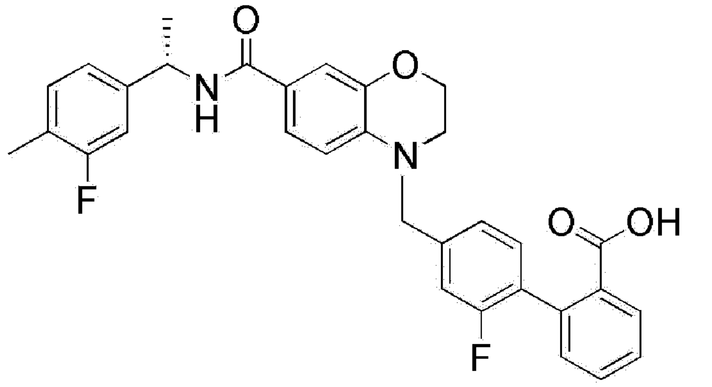

The target compound was obtained by the same manner as described in Example 162 except that (S)-1-(3-fluoro-4-methylphenyl)ethaneamine was used instead of (S)-1-phenylethylamine.
1H NMR (400 MHz, $CDCl_3$) δ 8.07-8.05 (m, 1H), 7.64-7.60 (m, 1H), 7.51-7.47 (m, 1H), 7.40-7.36 (m, 1H), 7.30-7.26 (m, 3H), 7.15-7.08 (m, 2H), 7.02-6.79 (m, 1H), 6.63 (d, J = 8.4 Hz, 1H), 6.18 (d, J = 7.6 Hz, 1H), 5.36-5.28 (m, 1H), 4.55 (s, 2H), 4.29 (t, J = 4.4 Hz, 2H), 3.50 (t, J = 4.4 Hz, 2H), 1.58 (d, J = 7.8 Hz, 3H).

## <Comparative Example 1> SR1664

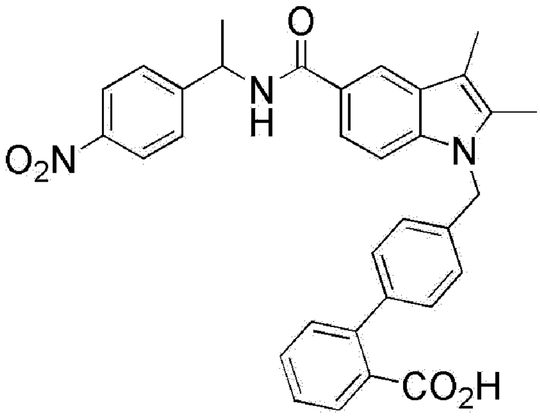

## <Comparative Example 2> SR1824

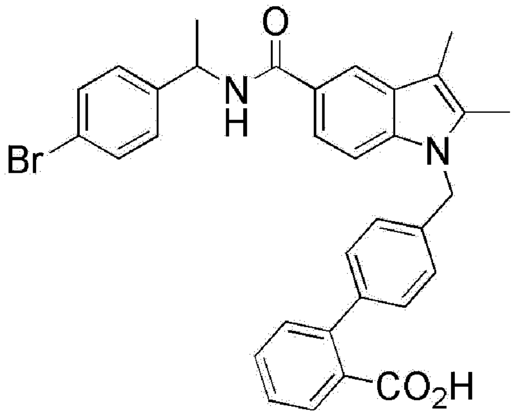

The chemical formulae of the compounds prepared in Examples 1 ~ 164 are shown in Table 1.

[Table 1]

| Example | Formula | Example | Formula |
|---|---|---|---|
| 1 | 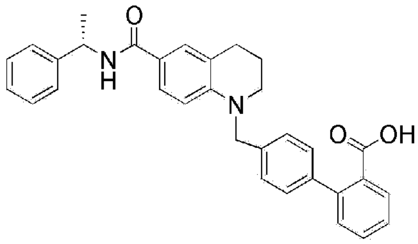 | 83 | 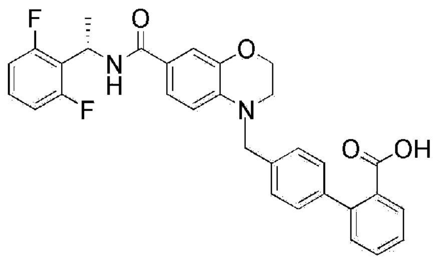 |
| 2 | 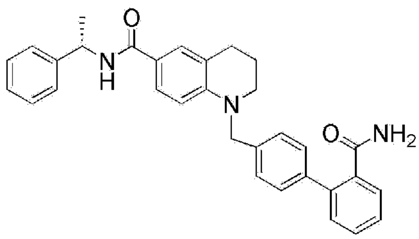 | 84 | 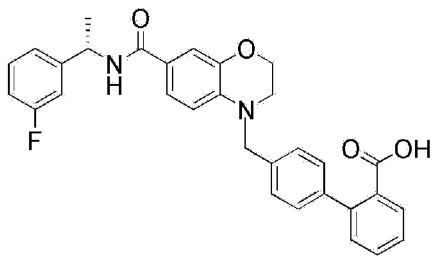 |

(continued)

| Example | Formula | Example | Formula |
|---|---|---|---|
| 3 | 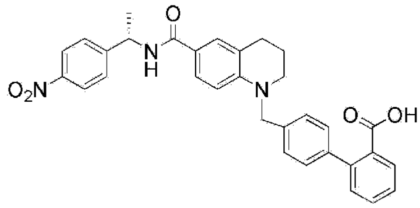 | 85 | 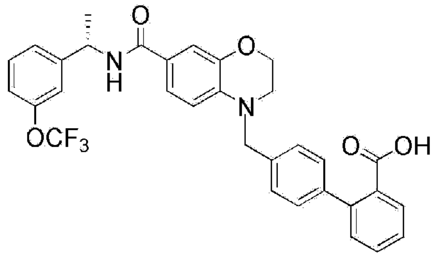 |
| 4 | 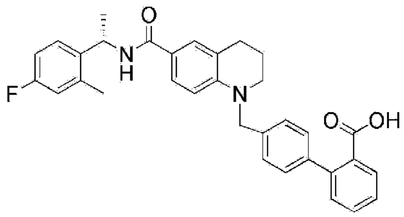 | 86 | 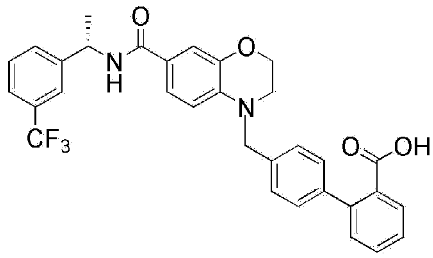 |
| 5 | 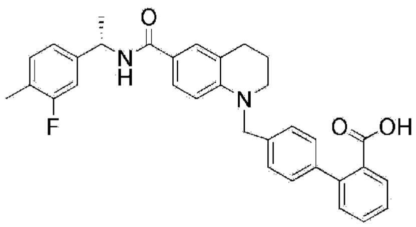 | 87 | 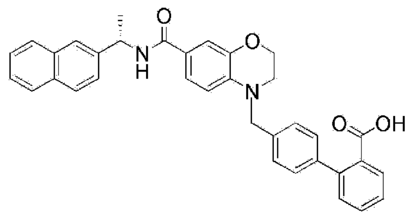 |
| 6 | 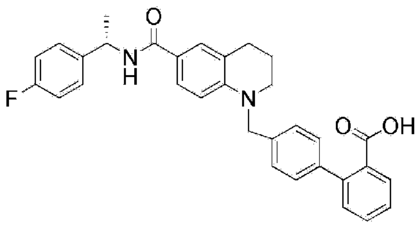 | 88 | 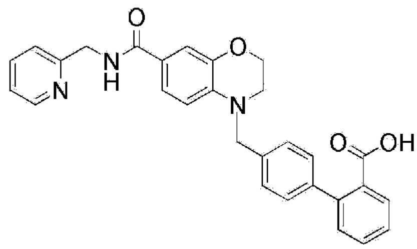 |
| 7 | 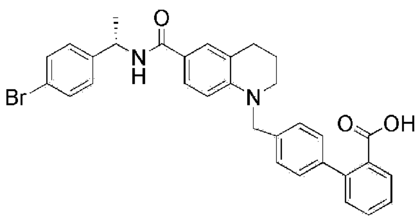 | 89 | 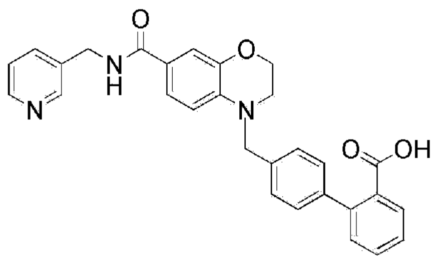 |
| 8 | 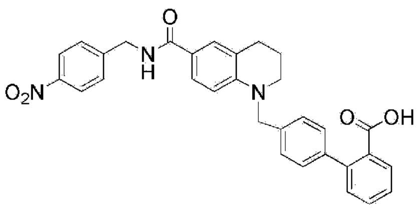 | 90 | 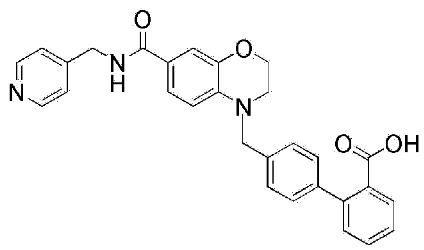 |

(continued)

| Example | Formula | Example | Formula |
|---|---|---|---|
| 9 | 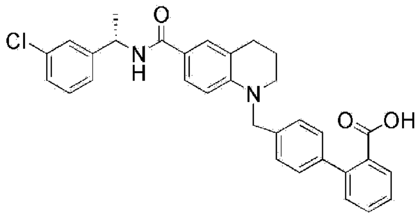 | 91 | 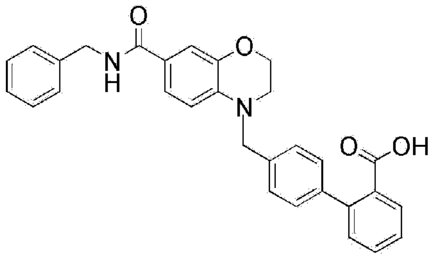 |
| 10 | 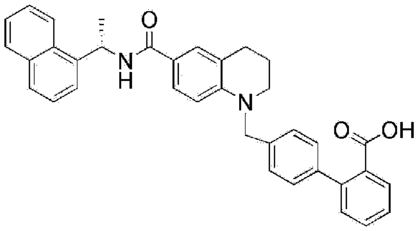 | 92 | 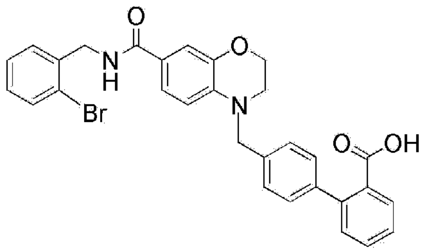 |
| 11 | 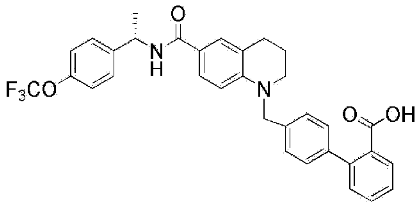 | 93 | 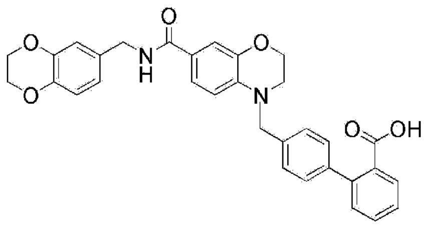 |
| 12 | 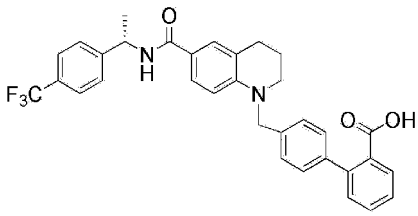 | 94 | 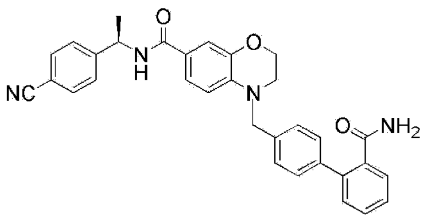 |
| 13 | 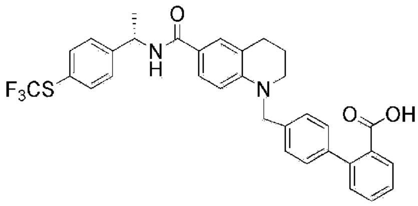 | 95 | 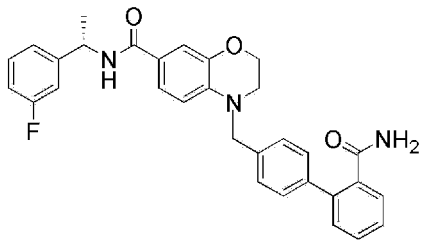 |
| 14 | 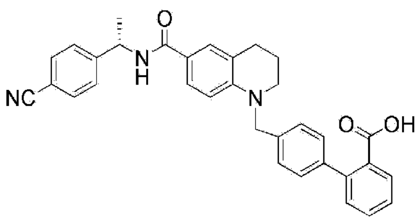 | 96 | 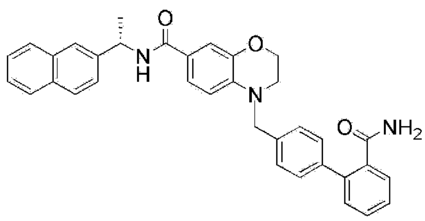 |

(continued)

| Example | Formula | Example | Formula |
|---|---|---|---|
| 15 | 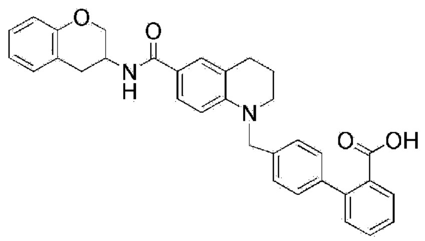 | 97 | 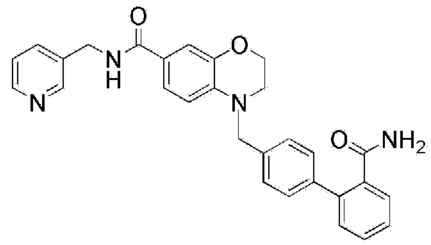 |
| 16 | 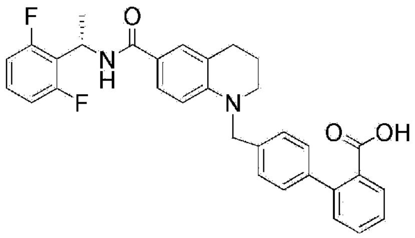 | 98 | 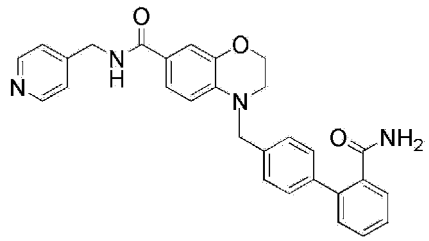 |
| 17 | 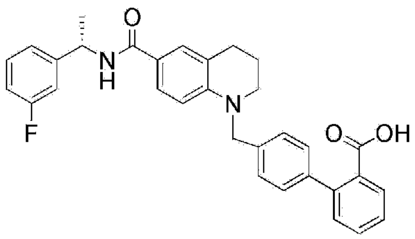 | 99 | 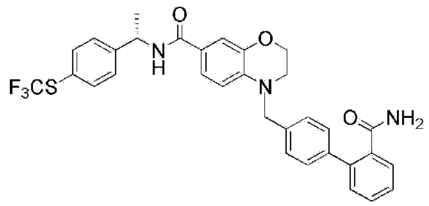 |
| 18 | 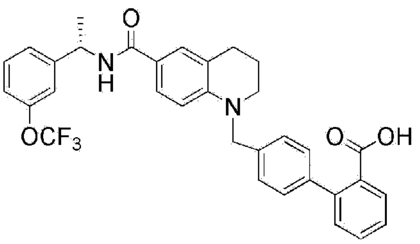 | 100 | 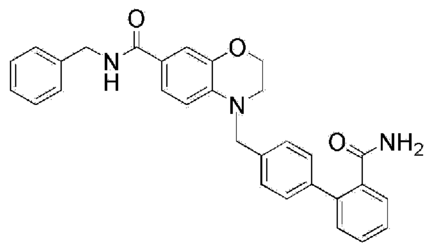 |
| 19 | 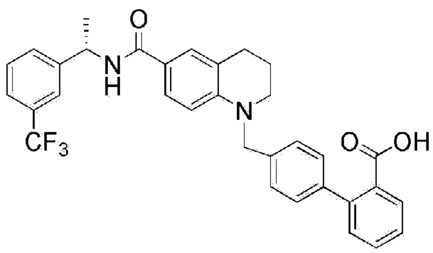 | 101 | 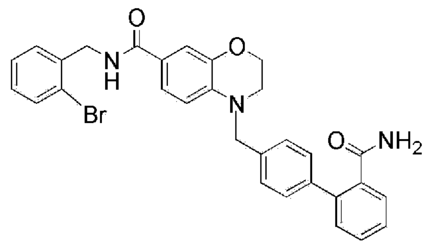 |
| 20 | 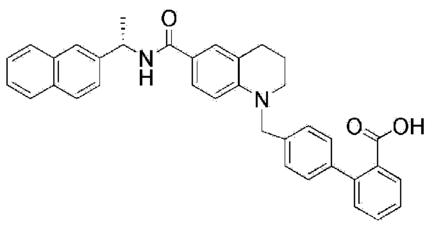 | 102 | 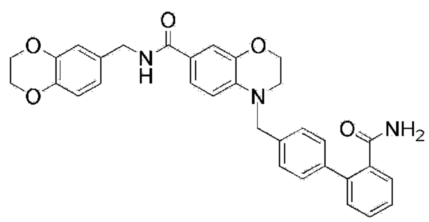 |

(continued)

| Example | Formula | Example | Formula |
|---|---|---|---|
| 21 | 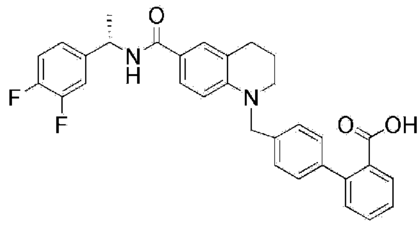 | 103 | 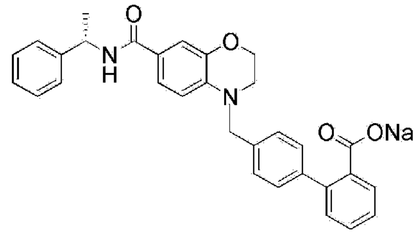 |
| 22 | 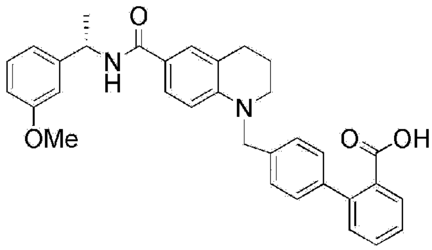 | 104 | 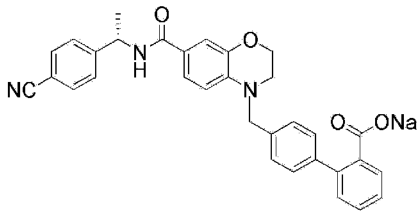 |
| 23 | 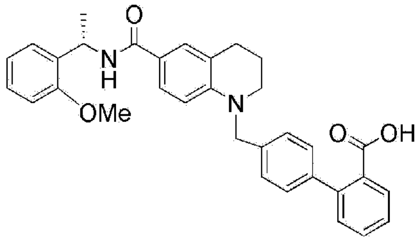 | 105 | 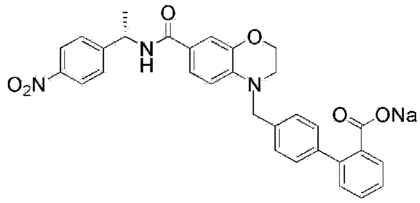 |
| 24 | 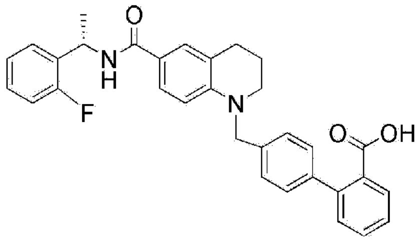 | 106 | 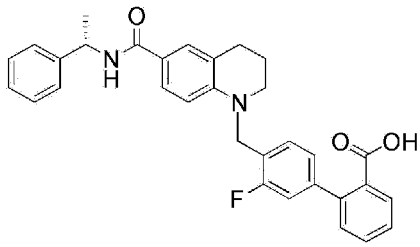 |
| 25 | 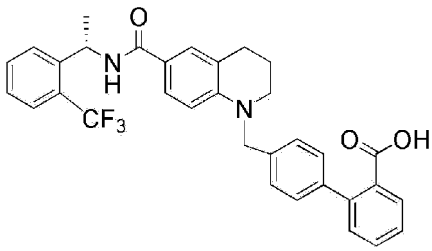 | 107 | 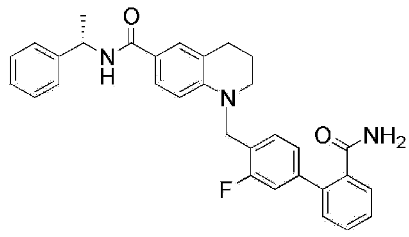 |
| 26 | 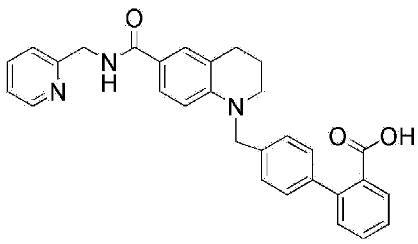 | 108 | 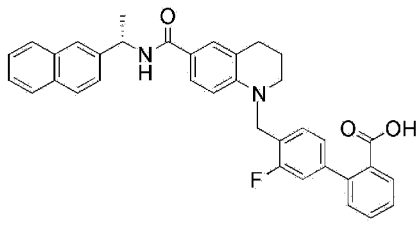 |

(continued)

| Example | Formula | Example | Formula |
|---|---|---|---|
| 27 | 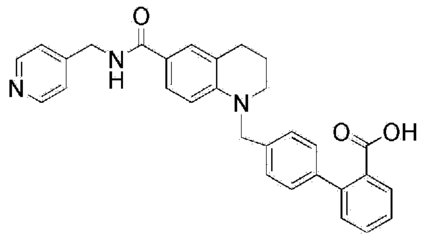 | 109 | 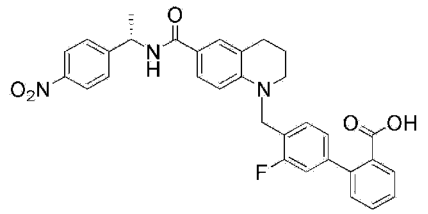 |
| 28 | 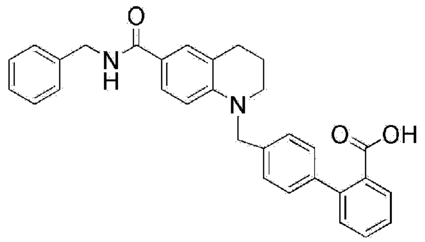 | 110 | 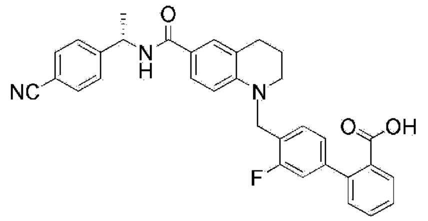 |
| 29 | 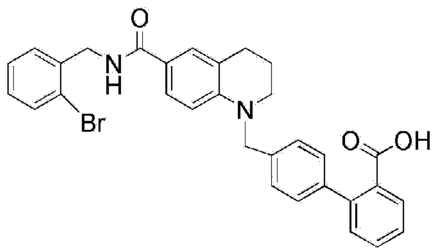 | 111 | 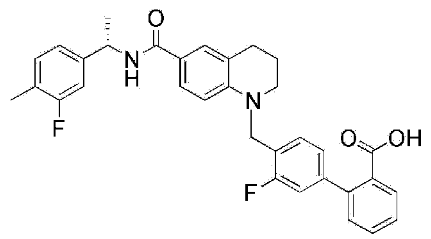 |
| 30 | 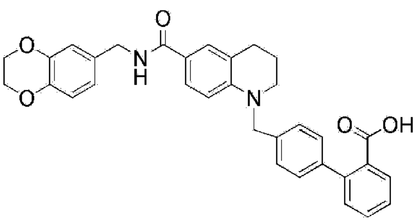 | 112 | 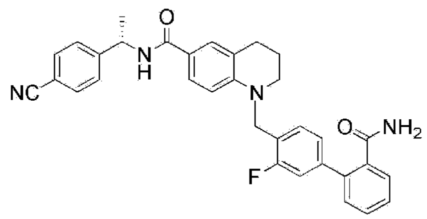 |
| 31 | 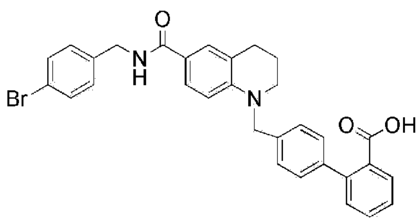 | 113 | 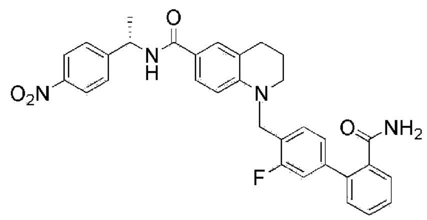 |
| 32 | 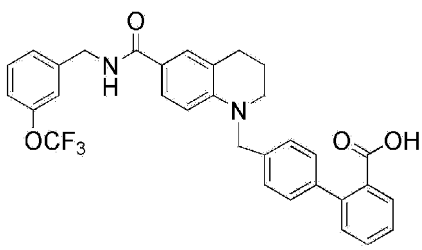 | 114 | 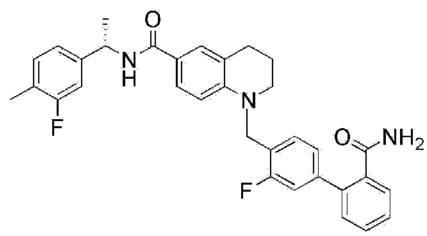 |

(continued)

| Example | Formula | Example | Formula |
|---|---|---|---|
| 33 | 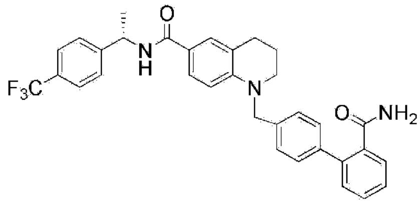 | 115 | 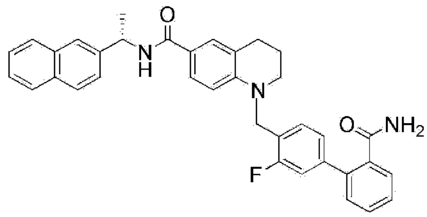 |
| 34 | 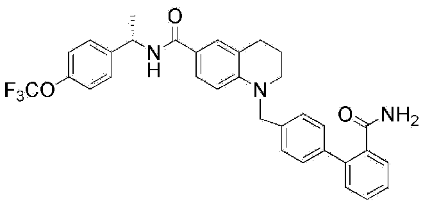 | 116 | 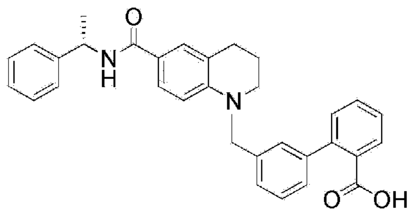 |
| 35 | 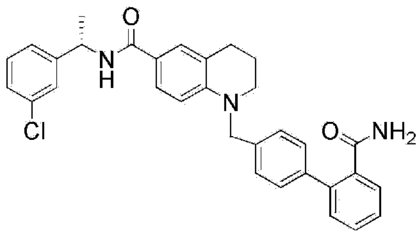 | 117 | 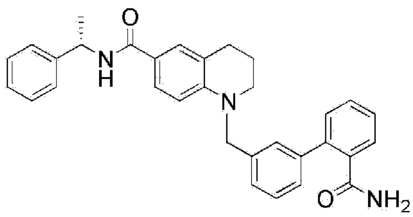 |
| 36 | 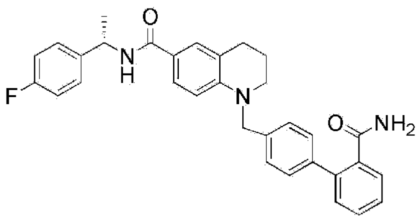 | 118 | 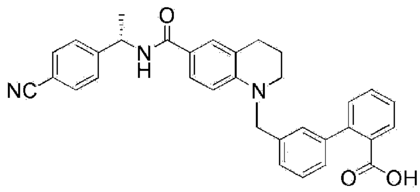 |
| 37 | 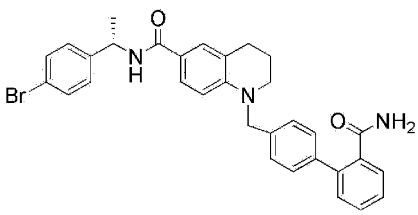 | 119 | 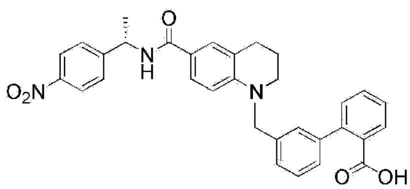 |
| 38 | 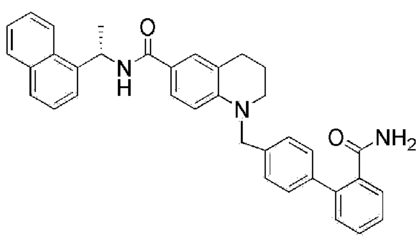 | 120 | 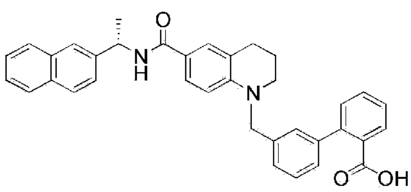 |
| 39 | 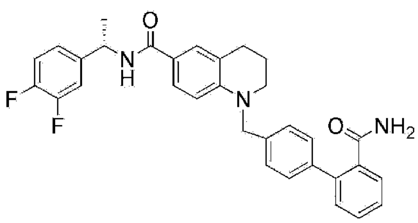 | 121 | 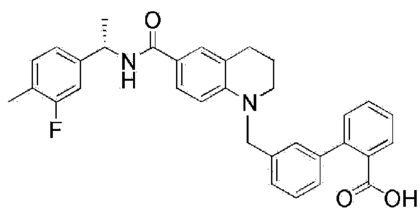 |

(continued)

| Example | Formula | Example | Formula |
|---|---|---|---|
| 40 | 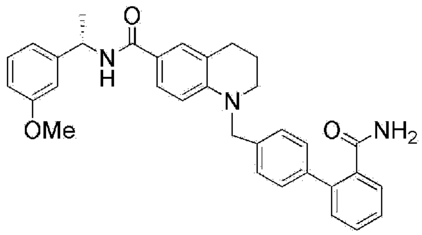 | 122 | 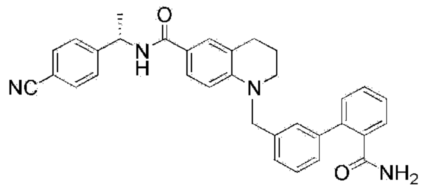 |
| 41 | 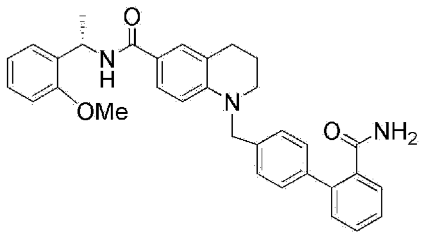 | 123 | 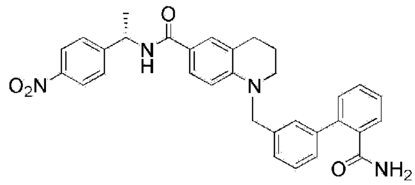 |
| 42 | 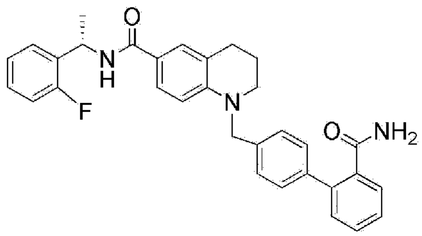 | 124 | 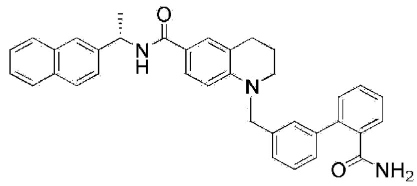 |
| 43 | 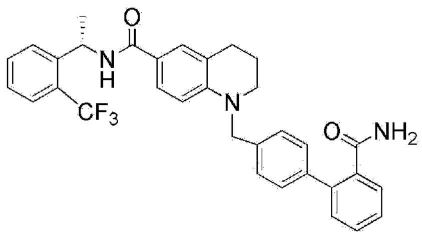 | 125 | 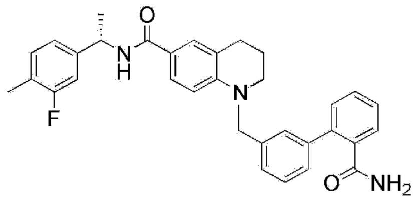 |
| 44 | 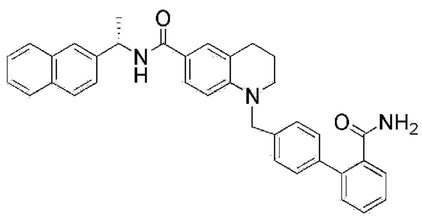 | 126 | 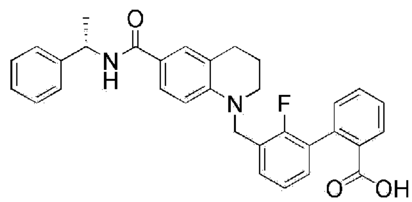 |
| 45 | 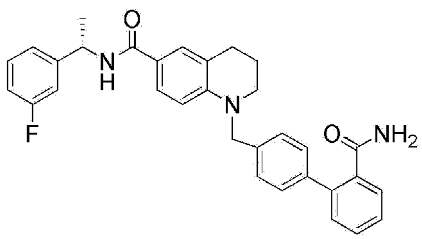 | 127 | 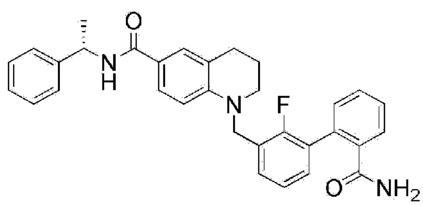 |

(continued)

| Example | Formula | Example | Formula |
|---|---|---|---|
| 46 | 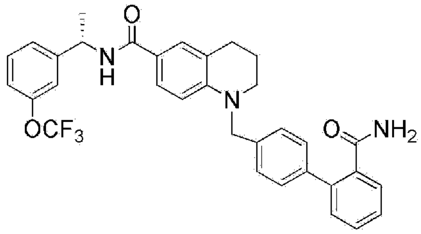 | 128 | 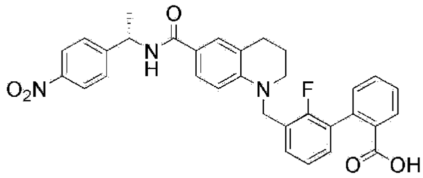 |
| 47 | 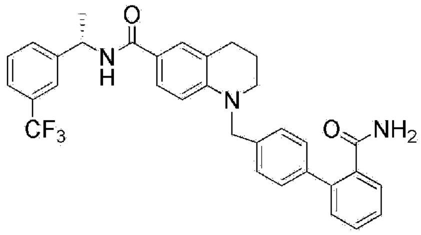 | 129 | 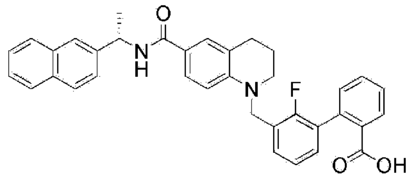 |
| 48 | 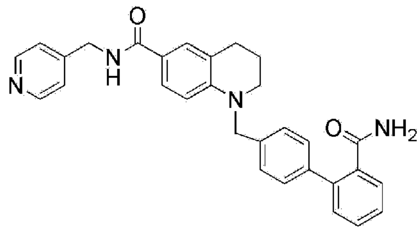 | 130 | 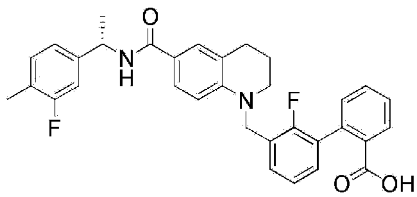 |
| 49 | 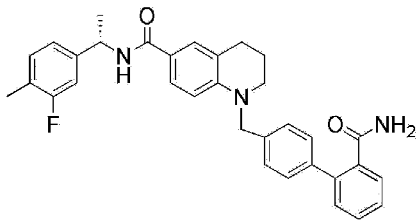 | 131 | 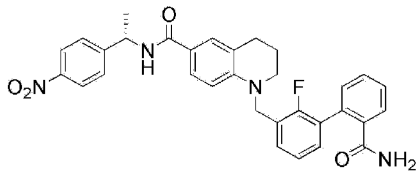 |
| 50 | 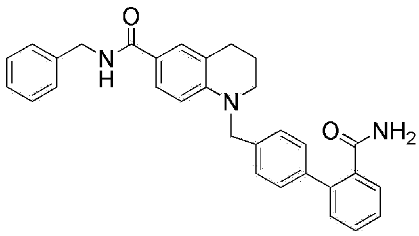 | 132 | 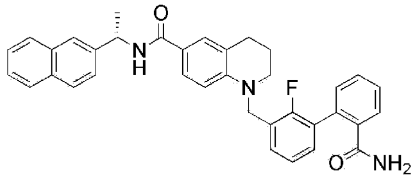 |
| 51 | 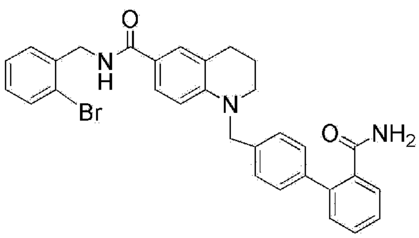 | 133 | 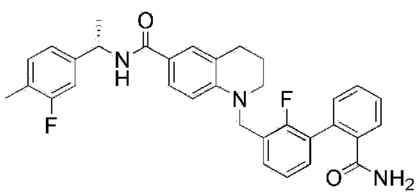 |

(continued)

| Example | Formula | Example | Formula |
|---|---|---|---|
| 52 | 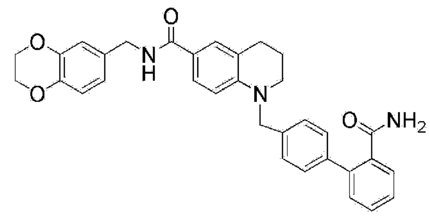 | 134 | 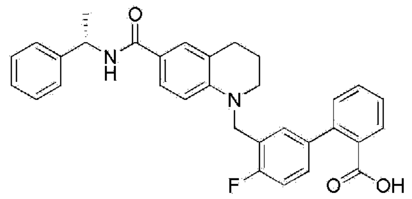 |
| 53 | 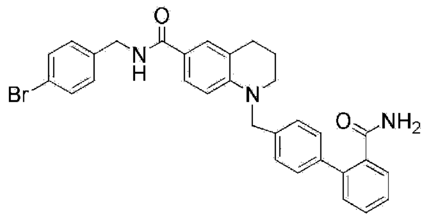 | 135 | 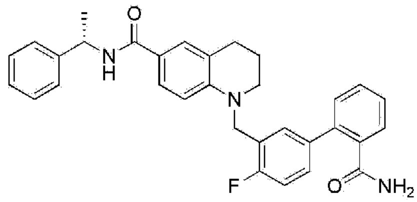 |
| 54 | 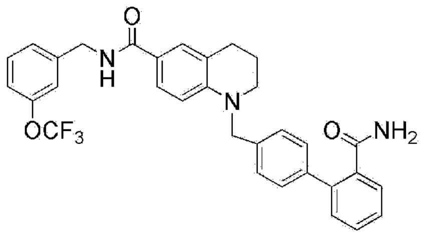 | 136 | 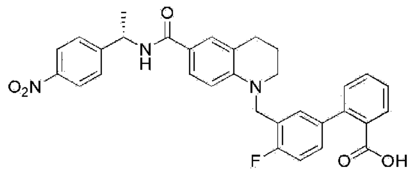 |
| 55 | 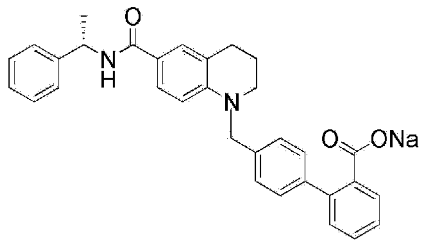 | 137 | 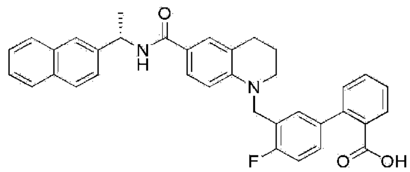 |
| 56 | 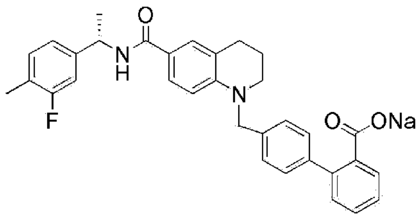 | 138 | 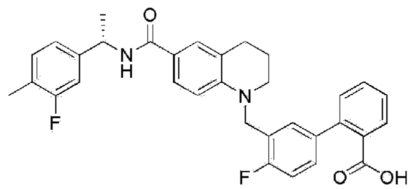 |
| 57 | 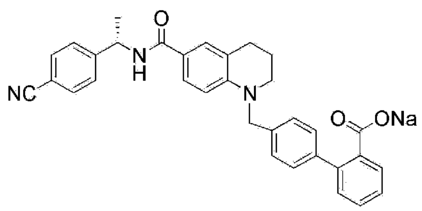 | 139 | 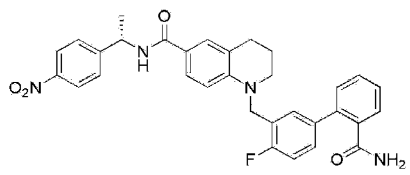 |

(continued)

| Example | Formula | Example | Formula |
|---|---|---|---|
| 58 | 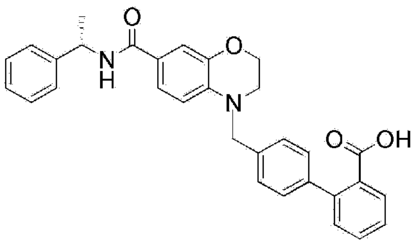 | 140 | 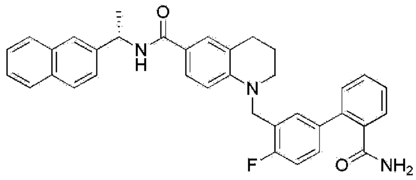 |
| 59 | 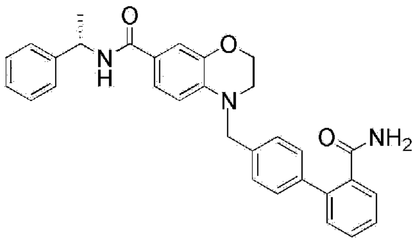 | 141 | 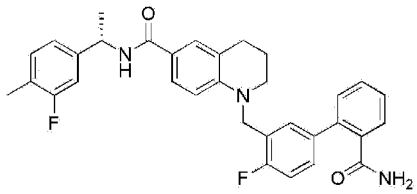 |
| 60 | 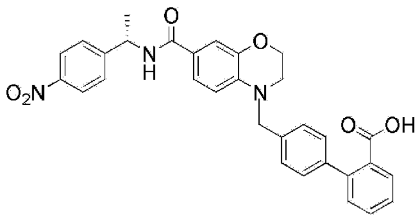 | 142 | 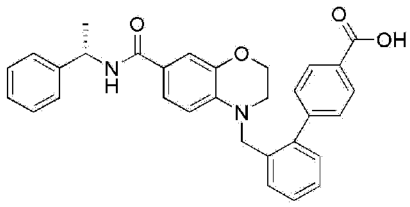 |
| 61 | 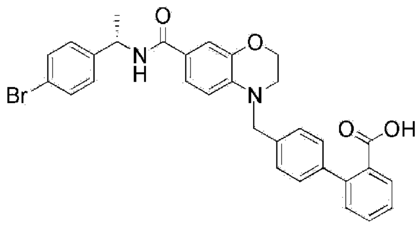 | 143 | 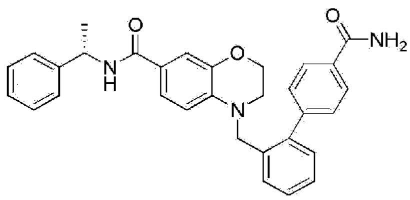 |
| 62 | 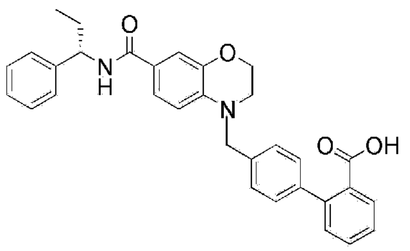 | 144 | 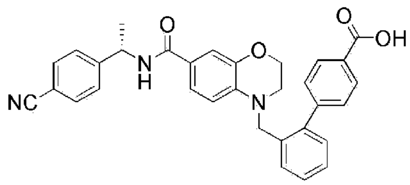 |
| 63 | 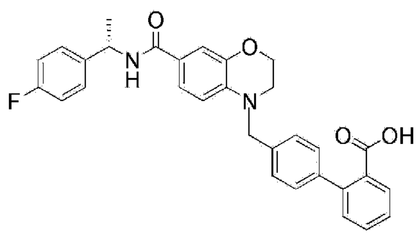 | 145 | 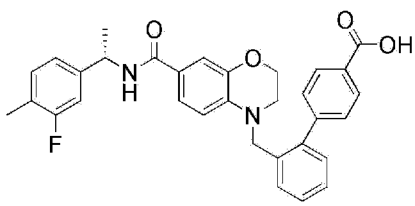 |

(continued)

| Example | Formula | Example | Formula |
|---|---|---|---|
| 64 | 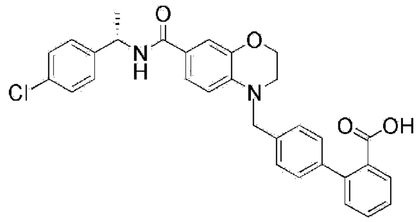 | 146 | 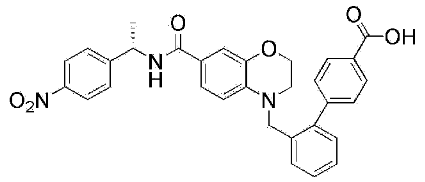 |
| 65 | 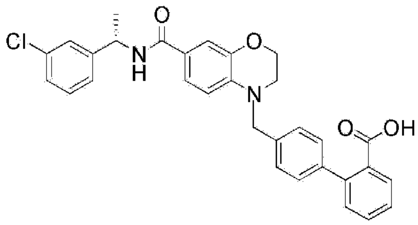 | 147 | 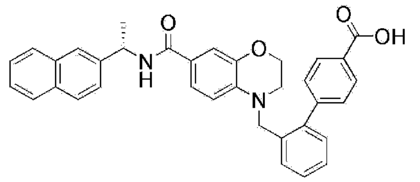 |
| 66 | 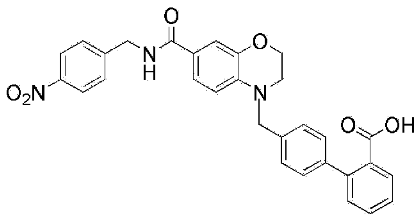 | 148 | 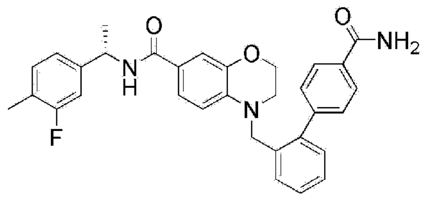 |
| 67 | 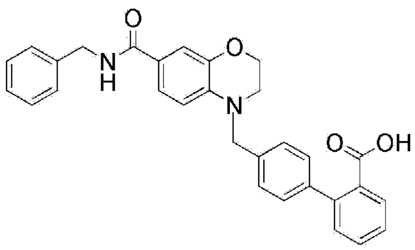 | 149 | 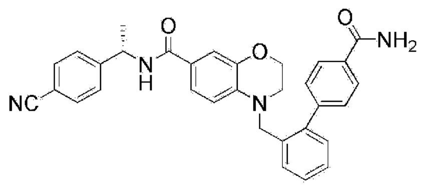 |
| 68 | 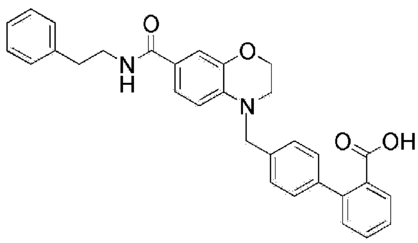 | 150 | 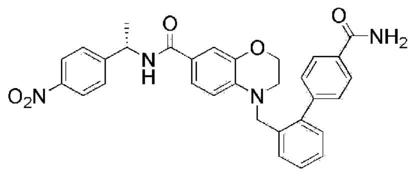 |
| 69 | 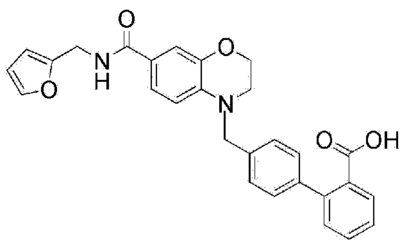 | 151 | 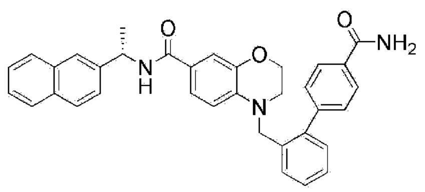 |

(continued)

| Example | Formula | Example | Formula |
|---|---|---|---|
| 70 | 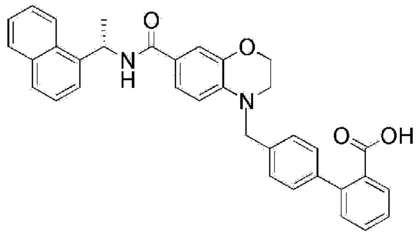 | 152 | 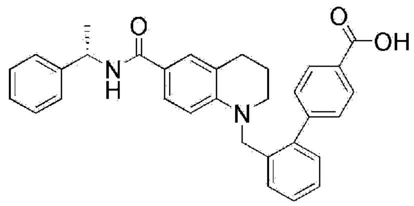 |
| 71 | 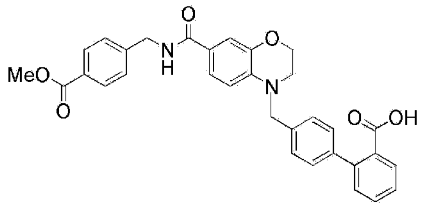 | 153 | 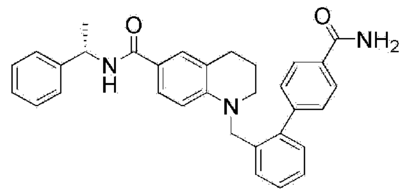 |
| 72 | 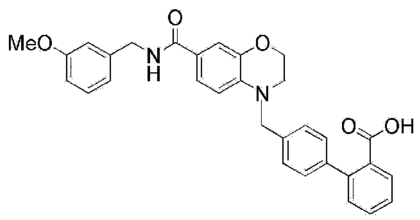 | 154 | 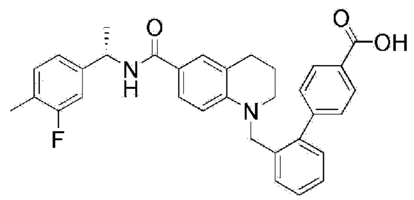 |
| 73 | 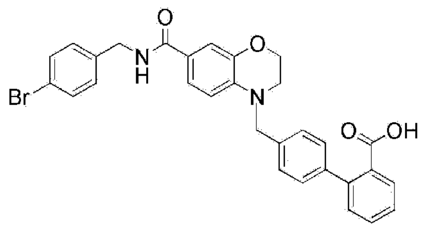 | 155 | 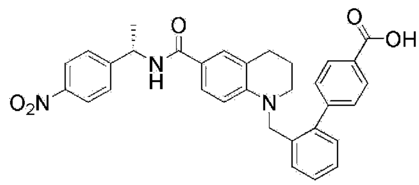 |
| 74 | 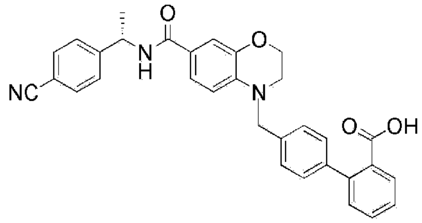 | 156 | 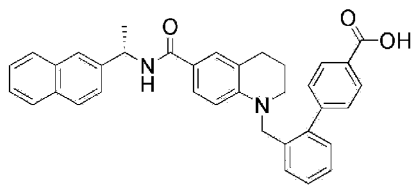 |
| 75 | 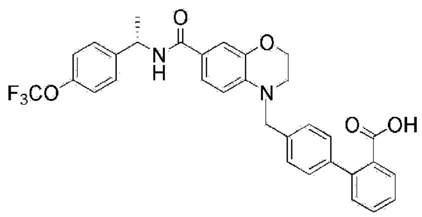 | 157 | 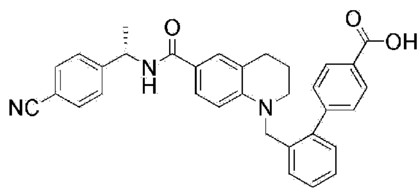 |
| 76 | 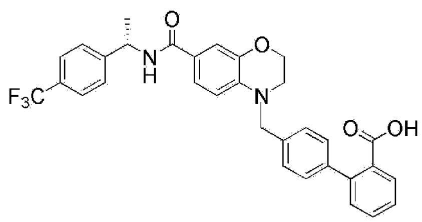 | 158 | 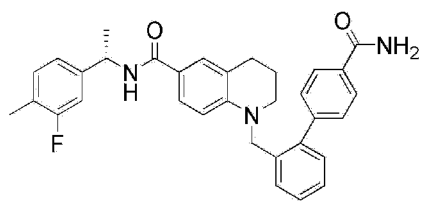 |

(continued)

| Example | Formula | Example | Formula |
|---|---|---|---|
| 77 | 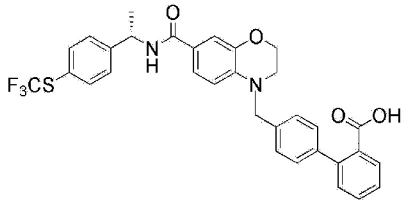 | 159 | 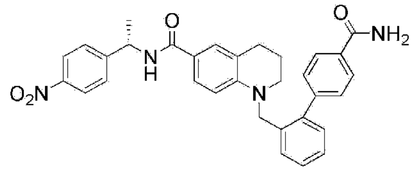 |
| 78 | 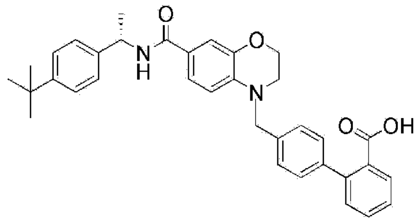 | 160 | 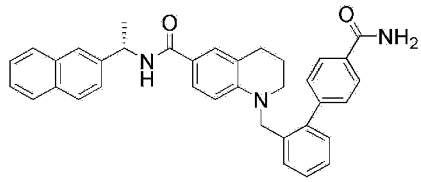 |
| 79 | 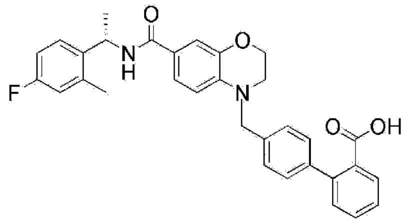 | 161 | 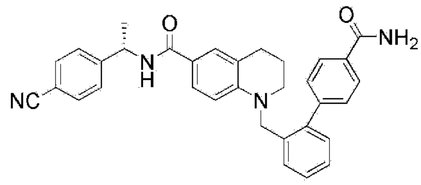 |
| 80 | 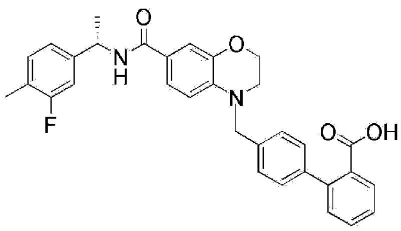 | 162 | 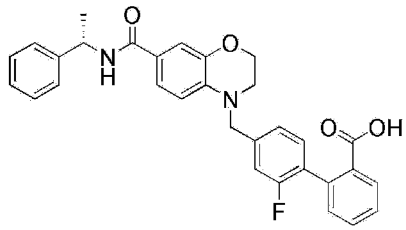 |
| 81 | 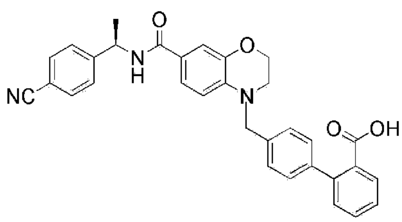 | 163 | 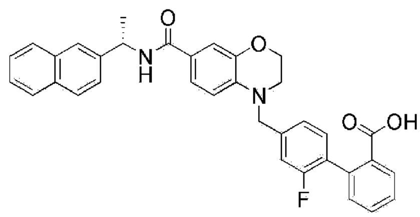 |
| 82 | 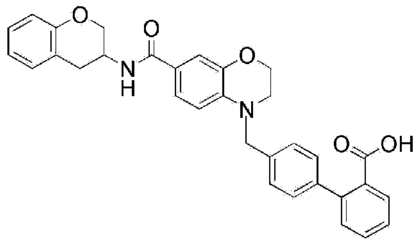 | 164 | 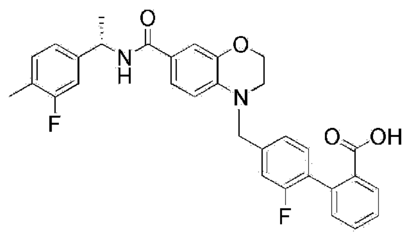 |

**<Experimental Example 1> Evaluation of CDK5 (cyclin-dependant kinase 5) phosphorylation suppressing activity**

The following experiment was performed to investigate whether or not the compounds prepared in the examples of the invention could bind to PPARG but did not act as a CDK5 (cyclin-dependant kinase 5) promoter so that it did inhibit the phosphorylation of the 273rd amino acid serine of PPARG.

The compounds of examples 1 ~ 164 and SR1664 and SR1824 of comparative examples were diluted in DMSO (dimethyl sulfoxide) to meet the final test concentration 2000×. The 2000× compounds were diluted in 50% DMSO (DMSO:DW=1:1) at the ratio of 1:100. Premix in total volume of 36 μℓ was prepared by mixing 0.43 μg of PPARG-LBD (ligand binding domain) (human recombinant) (Cayman, 10007941), Cdk5/p35, 100 ng of active (Millipore, 14-477), 10× kinase buffer (Cell Signaling, 9802S), and DW (Data warehousing) (the premix was prepared on the ice and stored in the ice).

2 μℓ of the compound of the invention was mixed in 36 μℓ of the premix, followed by reaction in the ice for 10 minutes. 2 μℓ of 10 mM ATP (negative control: DW 2 μℓ) was added thereto, followed by reaction in a 37°C water bath for 15 minutes. Upon completion of the reaction, the mixture was transferred onto the ice swiftly so that the mixture was cooled down for 1 ~ 2 minutes. 10 μℓ of 5× sample buffer was mixed with the reaction mixture, followed by heating at 95°C in heat block for 8 ~ 10 minutes. Upon completion of the treatment in heat block, the mixture was taken out and cooled down for a while, followed by SDS-PAGE (sodium dodecyl sulfate-polyacryl amide gel electrophoresis) on 10% SDS-gel.

Then, the protein on SDS-gel was transferred onto nitrocellulose membrane (IB3010-32, Invitrogen) by using iBlot (IB1001, Invitrogen).

Upon completion of the transfer, the membrane proceeded to blocking in 5% BSA-TBST (bovine serum albumin-tris buffered saline and tween 20) for 30 minutes ~ 1 hour, followed by reaction with the primary antibody phospho-PPARG-Ser273 (Hyundai Pharm. Co., Ltd.) for at least 18 hours in a 4°C refrigerator. The membrane was washed with TBST (Tris-Buffered Saline and Tween 20), followed by reaction with the secondary antibody Anti-rabbit IgG (HRP-linked antibody, 7074S, Cell Signaling) at room temperature for 1 hour. Upon completion of the reaction, the membrane was washed with TBST, followed by reaction in ECL (enhanced chemiluminescence) solution (NCI34095KR, Thermo Scientific). The band signal for phospho-PPARG-Ser273 was measured by Las4000mini (Fujifilm corp). After measuring the phospho-Ser273, the membrane was reacted in stripping buffer (NCI1059KR, Thermo Scientific) for 30 minutes, and then was washed, which was the ready for the total PPARG measurement.

The membrane was reacted with PPARG(E-8) antibody (sc-7273, Santa Cruz), the primary antibody, at room temperature for 1 hour, followed by another reaction with anti-mouse IgG (HRP-linked antibody, sc-2005, Santa Cruz), the secondary antibody, for 1 hour.

Upon completion of the reaction, the membrane was washed and reacted in ECL solution (RPN2232, GE Healthcare). The total PPARG band signal was measured by Las4000mini (Fujifilm corp).

The compounds prepared in the examples of the present invention were confirmed to bind to PPARG (peroxisome proliferator activated receptor-gamma) but not to act as a CDK5 (cyclin-dependant kinase 5) promoter, indicating that they are excellent in suppressing the phosphorylation of serine, the 273rd amino acid of PPARG.

Therefore, the compounds of examples of the invention bind to PPARG (peroxisome proliferator activated receptor-gamma) but do not activate CDK5 (cyclin-dependant kinase 5), the phosphorylation inducer, so that they can be effectively used as a therapeutic agent for PPARG-related disease by preventing side effects accompanied by the phosphorylation of serine, the 273rd amino acid of PPARG.

**<Experimental Example 2> Evaluation of PPARG (peroxisome proliferator activated receptor-gamma) binding capacity and transcriptional activity**

The following experiment was performed to evaluate the PPARG (peroxisome proliferator activated receptor-gamma) binding capacity and transcriptional activity of the compounds prepared in the examples of the invention.

For the experiment, LanthaScreen™ TR-FRET PPARG Competitive Binding Assay Kit (PV4894, Invitrogen) was used according to the manufacturer's instruction.

30 μℓ of the compound or the control substance (SR-1664, Rosiglitazone) diluted in assay buffer (50×) in a 96-well plate (SPL, 34096) was mixed with 15 μℓ of 5 nM Fluormone™ Pan-PPAR Green, to which 15 μℓ of 5 nM GST-PPARG-LBD and 5 nM Tb-GST-antibody were added. The mixture was loaded in a 384-well plate (Greiner, 784075) by 20 μℓ per well, followed by reaction at room temperature for 1 hour.

Upon completion of the reaction, fluorescence value was measured by using Flexstation 3 (Molecular Devices) with time-resolved fluorescence (RFU) mode (excitation 1: 340 nm, emission 1: 518 nm, excitation 2: 340 nm, emission 2: 488 nm, integration delay 50 us, integration 400 us). The results were calculated by using 518 nm RFUs/488 nm RFUs ratio. The activity of the compounds of the invention was compared with that of the vehicle, which was calculated by ratio according to the formula [100% - compound of the example ratio/vehicle ratio]. At this time, the binding activity ratio to rosiglitazone was compared.

The following experiment was performed to evaluate PPARG (peroxisome proliferator activated receptor-gamma) transcriptional activity of the compounds of the invention.

HEK293 cells were plated in a 24-well plate (SPL, 30024) at the density of $2.5\times10^4$ cells/well. The cells were transfected with PPRE, PPARG or PPARα, RXR, and Renilla DNA by using FuGENE HD (Promega, E2312). Upon

completion of the gene transfection, the cells were treated with rosiglitazone, SR1664, and the compounds of the invention respectively for 24 hours. 24 hours later, the cells were collected, followed by reporter gene assay with Dual Reporter gene assay kit (Promega, E1980). Luciferase assay activity was calculated by normalization of renilla activity.

[Table 2]

| Example | Binding assay (1 uM) | PPRE EC50 (nM) |
|---|---|---|
| 1 | +++ | * |
| 2 | +++ | *** |
| 3 | + | NA |
| 4 | +++ | *** |
| 5 | ++ | NA |
| 6 | +++ | * |
| 7 | ++++ | * |
| 8 | +++ | * |
| 9 | ++++ | *** |
| 10 | +++ | NA |
| 11 | ++++ | *** |
| 12 | ++++ | *** |
| 13 | ++++ | NA |
| 14 | ++ | NA |
| 15 | + | * |
| 16 | +++ | ** |
| 17 | +++ | ** |
| 18 | +++ | *** |
| 19 | +++ | *** |
| 20 | ++++ | NA |
| 21 | +++ | * |
| 22 | +++ | * |
| 23 | +++ | * |
| 24 | +++ | ** |
| 25 | +++ | NA |
| 26 | ++ | * |
| 27 | + | NA |
| 28 | ++++ | ** |
| 29 | ++++ | NA |
| 30 | +++ | * |
| 31 | ++++ | *** |
| 32 | +++ | *** |
| 33 | +++ | NA |
| 34 | ++++ | NA |
| 35 | +++ | *** |

(continued)

| Example | Binding assay (1 uM) | PPRE EC50 (nM) |
|---|---|---|
| 36 | +++ | NA |
| 37 | +++ | NA |
| 38 | ++ | NA |
| 39 | +++ | *** |
| 40 | +++ | *** |
| 41 | +++ | NA |
| 42 | +++ | NA |
| 43 | ++ | NA |
| 44 | +++ | NA |
| 45 | +++ | *** |
| 46 | +++ | NA |
| 47 | +++ | NA |
| 48 | + | NA |
| 49 | +++ | NA |
| 50 | +++ | *** |
| 51 | +++ | NA |
| 52 | +++ | NA |
| 53 | +++ | *** |
| 54 | +++ | *** |
| 55 | NT | NT |
| 56 | ++++ | NA |
| 57 | +++ | NA |
| 58 | + | NA |
| 59 | ++ | *** |
| 60 | ++ | NA |
| 61 | ++ | NA |
| 62 | +++ | * |
| 63 | + | NA |
| 64 | + | NA |
| 65 | ++ | NA |
| 66 | + | NA |
| 67 | ++ | * |
| 68 | ++ | * |
| 69 | + | NA |
| 70 | ++ | NA |
| 71 | + | * |
| 72 | + | NA |
| 73 | + | * |

(continued)

| Example | Binding assay (1 uM) | PPRE EC50 (nM) |
|---|---|---|
| 74 | + | NA |
| 75 | +++ | * |
| 76 | +++ | NA |
| 77 | +++ | * |
| 78 | ++ | NA |
| 79 | ++ | * |
| 80 | ++ | NA |
| 81 | +++ | * |
| 82 | +++ | * |
| 83 | ++ | * |
| 84 | ++ | * |
| 85 | +++ | *** |
| 86 | +++ | NA |
| 87 | ++++ | NA |
| 88 | + | * |
| 89 | + | NA |
| 90 | + | NA |
| 91 | +++ | * |
| 92 | +++ | NA |
| 93 | ++ | * |
| 94 | ++ | *** |
| 95 | +++ | *** |
| 96 | +++ | NA |
| 97 | + | NA |
| 98 | + | NA |
| 99 | +++ | NA |
| 100 | ++ | *** |
| 101 | ++ | * |
| 102 | + | NA |
| 103 | ++ | NA |
| 104 | NT | NT |
| 105 | + | NA |
| 106 | ++++ | *** |
| 107 | +++ | *** |
| 108 | ++++ | NA |
| 109 | +++ | * |
| 110 | +++ | NA |
| 111 | ++++ | *** |

(continued)

| Example | Binding assay (1 uM) | PPRE EC50 (nM) |
|---|---|---|
| 112 | +++ | NA |
| 113 | ++ | NA |
| 114 | +++ | NA |
| 115 | +++ | NA |
| 116 | +++ | * |
| 117 | +++ | *** |
| 118 | + | NA |
| 119 | ++ | * |
| 120 | ++++ | * |
| 121 | +++ | ** |
| 122 | ++ | NA |
| 123 | +++ | NA |
| 124 | ++++ | ** |
| 125 | +++ | NA |
| 126 | +++ | * |
| 127 | +++ | * |
| 128 | ++ | * |
| 129 | ++++ | ** |
| 130 | +++ | *** |
| 131 | +++ | NA |
| 132 | ++++ | * |
| 133 | +++ | NA |
| 134 | ++++ | * |
| 135 | +++ | *** |
| 136 | +++ | * |
| 137 | ++++ | *** |
| 138 | ++++ | *** |
| 139 | +++ | NA |
| 140 | ++++ | *** |
| 141 | +++ | NA |
| 142 | ++ | * |
| 143 | ++ | NA |
| 144 | + | *** |
| 145 | ++ | NA |
| 146 | + | * |
| 147 | +++ | * |
| 148 | ++ | NA |
| 149 | + | NA |

(continued)

| Example | Binding assay (1 uM) | PPRE EC50 (nM) |
|---|---|---|
| 150 | ++ | NA |
| 151 | +++ | *** |
| 152 | ++ | ** |
| 153 | ++ | *** |
| 154 | + | NA |
| 155 | + | NA |
| 156 | + | NA |
| 157 | ++ | NA |
| 158 | + | NA |
| 159 | + | NA |
| 160 | ++ | *** |
| 161 | ++ | NA |
| 162 | +++ | NT |
| 163 | +++ | NT |
| 164 | +++ | NT |

In Table 2,

NT (No Tested) means that the experiment was not performed,
"+" indicates the degree of the binding activity and as the number of "+" is getting more the binding activity is getting stronger,
"*" indicates the degree of the transcriptional activity and as the number of "*" is getting more the transcription activity is getting stronger, and "NA" means there is no transcriptional activity detected since the compound has not been acting as an agonist.

As shown in Table 2, the compounds of the examples of present invention displayed excellent binding activity to PPARG (peroxisome proliferator activated receptor-gamma). At this time, the binding activity number indicates the binding or no binding and does not relate directly to the pharmacological activity.

The compounds of the examples of present invention were confirmed not to induce the transcription of PPARG (peroxisome proliferator activated receptor-gamma).

Therefore, the compounds of the examples of the present invention bind specifically to PPARG (peroxisome proliferator activated receptor-gamma) but do not induce the transcription of PPARG gene and thereby prevent the side effects carried by PPARG phosphorylation, suggesting that the compounds can be effectively used as a therapeutic agent for PPARG-related diseases.

**<Experimental Example 3> Evaluation of CYP (cytochrome P450) suppressing effect**

To evaluate the CYP (cytochrome P450) suppressing activity of the compounds prepared in the examples of the invention, the following experiment was performed.

The analysis of CYP1A2, 2C19, and 3A4 suppressing activity was performed by using BD GENTEST (459100, 459400, and 459500) kit and the analysis of CYP 2C9 and 2D6 suppressing activity was performed by using Invitrogen (P2861 and P2862) kit.

When the Invitrogen kit was used, the compounds of the present invention were diluted in Vivid CYP450 reaction buffer (1×) at the final concentration of 2.5×. P450 BACULOSOMES sample and Regeneration system (100×) were diluted in Vivid CYP450 reaction buffer (1×) at different proper concentrations. 80 $\mu\ell$ of the compound of the invention (2.5×) and 100 $\mu\ell$ of the diluted P450 BACULOSOMES sample mixture were mixed in a U-bottom 96-well plate, followed by pre-incubation for 10 minutes. Vivid CYP450 Substrate and NADP+ (100×) were diluted in Vivid CYP450 reaction buffer (1×) at a desired concentration respectively according to their corresponding types of CYP450 and substrates.

20 μℓ of Substrate-NADP+ mix was loaded in the plate when the pre-incubation had been completed, followed by reaction for 1 hour. Upon completion of the reaction, the reaction mixture was transferred in a white plate and fluorescent wavelength was measured with a microplate reader (2C9 excitation 415 nm, emission 460 nm, 2D6 excitation 400 nm, emission 502 nm).

When the BD GENTEST kit was used, the compounds of the present invention were diluted in acetonitrile at the final concentration of 50×. NADPH-Cofactor mix was prepared by diluting the Cofactor, G6PDH, and control protein provided by the kit in DW according to the instructed concentration. 4 μℓ of the compound of the example (50×) was mixed with 96 μℓ of NADPH-Cofactor mix in a 96-well plate, followed by pre-incubation in a 37°C incubator for 10 minutes.

The enzyme/substrate mixture was diluted in the buffer (0.5 M potassium phosphate, pH 7.4) provided by the kit according to the instructed concentration. Each CYP450 enzyme substrate was diluted in DW at a desired concentration respectively according to the corresponding types of CYP450. 100 μℓ of the enzyme/substrate mixture was loaded in the plate when the pre-incubation had been completed, followed by reaction in a 37°C incubator for 15 minutes (1A2) and 30 minutes (3A4 and 2C19). Upon completion of the reaction, the mixture was transferred in a white plate and fluorescent wavelength was measured with a microplate reader (1A2, 2C19 excitation 410 nm, emission 460 nm, 3A4 excitation 409 nm, emission 530 nm).

The test results were calculated by the following formula:

(1-(fluorescence value of the test material/ fluorescence value of the vehicle)) x 100%.

[Table 3]

| Example | CYP(10uM, %) | | | | |
|---|---|---|---|---|---|
| | 1A2 | 2C9 | 2C19 | 2D6 | 3A4 |
| 14 | 97.99% | 64.28% | 79.96% | 86.13% | 71.24% |
| 36 | 98.84% | 51.03% | 52.68% | 75.73% | 58.18% |
| 44 | 94.56% | 56.40% | 59.55% | 97.07% | 70.42% |
| 56 | 100.73% | 55.36% | 85.71% | 94.15% | 54.62% |
| 57 | 100.32% | 58.87% | 75.69% | 82.59% | 59.17% |
| 58 | 100.93% | 57.47% | 70.53% | 55.23% | 56.92% |
| 60 | 100.63% | 80.25% | 69.60% | 62.27% | 70.64% |
| 74 | 102.89% | 80.68% | 91.36% | 83.97% | 81.98% |
| 96 | 92.41% | 57.63% | 52.35% | 98.66% | 98.04% |
| 110 | 97.28% | 55.18% | 69.53% | 88.44% | 53.03% |
| 113 | 97.27% | 67.73% | 54.70% | 78.65% | 85.01% |
| 115 | 97.62% | 63.87% | 59.33% | 95.56% | 77.73% |
| 118 | 101.50% | 52.02% | 74.03% | 94.83% | 71.21% |
| 123 | 102.45% | 59.36% | 58.85% | 82.48% | 52.65% |
| 131 | 94.17% | 52.84% | 54.84% | 86.92% | 55.88% |
| 139 | 94.00% | 63.24% | 53.50% | 80.63% | 60.95% |
| Comparative Example 1 (SR1664) | 60.95% | 3.52% | 33.88% | 50.18 % | 58.56% |
| Comparative Example 2 (SR1824) | 64.12% | 6.43% | 32.45% | 68.34% | 71.88% |

As shown in Table 3, the compounds of the examples of 14, 36, 44, 56, 57, 58, 60, 74, 96, 110, 113, 115, 118, 123, 131, and 139 of the invention suppressed the CYP isozymes 1A2, 2C9, 2C19, and 2D6 comparatively less than the compounds of the comparative examples (SR1664 and SR1824) did.

Particularly, SR1664 suppressed the CYP isozyme 2C9 activity significantly, so that about 96% of the drug stayed without being decomposed. Therefore, when SR1664 was used as a drug, the amount that reached a target was more than the proper dose as a drug, so that it could cause toxicity.

However, the compounds of the present invention suppressed the CYP activity comparatively moderately, compared with SR1664, so the drug decomposition rate was at least 10 times higher than SR1664, suggesting that a proper amount of the drug would reach a target. Therefore, the compounds of the present invention were confirmed to be more effective as a drug than the conventional SR1664 and SR1824.

**<Experimental Example 4> Evaluation of cardiotoxicity**

To evaluate the cardiotoxicity of the compounds prepared in the examples of the present invention, the following experiment was performed.

HEK293 cells expressing HERG (Human Ether-a-go-go-Related Gene) channel safely were cultured in MEM (Minimum Essential Medium) supplemented with 10% fetal bovine serum (FBS), 1 mM sodium pyruvate, 0.1 mM non-essential amino acid solution, 100 units/ml penicillin-streptomycin, 100 $\mu$g/ml streptomycin sulfate and 100 $\mu$g/ml zeocin under the condition of air:$CO_2$ = 95:5. When the cells were grown to 60 ~ 80% confluency, the cells were loaded in the medium supplemented with 0.25% trypsin and 0.02% EDTA (ethylenediaminetertraacetic acid) for 3 minutes. The cells were then washed with a fresh medium and then dispensed on a new plastic culture dish. For the electrophysiological record, the cells were seeded on a glass cover slip of 5 mm in diameter, followed by incubation in a 24-well plate for 5 ~ 24 hours. Then, the cells were transferred into the recording chamber.

The current value of all the cells was measured by the conventional patch-clamp technique or Port-a-Patch (Nanion, Germany) semiautomatic technique. PH of the external bath solution containing 136 mM NaCl, 5.4 mM KCl, 1.8mM $CaCl_2$, 1 mM $MgCl_2$, 10 mM glucose, and 10 mM HEPES (4-(2-hydroxyethyl)-1-piperazineethanesulfonic acid) was adjusted to be 7.4 by using NaOH. PH of the intracellular pipette solution containing 130 mM KCl, 1 mM $MgCl_2$, 10 mM EGTA (ethylene glycol tetraacetic acid), 5 mM Mg-ATP, and 10 mM HEPES was adjusted to be 7.2 by using KOH.

In the conventional patch-clamp technique, the patch pipette was pulled by PP-83 pipette puller (Narishige, Tokyo, Japan), so that it had 2~4 M$\Omega$ of resistance in the external bath solution. The ionic current was recorded by using EPC7 pulse amplifier (HEKA electronic, Germany). The voltage-clamp amplification was regulated by using pClamp computer program (Axon Instruments, USA) and the obtained data was analyzed. During the recording, the solution above was continually spreaded on HEK293 cells in the chamber. For the recording, Port-a-Patch (Nanion, Germany) semiautomatic patch system was used and the amplifier used herein was EPC10.

The membrane potential was maintained at -80 mV, which was changed into +20 mV for 4 seconds and then changed into -50 mV for 6 seconds and then maintained again at -80 mV. Those changes had been made every 25 seconds.

To obtain the concentration-reaction curve in the presence of the drug, dose-dependent inhibition was put in Hill formula represented by the mathematical formula 1 below.

【Mathematical Formula 1】

$$\frac{I_{drug}}{I_{control}} = \frac{1}{1+(\frac{[D]}{IC_{50}})^{h}}$$

In the mathematical formula 1,

$I_{drug}$ indicates the peak tail current in the presence of a drug,
$I_{control}$ indicates the maximum peak tail current in the absence of a drug,
D indicates the concentration of a drug,
h indicates Hill constant, and
$IC_{50}$ indicates the concentration when the half-maximum peak tail current was suppressed.

[Table 4]

| Example | $IC_{50}$ (uM) |
|---|---|
| 157 | 42.67 |
| 156 | 55.21 |
| 155 | 73.60 |
| 56 | 72.60 |
| Comparative Example 1 (SR-1664) | 2.7 |

As shown in Table 4, SR-1664 displayed a significantly lower $IC_{50}$ (2.7 uM) than $IC_{50}$ that can induce cardiotoxicity (10 uM), suggesting that when it is used as a drug there is a high chance of cardiotoxicity.

However, the compounds of the invention displayed a much higher $IC_{50}$ than $IC_{50}$ that can induce cardiotoxicity (10 uM), suggesting that there is a low chance of cardiotoxicity when they are used as a drug.

Therefore, the compounds of the examples of the invention can be effectively used as a therapeutic agent for PPARG-related disease with significantly low chance of causing cardiotoxicity.

**<Experimental Example 5> Evaluation of blood glucose lowering effect and body weight reducing effect through DIO (diet-induced obesity) model**

To evaluate the blood glucose lowering effect and body weight reducing effect of the compounds prepared in the examples of the invention, the following experiment was performed.

The male C57BL/6 mice at 4 seeks were given with high fat diet (Lab. Diet Co.) to construct a high fat diet-induced obesity (DIO) mouse model.

Among those mice that gained more weight by high fat diet, those that were weighed more than 40 g were selected. Groups were divided with the randomly selected mice (n=8). Each group was treated separately with vehicle, the positive control drug, and the test compounds for 4 weeks at the different concentrations. Weight measurement was performed twice a week during the 4 week treatment period and the weight changes were recorded.

Then, oral glucose tolerance test (OGTT) was performed as follows.

30 minutes after the treatment of vehicle or the compounds of the example, the mice were orally administered with 2 g/kg of glucose. Blood glucose was measured by using Accu-chek active strip (Roche Diagnostic Co.). The time point for the measurement was -30, 0, 20, 40, 60, and 120 minutes from the glucose administration via tail vein puncture. The obtained AUC results and the weight changes are shown in Table 5.

[Table 5]

| | Comparative Example 1 | Example | | | |
|---|---|---|---|---|---|
| | SR-1664 [20mpk] | 56 [10mpk] | 155 [10mpk] | 156 [10mpk] | 157 [10mpk] |
| Weight change | 1.6% ↓ | 10.6% ↓ | 5.24% ↓ | 6.26% ↓ | 8.76% ↓ |
| Blood glucose (oGTT) | 14% ↓ | 37.5% ↓ | 25.0% ↓ | 34.2% ↓ | 24.6% ↓ |

As shown in Table 5, the comparative example compound SR-1664 (20 mpk) reduced the weight of the mouse by 1.6%, while the compounds of the examples of 56, 155, 156, and 157 (10 mpk each) reduced the mouse weight at least 5% even at a low dose.

In the meantime, the comparative example compound SR-1664 (20 mpk) reduced blood glucose by 14%, while the compounds of the examples 56, 155, 156, and 157 (10 mpk each) reduced blood glucose at least 24% even at a low dose.

Therefore, the compounds of the examples of the present invention had a significant effect of lowering body weight and blood glucose even at a low dose, so that they can be effectively used as a therapeutic agent for PPARG-related disease.

**<Manufacturing Example 1> Preparation of pharmaceutical formulations**

**1-1. Preparation of powders**

| | |
|---|---|
| The compound represented by formula 1 | 500 mg |
| Lactose | 100 mg |
| Talc | 10 mg |

Powders were prepared by mixing all the above components, which were filled in airtight packs according to the conventional method for preparing powders.

**1-2. Preparation of tablets**

| | |
|---|---|
| The compound represented by formula 1 | 500 mg |
| Corn starch | 100 mg |
| Lactose | 100 mg |
| Magnesium stearate | 2 mg |

Tablets were prepared by mixing all the above components by the conventional method for preparing tablets.

**1-3. Preparation of capsules**

| | |
|---|---|
| The compound represented by formula 1 | 500 mg |
| Corn starch | 100 mg |
| Lactose | 100 mg |
| Magnesium stearate | 2 mg |

Capsules were prepared by mixing all the above components, which were filled in gelatin capsules according to the conventional method for preparing capsules.

**1-4. Preparation of injectable solution**

| | |
|---|---|
| The compound represented by formula 1 | 500 mg |
| Sterilized distilled water | proper amount |
| pH regulator | proper amount |

Injectable solutions were prepared by mixing all the above components, putting the mixture into 2 Mℓ ampoules and sterilizing thereof by the conventional method for preparing injectable solutions.

**1-5. Preparation of liquid formulations**

| | |
|---|---|
| The compound represented by formula 1 | 100 mg |
| Isomerized sugar | 10 g |
| Mannitol | 5 g |
| Purified water | proper amount |

All the above components were dissolved in purified water. After adding lemon flavor, total volume was adjusted to be 100 **Mℓ** by adding purified water. Liquid formulations were prepared by putting the mixture into brown bottles and sterilizing thereof by the conventional method for preparing liquid formulations.

**INDUSTRIAL APPLICABILITY**

The compound represented by formula 1 or the optical isomer thereof of the present invention binds to PPARG at a high affinity but does not act as an agonist and thereby does not induce gene transcription. The compound also blocks CDK5, which is the cause of PPARG phosporylation, so that the compound can suppress side effects of the conventional antidiabetic agents and at the same time can improve pharmacophysical properties enough to bring PPARG-related disease treating effect. Therefore, the compound of the invention can be effectively used as a pharmaceutical composition for treating PPARG-related disease.

## Claims

1. A compound represented by formula 1 below, an optical isomer thereof, or a pharmaceutically acceptable salt thereof;

[Formula 1]

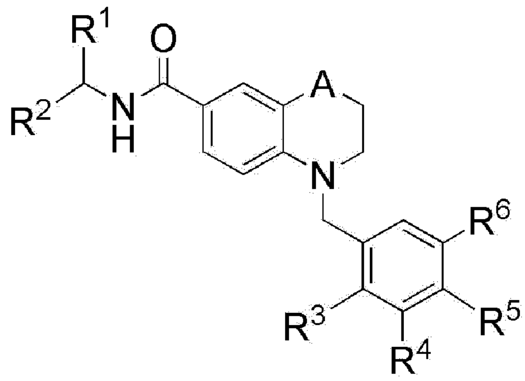

(In the formula 1,

$R^1$ is H, unsubstituted or substituted $C_{1-10}$ straight or branched alkyl wherein one or more halogens are substituted, or unsubstituted or substituted $C_{1-10}$ straight or branched alkoxy wherein one or more halogens are substituted;
$R^2$ is unsubstituted or substituted $C_{6-10}$ aryl, unsubstituted or substituted $C_{6-10}$ aryl $C_{1-10}$ straight or branched alkyl, or unsubstituted or substituted 5 ~ 10 membered heteroaryl containing one or more hetero atoms selected from the group consisting of N, O, and S.
In the said substituted $C_{6-10}$ aryl, the substituted $C_{6-10}$ aryl $C_{1-10}$ straight or branched alkyl, and the substituted 5 ~ 10 membered heteroaryl, one or more substituents selected from the group consisting of $C_{1-10}$ straight or branched alkyl unsubstituted or substituted with one or more halogens, $C_{1-10}$ straight or branched alkoxy unsubstituted or substituted with one or more halogens, $C_{1-10}$ straight or branched alkylsulfonyl unsubstituted or substituted with one or more halogens, $C_{1-10}$ straight or branched alkoxycarbonyl, halogen, nitrile, and nitro can be substituted,
In the said unsubstituted or substituted $C_{6-10}$ aryl, phenyl or 5 ~ 8 membered heterocycloalkyl containing one or more hetero atoms selected from the group consisting of N, O, and S can be included;
$R^1$ and $R^2$ can form $C_{5-10}$ cycloalkyl, 5 ~ 10 membered heterocycloalkyl containing one or more hetero atoms selected from the group consisting of N, O, and S, or 5 ~ 10 membered heteroaryl containing one or more hetero atoms selected from the group consisting of N, O, and S along with the carbon atoms which are conjugated to the same,
In the said $C_{5-10}$ cycloalkyl, the 5 ~ 10 membered heterocycloalkyl, or the 5 ~ 10 membered heteroaryl, phenyl can be fused;
$R^3$, $R^4$, $R^5$, and $R^6$ are independently H, halogen, unsubstituted or substituted $C_{1-10}$ straight or branched alkyl wherein one or more halogens are substituted, unsubstituted or substituted $C_{1-10}$ straight or branched alkoxy wherein one or more halogens are substituted, or unsubstituted or substituted $C_{6-10}$ aryl,
In the said substituted $C_{6-10}$ aryl, one or more substituents selected from the group consisting of halogen, unsubstituted or substituted $C_{1-10}$ straight or branched alkyl wherein one or more halogens are substituted, hydroxycarbonyl, aminocarbonyl, and sodiumoxycarbonyl can be substituted;

A is $-CH_2-$ or -O-).

2. The compound represented by formula 1, the optical isomer thereof, or the pharmaceutically acceptable salt thereof according to claim 1, wherein:

$R^1$ is H, unsubstituted or substituted $C_{1-5}$ straight or branched alkyl wherein one or more halogens are substituted, or unsubstituted or substituted $C_{1-5}$ straight or branched alkoxy wherein one or more halogens are substituted;
$R^2$ is unsubstituted or substituted $C_{6-8}$ aryl, unsubstituted or substituted $C_{6-8}$ aryl $C_{1-5}$ straight or branched alkyl,

or unsubstituted or substituted 5 ~ 8 membered heteroaryl containing one or more hetero atoms selected from the group consisting of N, O, and S,
In the said substituted $C_{6-8}$ aryl, the substituted $C_{6-8}$ aryl $C_{1-5}$ straight or branched alkyl, and the substituted 5 ~ 8 membered heteroaryl, one or more substituents selected from the group consisting of $C_{1-5}$ straight or branched alkyl unsubstituted or substituted with one or more halogens, $C_{1-5}$ straight or branched alkoxy unsubstituted or substituted with one or more halogens, $C_{1-5}$ straight or branched alkylsulfonyl unsubstituted or substituted with one or more halogens, $C_{1-5}$ straight or branched alkoxycarbonyl, halogen, nitrile, and nitro can be substituted,
In the said unsubstituted or substituted $C_{6-8}$ aryl, phenyl or 5 ~ 8 membered heterocycloalkyl containing one or more hetero atoms selected from the group consisting of N, O, and S can be fused;
$R^1$ and $R^2$ can form $C_{5-8}$ cycloalkyl, 5 ~ 8 membered heterocycloalkyl containing one or more hetero atoms selected from the group consisting of N, O, and S, or 5 ~ 8 membered heteroaryl containing one or more hetero atoms selected from the group consisting of N, O, and S along with the carbon atoms which are conjugated to the same,
In the said $C_{5-8}$ cycloalkyl, the 5 ~ 8 membered heterocycloalkyl, or the 5 ~ 8 membered heteroaryl, phenyl can be fused;
$R^3$, $R^4$, $R^5$, and $R^6$ are independently H, halogen, unsubstituted or substituted $C_{1-5}$ straight or branched alkyl wherein one or more halogens are substituted, unsubstituted or substituted $C_{1-5}$ straight or branched alkoxy wherein one or more halogens are substituted, or unsubstituted or substituted $C_{6-8}$ aryl,
In the said substituted $C_{6-8}$ aryl, one or more substituents selected from the group consisting of halogen, unsubstituted or substituted $C_{1-5}$ straight or branched alkyl wherein one or more halogens are substituted, hydroxycarbonyl, aminocarbonyl, and sodiumoxycarbonyl can be substituted;

A is $-CH_2-$ or -O-.

**3.** The compound represented by formula 1, the optical isomer thereof, or the pharmaceutically acceptable salt thereof according to claim 1, wherein:

$R^1$ is H, methyl, or ethyl;
$R^2$ is unsubstituted or substituted phenyl, unsubstituted or substituted benzyl, or unsubstituted or substituted 5 ~ 6 membered heteroaryl containing one or more hetero atoms selected from the group consisting of N and O,
In the said substituted phenyl, the substituted benzyl, and the substituted 5 ~ 6 membered heteroaryl, one or more substituents selected from the group consisting of $C_{1-5}$ straight or branched alkyl unsubstituted or substituted with one or more halogens, $C_{1-5}$ straight or branched alkoxy unsubstituted or substituted with one or more halogens, $C_{1-5}$ straight or branched alkylsulfonyl unsubstituted or substituted with one or more halogens, $C_{1-5}$ straight or branched alkoxycarbonyl, halogen, nitrile, and nitro can be substituted,
In the said unsubstituted or substituted phenyl, phenyl or 6 membered heterocycloalkyl containing one or more hetero atoms selected from the group consisting of N and O can be fused;
$R^1$ and $R^2$ can form $C_{5-8}$ cycloalkyl, 5 ~ 8 membered heterocycloalkyl containing one or more hetero atoms selected from the group consisting of N, O, and S, or 5 ~ 8 membered heteroaryl containing one or more hetero atoms selected from the group consisting of N, O, and S along with the carbon atoms which are conjugated to the same,
In the said $C_{5-8}$ cycloalkyl, the 5 ~ 8 membered heterocycloalkyl, or the 5 ~ 8 membered heteroaryl, phenyl can be fused;
$R^3$, $R^4$, $R^5$, and $R^6$ are independently H, fluoro, or unsubstituted or substituted phenyl,
In the said substituted phenyl, one or more substituents selected from the group consisting of hydroxycarbonyl, aminocarbonyl, and sodiumoxycarbonyl can be substituted;

A is $-CH_2-$ or -O-.

**4.** The compound represented by formula 1, the optical isomer thereof, or the pharmaceutically acceptable salt thereof according to claim 1, wherein:

$R^1$ is H, methyl, or ethyl;

$R^2$ is 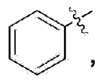, 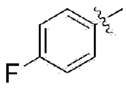, 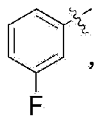, 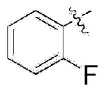, 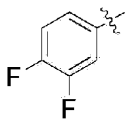, 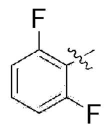, 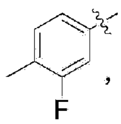,

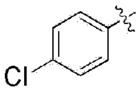, 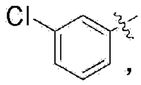, 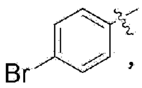, 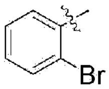, 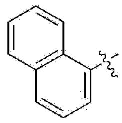, 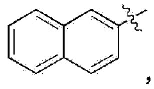,

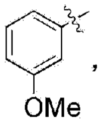, 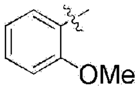, 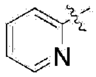, 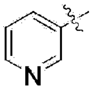, 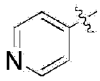, 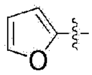,

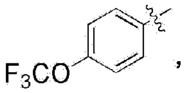, 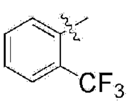, 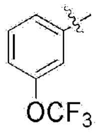, 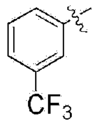, 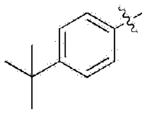, 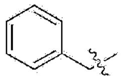,

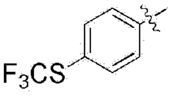, 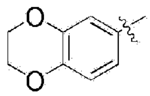, 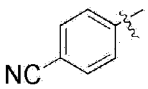, 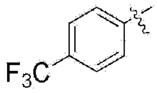, 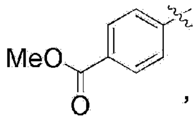,

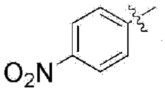 or 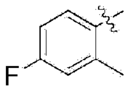;

$R^3$ is H, F,

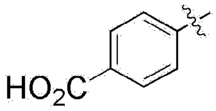

or

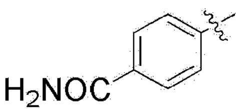;

$R^4$ is H, F,

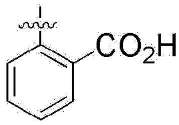

or

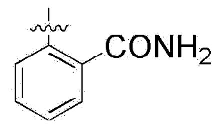

;

;

$R^5$ is H,

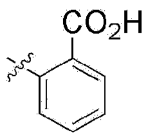

,

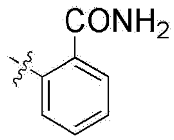

or

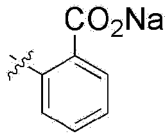

;

$R^6$ is H,

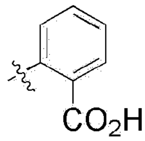

or

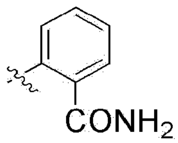

;

and

A is $-CH_2-$ or -O-.

**5.** The compound represented by formula 1, the optical isomer thereof, or the pharmaceutically acceptable salt thereof according to claim 1, wherein the compound represented by formula 1 is selected from the group consisting of the following compounds:

(1) (S)-4'-((6-((1-phenylethyl)carbamoyl)-3,4-dihydroquinoline-1(2H)-yl)methyl)-[1,1'-biphenyl]-2-carboxylic acid;

(2) (S)-1-((2'-carbamoyl-[1,1'-biphenyl]-4-yl)methyl)-N-(1-phenylethyl)-1,2,3,4-tetrahydroquinoline-6-carboxamide;

(3) (S)-4'-((6-((1-(4-nitrophenyl)ethyl)carbamoyl)-3,4-dihydroquinoline-1(2H)-yl)methyl)-[1,1'-biphenyl]-2-carboxylic acid;

(4) (S)-4'-((6-((1-(4-fluoro-2-methylphenyl)ethyl)carbamoyl)-3,4-dihydroquinoline-1(2H)-yl)methyl)-[1,1'-biphenyl]-2-carboxylic acid;

(5) (S)-4'-((6-((1-(3-fluoro-4-methylphenyl)ethyl)carbamoyl)-3,4-dihydroquinoline-1(2H)-yl)methyl)-[1,1'-biphenyl]-2-carboxylic acid;

(6) (S)-4'-((6-((1-(4-fluorophenyl)ethyl)carbamoyl)-3,4-dihydroquinoline-1(2H)-yl)methyl)-[1,1'-biphenyl]-2-carboxylic acid;

(7) (S)-4'-((6-((1-(4-bromophenyl)ethyl)carbamoyl)-3,4-dihydroquinoline-1(2H)-yl)methyl)-[1,1'-biphenyl]-2-carboxylic acid;
(8) 4'-((6-((4-nitrobenzyl)carbamoyl)-3,4-dihydroquinoline-1(2H)-yl)methyl)-[1,1'-biphenyl]-2-carboxylic acid;
(9) (S)-4'-((6-((1-(3-chlorophenyl)ethyl)carbamoyl)-3,4-dihydroquinoline-1(2H)-yl)methyl)-[1,1'-biphenyl]-2-carboxylic acid;
(10) (S)-4'-((6-((1-(naphthalene-1-yl)ethyl)carbamoyl)-3,4-dihydroquinoline-1(2H)-yl)methyl)-[1,1'-biphenyll-2-carboxylic acid;
(11) (S)-4'-((6-((1-(4-(trifluoromethoxy)phenyl)ethyl)carbamoyl)-3,4-dihydroquinoline-1(2H)-yl)methyl)-[1,1'-biphenyl]-2-carboxylic acid;
(12) (S)-4'-((6-((1-(4-(trifluoromethyl)phenyl)ethyl)carbamoyl)-3,4-dihydroquinoline-1(2H)-yl)methyl)-[1,1'-biphenyl]-2-carboxylic acid;
(13) (S)-4'-((6-((1-(4-((trifluoromethyl)thio)phenyl)ethyl)carbamoyl)-3,4-dihydroquinoline-1(2H)-yl)methyl)-[1,1'-biphenyl]-2-carboxylic acid;
(14) (S)-4'-((6-((1-(4-cyanophenyl)ethyl)carbamoyl)-3,4-dihydroquinoline-1(2H)-yl)methyl)-[1,1'-biphenyl]-2-carboxylic acid;
(15) 4'-((6-(cromene-3-ylcarbamoyl)-3,4-dihydroquinoline-1(2H)-yl)methyl)-[1,1'-biphenyl]-2-carboxylic acid;
(16) (S)-4'-((6-((1-(2,6-difluorophenyl)ethyl)carbamoyl)-3,4-dihydroquinoline-1(2H)-yl)methyl)-[1,1'-biphenyl]-2-carboxylic acid;
(17) (S)-4'-((6-((1-(3-fluorophenyl)ethyl)carbamoyl)-3,4-dihydroquinoline-1(2H)-yl)methyl)-[1,1'-biphenyl]-2-carboxylic acid;
(18) (S)-4'-((6-((1-(3-(trifluoromethoxy)phenyl)ethyl)carbamoyl)-3,4-dihydroquinoline-1(2H)-yl)methyl)-[1,1'-biphenyl]-2-carboxylic acid;
(19) (S)-4'-((6-((1-(3-(trifluoromethyl)phenyl)ethyl)carbamoyl)-3,4-dihydroquinoline-1(2H)-yl)methyl)-[1,1'-biphenyl]-2-carboxylic acid;
(20) (S)-4'-((6-((1-(naphthalene-2-yl)ethyl)carbamoyl)-3,4-dihydroquinoline-1(2H)-yl)methyl)-[1,1'-biphenyl]-2-carboxylic acid;
(21) (S)-4'-((6-((1-(3,4-difluorophenyl)ethyl)carbamoyl)-3,4-dihydroquinoline-1(2H)-yl)methyl)-[1,1'-biphenyl]-2-carboxylic acid;
(22) (S)-4'-((6-((1-(3-methoxyphenyl)ethyl)carbamoyl)-3,4-dihydroquinoline-1(2H)-yl)methyl)-[1,1'-biphenyl]-2-carboxylic acid;
(23) (S)-4'-((6-((1-(2-methoxyphenyl)ethyl)carbamoyl)-3,4-dihydroquinoline-1(2H)-yl)methyl)-[1,1'-biphenyl]-2-carboxylic acid;
(24) (S)-4'-((6-((1-(2-fluorophenyl)ethyl)carbamoyl)-3,4-dihydroquinoline-1(2H)-yl)methyl)-[1,1'-biphenyl]-2-carboxylic acid;
(25) (S)-4'-((6-((1-(2-(trifluoromethyl)phenyl)ethyl)carbamoyl)-3,4-dihydroquinoline-1(2H)-yl)methyl)-[1,1'-biphenyl]-2-carboxylic acid;
(26) 4'-((6-((pyridine-2-ylmethyl)carbamoyl)-3,4-dihydroquinoline-1(2H)-yl)methyl)-[1,1'-biphenyl]-2-carboxylic acid;
(27) 4'-((6-((pyridine-4-ylmethyl)carbamoyl)-3,4-dihydroquinoline-1(2H)-yl)methyl)-[1,1'-biphenyl]-2-carboxylic acid;
(28) 4'-((6-(benzylcarbamoyl)-3,4-dihydroquinoline-1(2H)-yl)methyl)-[1,1'-biphenyl]-2-carboxylic acid;
(29) 4'-((6-((2-bromobenzyl)carbamoyl)-3,4-dihydroquinoline-1(2H)-yl)methyl)-[1,1'-biphenyl]-2-carboxylic acid;
(30) 4'-((6-(((2,3-dihydrobenzo[b][1,4]dioxin-6-yl)methyl)carbamoyl)-3,4-dihydroquinoline-1(2H)-yl)methyl)-[1,1'-biphenyl]-2-carboxylic acid;
(31) 4'-((6-((4-bromobenzyl)carbamoyl)-3,4-dihydroquinoline-1(2H)-yl)methyl)-[1,1'-biphenyl]-2-carboxylic acid;
(32) 4'-((6-((3-(trifluoromethoxy)benzyl)carbamoyl)-3,4-dihydroquinoline-1(2H)-yl)methyl)-[1,1'-biphenyl]-2-carboxylic acid;
(33) (S)-1-((2'-carbamoyl-[1,1'-biphenyl]-4-yl)methyl)-N-(1-(4-(trifluoromethyl)phenyl)ethyl)-1,2,3,4-tetrahydroquinoline-6-carboxamide
(34) (S)-1-((2'-carbamoyl-[1,1'-biphenyl]-4-yl)methyl)-N-(1-(4-(trifluoromethoxy)phenyl)ethyl)-1,2,3,4-tetrahydroquinoline-6-carboxamide;
(35) (S)-1-((2'-carbamoyl-[1,1'-biphenyl]-4-yl)methyl)-N-(1-(3-chlorophenyl)ethyl)-1,2,3,4-tetrahydroquinoline-6-carboxamide;
(36) (S)-1-((2'-carbamoyl-[1,1'-biphenyl]-4-yl)methyl)-N-(1-(4-fluorophenyl)ethyl)-1,2,3,4-tetrahydroquinoline-6-carboxamide;
(37) (S)-N-(1-(4-bromophenyl)ethyl)-1-((2'-carbamoyl-[1,1'-biphenyl]-4-yl)methyl)-1,2,3,4-tetrahydroquinoline-6-carboxamide;
(38) (S)-1-((2'-carbamoyl-[1,1'-biphenyl]-4-yl)methyl)-N-(1-(naphthalene-1-yl)ethyl)-1,2,3,4-tetrahydroquinoline-6-carboxamide;
(39) (S)-1-((2'-carbamoyl-[1,1'-biphenyl]-4-yl)methyl)-N-(1-(3,4-difluorophenyl)ethyl)-1,2,3,4-tetrahydroquinoline-6-carboxamide;
(40) (S)-1-((2'-carbamoyl-[1,1'-biphenyl]-4-yl)methyl)-N-(1-(3-methoxyphenyl)ethyl)-1,2,3,4-tetrahydroquinoline-6-carboxamide;
(41) (S)-1-((2'-carbamoyl-[1,1'-biphenyl]-4-yl)methyl)-N-(1-(2-methoxyphenyl)ethyl)-1,2,3,4-tetrahydroquinoline-6-carboxamide;
(42) (S)-1-((2'-carbamoyl-[1,1'-biphenyl]-4-yl)methyl)-N-(1-(2-fluorophenyl)ethyl)-1,2,3,4-tetrahydroquinoline-6-carboxamide;
(43) (S)-1-((2'-carbamoyl-[1,1'-biphenyl]-4-yl)methyl)-N-(1-(2-(trifluoromethyl)phenyl)ethyl)-1,2,3,4-tetrahydroquinoline-6-carboxamide;
(44) (S)-1-((2'-carbamoyl-[1,1'-biphenyl]-4-yl)methyl)-N-(1-(naphthalene-2-yl)ethyl)-1,2,3,4-tetrahydroquinoline-6-carboxamide;
(45) (S)-1-((2'-carbamoyl-[1,1'-biphenyl]-4-yl)methyl)-N-(1-(3-fluorophenyl)ethyl)-1,2,3,4-tetrahydroquinoline-6-carboxamide;
(46) (S)-1-((2'-carbamoyl-[1,1'-biphenyl]-4-yl)methyl)-N-(1-(3-(trifluoromethoxy)phenyl)ethyl)-1,2,3,4-tetrahydroquinoline-6-carboxamide;
(47) (S)-1-((2'-carbamoyl-[1,1'-biphenyl]-4-yl)methyl)-N-(1-(3-(trifluoromethyl)phenyl)ethyl)-1,2,3,4-tetrahydroquinoline-6-carboxamide;
(48) 1-((2'-carbamoyl-[1,1'-biphenyl]-4-yl)methyl)-N-(pyridine-4-ylmethyl)-1,2,3,4-tetrahydroquinoline-6-carboxamide;
(49) (S)-1-((2'-carbamoyl-[1,1'-biphenyl]-4-yl)methyl)-N-(1-(3-fluoro-4-methylphenyl)ethyl)-1,2,3,4-tetrahydroquinoline-6-carboxamide;
(50) N-benzyl-1-((2'-carbamoyl-[1,1'-biphenyl]-4-yl)methyl)-1,2,3,4-tetrahydroquinoline-6-carboxamide;
(51) N-(2-bromobenzyl)-1-((2'-carbamoyl-[1,1'-biphenyl]-4-yl)methyl)-1,2,3,4-tetrahydroquinoline-6-carboxamide;
(52) 1-((2'-carbamoyl-[1,1'-biphenyl]-4-yl)methyl)-N-((2,3-dihydrobenzo[b][1,4]dioxin-6-yl)methyl)-1,2,3,4-tetrahydroquinoline-6-carboxamide;
(53) N-(4-bromobenzyl)-1-((2'-carbamoyl-[1,1'-biphenyl]-4-yl)methyl)-1,2,3,4-tetrahydroquinoline-6-carboxamide;
(54) 1-((2'-carbamoyl-[1,1'-biphenyl]-4-yl)methyl)-N-(3-(trifluoromethoxy)benzyl)-1,2,3,4-tetrahydroquinoline-6-carboxamide;
(55) sodium (S)-4'-((6-((1-phenylethyl)carbamoyl)-3,4-dihydroquinoline-1(2H)-yl)methyl)-[1,1'-biphenyl]-2-carboxylate;
(56) sodium (S)-4'-((6-((1-(3-fluoro-4-methylphenyl)ethyl)carbamoyl)-3,4-dihydroquinoline-1(2H)-yl)methyl)-[1,1'-biphenyl]-2-carboxylate;
(57) sodium (S)-4'-((6-((1-(4-cyanophenyl)ethyl)carbamoyl)-3,4-dihydroquinoline-1(2H)-yl)methyl)-[1,1'-biphenyl]-2-carboxylate;
(58) (S)-4'-((7-((1-phenylethyl)carbamoyl)-2H-benzo[b][1,4]oxazine-4(3H)-yl)methyl)-[1,1'-biphenyl]-2-carboxylic acid;
(59) (S)-4-((2'-carbamoyl-[1,1'-biphenyl]-4-yl)methyl)-N-(1-phenylethyl)-3,4-dihydro-2H-benzo[b][1,4]oxazine-7-carboxamide;
(60) (S)-4'-((7-((1-(4-nitrophenyl)ethyl)carbamoyl)-2H-benzo[b][1,4]oxazine-4(3H)-yl)methyl)-[1,1'-biphenyl]-2-carboxylic acid;
(61) (S)-4'-((7-((1-(4-bromophenyl)ethyl)carbamoyl)-2H-benzo[b][1,4]oxazine-4(3H)-yl)methyl)-[1,1'-biphenyl]-2-carboxylic acid;
(62) (S)-4'-((7-((1-phenylpropyl)carbamoyl)-2H-benzo[b][1,4]oxazine-4(3H)-yl)methyl)-[1,1'-biphenyl]-2- carboxylic acid;
(63) (S)-4'-((7-((1-(4-fluorophenyl)ethyl)carbamoyl)-2H-benzo[b][1,4]oxazine-4(3H)-yl)methyl)-[1,1'-biphenyl]-2-carboxylic acid;
(64) (S)-4'-((7-((1-(4-chlorophenyl)ethyl)carbamoyl)-2H-benzo[b][1,4]oxazine-4(3H)-yl)methyl)-[1,1'-biphenyl]-2-carboxylic acid;
(65) (S)-4'-((7-((1-(3-chlorophenyl)ethyl)carbamoyl)-2H-benzo[b][1,4]oxazine-4(3H)-yl)methyl)-[1,1'-biphenyl]-2-carboxylic acid;
(66) 4'-((7-((4-nitrobenzyl)carbamoyl)-2H-benzo[b][1,4]oxazine-4(3H)-yl)methyl)-[1,1'-biphenyl]-2-carboxylic acid;

(67) 4'-((7-(benzylcarbamoyl)-2H-benzo[b][1,4]oxazine-4(3H)-yl)methyl)-[1,1'-biphenyl]-2-carboxylic acid;
(68) 4'-((7-(phenethylcarbamoyl)-2H-benzo[b][1,4]oxazine-4(3H)-yl)methyl)-[1,1'-biphenyl]-2-carboxylic acid;
(69) 4'-((7-((furan-2-ylmethyl)carbamoyl)-2H-benzo[b][1,4oxazine-4(3H)-yl)methyl)-[1,1'-biphenyl]-2-carboxylic acid;
(70) (S)-4'-((7-((1-(naphthalene-1-yl)ethyl)carbamoyl)-2H-benzo[b][1,4]oxazine-4(3H)-yl)methyl)-[1,1'-biphenyl]-2-carboxylic acid;
(71) 4'-((7-((4-(methoxycarbonyl)benzyl)carbamoyl)-2H-benzo[b][1,4]oxazine-4(3H)-yl)methyl)-[1,1'-biphenyl]-2-carboxylic acid;
(72) 4'-((7-((3-methoxybenzyl)carbamoyl)-2H-benzo[b][1,4]oxazine-4(3H)-yl)methyl)-[1,1'-biphenyl]-2-carboxylic acid;
(73) 4'-((7-((4-bromobenzyl)carbamoyl)-2H-benzo[b][1, 4]oxazine-4(3H)-yl)methyl)-[1,1'-biphenyl]-2-carboxylic acid;
(74) (S)-4'-((7-((1-(4-cyanophenyl)ethyl)carbamoyl)-2H-benzo[b][1,4]oxazine-4(3H)-yl)methyl)-[1,1'-biphenyl]-2-carboxylic acid;
(75) (S)-4'-((7-((1-(4-(trifluoromethoxy)phenyl)ethyl)carbamoyl)-2H-benzo[b][1,4]oxazine-4(3H)-yl)methyl)-[1,1'-biphenyl]-2-carboxylic acid;
(76) (S)-4'-((7-((1-(4-(trifluoromethyl)phenyl)ethyl)carbamoyl)-2H-benzo [b][1,4]oxazine-4(3H)-yl)methyl)-[1,1'-biphenyl]-2-carboxylic acid;
(77) (S)-4'-((7-((1-(4-((trifluoromethyl)thio)phenyl)ethyl)carbamoyl)-2H-benzo[b][1,4oxazine-4(3H)-yl)methyl)-[1,1'-biphenyl]-2-carboxylic acid ;
(78) (S)-4'-((7-((1-(4-(tert-butyl)phenyl)ethyl)carbamoyl)-2H-benzo[b][1,4]oxazine-4(3H)-yl)methyl)-[1,1'-biphenyl]-2-carboxylic acid;
(79) (S)-4'-((7-((1-(4-fluoro-2-methylphenyl)ethyl)carbamoyl)-2H-benzo[b][1,4]oxazine-4(3H)-yl)methyl)-[1,1'-biphenyl]-2-carboxylic acid;
(80) (S)-4'-((7-((1-(3-fluoro-4-methylphenyl)ethyl)carbamoyl)-2H-benzo[b][1,4]oxazine-4(3H)-yl)methyl)-[1,1'-biphenyl]-2-carboxylic acid;
(81) (R)-4'-((7-((1-(4-cyanophenyl)ethyl)carbamoyl)-2H-benzo[b][1,4]oxazine-4(3H)-yl)methyl)-[1,1'-biphenyl]-2-carboxylic acid;
(82) 4'-((7-(cromene-3-ylcarbamoyl)-2H-benzo[b][1,4]oxazine-4(3H)-yl)methyl)-[1,1'-biphenyl]-2-carboxylic acid;
(83) (S)-4'-((7-((1-(2,6-difluorophenyl)ethyl)carbamoyl)-2H-benzo[b][1,4]oxazine-4(3H)-yl)methyl)-[1,1'-biphenyl]-2-carboxylic acid;
(84) (S)-4'-((7-((1-(3-fluorophenyl)ethyl)carbamoyl)-2H-benzo[b][1,4]oxazine-4(3H)-yl)methyl)-[1,1'-biphenyl]-2-carboxylic acid;
(85) (S)-4'-((7-((1-(3-(trifluoromethoxy)phenyl)ethyl)carbamoyl)-2H-benzo[b][1,4]oxazine-4(3H)-yl)methyl)-[1,1'-biphenyl]-2-carboxylic acid;
(86) (S)-4'-((7-((1-(3-(trifluoromethyl)phenyl)ethyl)carbamoyl)-2H-benzo[b][1,4]oxazine-4(3H)-yl)methyl)-[1,1'-biphenyl]-2-carboxylic acid;
(87) (S)-4'-((7-((1-(naphthalene-2-yl)ethyl)carbamoyl)-2H-benzo[b][1,4]oxazine-4(3H)-yl)methyl)-[1,1'-biphenyl]-2-carboxylic acid;
(88) 4'-((7-((pyridine-2-ylmethyl)carbamoyl)-2H-benzo[b][1,4]oxazine-4(3H)-yl)methyl)-[1,1'-biphenyl]-2-carboxylic acid;
(89) 4'-((7-((pyridine-3-ylmethyl)carbamoyl)-2H-benzo[b][1,4]oxazine-4(3H)-yl)methyl)-[1,1'-biphenyl]-2-carboxylic acid;
(90) 4'-((7-((pyridine-4-ylmethyl)carbamoyl)-2H-benzo[b][1,4]oxazine-4(3H)-yl)methyl)-[1,1'-biphenyl]-2-carboxylic acid;
(91) 4'-((7-(benzylcarbamoyl)-2H-benzo[b][1,4]oxazine-4(3H)-yl)methyl)-[1,1'-biphenyl]-2-carboxylic acid;
(92) 4'-((7-((2-bromobenzyl)carbamoyl)-2H-benzo[b][1,4]oxazine-4(3H)-yl)methyl)-[1,1'-biphenyl]-2-carboxylic acid;
(93) 4'-((7-(((2,3-dihydrobenzo[b][1,4]dioxin-6-yl)methyl)carbamoyl)-2H-benzo[b][1,4]oxazine-4(3H)-yl)methyl)-[1,1'-biphenyl]-2-carboxylic acid;
(94) (R)-4-((2'-carbamoyl-[1,1'-biphenyl]-4-yl)methyl)-N-(1-(4-cyanophenyl)ethyl)-3,4-dihydro-2H-benzo[b][1,4]oxazine-7-carboxamide;
(95) (S)-4-((2'-carbamoyl-[1,1'-biphenyl]-4-yl)methyl)-N-(1-(3-fluorophenyl)ethyl)-3,4-dihydro-2H-benzo[b][1,4]oxazine-7-carboxamide;
(96) (S)-4-((2'-carbamoyl-[1,1'-biphenyl]-4-yl)methyl)-N-(1-(naphthalene-2-yl)ethyl)-3,4-dihydro-2H-benzo[b][1,4]oxazine-7-carboxamide;
(97) 4-((2'-carbamoyl-[1,1'-biphenyl]-4-yl)methyl)-N-(pyridine-3-ylmethyl)-3,4-dihydro-2H-benzo[b][1,4]oxazine-7-carboxamide;

(98) 4-((2'-carbamoyl-[1,1'-biphenyl]-4-yl)methyl)-N-(pyridine-4-ylmethyl)-3,4-dihydro-2H-benzo[b][1,4]oxazine-7-carboxamide;

(99) (S)-4-((2'-carbamoyl-[1,1'-biphenyl]-4-yl)methyl)-N-(1-(4-((trifluoromethyl)thio)phenyl)ethyl)-3,4-dihydro-2H-benzo[b][1,4]oxazine-7-carboxamide;

(100) N-benzyl-4-((2'-carbamoyl-[1,1'-biphenyl]-4-yl)methyl)-3,4-dihydro-2H-benzo[b][1,4]oxazine-7-carboxamide;

(101) N-(2-bromobenzyl)-4-((2'-carbamoyl-[1,1'-biphenyl]-4-yl)methyl)-3,4-dihydro-2H-benzo[b][1,4]oxazine-7-carboxamide;

(102) 4-((2'-carbamoyl-[1,1'-biphenyl]-4-yl)methyl)-N-((2,3-dihydrobenzo[b][1,4]dioxin-6-yl)methyl)-3,4-dihydro-2H-benzo[b][1,4]oxazine-7-carboxamide;

(103) sodium (S)-4'-((7-((1-phenylethyl)carbamoyl)-2H-benzo[b][1,4]oxazine-4(3H)-yl)methyl)-[1,1'-biphenyl]-2-carboxylate;

(104) sodium (S)-4'-((7-((1-(4-cyanophenyl)ethyl)carbamoyl)-2H-benzo[b][1,4]oxazine-4(3H)-yl)methyl)-[1,1'-biphenyl]-2-carboxylate;

(105) sodium (S)-4'-((7-((1-(4-nitrophenyl)ethyl)carbamoyl)-2H-benzo[b][1,4]oxazine-4(3H)-yl)methyl)-[1,1'-biphenyl]-2-carboxylate;

(106) (S)-1-((2'-carbamoyl-3-fluoro-[1,1'-biphenyl]-4-yl)methyl)-N-(1-phenylethyl)-1,2,3,4-tetrahydroquinoline-6-carboxylic acid;

(107) (S)-1-((2'-carbamoyl-3-fluoro-[1,1'-biphenyl]-4-yl)methyl)-N-(1-phenylethyl)-1,2,3,4-tetrahydroquinoline-6-carboxamide;

(108) (S)-3'-fluoro-4'-((6-((1-(naphthalene-2-yl)ethyl)carbamoyl)-3,4-dihydro quinoline-1(2H)-yl)methyl)-[1,1'-biphenyll-2-carboxylic acid;

(109) (S)-3'-fluoro-4'-((6-((1-(4-nitrophenyl)ethyl)carbamoyl)-3,4-dihydroquinoline-1(2H)-yl)methyl)-[1,1'-biphenyl]-2-carboxylic acid;

(110) (S)-4'-((6-((1-(4-cyanophenyl)ethyl)carbamoyl)-3,4-dihydroquinoline-1(2H)-yl)methyl)-3'-fluoro-[1,1'-biphenyl]-2-carboxylic acid;

(111) (S)-3'-fluoro-4'-((6-((1-(3-fluoro-4-methylphenyl)ethyl)carbamoyl)-3,4-dihydroquinoline-1(2H)-yl)methyl)-[1,1'-biphenyl]-2-carboxylic acid;

(112) (S)-1-((2'-carbamoyl-3-fluoro-[1,1'-biphenyl]-4-yl)methyl)-N-(1-(4-cyanophenyl)ethyl)-1,2,3,4-tetrahydroquinoline-6-carboxamide;

(113) (S)-1-((2'-carbamoyl-3-fluoro-[1,1'-biphenyl]-4-yl)methyl)-N-(1-(4-nitrophenyl)ethyl)-1,2,3,4-tetrahydroquinoline-6-carboxamide;

(114) (S)-1-((2'-carbamoyl-3-fluoro-[1,1'-biphenyl]-4-yl)methyl)-N-(1-(3-fluoro-4-methylphenyl)ethyl)-1,2,3,4-tetrahydroquinoline-6-carboxamide;

(115) (S)-1-((2'-carbamoyl-3-fluoro-[1,1'-biphenyl]-4-yl)methyl)-N-(1-(naphthalene-2-yl)ethyl)-1,2,3,4-tetrahydroquinoline-6-carboxamide;

(116) (S)-3'-((6-((1-phenylethyl)carbamoyl)-3,4-dihydroquinoline-1(2H)-yl)methyl)-[1,1'-biphenyl]-2-carboxylic acid;

(117) (S)-1-((2'-carbamoyl-[1,1'-biphenyl]-3-yl)methyl)-N-(1-phenylethyl)-1,2,3,4-tetrahydroquinoline-6-carboxamide;

(118) (S)-3'-((6-((1-(4-cyanophenyl)ethyl)carbamoyl)-3,4-dihydroquinoline-1(2H)-yl)methyl)-[1,1'-biphenyl]-2-carboxylic acid;

(119) (S)-3'-((6-((1-(4-nitrophenyl)ethyl)carbamoyl)-3,4-dihydroquinoline-1(2H)-yl)methyl)-[1,1'-biphenyl]-2-carboxylic acid;

(120) (S)-3'-((6-((1-(naphthalene-2-yl)ethyl)carbamoyl)-3,4-dihydroquinoline-1(2H)-yl)methyl)-[1,1'-biphenyl]-2-carboxylic acid;

(121) (S)-3'-((6-((1-(3-fluoro-4-methylphenyl)ethyl)carbamoyl)-3,4-dihydroquinoline-1(2H)-yl)methyl)-[1,1'-biphenyl]-2-carboxylic acid;

(122) (S)-1-((2'-carbamoyl-[1,1'-biphenyl]-3-yl)methyl)-N-(1-(4-cyanophenyl)ethyl)-1,2,3,4-tetrahydroquinoline-6-carboxamide;

(123) (S)-1-((2'-carbamoyl-[1,1'-biphenyl]-3-yl)methyl)-N-(1-(4-nitrophenyl)ethyl)-1,2,3,4-tetrahydroquinoline-6-carboxamide;

(124) (S)-1-((2'-carbamoyl-[1,1'-biphenyl]-3-yl)methyl)-N-(1-(naphthalene-2-yl)ethyl)-1,2,3,4-tetrahydroquinoline-6-carboxamide;

(125) (S)-1-((2'-carbamoyl-[1,1'-biphenyl]-3-yl)methyl)-N-(1-(3-fluoro-4-methylphenyl)ethyl)-1,2,3,4-tetrahydroquinoline-6-carboxamide;

(126) (S)-2'-fluoro-3'-((6-((1-phenylethyl)carbamoyl)-3,4-dihydroquinoline- 1(2H)-yl)methyl)-[1,1'-biphenyl]-2-carboxylic acid;
(127) (S)-1-((2'-carbamoyl-2-fluoro-[1,1'-biphenyl]-3-yl)methyl)-N-(1-phenylethyl)-1,2,3,4-tetrahydroquinoline-6-carboxamide compound;
(128) (S)-2'-fluoro-3'-((6-((1-(4-nitrophenyl)ethyl)carbamoyl)-3,4-dihydroquinoline-1(2H)-yl)methyl)-[1,1'-biphenyl]-2-carboxylic acid;
(129) (S)-2'-fluoro-3'-((6-((1-(naphthalene-2-yl)ethyl)carbamoyl)-3,4-dihydroquinoline-1(2H)-yl)methyl)-[1,1'-biphenyl]-2-carboxylic acid;
(130) (S)-2'-fluoro-3'-((6-((1-(3-fluoro-4-methylphenyl)ethyl)carbamoyl)-3,4-dihydroquinoline-1(2H)-yl)methyl)-[1,1'-biphenyl]-2-carboxylic acid ;
(131) (S)-1-((2'-carbamoyl-2-fluoro-[1,1'-biphenyl]-3-yl)methyl)-N-(1-(4-nitrophenyl)ethyl)-1,2,3,4-tetrahydroquinoline-6-carboxamide;
(132) (S)-1-((2'-carbamoyl-2-fluoro-[1,1'-biphenyl]-3-yl)methyl)-N-(1-(naphthalene-2-yl)ethyl)-1,2,3,4-tetrahydroquinoline-6-carboxamide;
(133) (S)-1-((2'-carbamoyl-2-fluoro-[1,1'-biphenyl]-3-yl)methyl)-N-(1-(3-fluoro-4-methylphenyl)ethyl)-1,2,3,4-tetrahydroquinoline-6-carboxamide;
(134) (S)-4'-fluoro-3'-((6-((1-phenylethyl)carbamoyl)-3,4-dihydroquinoline-1(2H)-yl)methyl)-[1,1'-biphenyl]-2-carboxylic acid;
(135) (S)-1-((2'-carbamoyl-4-fluoro-[1,1'-biphenyl]-3-yl)methyl)-N-(1-phenylethyl)-1,2,3,4-tetrahydroquinoline-6-carboxamide;
(136) (S)-4'-fluoro-3'-((6-((1-(4-nitrophenyl)ethyl)carbamoyl)-3,4-dihydroquinoline-1(2H)-yl)methyl)-[1,1'-biphenyl]-2-carboxylic acid;
(137) (S)-4'-fluoro-3'-((6-((1-(naphthalene-2-yl)ethyl)carbamoyl)-3,4-dihydroquinoline-1(2H)-yl)methyl)-[1,1'-biphenyll-2-carboxylic acid;
(138) (S)-4'-fluoro-3'-((6-((1-(3-fluoro-4-methylphenyl)ethyl)carbamoyl)-3,4-dihydroquinoline-1(2H)-yl)methyl)-[1,1'-biphenyl]-2-carboxylic acid;
(139) (S)-1-((2'-carbamoyl-4-fluoro-[1,1'-biphenyl]-3-yl)methyl)-N-(1-(4-nitrophenyl)ethyl)-1,2,3,4-tetrahydroquinoline-6-carboxamide;
(140) (S)-1-((2'-carbamoyl-4-fluoro-[1,1'-biphenyl]-3-yl)methyl)-N-(1-(naphthalene-2-yl)ethyl)-1,2,3,4-tetrahydroquinoline-6-carboxamide;
(141) (S)-1-((2'-carbamoyl-4-fluoro-[1,1'-biphenyl]-3-yl)methyl)-N-(1-(3-fluoro-4-methylphenyl)ethyl)-1,2,3,4-tetrahydroquinoline-6-carboxamide;
(142) (S)-2'-((7-((1-phenylethyl)carbamoyl)-2H-benzo[b][1,4]oxazine-4(3H)-yl)methyl)-[1,1'-biphenyl]-4-carboxylic acid;
(143) (S)-4-((4'-carbamoyl-[1,1'-biphenyl]-2-yl)methyl)-N-(1-phenylethyl)-3,4-dihydro-2H-benzo[b][1,4]oxazine-7-carboxamide;
(144) (S)-2'-((7-((1-(4-cyanophenyl)ethyl)carbamoyl)-2H-benzo[b][1,4]oxazine-4(3H)-yl)methyl)-[1,1'-biphenyl]-4-carboxylic acid;
(145) (S)-2'-((7-((1-(3-fluoro-4-methylphenyl)ethyl)carbamoyl)-2H-benzo[b][1,4]oxazine-4(3H)-yl)methyl)-[1,1'-biphenyl]-4-carboxylic acid;
(146) (S)-2'-((7-((1-(4-nitrophenyl)ethyl)carbamoyl)-2H-benzo[b][1,4]oxazine-4(3H)-yl)methyl)-[1,1'-biphenyl]-4-carboxylic acid;
(147) (S)-2'-((7-((1-(naphthalene-2-yl)ethyl)carbamoyl)-2H-benzo[b][1,4]oxazine-4(3H)-yl)methyl)-[1,1'-biphenyl]-4-carboxylic acid;
(148) (S)-4-((4'-carbamoyl-[1,1'-biphenyl]-2-yl)methyl)-N-(1-(3-fluoro-4-methylphenyl)ethyl)-3,4-dihydro-2H-benzo[b][1,4]oxazine-7-carboxamide;
(149) (S)-4-((4'-carbamoyl-[1,1'-biphenyl]-2-yl)methyl)-N-(1-(4-cyanophenyl)ethyl)-3,4-dihydro-2H-benzo[b][1,4]oxazine-7-carboxamide;
(150) (S)-4-((4'-carbamoyl-[1,1'-biphenyl]-2-yl)methyl)-N-(1-(4-nitrophenyl)ethyl)-3,4-dihydro-2H-benzo[b][1,4]oxazine-7-carboxamide;
(151) (S)-4-((4'-carbamoyl-[1,1'-biphenyl]-2-yl)methyl)-N-(1-(naphthalene-2-yl)ethyl)-3,4-dihydro-2H-benzo[b][1,4]oxazine-7-carboxamide;
(152) (S)-2'-((6-((1-phenylethyl)carbamoyl)-3,4-dihydroquinoline-1(2H)-yl)methyl)-[1,1'-biphenyl]-4-carboxylic acid;
(153) (S)-1-((4'-carbamoyl-[1,1'-biphenyl]-2-yl)methyl)-N-(1-phenylethyl)-1,2,3,4-tetrahydroquinoline-6-carboxamide;
(154) (S)-2'-((6-((1-(3-fluoro-4-methylphenyl)ethyl)carbamoyl)-3,4-dihydroquinoline-1(2H)-yl)methyl)-[1,1'-biphenyl]-4-carboxylic acid;
(155) (S)-2'-((6-((1-(4-nitrophenyl)ethyl)carbamoyl)-3,4-dihydroquinoline-1(2H)-yl)methyl)-[1,1'-biphenyl]-4-carboxylic acid;
(156) (S)-2'-((6-((1-(naphthalene-2-yl)ethyl)carbamoyl)-3,4-dihydroquinoline-1(2H)-yl)methyl)-[1,1'-biphenyl]-4-carboxylic acid;
(157) (S)-2'-((6-((1-(4-cyanophenyl)ethyl)carbamoyl)-3,4-dihydroquinoline-1(2H)-yl)methyl)-[1,1'-biphenyl]-4-carboxylic acid;
(158) (S)-1-((4'-carbamoyl-[1,1'-biphenyl]-2-yl)methyl)-N-(1-(3-fluoro-4-methylphenyl)ethyl)-1,2,3,4-tetrahydroquinoline-6-carboxamide;
(159) (S)-1-((4'-carbamoyl-[1,1'-biphenyl]-2-yl)methyl)-N-(1-(4-nitrophenyl)ethyl)-1,2,3,4-tetrahydroquinoline-6-carboxamide;
(160) (S)-1-((4'-carbamoyl-[1,1'-biphenyl]-2-yl)methyl)-N-(1-(naphthalene-2-yl)ethyl)-1,2,3,4-tetrahydroquinoline-6-carboxamide;
(161) (S)-1-((4'-carbamoyl-[1,1'-biphenyl]-2-yl)methyl)-N-(1-(4-cyanophenyl)ethyl)-1,2,3,4-tetrahydroquinoline-6-carboxamide;
(162) (S)-2'-fluoro-4'-((7-((1-phenylethyl)carbamoyl)-2H-benzo[b][1,4]oxazine-4(3H)-yl)methyl)-[1,1'-biphenyl]-2-carboxylic acid;
(163) (S)-2'-fluoro-4'-((7-((1-(naphthalene-2-yl)ethyl)carbamoyl)-2H-benzo[b][1,4]oxazine-4(3H)-yl)methyl)-[1,1'-biphenyll-2-carboxylic acid; and
(164) (S)-2'-fluoro-4'-((7-(((3-fluoro-4-methylphenyl)ethyl)carbamoyl)-2H-benzo[b][1,4]oxazine-4(3H)-yl)methyl)-[1,1'-biphenyl]-2-carboxylic acid.

**6.** A method for preparing the compound represented by formula 1 of claim 1 comprising the following steps, as shown in reaction formula 1 below:

preparing the compound represented by formula 4 by reacting the compound represented by formula 2 with the compound represented by formula 3 (step 1);
preparing the compound represented by formula 5 by substituting methoxycarbonyl group of the compound represented by formula 4 prepared in step 1 with carboxy group (step 2);
preparing the compound represented by formula 7 by reacting the compound represented by formula 5 prepared in step 2 with the compound represented by formula 6 (step 3); and
substituting t-butoxy group of the compound represented by formula 7 prepared in step 3 with -OH group (step 4).

[Reaction Formula 1]

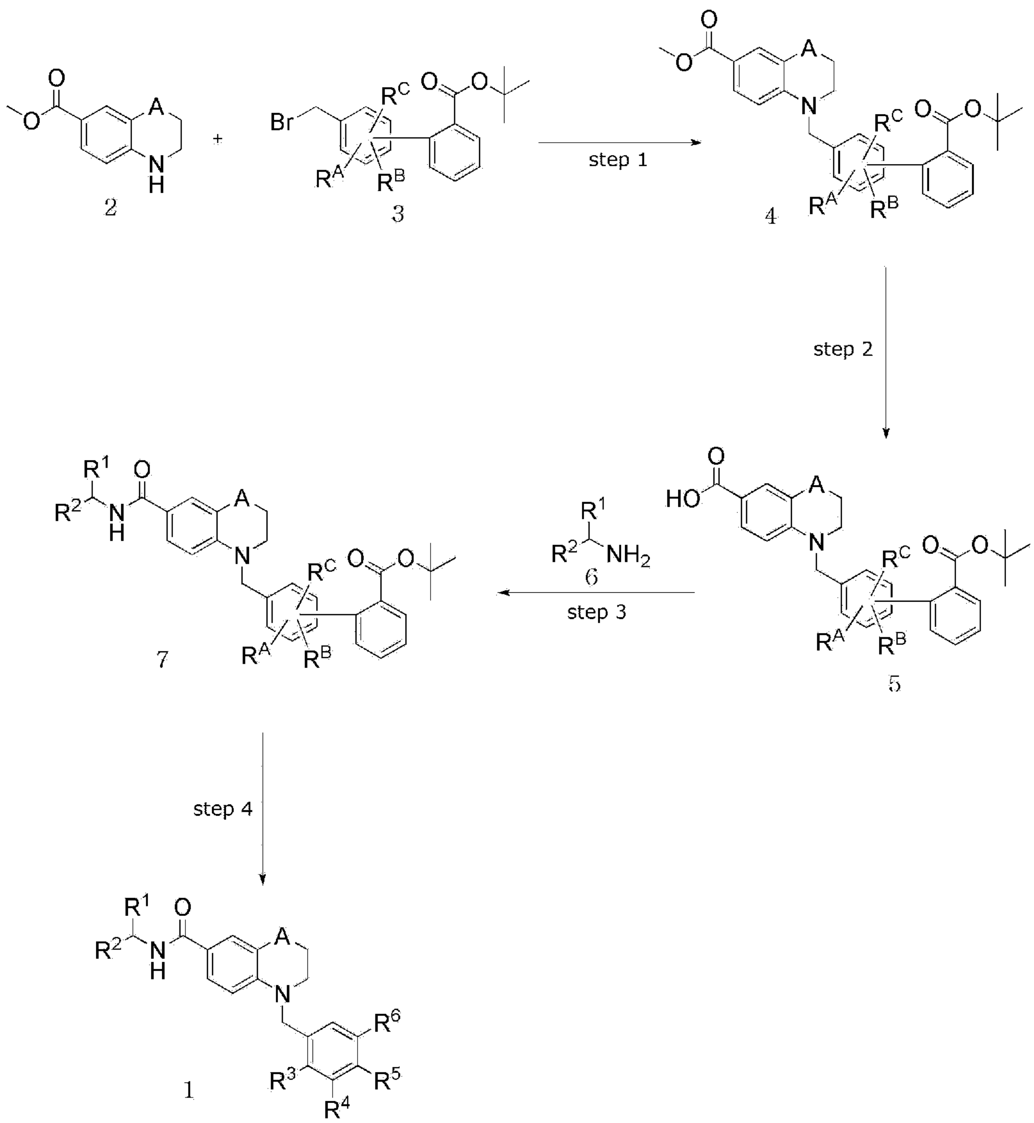


(In the reaction formula 1,

$R^1 \sim R^6$ and A are as defined in formula 1, and
$R^A$, $R^B$, and $R^C$ are independently as defined in $R^3 \sim R^6$).

**7.** A method for preparing the compound represented by formula 1 of claim 1 comprising the following steps, as shown in reaction formula 2 below:

preparing the compound represented by formula 9 by reacting the compound represented by formula 8 with the compound represented by formula 6 (step 1);
preparing the compound represented by formula 7 by reacting the compound represented by formula 9 prepared in step 1 with the compound represented by formula 3 (step 2); and
substituting t-butoxy group of the compound represented by formula 7 prepared in step 2 with -OH group (step 3).

[Reaction Formula 2]

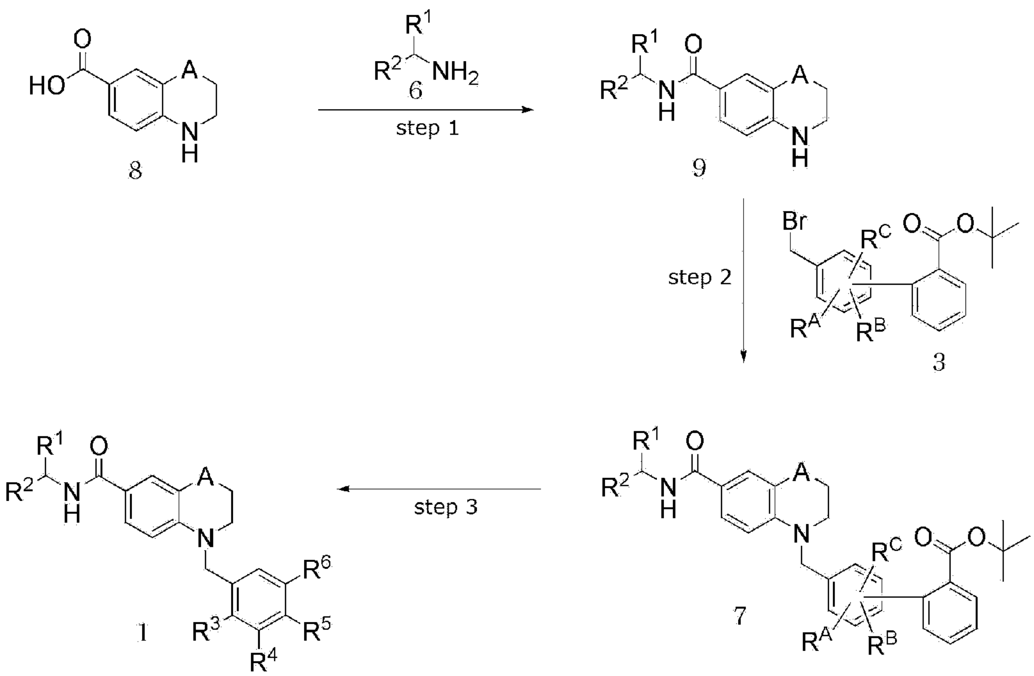


(In the reaction formula 2,

$R^1 \sim R^6$ and A are as defined in formula 1, and
$R^A$, $R^B$, and $R^C$ are independently as defined in $R^3 \sim R^6$).

**8.** A method for preparing the compound represented by formula 1 of claim 1, as shown in reaction formula 3 below, comprising the step of substituting carboxyl group of the compound represented by formula 1A with sodiumoxycarbonyl group or amide group (step 1).

[Reaction Formula 3]

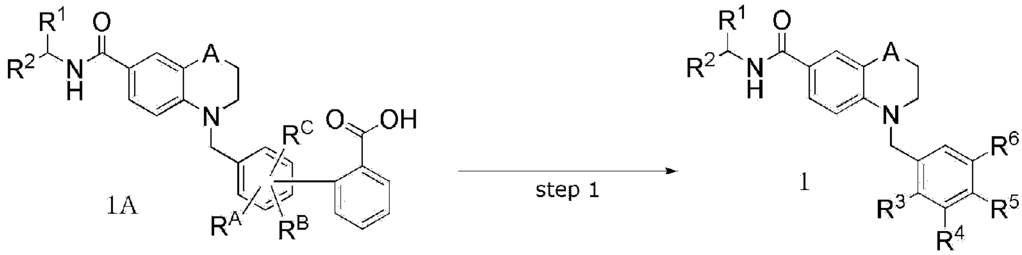


(In the reaction formula 3,

$R^1 \sim R^6$ and A are as defined in formula 1, and
$R^A$, $R^B$, and $R^C$ are independently as defined in $R^3 \sim R^6$).

**9.** A pharmaceutical composition for treating PPARG-related disease comprising the compound represented by formula 1, the optical isomer thereof, or the pharmaceutically acceptable salt of the same as an active ingredient.

**10.** The pharmaceutical composition for treating PPARG-related disease according to claim 9, wherein the PPARG-related disease is selected from the group consisting of diabetes, insulin resistance, impaired glucose tolerance, pre-diabetes, hyperglycemia, hyperinsulinemia, obesity, and inflammation.

**11.** The pharmaceutical composition for treating PPARG-related disease according to claim 9, wherein the pharmaceutical composition specifically binds to PPARG but does not act as an agonist thereof.

**12.** The pharmaceutical composition for treating PPARG-related disease according to claim 9, wherein the pharmaceutical composition specifically binds to PPARG but does not act as an agonist of CDK5 (cyclin dependent kinase 5).

**13.** The pharmaceutical composition for treating PPARG-related disease according to claim 12, wherein the pharmaceutical composition is **characterized by** not acting as an agonist of CDK5 that induces phosphorylation of serine, the $273^{rd}$ amino acid of PPARG.

**14.** The pharmaceutical composition for treating PPARG-related disease according to claim 13, wherein the pharmaceutical composition is **characterized by** not inducing side effect caused by the phosphorylation of serine, the $273^{rd}$ amino acid of PPARG.

**15.** The pharmaceutical composition for treating PPARG-related disease according to claim 14, wherein the side effect caused by the phosphorylation of serine, the $273^{rd}$ amino acid of PPARG is one or more side effects selected from the group consisting of weight gain, edema, impairment of bone growth or formation, and cardiac hypertrophy.

## INTERNATIONAL SEARCH REPORT

International application No.
**PCT/KR2015/001941**

A. CLASSIFICATION OF SUBJECT MATTER

***C07D 265/36(2006.01)i, C07D 215/04(2006.01)i, A61K 31/538(2006.01)i, A61P 3/10(2006.01)i***

According to International Patent Classification (IPC) or to both national classification and IPC

B. FIELDS SEARCHED

Minimum documentation searched (classification system followed by classification symbols)
C07D 265/36; C07D 209/42; C07D 401/10; C07D 215/04; A61K 31/538; A61P 3/10

Documentation searched other than minimum documentation to the extent that such documents are included in the fields searched
Korean Utility models and applications for Utility models: IPC as above
Japanese Utility models and applications for Utility models: IPC as above

Electronic data base consulted during the international search (name of data base and, where practicable, search terms used)
eKOMPASS (KIPO internal) & Keywords: Peroxisome proliferator activated receptor-gammar, PPAR-gammar, benzoxazines, Cyclin-dependant kinase 5, CDK5, inhibitor, diabetes, diabetes mellitus, insulin resistance.

C. DOCUMENTS CONSIDERED TO BE RELEVANT

| Category* | Citation of document, with indication, where appropriate, of the relevant passages | Relevant to claim No. |
|---|---|---|
| A | WO 2012-170554 A1 (KAMENECKA, T. M. et al.) 13 December 2012<br>See abstract, example 1 and claims 1-25. | 1-15 |
| A | LAMOTTE, Y. et al., "Synthesis and biological activities of novel indole derivatives as potent and selective PPARG modulators", Bioorganic & Medicinal Chemistry Letters, 2010, Vol. 20, pages 1399-1404<br>See abstract, chemical equations 2-4 and compounds 6a, 7a-e, 10, 16a, 17a-b. | 1-15 |
| A | CHOI, J. H. et al., "Antidiabetic actions of a non-agonist PPARG ligand blocking Cdk5-mediated phosphorylation", Nature, 2011, Vol. 477, pages 477-481<br>See abstract and figure 1. | 1-15 |

☐ Further documents are listed in the continuation of Box C.  ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 16 JUNE 2015 (16.06.2015) | **16 JUNE 2015 (16.06.2015)** |

| Name and mailing address of the ISA/**KR** | Authorized officer |
|---|---|
| Korean Intellectual Property Office<br>Government Complex-Daejeon, 189 Seonsa-ro, Daejeon 302-701, Republic of Korea<br>Facsimile No. 82-42-472-7140 | Telephone No. |

Form PCT/ISA/210 (second sheet) (July 2009)

INTERNATIONAL SEARCH REPORT
Information on patent family members

International application No.
PCT/KR2015/001941

| Patent document cited in search report | Publication date | Patent family member | Publication date |
|---|---|---|---|
| WO 2012-170554 A1 | 13/12/2012 | US 2012-309757 A1 | 06/12/2012 |
| | | US 8957093 B2 | 17/02/2015 |

Form PCT/ISA/210 (patent family annex) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20120309757 A1